(19) Europäisches Patentamt
European Patent Office
Office européen des brevets

(11) **EP 3 512 841 B1**

(12) **EUROPÄISCHE PATENTSCHRIFT**

(45) Veröffentlichungstag und Bekanntmachung des Hinweises auf die Patenterteilung:
**26.04.2023 Patentblatt 2023/17**

(21) Anmeldenummer: **17761905.3**

(22) Anmeldetag: **11.09.2017**

(51) Internationale Patentklassifikation (IPC):
***C07D 401/14*** (2006.01) ***C07D 405/14*** (2006.01)
***C07D 401/04*** (2006.01) ***C07D 403/04*** (2006.01)
***C07D 403/10*** (2006.01) ***C07D 403/14*** (2006.01)
***C07D 471/04*** (2006.01) ***C07D 471/14*** (2006.01)
***H01L 51/00*** (2006.01) ***C09K 11/06*** (2006.01)
***C07D 487/04*** (2006.01) ***C07D 491/147*** (2006.01)
***C07D 495/04*** (2006.01) ***C07D 495/14*** (2006.01)
***C07D 498/04*** (2006.01) ***C07D 498/14*** (2006.01)
***C07D 513/04*** (2006.01) ***H05B 33/10*** (2006.01)

(52) Gemeinsame Patentklassifikation (CPC):
**C07D 401/10; C07D 401/04; C07D 401/14; C07D 403/04; C07D 403/10; C07D 403/14; C07D 405/14; C07D 471/04; C07D 471/14; C07D 487/04; C07D 491/147; C07D 495/04; C07D 495/14; C07D 498/04; C07D 498/14;** (Forts.)

(86) Internationale Anmeldenummer:
**PCT/EP2017/072701**

(87) Internationale Veröffentlichungsnummer:
**WO 2018/050584 (22.03.2018 Gazette 2018/12)**

(54) **VERBINDUNGEN MIT SPIROBIFLUOREN-STRUKTUREN**

COMPOUNDS WITH SPIROBIFLUORENE-STRUCTURES

COMPOSÉS À STRUCTURES SPIROBIFLUORÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.09.2016 EP 16188675**

(43) Veröffentlichungstag der Anmeldung:
**24.07.2019 Patentblatt 2019/30**

(73) Patentinhaber: **Merck Patent GmbH**
**64293 Darmstadt (DE)**

(72) Erfinder:
- **PARHAM, Amir**
**60486 Frankfurt Am Main (DE)**
- **GROSSMANN, Tobias**
**64297 Darmstadt (DE)**
- **LUDEMANN, Aurélie**
**60322 Frankfurt Am Main (DE)**
- **JOOSTEN, Dominik**
**60487 Frankfurt Am Main (DE)**
- **KROEBER, Jonas**
**60311 Frankfurt Am Main (DE)**

(56) Entgegenhaltungen:
**WO-A1-2015/192939 KR-A- 20140 099 759**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
**C07D 513/04; C07D 513/14; C09K 11/06;**
**H10K 85/654; H10K 85/6572; H10K 85/6574;**
H10K 50/18; H10K 85/342; H10K 2101/90;
Y02E 10/549

## Beschreibung

Die vorliegende Erfindung beschreibt Spirobifluorenderivate, welche mit Elektronentransportgruppen substituiert sind, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

In organischen Elektrolumineszenzvorrichtungen (OLEDs) werden als emittierende Materialien häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Gemäß dem Stand der Technik werden als Matrixmaterialien für phosphoreszierende Verbindungen sowie als Elektronentransportmaterialien häufig heteroaromatische Verbindungen eingesetzt, wie zum Beispiel Triazinderivate oder Pyrimidinderivate. Weiterhin werden als Matrixmaterialien auch Carbazolderivate verwendet, wobei auch Verbindungen bekannt sind, die sowohl Carbazol-Strukturen als auch Struktren aufweisen, die von Triazinen oder Pyrimidinen abgeleitet sind. Beispielsweise sind in WO 2015/156587 A1, US 2015/001511 A1 und KR 2015/0065383 A entsprechende Verbindungen beschrieben. Allerdings weisen diese Verbindungen keine Spirobifluorengruppe auf.

Aus KR 2014-0099759 A sind Benzoindenocarbazolstrukturen bekannt, welche mit einer Diarylaminogruppe substituiert sind, wobei auch Verbindungen mit einer Spiro-Struktur offenbart sind.

Generell besteht bei diesen Materialien, beispielsweise für die Verwendung als Matrixmaterialien, noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Deviceeigenschaften führen, sowie die Bereitstellung der entsprechenden elektronischen Vorrichtung.

Insbesondere ist es die Aufgabe der vorliegenden Erfindung, Verbindungen zur Verfügung zu stellen, die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden OLED eignen, insbesondere als Matrixmaterial. Insbesondere ist es eine Aufgabe der vorliegenden Erfindung, Matrixmaterialien bereitzustellen, welche sich für rot, gelb und grün phosphoreszierende OLEDs eignen.

Weiterhin sollten sich die Verbindungen möglichst einfach verarbeiten lassen und insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen.

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere organischer Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Ausführungsformen sind daher Gegenstand der vorliegenden Erfindung.

Gegenstand der vorliegenden Erfindung ist daher eine Verbindung, umfassend mindestens eine Struktur gemäß der folgenden Formel (I),

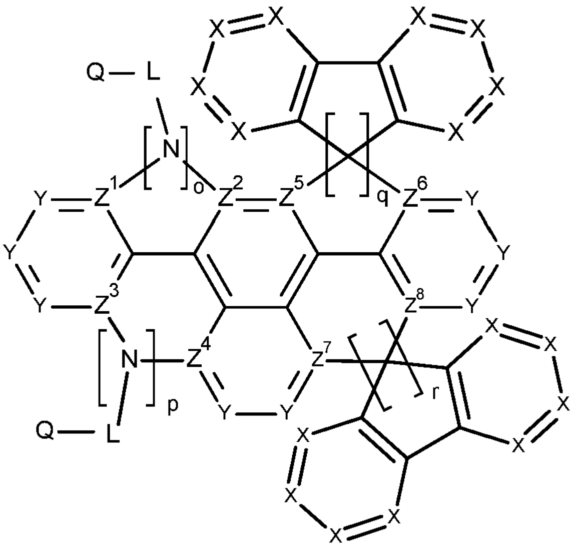

Formel (I)

wobei für die verwendeten Symbole gilt:

| | |
|---|---|
| $Z^1$, $Z^2$, $Z^3$, $Z^4$, $Z^5$, $Z^6$, $Z^7$, $Z^8$ | ist bei jedem Auftreten gleich oder verschieden Y oder C; |
| Y | ist bei jedem Auftreten gleich oder verschieden N, $CR^1$, oder zwei benachbarte Gruppen Y stehen zusammen für O, S oder $NR^1$ mit der Maßgabe, dass ein 5- oder ein 6-Ring gebildet wird; |
| X | ist bei jedem Auftreten gleich oder verschieden N oder $CR^1$, vorzugsweise $CR^1$; |
| o, p, q, r | ist 0 oder 1 ; |
| L | ist eine Bindung oder ein aromatisches oder heteroaromatisches Ringsystem mit 5 bis 24 aromatischen Ringatomen, welches durch einen oder mehrere Reste $R^1$ substituiert sein kann; |
| Q | ist eine Elektronentransportgruppe, ausgewählt aus einer optional substituierten Fünfring-Heteroarylgruppe, die mindestens zwei Heteroatome enthält, oder einer optional substituierten Sechsring-Heteroarylgruppe, wobei die Gruppen auch Teil eines aromatischen Ringsystems sein können und wobei an diese Gruppen auch noch weitere Aryl- oder Heteroarylgruppen ankondensiert sein können; |
| $R^1$ | ist bei jedem Auftreten gleich oder verschieden H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $NO_2$, $P(=O)(R^2)_2$, $OSO_2R^2$, $OR^2$, $S(=O)R^2$, $S(=O)_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, wobei eine oder mehrere nicht benachbarte $CH_2$-Gruppen durch $-R^2C=CR^2-$, -C=C-, $Si(R^2)_2$, $Ge(R^2)_2$, $Sn(R^2)_2$, C=O, C=S, C=Se, $C=NR^2$, $NR^2$, $P(=O)(R^2)$, -C(=O)O-, $-C(=O)NR^2-$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Aralkyl- oder Heteroaralkylgruppe mit 5 bis 40 aromatischen Ringatomen, die durch einen oder mehrere Reste $R^2$ substituiert sein kann, oder eine Kombination dieser Systeme; dabei können zwei oder mehrere Reste $R^1$ miteinander ein Ringsystem bilden; |

$R^2$ ist bei jedem Auftreten gleich oder verschieden H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C=C-, $Si(R^3)_2$, $Ge(R^3)_2$, $Sn(R^3)_2$, C=O, C=S, C=Se, $C=NR^3$, $NR^3$, $P(=O)(R^3)$, -C(=O)O-, $-C(=O)NR^3-$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Aralkyl- oder Heteroaralkylgruppe mit 5 bis 40 aromatischen Ringatomen, die durch einen oder mehrere Reste $R^3$ substituiert sein kann, oder eine Kombination dieser Systeme; dabei können zwei oder mehrere Substituenten $R^2$ auch miteinander ein mono- oder polycyclisches, aliphatisches, heteroaliphatisches, aromatisches oder heteroaromatisches Ringsystem, vorzugsweise ein mono- oder polycyclisches, aliphatisches oder aromatisches Ringsystem bilden;

$R^3$ ist bei jedem Auftreten gleich oder verschieden H, D, F oder ein aliphatischer, aromatischer und/oder heteroaromatischer organischer Rest, bevorzugt ein Kohlenwasserstoffrest mit 1 bis 20 C-Atomen, in dem auch H-Atome durch F ersetzt sein können; dabei können zwei oder mehrere Substituenten $R^3$ auch miteinander ein mono- oder polycyclisches, aliphatisches, heteroaliphatisches, aromatisches oder heteroaromatisches Ringsystem, vorzugsweise ein mono- oder polycyclisches, aliphatisches oder aromatisches Ringsystem bilden;

mit der Maßgabe, dass o + p = 1 ist, wobei für den Fall, dass o = 1 ist, $Z^1$, $Z^2$ C darstellen und $Z^3$, $Z^4$ Y darstellen, und für den Fall, dass p = 1 ist, $Z^1$, $Z^2$ Y darstellen und $Z^3$, $Z^4$ C darstellen; und

mit der Maßgabe, dass q + r = 1 ist, wobei für den Fall, dass q = 1 ist, $Z^5$, $Z^6$ C darstellen und $Z^7$, $Z^8$ Y darstellen, und für den Fall, dass r = 1 ist, $Z^5$, $Z^6$ Y darstellen und $Z^7$, $Z^8$ C darstellen;

weiterhin sind für o bzw. p = 0 das entsprechende Stickstoffatom und die daran gebundene Gruppen nicht vorhanden, und für q bzw. r = 0 sind das entsprechende Kohlenstoffatom und die daran gebundenen Gruppen nicht vorhanden.

Wenn der Index o bzw. p = 0 ist, bedeutet dies, dass das entsprechende Stickstoffatom und die daran gebundenen Gruppen nicht vorhanden sind. Wenn der Index q bzw. r = 0 ist, bedeutet dies, dass das entsprechende Kohlenstoffatom und die daran gebundenen Gruppen nicht vorhanden sind.

Bevorzugt ist o = q = 1 und p = r = 0.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

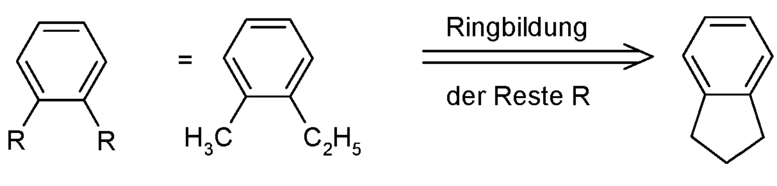


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das

Wasserstoffatom gebunden war, bindet. Dies soll durch das folgende Schema verdeutlicht werden:

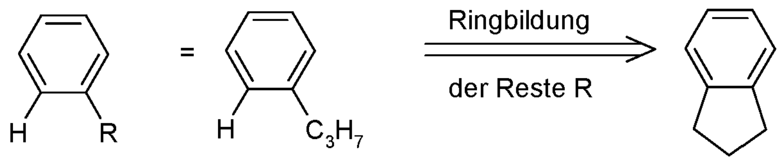


Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindestens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren,

Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugt ist die Verbindung, umfassend mindestens eine Struktur gemäß der folgenden Formel (I), eine Verbindung gemäß Formel (I).

In einer bevorzugten Ausgestaltung können die erfindungsgemäßen Verbindungen eine Struktur gemäß einer der Formeln (IIa), (IIb), (IIc) oder (IId) umfassen,

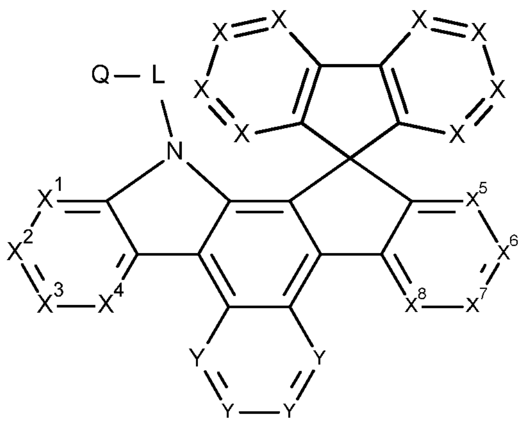

Formel (IIa)

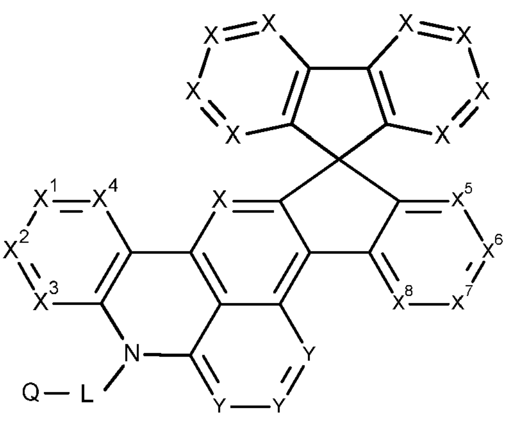

Formel (IIb)

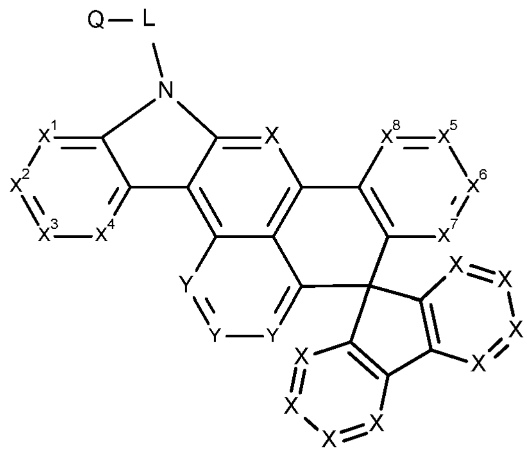

Formel (IIc)

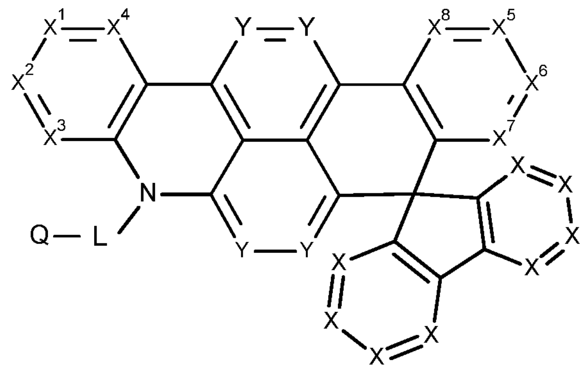

Formel (IId)

wobei die verwendeten Symbole L, Q, Y und X die zuvor, insbesondere für Formel (I) genannte Bedeutung haben und $X^1$, $X^2$, $X^3$, $X^4$, $X^5$, $X^6$, $X^7$, $X^8$ gleich oder verschieden bei jedem Auftreten N oder $CR^1$, vorzugsweise $CR^1$ ist, wobei vorzugsweise maximal zwei Gruppen X, $X^1$, $X^2$, $X^3$, $X^4$, $X^5$, $X^6$, $X^7$, $X^8$ pro Ring für N stehen.

Vorzugsweise können die erfindungsgemäßen Verbindungen Strukturen gemäß Formeln (IIIa), (IIIb), (IIIc) oder (IIId) umfassen,

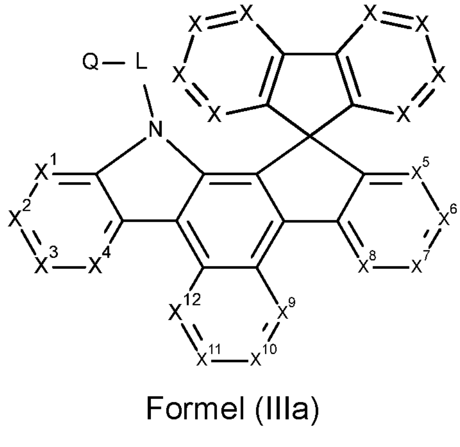

Formel (IIIa)

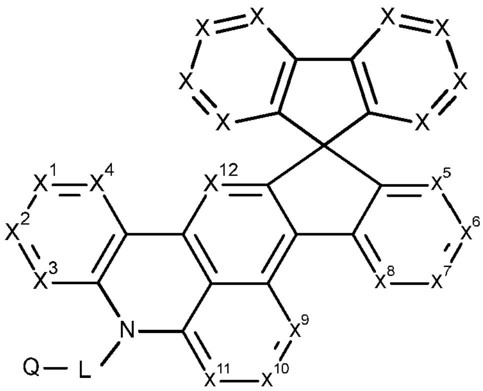

Formel (IIIb)

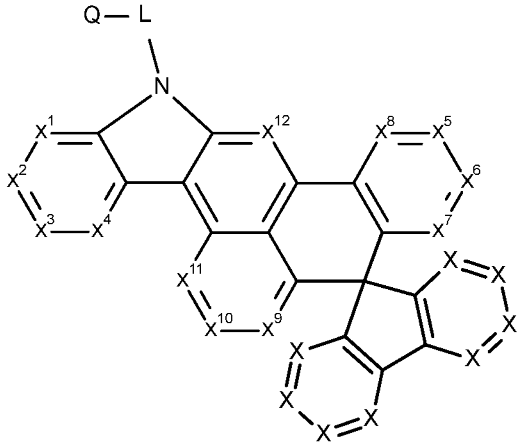

Formel (IIIc)

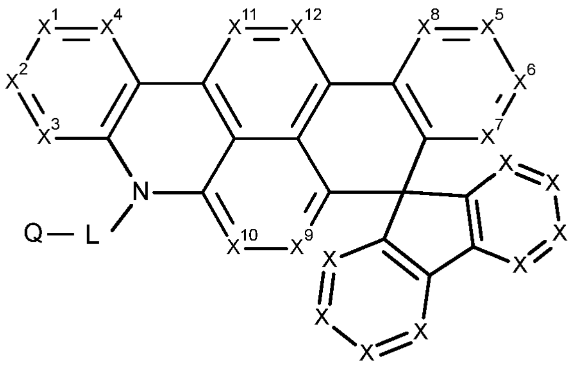

Formel (IIId)

wobei die verwendeten Symbole L, Q und X die zuvor, insbesondere für Formel (I) genannte Bedeutung haben und $X^1$, $X^2$, $X^3$, $X^4$, $X^5$, $X^6$, $X^7$, $X^8$, $X^9$, $X^{10}$, $X^{11}$, $X^{12}$ gleich oder verschieden N oder $CR^1$, vorzugsweise $CR^1$ ist, wobei vorzugsweise maximal zwei Gruppen X, $X^1$, $X^2$, $X^3$, $X^4$, $X^5$, $X^6$, $X^7$, $X^8$, $X^9$, $X^{10}$, $X^{11}$, $X^{12}$ pro Ring für N stehen.

Ferner sind Verbindungen mit Strukturen gemäß Formeln (IIIa), (IIIb), (IIIc) oder (IIId) bevorzugt, in der mindestens neun, vorzugweise mindestens elf der Symbole $X^1$, $X^2$, $X^3$, $X^4$, $X^5$, $X^6$, $X^7$, $X^8$, $X^9$, $X^{10}$, $X^{11}$, $X^{12}$ für $CR^1$ stehen, besonders bevorzugt mindestens sechs der Symbole $X^1$, $X^2$, $X^3$, $X^4$, $X^5$, $X^6$, $X^7$, $X^8$, $X^9$, $X^{10}$, $X^{11}$, $X^{12}$ ausgewählt sind aus C-H und C-D. Besonders bevorzugt stehen alle Symbole $X^1$, $X^2$, $X^3$, $X^4$, $X^5$, $X^6$, $X^7$, $X^8$, $X^9$, $X^{10}$, $X^{11}$, $X^{12}$ für $CR^1$,

wobei besonders bevorzugt mindestens acht der Symbole $X^1$, $X^2$, $X^3$, $X^4$, $X^5$, $X^6$, $X^7$, $X^8$, $X^9$, $X^{10}$, $X^{11}$, $X^{12}$ ausgewählt sind aus C-H und C-D.

Vorzugsweise können die erfindungsgemäßen Verbindungen Strukturen gemäß Formeln (IIIa-1), (IIIb-1), (IIIc-1) oder (IIId-1) umfassen,

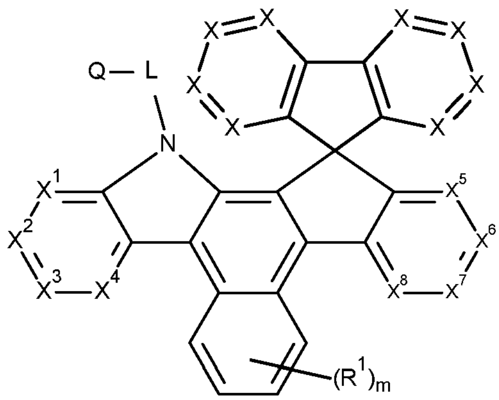

Formel (IIIa-1)

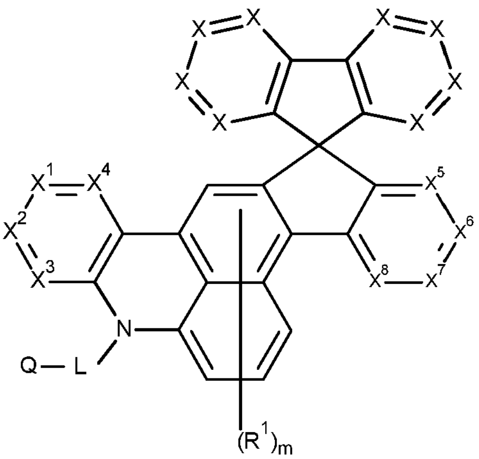

Formel (IIIb-1)

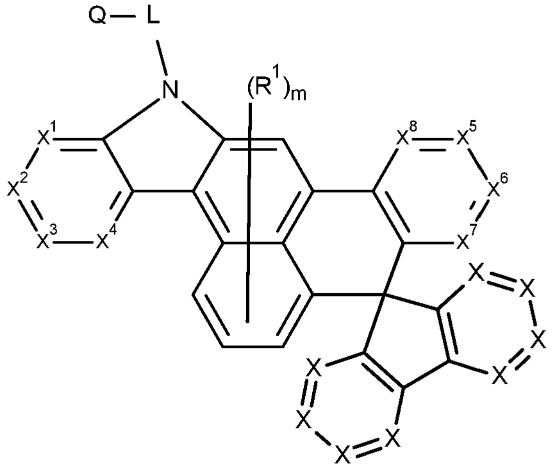

Formel (IIIc-1)

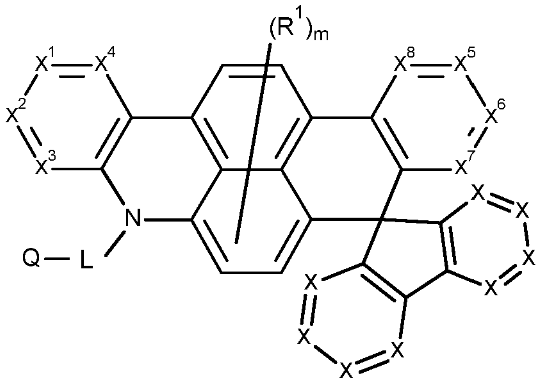

Formel (IIId-1)

wobei die verwendeten Symbole L, $R^1$, Q und X die zuvor, insbesondere für Formel (I) dargelegte Bedeutung aufweisen, $X^1$, $X^2$, $X^3$, $X^4$, $X^5$, $X^6$, $X^7$, $X^8$ gleich oder verschieden bei jedem Auftreten N oder $CR^1$, vorzugsweise $CR^1$ ist, und m 0, 1, 2, 3 oder 4, vorzugsweise 0, 1 oder 2 ist, wobei vorzugsweise maximal zwei Gruppen X, $X^1$, $X^2$, $X^3$, $X^4$, $X^5$, $X^6$, $X^7$, $X^8$ pro Ring für N stehen.

Ferner sind Verbindungen mit Strukturen gemäß Formeln (IIIa-1), (IIIb-1), (IIIc-1) oder (IIId-1) bevorzugt, in der mindestens fünf, vorzugweise mindestens sieben der Symbole $X^1$, $X^2$, $X^3$, $X^4$, $X^5$, $X^6$, $X^7$, $X^8$ für $CR^1$ stehen, besonders bevorzugt mindestens sechs der Symbole $X^1$, $X^2$, $X^3$, $X^4$, $X^5$, $X^6$, $X^7$, $X^8$ ausgewählt sind aus C-H und C-D. Besonders bevorzugt stehen alle Symbole $X^1$, $X^2$, $X^3$, $X^4$, $X^5$, $X^6$, $X^7$, $X^8$ für $CR^1$, wobei besonders bevorzugt mindestens sechs der Symbole $X^1$, $X^2$, $X^3$, $X^4$, $X^5$, $X^6$, $X^7$, $X^8$ ausgewählt sind aus C-H und C-D.

Bevorzugt können die erfindungsgemäßen Verbindungen Strukturen gemäß Formeln (IIIa-2), (IIIb-2), (IIIc-2) oder (IIId-2) umfassen,

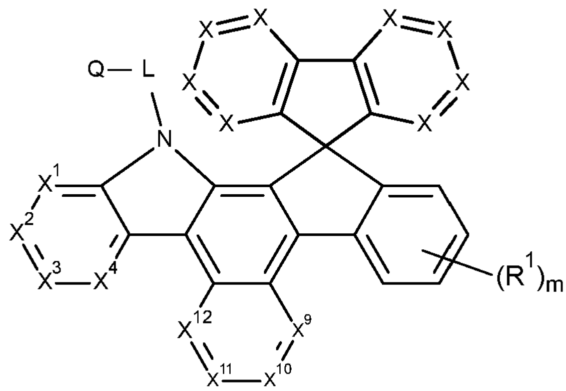

Formel (IIIa-2)

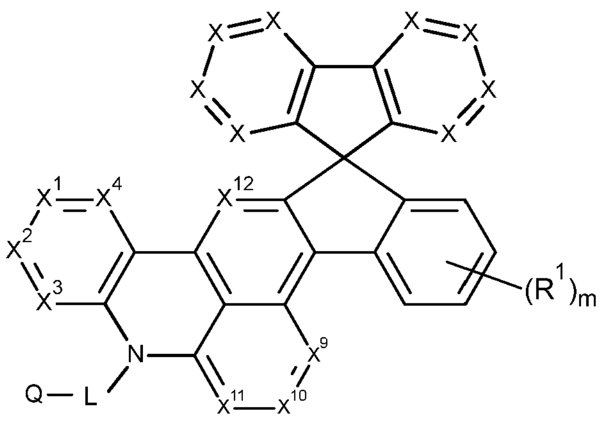

Formel (IIIb-2)

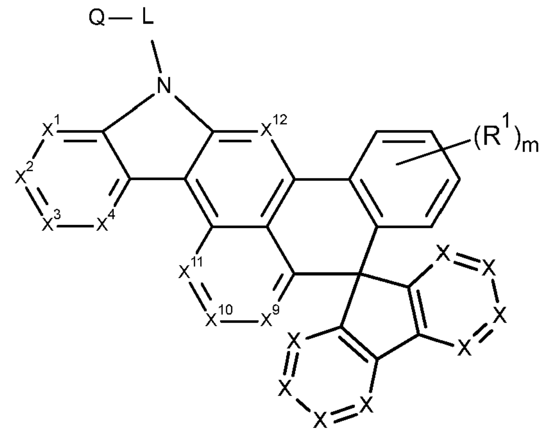

Formel (IIIc-2)

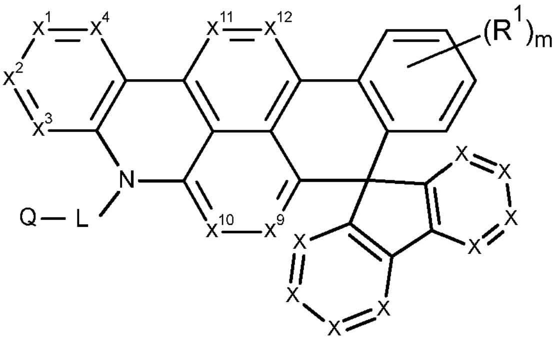

Formel (IIId-2)

wobei die verwendeten Symbole L, $R^1$, Q und X die zuvor, insbesondere für Formel (I) dargelegte Bedeutung aufweisen, $X^1$, $X^2$, $X^3$, $X^4$, $X^9$, $X^{10}$, $X^{11}$, $X^{12}$ gleich oder verschieden bei jedem Auftreten N oder $CR^1$, vorzugsweise $CR^1$ ist, und m 0, 1, 2, 3 oder 4, vorzugsweise 0, 1 oder 2 ist, wobei vorzugsweise maximal zwei Gruppen X, $X^1$, $X^2$, $X^3$, $X^4$, $X^9$, $X^{10}$, $X^{11}$, $X^{12}$ pro Ring für N stehen.

Darüber hinaus sind Verbindungen mit Strukturen gemäß Formeln (IIIa-2), (IIIb-2), (IIIc-2) oder (IIId-2) bevorzugt, in der mindestens fünf, vorzugweise mindestens sieben der Symbole $X^1$, $X^2$, $X^3$, $X^4$, $X^9$, $X^{10}$, $X^{11}$, $X^{12}$ für $CR^1$ stehen, besonders bevorzugt mindestens sechs der Symbole $X^1$, $X^2$, $X^3$, $X^4$, $X^9$, $X^{10}$, $X^{11}$, $X^{12}$ ausgewählt sind aus C-H und C-D. Besonders bevorzugt stehen alle Symbole $X^1$, $X^2$, $X^3$, $X^4$, $X^9$, $X^{10}$, $X^{11}$, $X^{12}$ für $CR^1$, wobei besonders bevorzugt mindestens sechs der Symbole $X^1$, $X^2$, $X^3$, $X^4$, $X^9$, $X^{10}$, $X^{11}$, $X^{12}$ ausgewählt sind aus C-H und C-D.

In einer weiterhin bevorzugten Ausführungsform können die erfindungsgemäßen Verbindungen mindestens eine Struktur gemäß mindestens einer der Formeln (IIIa-3), (IIIb-3), (IIIc-3) oder (IIId-3) aufweisen,

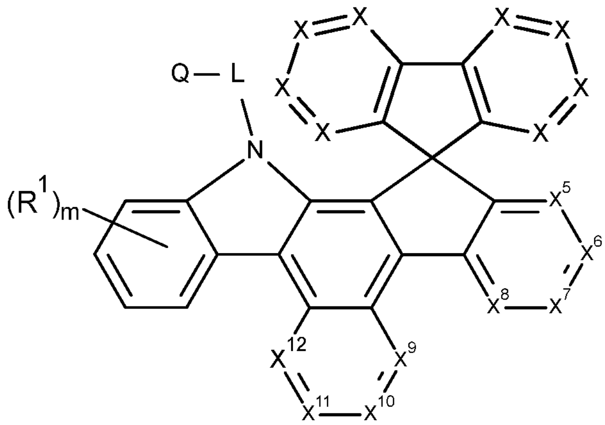

Formel (IIIa-3)

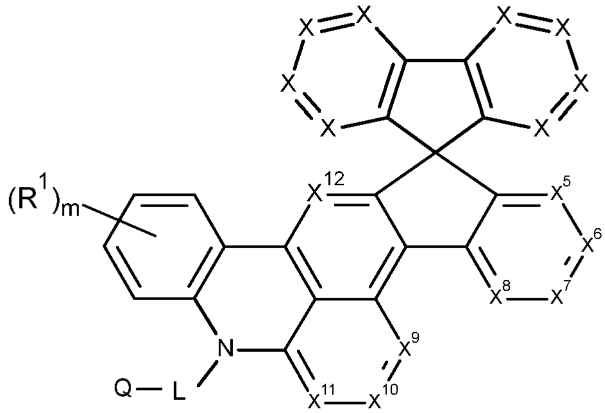

Formel (IIIb-3)

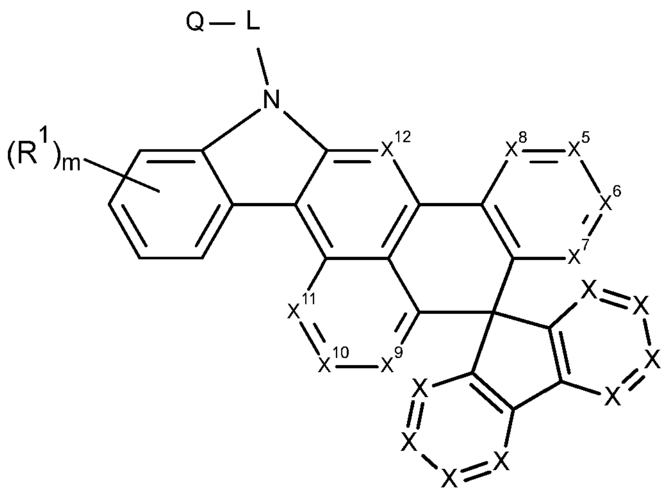

Formel (IIIc-3)

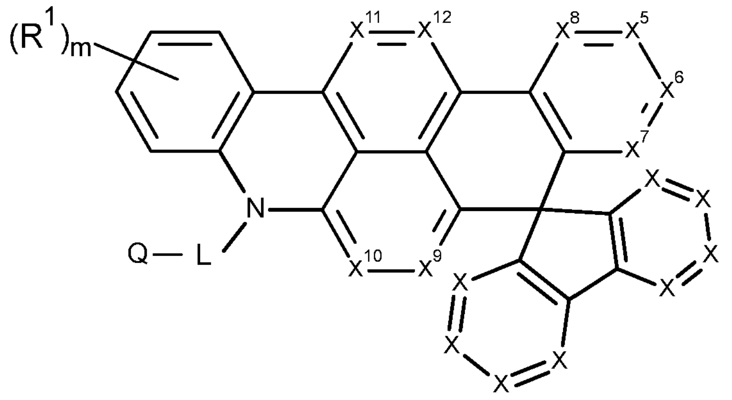

Formel (IIId-3)

wobei die verwendeten Symbole L, $R^1$, Q und X die zuvor, insbesondere für Formel (I) genannte Bedeutung aufweisen, $X^5$, $X^6$, $X^7$, $X^8$, $X^9$, $X^{10}$, $X^{11}$, $X^{12}$ gleich oder verschieden bei jedem Auftreten N oder $CR^1$, vorzugsweise $CR^1$ ist, und m 0, 1, 2, 3 oder 4, vorzugsweise 0, 1 oder 2 ist, wobei vorzugsweise maximal zwei Gruppen X, $X^5$, $X^6$, $X^7$, $X^8$, $X^9$, $X^{10}$, $X^{11}$, $X^{12}$ pro Ring für N stehen.

Weiterhin sind Verbindungen mit Strukturen gemäß Formeln (IIIa-3), (IIIb-3), (IIIc-3) oder (IIId-3) bevorzugt, in der mindestens fünf, vorzugweise mindestens sieben der Symbole $X^5$, $X^6$, $X^7$, $X^8$, $X^9$, $X^{10}$, $X^{11}$, $X^{12}$ für $CR^1$ stehen, besonders bevorzugt mindestens sechs der Symbole $X^5$, $X^6$, $X^7$, $X^8$, $X^9$, $X^{10}$, $X^{11}$, $X^{12}$ ausgewählt sind aus C-H und C-D. Besonders bevorzugt stehen alle Symbole $X^5$, $X^6$, $X^7$, $X^8$, $X^9$, $X^{10}$, $X^{11}$, $X^{12}$ für $CR^1$, wobei besonders bevorzugt mindestens sechs der Symbole $X^5$, $X^6$, $X^7$, $X^8$, $X^9$, $X^{10}$, $X^{11}$, $X^{12}$ ausgewählt sind aus C-H und C-D.

Darüber hinaus können die erfindungsgemäßen Verbindungen mindestens eine Struktur gemäß mindestens einer der Formeln (IIIa-4), (IIIb-4), (IIIc-4) oder (IIId-4) aufweisen,

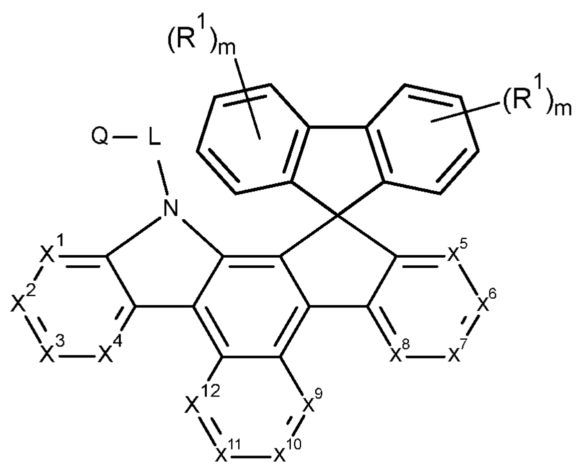

Formel (IIIa-4)

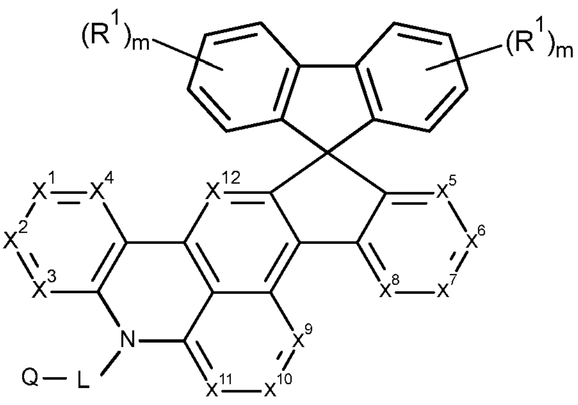

Formel (IIIb-4)

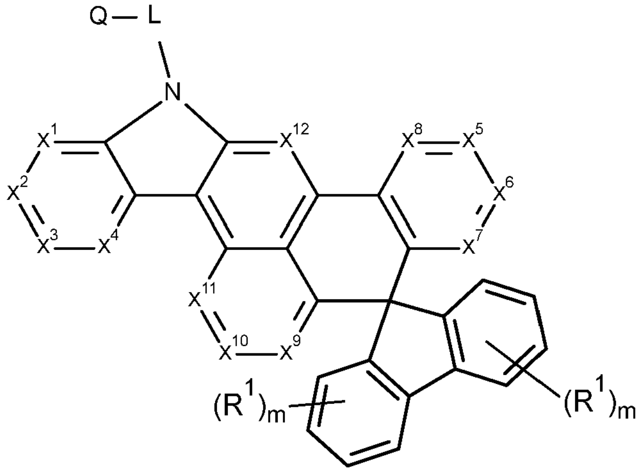

Formel (IIIc-4)

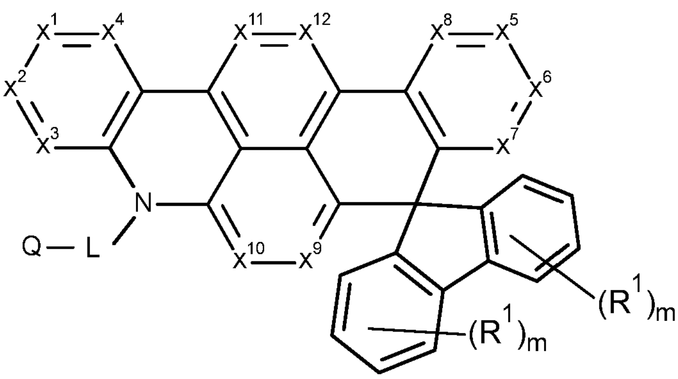

Formel (IIId-4)

wobei die verwendeten Symbole L, $R^1$ und Q die zuvor, insbesondere für Formel (I) genannte Bedeutung aufweisen, $X^1$, $X^2$, $X^3$, $X^4$, $X^5$, $X^6$, $X^7$, $X^8$, $X^9$, $X^{10}$, $X^{11}$, $X^{12}$ gleich oder verschieden bei jedem Auftreten N oder $CR^1$, vorzugsweise $CR^1$ ist, und m 0, 1, 2, 3 oder 4, vorzugsweise 0, 1 oder 2 ist, wobei vorzugsweise maximal zwei Gruppen $X^1$, $X^2$, $X^3$, $X^4$, $X^5$, $X^6$, $X^7$, $X^8$, $X^9$, $X^{10}$, $X^{11}$, $X^{12}$ pro Ring für N stehen.

Weiterhin sind Verbindungen mit Strukturen gemäß Formeln (IIIa-4), (IIIb-4), (IIIc-4) oder (IIId-4) bevorzugt, in der mindestens neun, vorzugweise mindestens elf der Symbole $X^1$, $X^2$, $X^3$, $X^4$, $X^5$, $X^6$, $X^7$, $X^8$, $X^9$, $X^{10}$, $X^{11}$, $X^{12}$ für $CR^1$ stehen, besonders bevorzugt mindestens sechs der Symbole $X^1$, $X^2$, $X^3$, $X^4$, $X^5$, $X^6$, $X^7$, $X^8$, $X^9$, $X^{10}$, $X^{11}$, $X^{12}$ ausgewählt

sind aus C-H und CD. Besonders bevorzugt stehen alle Symbole $X^1$, $X^2$, $X^3$, $X^4$, $X^5$, $X^6$, $X^7$, $X^8$, $X^9$, $X^{10}$, $X^{11}$, $X^{12}$ für $CR^1$, wobei besonders bevorzugt mindestens acht der Symbole $X^1$, $X^2$, $X^3$, $X^4$, $X^5$, $X^6$, $X^7$, $X^8$, $X^9$, $X^{10}$, $X^{11}$, $X^{12}$ ausgewählt sind aus C-H und C-D.

Ferner kann vorgesehen sein, dass erfindungsgemäße Verbindungen Strukturen der der Formeln (IVa), (IVb) oder (IVc) umfassen,

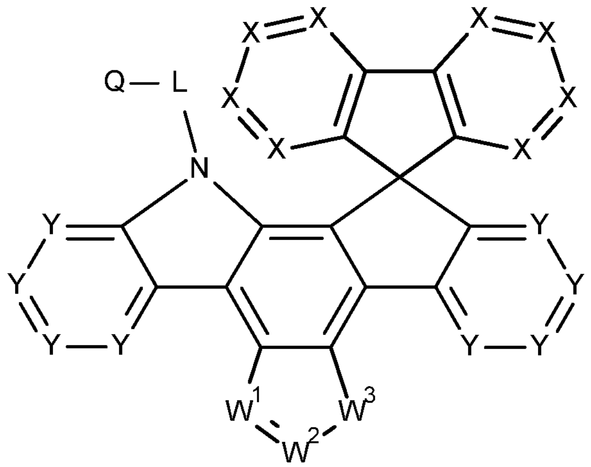

Formel (IVa)

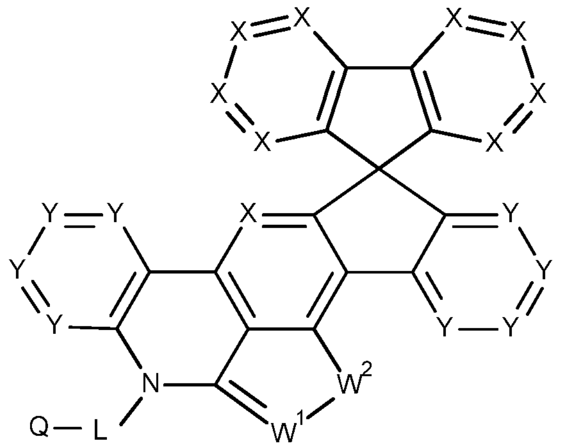

Formel (IVb)

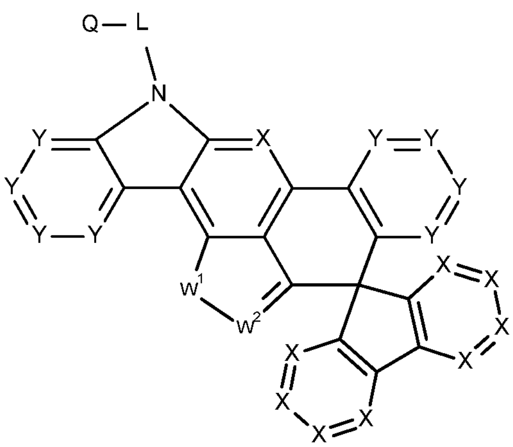

Formel (IVc)

wobei die verwendeten Symbole L, Q, X und Y die zuvor, insbesondere für Formel (I) dargelegte Bedeutung aufweisen und $W^1$, $W^2$ und $W^3$ bei jedem Auftreten gleich oder verschieden N, $CR^1$, O, S oder $NR^1$ ist, wobei genau eine der Gruppen $W^1$, $W^2$ und $W^3$ für O, S oder $NR^1$ steht und mindestens eine der Gruppen $W^1$, $W^2$ und $W^3$ für N oder $CR^1$ steht, wobei $R^1$ die für Formel (I) genannte Bedeutung aufweist, wobei vorzugsweise nicht zwei der Gruppen $W^1$, $W^2$ und $W^3$ für N und eine der Gruppen $W^1$, $W^2$ und $W^3$ für $NR^1$ steht. Dabei steht $W^2$ in Formel (IVa) bevorzugt nicht für O, S oder $NR^1$. Weiterhin steht $W^1$ in Formel (IVb) und $W^2$ in Formel (IVc) für $CR^1$ oder N.

Weiterhin sind Verbindungen, umfassend Strukturen der Formeln (Va), (Vb) oder (Vc) bevorzugt,

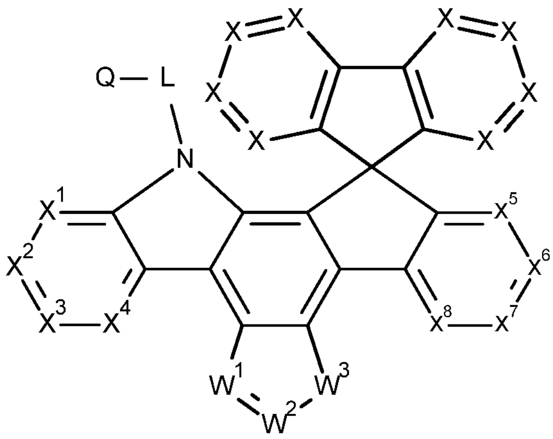

Formel (Va)

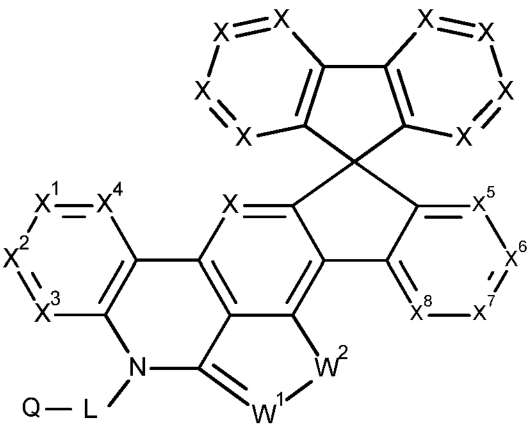

Formel (Vb)

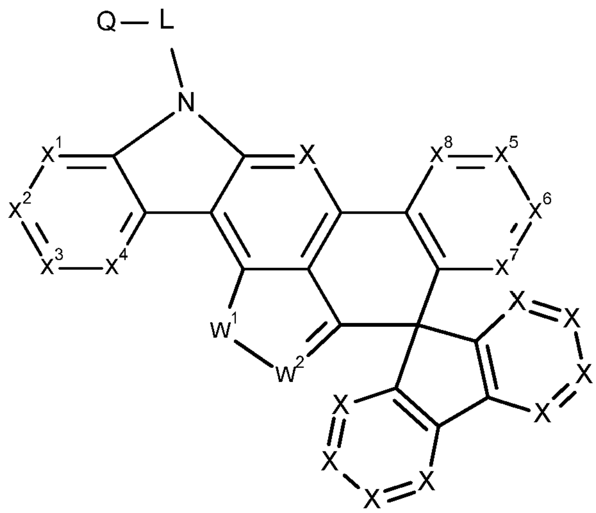

Formel (Vc)

wobei die verwendeten Symbole L, Q und X die zuvor, insbesondere für Formel (I) dargelegte Bedeutung, die Symbole $X^1$, $X^2$, $X^3$, $X^4$, $X^5$, $X^6$, $X^7$, $X^8$ die zuvor, insbesondere für Formel (II) dargelegte Bedeutung und die Symbole $W^1$, $W^2$ und $W^3$ die zuvor, insbesondere für Formel (IV) dargelegte Bedeutung aufweisen. Dabei steht $W^2$ in Formel (Va) bevorzugt nicht für O, S oder $NR^1$. Weiterhin steht $W^1$ in Formel (Vb) und $W^2$ in Formel (Vc) für $CR^1$ oder N.

In einer weiterhin bevorzugten Ausführungsform können die erfindungsgemäßen Verbindungen Strukturen gemäß Formel (Va-1), (Vb-1) und/oder (Vc-1) umfassen,

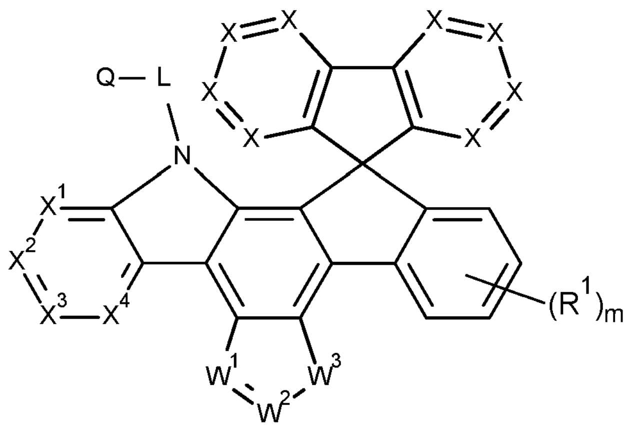

Formel (Va-1)

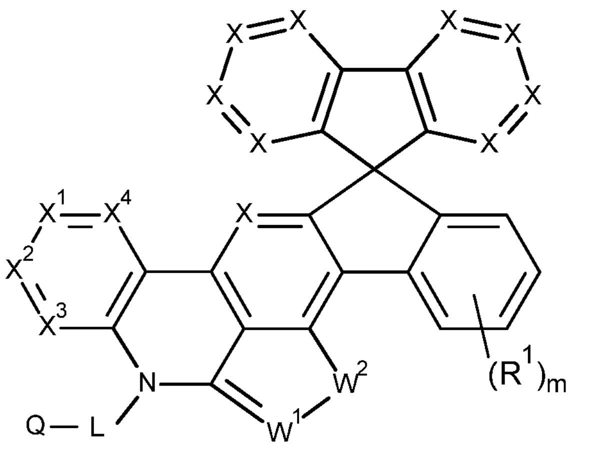

Formel (Vb-1)

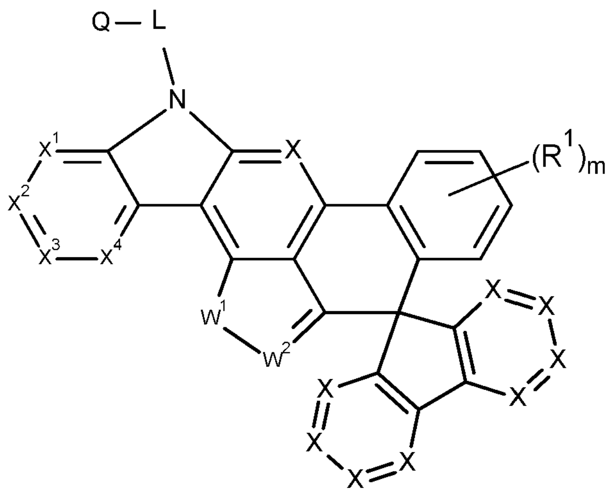

Formel (Vc-1)

wobei die verwendeten Symbole L, $R^1$, Q und X die zuvor, insbesondere für Formel (I) dargelegte Bedeutung, die Symbole $X^1$, $X^2$, $X^3$, $X^4$ die zuvor, insbesondere für Formel (II) dargelegte Bedeutung und die Symbole $W^1$, $W^2$ und $W^3$ die zuvor, insbesondere für Formel (IV) dargelegte Bedeutung aufweisen und m 0, 1, 2, 3 oder 4, vorzugsweise 0, 1 oder 2 ist. Dabei steht $W^2$ in Formel (Va-1) bevorzugt nicht für O, S oder $NR^1$. Weiterhin steht $W^1$ in Formel (Vb-1) und $W^2$ in Formel (Vc-1) für $CR^1$ oder N.

Weiterhin können die erfindungsgemäßen Verbindungen Strukturen gemäß Formel (Va-2), (Vb-2) und/oder (Vc-2) umfassen,

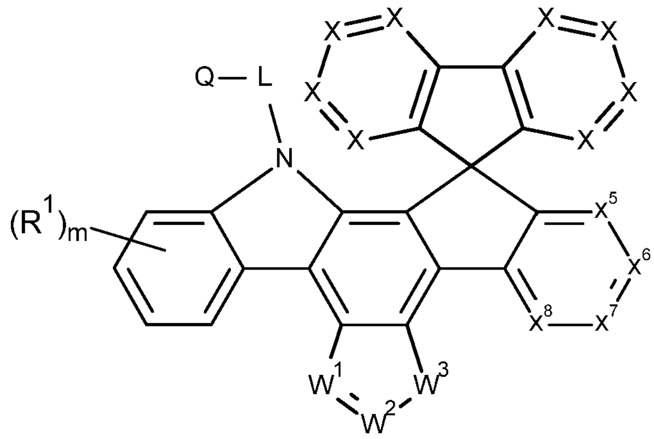

Formel (Va-2)

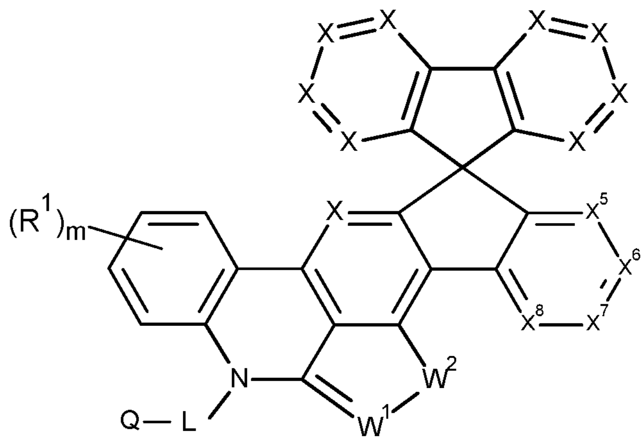

Formel (Vb-2)

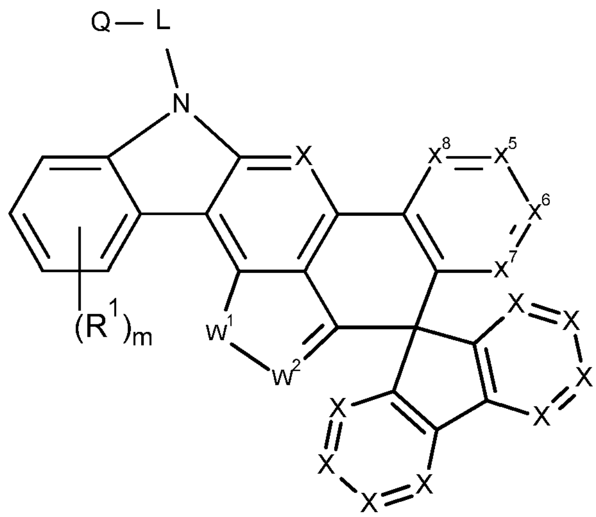

Formel (Vc-2)

wobei die verwendeten Symbole L, $R^1$, Q und X die zuvor, insbesondere für Formel (I) dargelegte Bedeutung, die Symbole $X^5$, $X^6$, $X^7$, $X^8$ die zuvor, insbesondere für Formel (II) dargelegte Bedeutung und die Symbole $W^1$, $W^2$ und $W^3$ die zuvor, insbesondere für Formel (IV) dargelegte Bedeutung aufweisen und m 0, 1, 2, 3 oder 4, vorzugsweise 0, 1 oder 2 ist. Dabei steht $W^2$ in Formel (Va-2) bevorzugt nicht für O, S oder $NR^1$. Weiterhin steht $W^1$ in Formel (Vb-2) und

$W^2$ in Formel (Vc-2) für $CR^1$ oder N.

Weiterhin sind Verbindungen, umfassend Strukturen der Formeln (Va-3), (Vb-3) oder (Vc-3) bevorzugt,

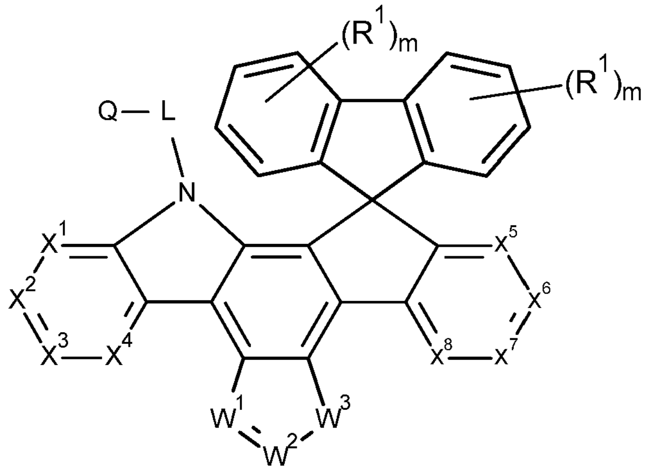

Formel (Va-3)

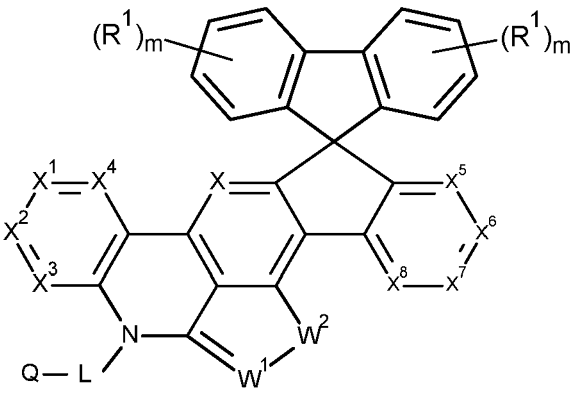

Formel (Vb-3)

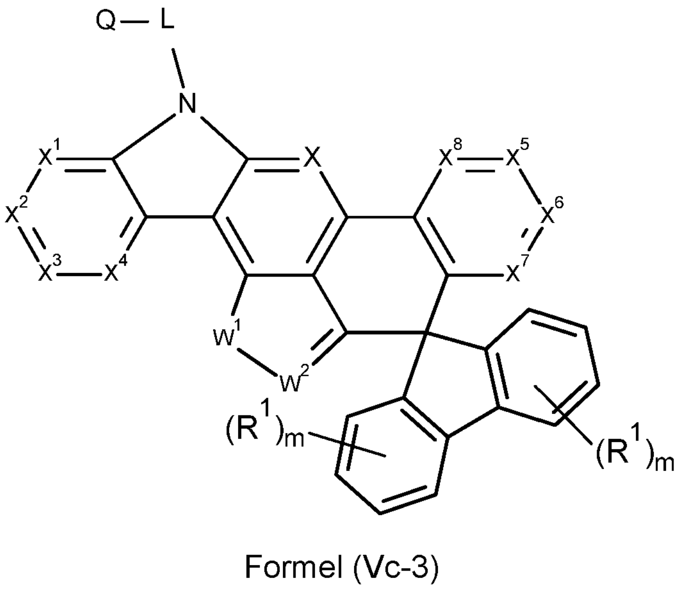

Formel (Vc-3)

wobei die verwendeten Symbole L, $R^1$, Q und X die zuvor, insbesondere für Formel (I) dargelegte Bedeutung, die

Symbole $X^1$, $X^2$, $X^3$, $X^4$, $X^5$, $X^6$, $X^7$, $X^8$ die zuvor, insbesondere für Formel (II) dargelegte Bedeutung und die Symbole $W^1$, $W^2$ und $W^3$ die zuvor, insbesondere für Formel (IV) dargelegte Bedeutung aufweisen und m 0, 1, 2, 3 oder 4, vorzugsweise 0, 1 oder 2 ist. Dabei steht $W^2$ in Formel (Va-3) bevorzugt nicht für O, S oder $NR^1$. Weiterhin steht $W^1$ in Formel (Vb-3) und $W^2$ in Formel (Vc-3) für $CR^1$ oder N.

Weiterhin sind Verbindungen bevorzugt, die dadurch gekennzeichnet sind, dass in Strukturen gemäß Formeln (I), (IVa), (IVb) und/oder (IVc) mindestens sechs, vorzugweise alle der Symbole Y für $CR^1$ stehen, besonders bevorzugt mindestens vier, vorzugsweise mindestens fünf der Gruppen Y ausgewählt sind aus C-H und C-D.

Ferner kann vorgesehen sein, dass in Formeln (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) mindestens eine Gruppe $W^1$, $W^2$ und $W^3$ für N steht.

In einer weiteren Ausführungsform sind Verbindungen mit Strukturen gemäß Formeln (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) bevorzugt, in denen mindestens eine Gruppe $W^1$, $W^2$ und $W^3$ für $CR^1$, vorzugsweise CH steht.

Weiterhin kann vorgesehen sein, dass in Formeln (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) eine Gruppe $W^1$, $W^2$ und $W^3$ für N und eine Gruppe $W^1$, $W^2$ und $W^3$ für $CR^1$, vorzugsweise CH steht.

Darüber hinaus sind Verbindungen bevorzugt, die dadurch gekennzeichnet sind, dass in Formeln (IIa), (IIb), (IIc), (IId), (IIIa), (IIIb), (IIIc), (IIId), (IIIa-1), (IIIb-1), (IIIc-1), (IIId-1), (Va), (Vb) und/oder (Vc) mindestens sechs, vorzugsweise mindestens sieben der Symbole $X^1$, $X^2$, $X^3$, $X^4$, $X^5$, $X^6$, $X^7$, $X^8$ für $CR^1$ stehen, besonders bevorzugt mindestens sechs der Symbole $X^1$, $X^2$, $X^3$, $X^4$, $X^5$, $X^6$, $X^7$, $X^8$ ausgewählt sind aus C-H und C-D. Besonders bevorzugt stehen alle Symbole $X^1$, $X^2$, $X^3$, $X^4$, $X^5$, $X^6$, $X^7$, $X^8$ für $CR^1$, wobei besonders bevorzugt mindestens sechs der Symbole $X^1$, $X^2$, $X^3$, $X^4$, $X^5$, $X^6$, $X^7$, $X^8$ ausgewählt sind aus C-H und C-D.

Darüber hinaus sind Verbindungen bevorzugt, die dadurch gekennzeichnet sind, dass in Formeln (IIIa), (IIIb), (IIIc), (IIId), (IIIa-2), (IIIb-2), (IIIc-2), (IIId-2), (IIIa-3), (IIIb-3), (IIIc-3) und/oder (IIId-3) mindestens zwei, vorzugsweise mindestens drei, besonders bevorzugt alle der Symbole $X^9$, $X^{10}$, $X^{11}$, $X^{12}$ für $CR^1$ stehen, besonders bevorzugt mindestens drei der Symbole $X^9$, $X^{10}$, $X^{11}$, $X^{12}$ ausgewählt sind aus C-H und C-D.

Weiterhin kann vorgesehen sein, dass die Substitutenten $R^1$ des aromatischen oder heteroaromatischen Ringssystems gemäß den Formeln (I), (IIa), (IIb), (IIc), (IId), (IIIa), (IIIb), (IIIe), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) mit den Ringatomen des aromatischen oder heteroaromatischen Ringsystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Substitutenten $R^1$ des heteroaromatischen Ringsystems gemäß den Formeln (I), (IIa), (IIb), (IIc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) mit den Ringatomen des heteroaromatischen Ringsystems kein Ringsystem bilden. Dies schließt die Bildung eines Ringsystems mit möglichen Substituenten $R^2$, $R^3$ ein, die an die Reste $R^1$ gebunden sein können.

Vorzugsweise weisen Verbindungen, umfassend Strukturen gemäß Formel (I), (IIa), (IIb), (IIc), (IId), (IIIa), (IIIb), (IIIe), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3), ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Die Gruppe Q stellt eine Elektronentransportgruppe dar. Elektronentransportgruppen sind in der Fachwelt weithin bekannt und fördern die Fähigkeit von Verbindungen, Elektronen zu transportieren und/oder zu leiten. Eine Elektronentransportgruppe im Sinne der vorliegenden Erfindung ist eine Fünfring-Heteroarylgruppe, die mindestens zwei Heteroatome enthält, oder eine Sechsring-Heteroarylgruppe, wobei diese Gruppen auch Teil eines heteroaromatischen Ringsystems sein können oder wobei an diese Gruppen auch noch weitere Aryl- oder Heteroarylgruppen ankondensiert sein können. Die Elektronentransportgruppe kann optional substituiert sein.

Weiterhin zeigen Verbindungen, umfassend mindestens eine Struktur gemäß Formel (I) oder deren bevorzugte Ausführungsformen überraschende Vorteile, bei denen in Formeln (I), (IIa), (IIb), (IIc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) die Gruppe Q mindestens eine Struktur umfasst, die aus der Gruppe der Pyridine, Pyrimidine, Pyrazine, Pyridazine, Triazine, Chinazoline, Chinoxaline, Chinoline, Isochinoline, Imidazole und/oder Benzimidazole ausgewählt ist, wobei Pyrimidine, Triazine und Chinazoline besonders bevorzugt sind.

Darüber hinaus sind Verbindungen bevorzugt, die dadurch gekennzeichnet sind, dass die Gruppe Q in Formel (I), (IIa), (IIb), (IIc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) ein heteroaromatisches Ringsystem mit mindestens zwei kondensierten Ringen ist, welches durch einen oder mehrere Reste $R^1$ substituiert sein kann, wobei die Ringatome der mindestens zwei kondensierten Ringe mindestens ein Stickstoffatom umfassen, wobei $R^1$ die zuvor, insbesondere für Formel (I) dargelegte Bedeutung aufweist.

In einer weiteren Ausgestaltung kann vorgesehen sein, dass die unter anderem in den Formeln (I), (IIa), (IIb), (IIc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) dargelegte Gruppe Q ein heteroaromatisches Ringsystem darstellt, wobei die Ringatome 1 bis 4 Stickstoffatome, bevorzugt 2 oder 3 Stickstoffatome umfassen und das Ringsystem durch einen oder mehrere Reste $R^1$ substituiert sein kann, wobei $R^1$ die zuvor, insbesondere für Formel (I) dargelegte Bedeutung aufweist.

Ferner kann vorgesehen sein, dass die unter anderem in den Formeln (I), (IIa), (IIb), (IIc), (IId), (IIIa), (IIIb), (IIIe), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) dargelegte Gruppe Q ein heteroaromatisches Ringsystem mit 9 bis 14, vorzugsweise 10 Ringatomen darstellt, welches durch einen oder mehrere Reste $R^1$ substituiert sein kann, wobei $R^1$ die zuvor, insbesondere für Formel (I) und/oder Formel (II) dargelegte Bedeutung aufweist.

Vorzugsweise kann die unter anderem in den Formeln (I), (IIa), (IIb), (IIc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) dargelegte Gruppe Q ausgewählt sein aus Strukturen der Formeln (Q-1), (Q-2), (Q-3), (Q-4) und/oder (Q-5),

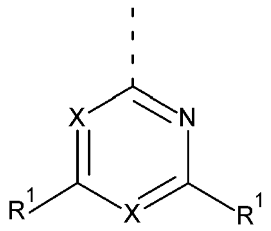

Formel (Q-1)

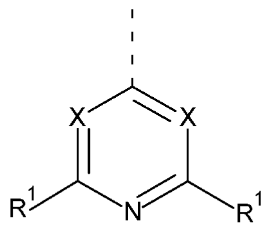

Formel (Q-2)

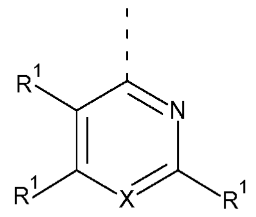

Formel (Q-3)

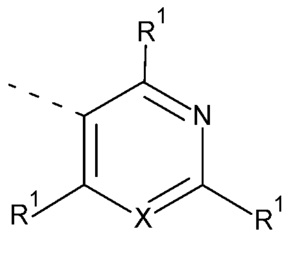

Formel (Q-4)

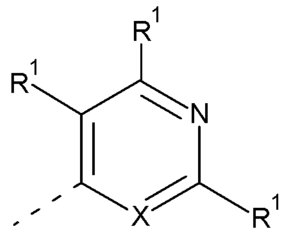

Formel (Q-5)

wobei das Symbol $R^1$ die zuvor unter anderem für Formel (I) genannte Bedeutung aufweist, X gleich oder verschieden bei jedem Auftreten N oder $CR^1$ ist und die gestrichelte Bindung die Anbindungsposition markiert, wobei X vorzugsweise ein Stickstoffatom darstellt.

In einer weiteren Ausführungsform kann unter anderem in den Formeln (I), (IIa), (IIb), (IIc), (IId), (IIIa), (IIIb), (IIIe), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) dargelegte Gruppe Q ausgewählt sein aus Strukturen der Formeln (Q-6), (Q-7), (Q-8), (Q-9), (Q-10), (Q 11) und/oder (Q-12)

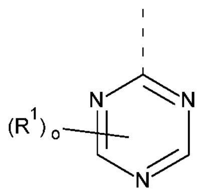

Formel (Q-6)

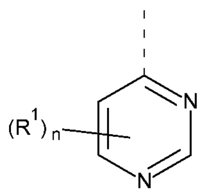

Formel (Q-7)

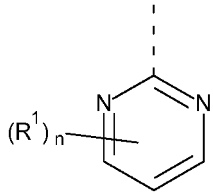

Formel (Q-8)

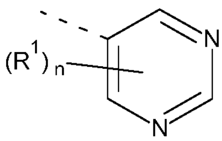

Formel (Q-9)

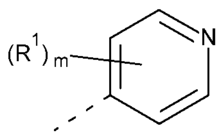

Formel (Q-10)

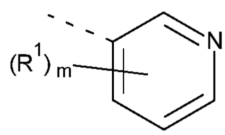

Formel (Q-11)

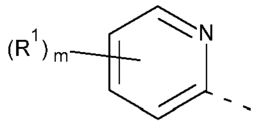

Formel (Q-12)

worin das Symbol $R^1$ die zuvor unter anderem für Formel (I) dargelegte Bedeutung aufweist, die gestrichelte Bindung die Anbindungsposition markiert und m 0, 1, 2, 3 oder 4, vorzugsweise 0, 1 oder 2, n 0, 1, 2 oder 3, vorzugsweise 0, 1 oder 2 und o 0,1 oder 2, vorzugsweise 1 oder 2 ist. Hierbei sind die Strukturen der Formeln (Q-6), (Q-7), (Q-8) und (Q-9) bevorzugt.

In einer weiteren Ausführungsform kann unter anderem in den Formeln (I), (IIa), (IIb), (IIc), (IId), (IIIa), (IIIb), (IIIe), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) dargelegte Gruppe Q ausgewählt sein aus Strukturen der Formeln (Q-13), (Q-14) und/oder (Q-15),

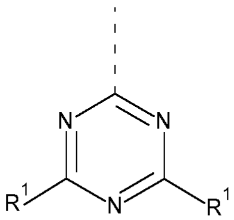

Formel (Q-13)

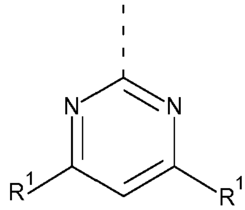

Formel (Q-14)

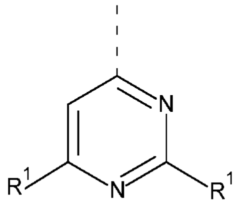

Formel (Q-15)

worin das Symbol $R^1$ die zuvor unter anderem für Formel (I) dargelegte Bedeutung aufweist und die gestrichelte Bindung die Anbindungsposition markiert.

In einer weiteren Ausführungsform kann unter anderem in den Formeln (I), (IIa), (IIb), (IIc), (IId), (IIIa), (IIIb), (IIIe), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) dargelegte Gruppe Q ausgewählt sein aus Strukturen der Formeln (Q-16), (Q-17) und/oder (Q-18),

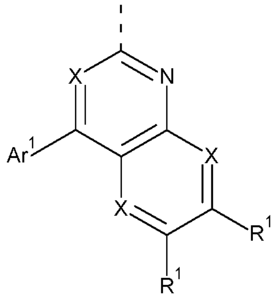

Formel (Q-16)

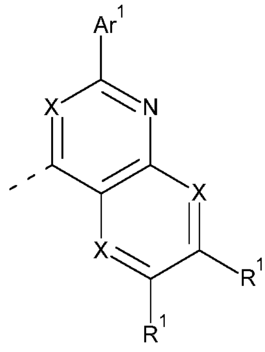

Formel (Q-17)

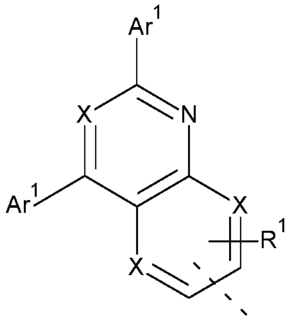

Formel (Q-18)

wobei $R^1$ die zuvor unter anderem für Formel (I) genannte Bedeutung aufweist, X gleich oder verschieden bei jedem Auftreten $CR^1$ oder N ist, die gestrichelte Bindung die Anbindungsposition markiert und $Ar^1$ ein aromatisches oder heteroaromatisches Ringsystem mit 5 bis 40 aromatischen Ringatomen, das jeweils mit einem oder mehreren Resten $R^1$ substituiert sein kann, darstellt, wobei optional zwei oder mehr benachbarte Substituenten $R^1$ ein mono- oder polycyclisches, aliphatisches, heteroaliphatisches, aromatisches oder heteroaromatisches Ringsystem, vorzugsweise ein mono- oder polycyclisches, aliphatisches Ringsystem bilden können, das mit einem oder mehreren Resten $R^2$ substituiert sein kann.

Vorzugsweise kann die unter anderem in den Formeln (I), (IIa), (IIb), (IIc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) dargelegte Gruppe Q ausgewählt sein aus Strukturen der Formeln (Q-19), (Q-20), (Q-21), (Q-22), (Q-23), (Q-24), (Q-25), (Q-26), (Q-27), (Q-28), (Q-29) und/oder (Q-30),

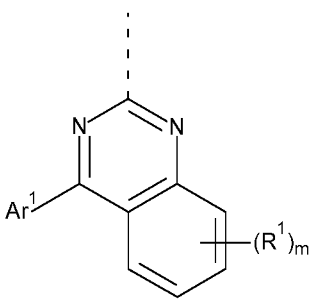

Formel (Q-19)

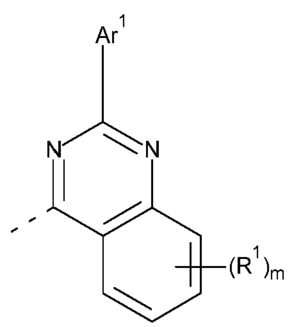

Formel (Q-20)

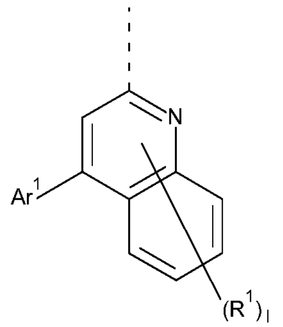

Formel (Q-21)

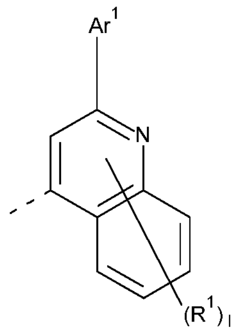

Formel (Q-22)

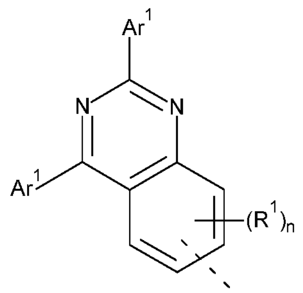

Formel (Q-23)

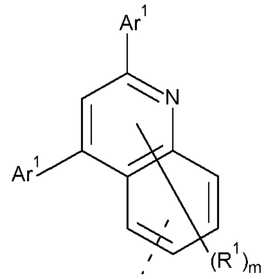

Formel (Q-24)

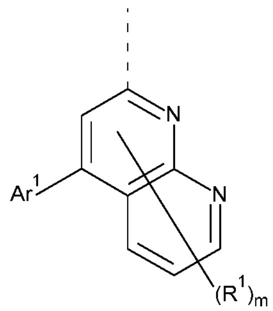

Formel (Q-25)

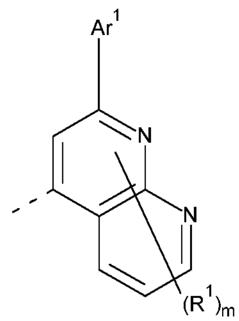

Formel (Q-26)

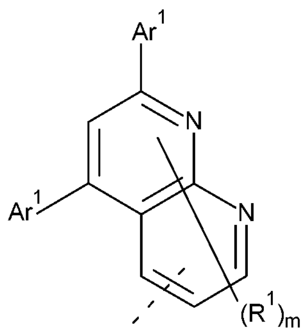

Formel (Q-27)

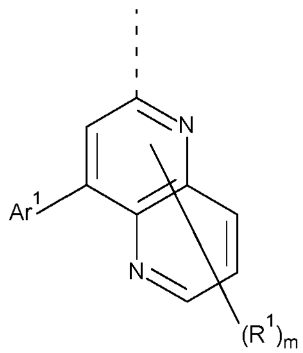

Formel (Q-28)

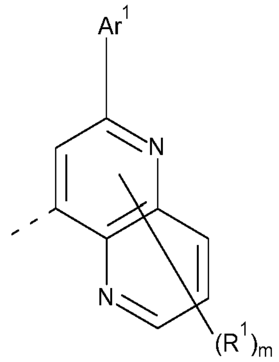

Formel (Q-29)

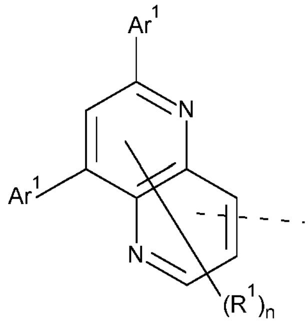

Formel (Q-30)

worin die Symbole $Ar^1$ die zuvor unter anderem für Formel (Q-16), (Q-17) oder (Q-18) und $R^1$ die zuvor unter anderem für Formel (I) dargelegte Bedeutung aufweisen, die gestrichelte Bindung die Anbindungsposition markiert und m 0, 1, 2, 3 oder 4, vorzugsweise 0, 1 oder 2, n 0, 1, 2 oder 3, vorzugsweise 0 oder 1 und I 0, 1, 2, 3, 4 oder 5, vorzugsweise 0, 1 oder 2 ist.

In einer weiteren bevorzugten Ausführungsform der Erfindung steht $Ar^1$ gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste $R^1$ substituiert sein kann, bevorzugt aber unsubstituiert ist, wobei $R^1$ die zuvor, insbesondere in Formel (I) dargestellte Bedeutung aufweisen kann. Beispiele für geeignete Gruppen $Ar^1$ sind ausgewählt aus der Gruppe bestehend aus Phenyl, ortho-, meta- oder para-Biphenyl, Terphenyl, insbesondere verzweigtem Terphenyl, Quaterphenyl, insbesondere verzweigtem Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Mit Vorteil stellt $Ar^1$ in den Formeln (Q-16) bis (Q-30) ein aromatisches Ringsystem mit 6 bis 12 aromatischen Ringatomen dar, welches mit einem oder mehreren Resten $R^1$ substituiert sein kann, vorzugsweise aber unsubstituiert ist, wobei $R^1$ die zuvor, insbesondere für Formel (I) dargestellte Bedeutung aufweisen kann.

In einer weiteren Ausführungsform der Erfindung kann die unter anderem in den Formeln (I), (IIa), (IIb), (IIc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) dargelegte Gruppe Q ausgewählt sein aus Strukturen der Formeln (Q-31) oder (Q-32),

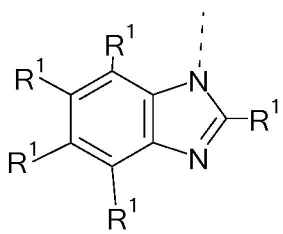

Formel (Q-31)

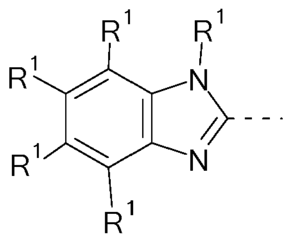

Formel (Q-32)

wobei das Symbol $R^1$ die zuvor, insbesondere für Formel (I) genannte Bedeutung aufweist und die gestrichelte Bindung jeweils die Anbindungsposition markiert, an die das Strukturelement gemäß Formel (Q-31) oder (Q-32) an L bindet.

Bevorzugt bilden die Reste $R^1$ in den Formeln (Q-1) bis (Q-30) mit den Ringatomen der Heteroarylgruppe, an die die Reste $R^1$ gebunden sind, kein kondensiertes Ringsystem. Dies schließt die Bildung eines kondensierten Ringsystems mit möglichen Substituenten $R^2$, $R^3$ ein, die an die Reste $R^1$ gebunden sein können.

Bevorzugt bilden die Reste $R^2$ mit den Ringatomen der Arylgruppe oder Heteroarylgruppe $Ar^1$, an die die Reste $R^2$ in den Formeln (Q-16) bis (Q-30) gebunden sein können, kein kondensiertes Ringsystem. Dies schließt die Bildung eines kondensierten Ringsystems mit möglichen Substituenten $R^3$ ein, die an die Reste $R^2$ gebunden sein können.

Wenn X für $CR^1$ steht bzw. wenn die aromatische und/oder heteroaromatische Gruppen des Grundgerüsts durch Substituenten $R^1$ substituiert sind, dann sind diese Substituenten $R^1$ bevorzugt gewählt aus der Gruppe bestehend

aus H, D, F, CN, C(=O)$Ar^1$, P(=O)$(Ar^1)_2$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, wobei eine oder mehrere nichtbenachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten $R^2$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^2$ substituiert sein kann; dabei können optional zwei Substituenten $R^1$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^1$ substituiert sein kann; wobei $Ar^1$ gleich oder verschieden bei jedem Auftreten ein aromatisches oder heteroaromatisches Ringsystem mit 6 bis 40 C-Atomen, das jeweils mit einem oder mehreren Resten $R^2$ substituiert sein kann, darstellt; wobei optional zwei oder mehr benachbarte Substituenten $R^2$ ein mono- oder polycyclisches, aliphatisches, heteroaliphatisches, aromatisches oder heteroaromatisches Ringsystem, vorzugsweise ein mono- oder polycyclisches, aliphatisches Ringsystem bilden können, das mit einem oder mehreren Resten $R^3$ substituiert sein kann, wobei das Symbol $R^2$ die zuvor, insbesondere für Formel (I) genannte Bedeutung aufweisen.

Vorzugsweise stellt $Ar^1$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^1$ sind ausgewählt aus der Gruppe bestehend aus Phenyl, ortho-, meta- oder para-Biphenyl, Terphenyl, insbesondere verzweigtem Terphenyl, Quaterphenyl, insbesondere verzweigtem Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Besonders bevorzugt sind diese Substituenten $R^1$ ausgewählt aus der Gruppe bestehend aus H, D, F, CN, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist; dabei können optional zwei Substituenten $R^1$, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten $R^2$ substituiert sein kann, bevorzugt aber unsubstituiert ist, wobei $Ar^1$ die zuvor dargelegte Bedeutung aufweisen kann.

Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtem Terphenyl, Quaterphenyl, insbesondere verzweigtem Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Weiterhin kann vorgesehen sein, dass in einer Struktur gemäß Formel (I), (IIa), (IIb), (IIc), (IId), (IIIa), (IIIb), (IIIe), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) mindestens ein Rest $R^1$ oder in Formel (Q-1) bis (Q-30) mindestens ein Rest $R^1$ oder $Ar^1$ für eine Gruppe steht, die ausgewählt ist aus den Formeln ($R^1$-1) bis ($R^1$- 80),

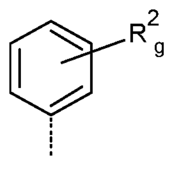

Formel ($R^1$-1)

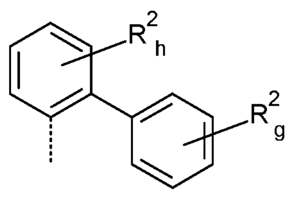

Formel ($R^1$-2)

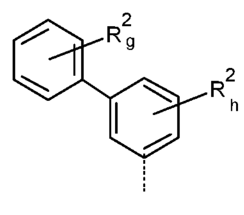

Formel ($R^1$-3)

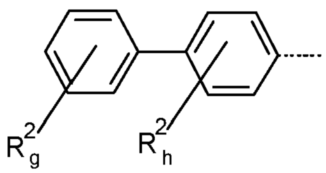

Formel ($R^1$-4)

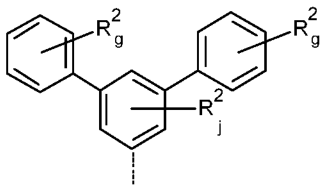

Formel ($R^1$-5)

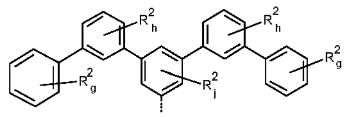

Formel ($R^1$-6)

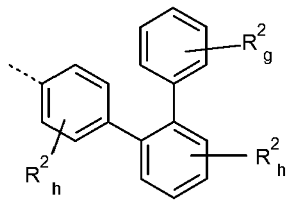

Formel ($R^1$-7)

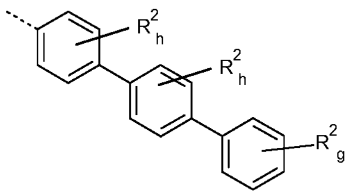

Formel ($R^1$-8)

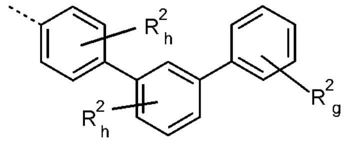

Formel ($R^1$-9)

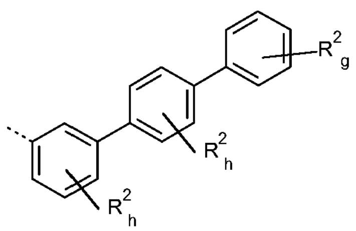

Formel ($R^1$-10)

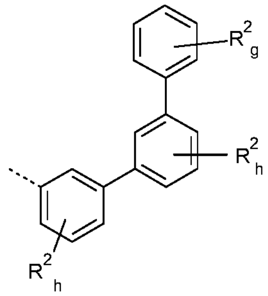

Formel ($R^1$-11)

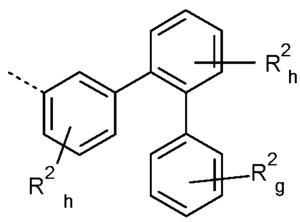

Formel ($R^1$-12)

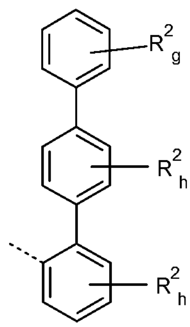

Formel ($R^1$-13)

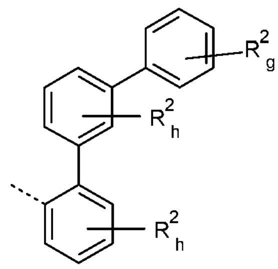

Formel ($R^1$-14)

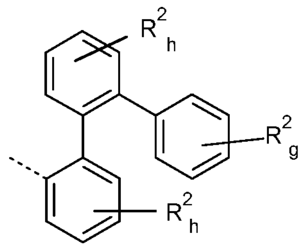

Formel ($R^1$-15)

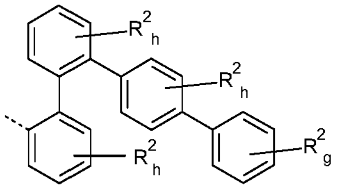

Formel ($R^1$-16)

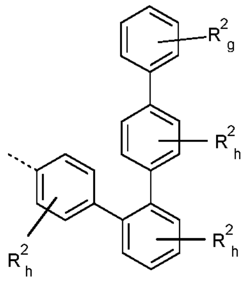

Formel ($R^1$-17)

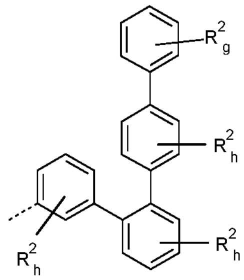

Formel ($R^1$-18)

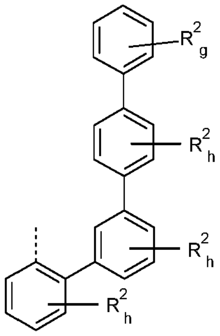

Formel ($R^1$-19)

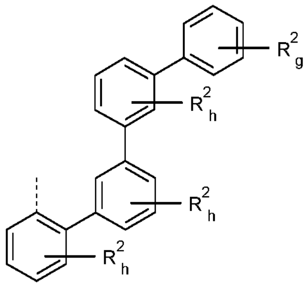

Formel ($R^1$-20)

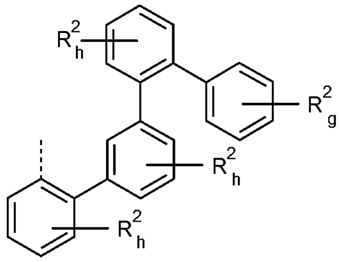

Formel ($R^1$-21)

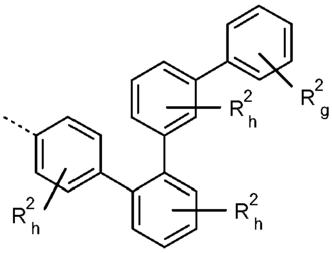

Formel ($R^1$-22)

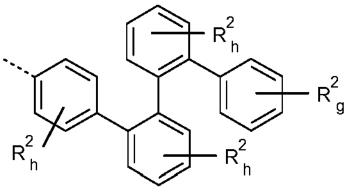

Formel ($R^1$-23)

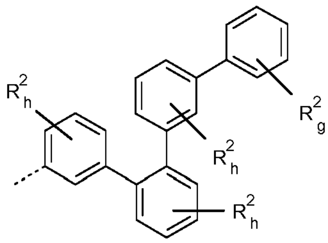

Formel ($R^1$-24)

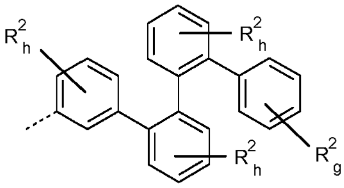

Formel ($R^1$-25)

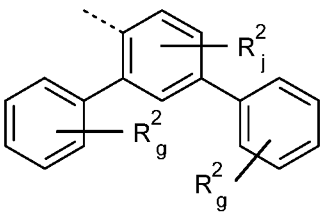

Formel ($R^1$-26)

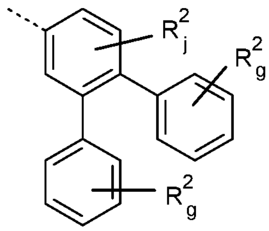

Formel ($R^1$-27)

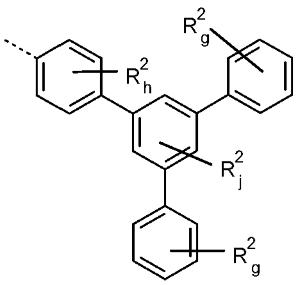

Formel ($R^1$-28)

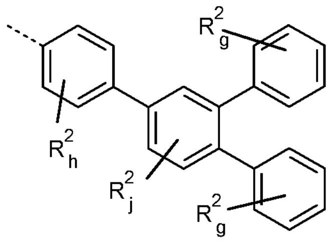

Formel ($R^1$-29)

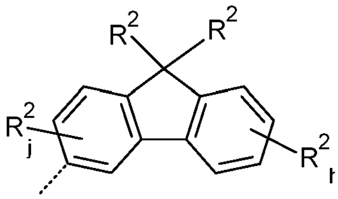

Formel ($R^1$-30)

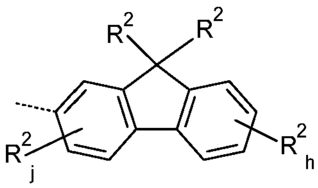

Formel ($R^1$-31)

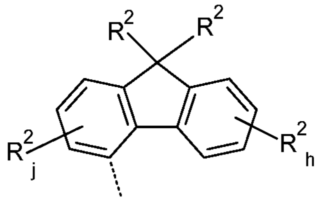

Formel ($R^1$-32)

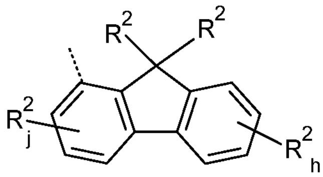

Formel ($R^1$-33)

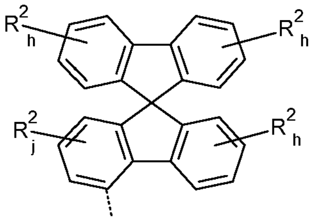

Formel ($R^1$-34)

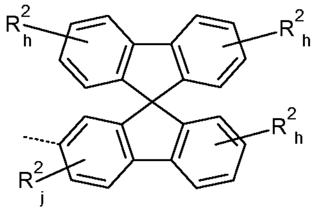

Formel ($R^1$-35)

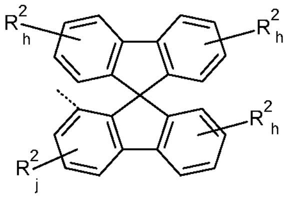

Formel ($R^1$-36)

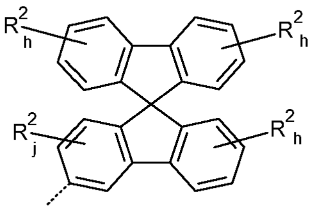

Formel ($R^1$-37)

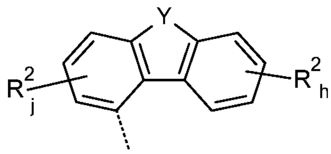

Formel ($R^1$-38)

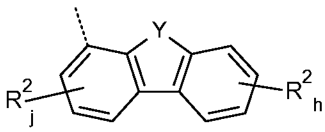

Formel ($R^1$-39)

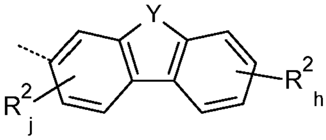

Formel ($R^1$-40)

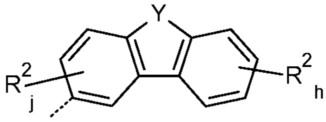

Formel ($R^1$-41)

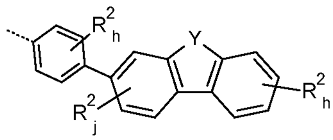

Formel ($R^1$-42)

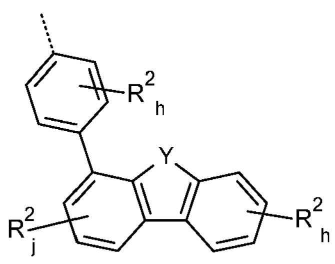
Formel ($R^1$-43)

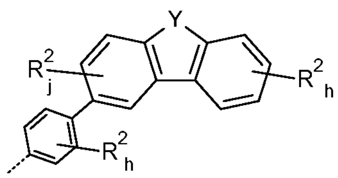
Formel ($R^1$-44)

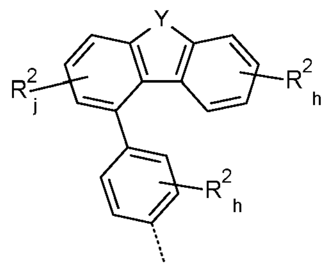
Formel ($R^1$-45)

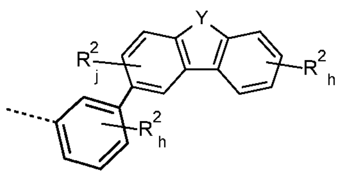
Formel ($R^1$-46)

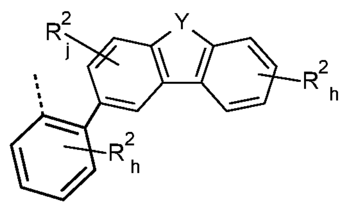
Formel ($R^1$-47)

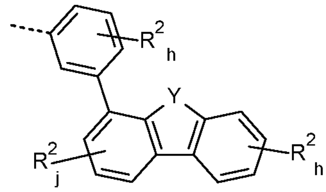
Formel ($R^1$-48)

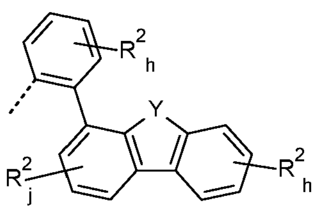
Formel ($R^1$-49)

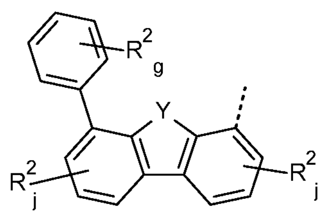
Formel ($R^1$-50)

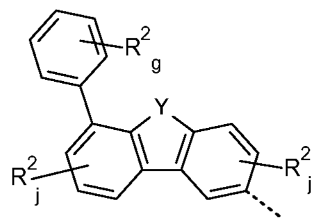
Formel ($R^1$-51)

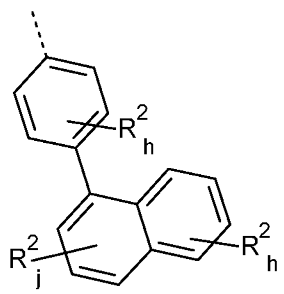
Formel ($R^1$-52)

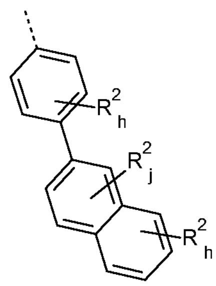
Formel ($R^1$-53)

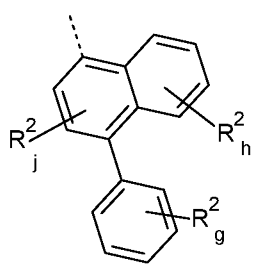
Formel ($R^1$-54)

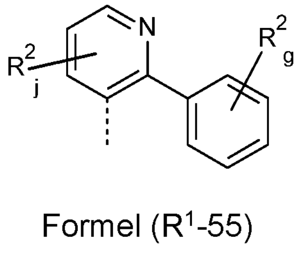
Formel ($R^1$-55)

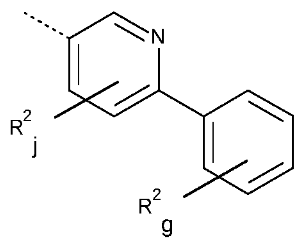
Formel ($R^1$-56)

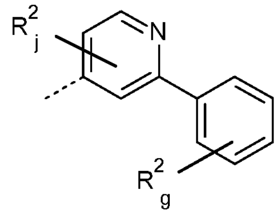
Formel ($R^1$-57)

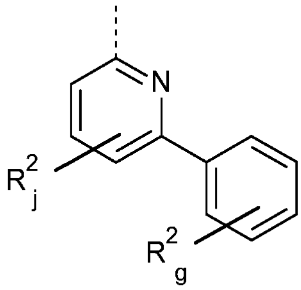

Formel ($R^1$-58)

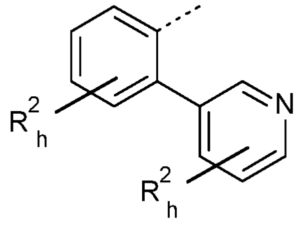

Formel ($R^1$-59)

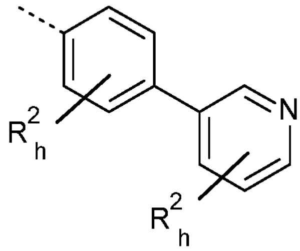

Formel ($R^1$-60)

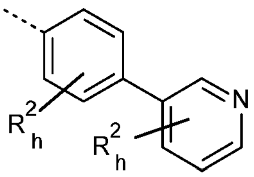

Formel ($R^1$-61)

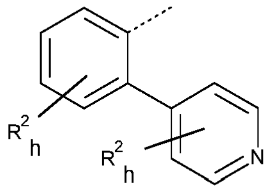

Formel ($R^1$-62)

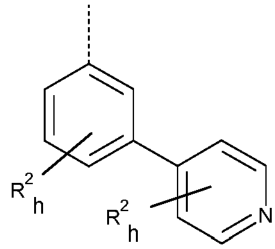

Formel ($R^1$-63)

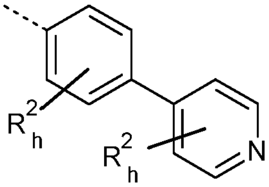

Formel ($R^1$-64)

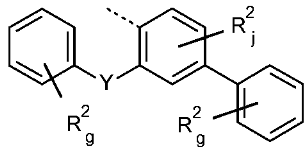

Formel ($R^1$-65)

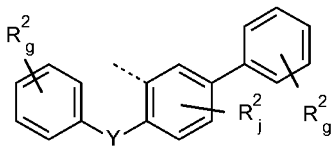

Formel ($R^1$-66)

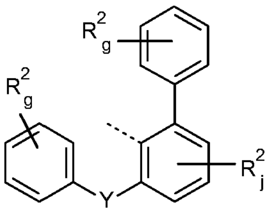

Formel ($R^1$-67)

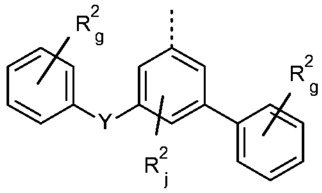

Formel ($R^1$-68)

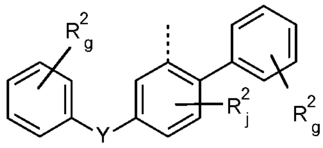

Formel ($R^1$-69)

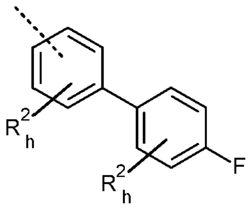

Formel ($R^1$-70)

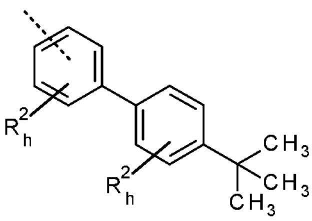

Formel ($R^1$-71)

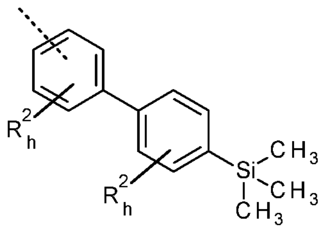

Formel ($R^1$-72)

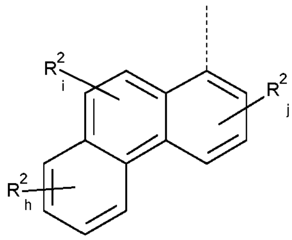
Formel ($R^1$-73)

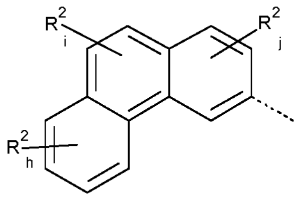
Formel ($R^1$-74)

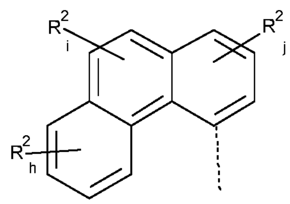
Formel ($R^1$-75)

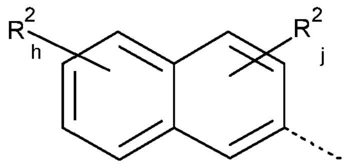
Formel ($R^1$-76)

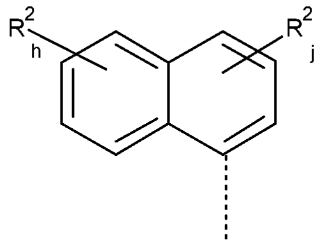
Formel ($R^1$-77)

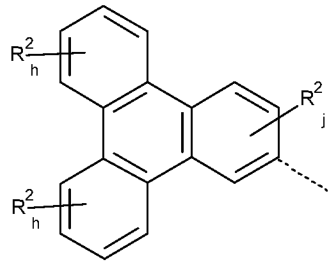
Formel ($R^1$-78)

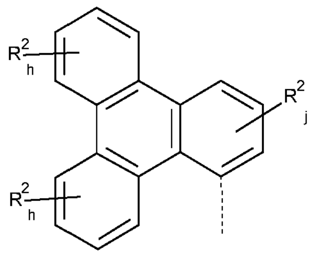
Formel ($R^1$-79)

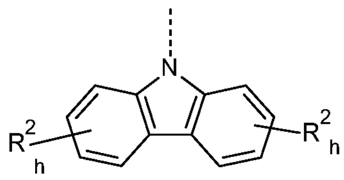
Formel ($R^1$-80)

wobei für die verwendeten Symbole gilt:

- Y ist O, S oder $NR^2$, vorzugsweise O oder S;
- i ist bei jedem Auftreten unabhängig 0, 1 oder 2;
- j ist bei jedem Auftreten unabhängig 0, 1, 2 oder 3;
- h ist bei jedem Auftreten unabhängig 0, 1, 2, 3 oder 4;
- g ist bei jedem Auftreten unabhängig 0, 1, 2, 3, 4 oder 5;
- $R^2$ kann die zuvor genannte, insbesondere für Formel (I) genannte Bedeutung aufweisen;

die gestrichelte Bindung markiert die Anbindungsposition.

Hierbei sind die Gruppen der Formeln $R^1$-1, $R^1$-3, R'-5, R'-6, R'-15, $R^1$-29, $R^1$-31, $R^1$-32, $R^1$-42, $R^1$-43, $R^1$-44 und/oder $R^1$-45 besonders bevorzugt.

Vorzugsweise kann vorgesehen sein, dass die Summe der Indices i, j, h und g in den Strukturen der Formel ($R^1$-1) bis ($R^1$-80) jeweils höchstens 3, vorzugsweise höchstens 2 und besonders bevorzugt höchstens 1 beträgt.

Bevorzugt bilden die Reste $R^2$ in den Formeln ($R^1$-1) bis ($R^1$-80) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

Bevorzugt kann die Gruppe L mit der Gruppe Q und dem heteroaromatischen Rest der Spirobifluorengruppe, an den die Gruppe L gemäß Formel (I), (IIa), (IIb), (IIc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) gebunden ist, eine durchgängige Konjugation ausbilden. Eine durchgängie Konjugation der aromatischen beziehungsweise heteroaromatischen Systeme

wird ausgebildet sobald direkte Bindungen zwischen benachbarten aromatischen oder heteroaromatischen Ringen gebildet werden. Bei einem Fluorensystem sind die beiden aromatischen Ringe unmittelbar gebunden, wobei das $sp^3$-hybridisierte Kohlenstoffatom in Position 9 zwar eine Kondensation dieser Ringe unterbindet, jedoch eine Konjugation erfolgen kann, da dieses $sp^3$-hybridisierte Kohlenstoffatom in Position 9 nicht zwingend zwischen der elektronentransportierenden Gruppe Q und der Fluorenstruktur liegt. Im Gegensatz hierzu kann bei einer zweiten Spirobifluorenstruktur eine durchgängie Konjugation ausgebildet werden, falls die Verbindung zwischen der Gruppe Q und dem aromatischen oder heteroaromatischen Rest der Spirobifluorengruppe der Formel (I), (IIa), (IIb), (IIc), (IId), (IIIa), (IIIb), (IIIe), (IIId), (IIIa-1 ), (IIIb-1 ), (IIIc-1 ), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) über die gleiche Phenylgruppe der Spirobifluorenstruktur oder über Phenylgruppen der Spirobifluorenstruktur, die unmittelbar aneinander gebunden sind und in einer Ebene liegen, erfolgt.

In einer weiteren bevorzugten Ausführungsform der Erfindung steht L für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen oder ein heteroaromatisches Ringsystem mit 5 bis 13 aromatischen Ringatomen, welches durch einen oder mehrere Reste $R^1$ substituiert sein kann, bevorzugt aber unsubstituiert ist, wobei $R^1$ die zuvor, insbesondere für Formel (I) genannte Bedeutung aufweisen kann. Besonders bevorzugt steht L für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^2$ substituiert sein kann, bevorzugt aber unsubstituiert ist, wobei $R^2$ die zuvor, insbesondere für Formel (I) genannte Bedeutung aufweisen kann.

Weiterhin bevorzugt steht das unter anderem in den Strukturen gemäß Formel (I), (IIa), (IIb), (IIc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) dargelegte Symbol L gleich oder verschieden bei jedem Auftreten für eine Bindung oder einen Arylen- oder Heteroarylenrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d. h. über ein Atom der aromatischen oder heteroaromatische Gruppe, an das jeweilige Atom der weiteren Gruppe gebunden ist.

Weiterhin kann vorgesehen sein, dass die unter anderem in den Strukturen gemäß Formel (I), (IIa), (IIb), (llc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) dargelegte Gruppe L ein aromatisches Ringsystem mit höchstens zwei kondensierten aromatischen und/oder heteroaromatischen 6-Ringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt. Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme L sind ausgewählt aus der Gruppe bestehend aus ortho-, meta- oder para-Phenylen, ortho-, meta- oder para-Biphenylen, Terphenylen, insbesondere verzweigtem Terphenylen, Quaterphenylen, insbesondere verzweigtem Quaterphenylen, Fluorenylen, Spirobifluorenylen, Dibenzofuranylen, Dibenzothienylen und Carbazolylen, die jeweils durch einen oder mehrere Reste $R^2$ substituiert sein können, bevorzugt aber unsubstituiert sind.

Ferner kann vorgesehen sein, dass die unter anderem in den Strukturen gemäß Formel (I), (IIa), (IIb), (llc), (IId), (IIIa), (IIIb), (lllc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) dargelegte Gruppe L höchstens ein Stickstoffatom, bevorzugt höchstens zwei Heteroatome, insbesondere bevorzugt höchstens ein Heteroatom und besonders bevorzugt kein Heteroatom aufweist.

Bevorzugt sind Verbindungen umfassend Strukturen der Formeln (I), (IIa), (IIb), (IIc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3), in denen die Gruppe L für eine Bindung oder für eine Gruppe steht, die ausgewählt ist aus den Formeln ($L^1$-1) bis ($L^1$-108),

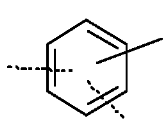

Formel ($L^1$-1)

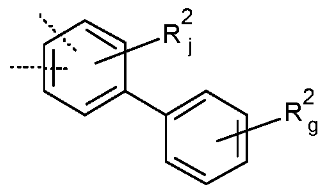

Formel ($L^1$-2)

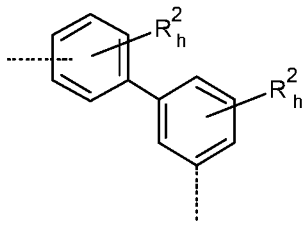

Formel ($L^1$-3)

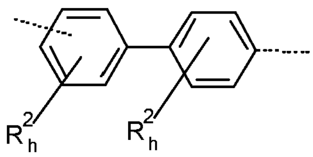

Formel ($L^1$-4)

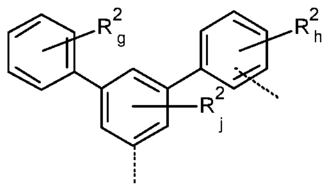

Formel ($L^1$-5)

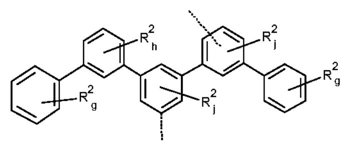

Formel ($L^1$-6)

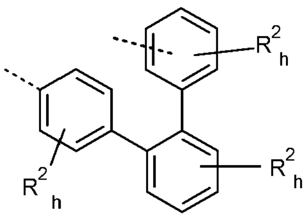

Formel ($L^1$-7)

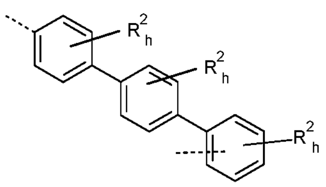

Formel ($L^1$-8)

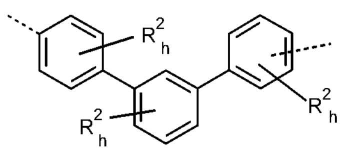

Formel ($L^1$-9)

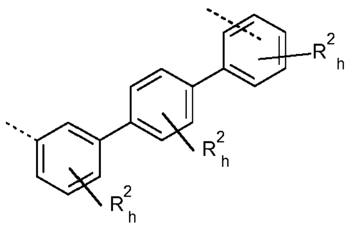

Formel ($L^1$-10)

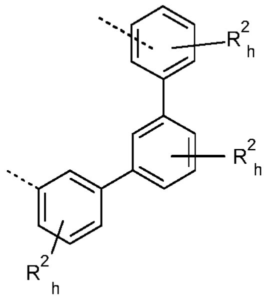

Formel ($L^1$-11)

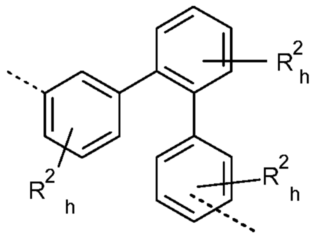

Formel ($L^1$-12)

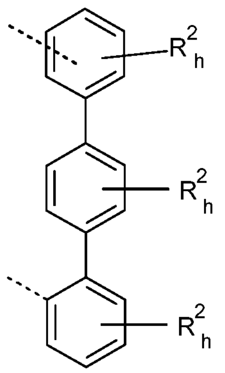

Formel ($L^1$-13)

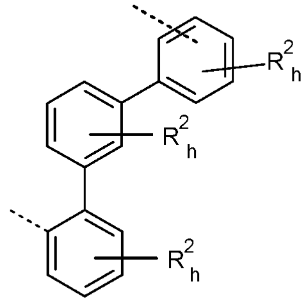

Formel ($L^1$-14)

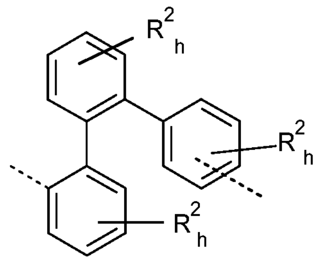

Formel ($L^1$-15)

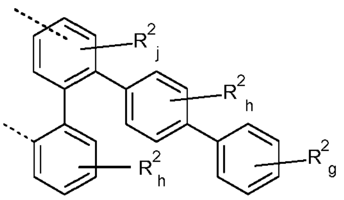

Formel ($L^1$-16)

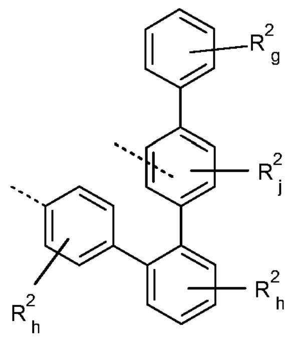

Formel ($L^1$-17)

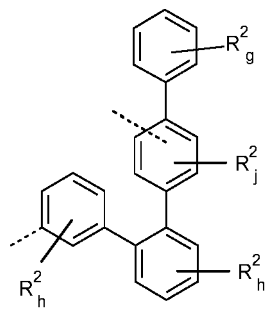

Formel ($L^1$-18)

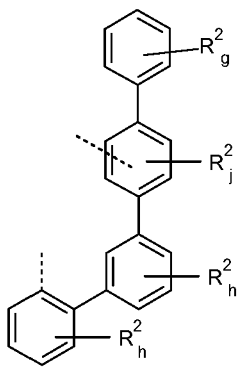

Formel ($L^1$-19)

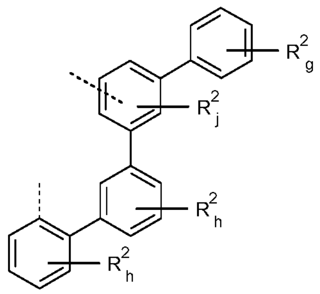

Formel ($L^1$-20)

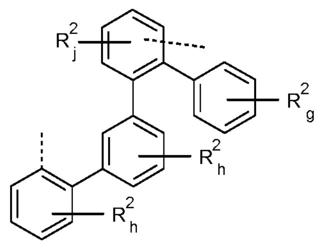

Formel ($L^1$-21)

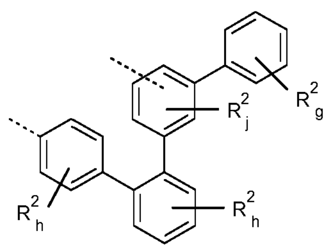

Formel ($L^1$-22)

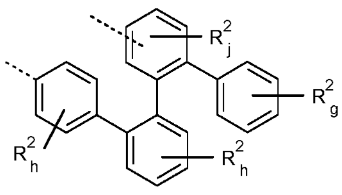

Formel ($L^1$-23)

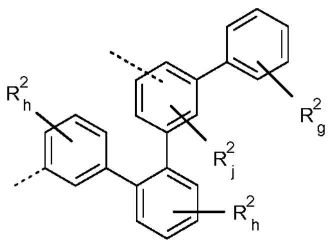

Formel ($L^1$-24)

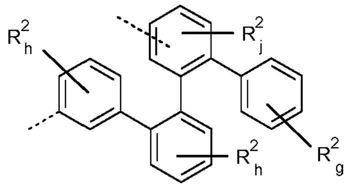

Formel ($L^1$-25)

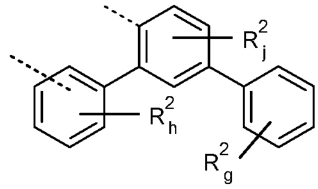

Formel ($L^1$-26)

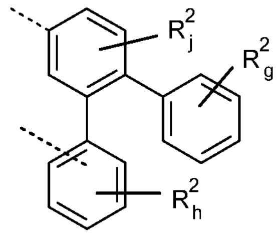

Formel ($L^1$-27)

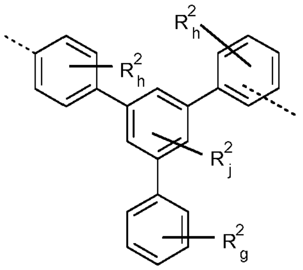

Formel ($L^1$-28)

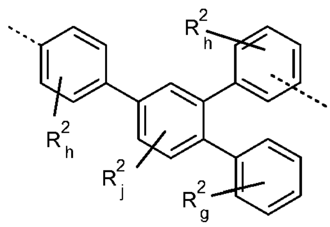

Formel ($L^1$-29)

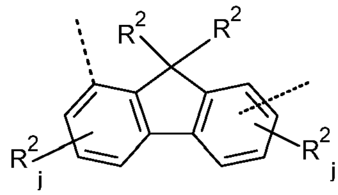

Formel ($L^1$-30)

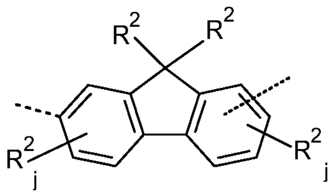

Formel ($L^1$-31)

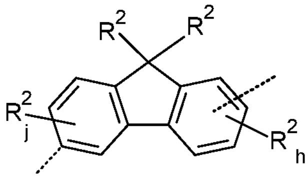

Formel ($L^1$-32)

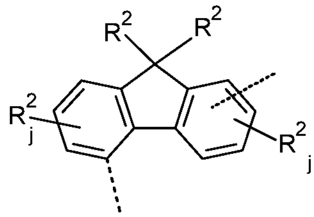

Formel ($L^1$-33)

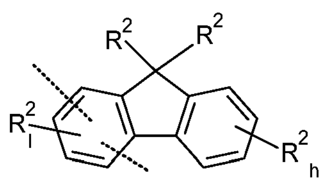

Formel ($L^1$-34)

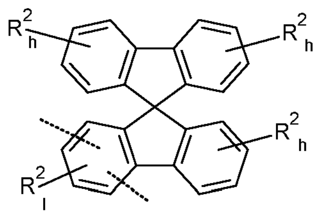

Formel ($L^1$-35)

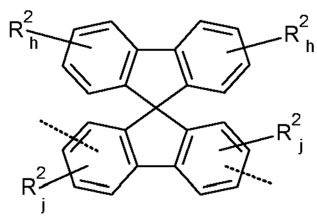

Formel ($L^1$-36)

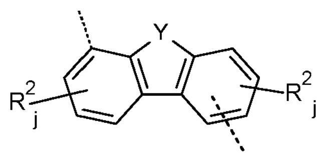

Formel ($L^1$-37)

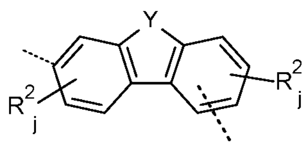

Formel ($L^1$-38)

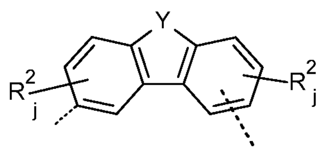

Formel ($L^1$-39)

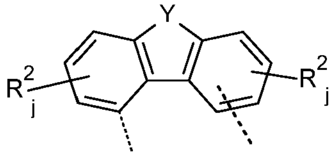
Formel ($L^1$-40)

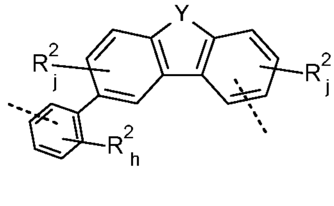
Formel ($L^1$-41)

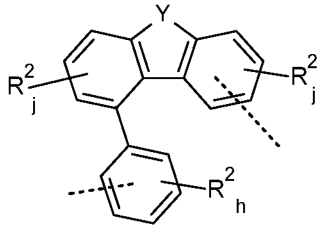
Formel ($L^1$-42)

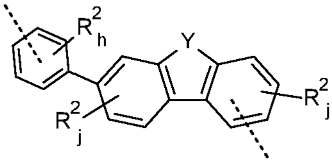
Formel ($L^1$-43)

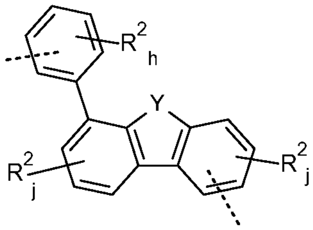
Formel ($L^1$-44)

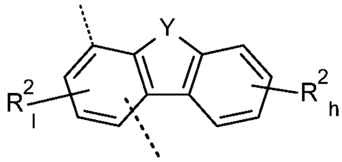
Formel ($L^1$-45)

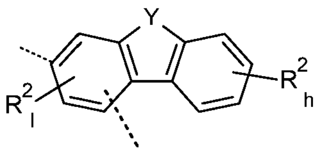
Formel ($L^1$-46)

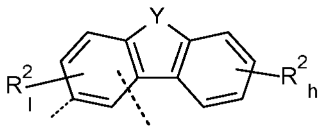
Formel ($L^1$-47)

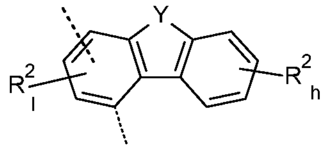
Formel ($L^1$-48)

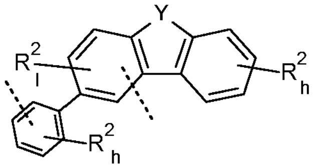
Formel ($L^1$-49)

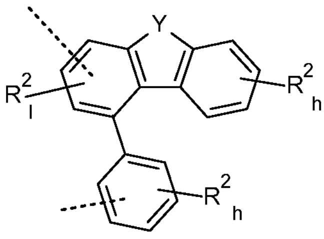
Formel ($L^1$-50)

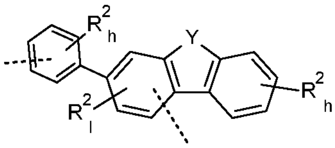
Formel ($L^1$-51)

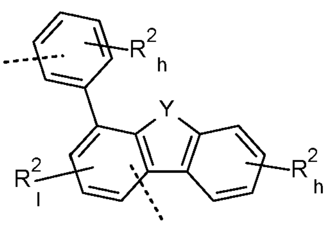
Formel ($L^1$-52)

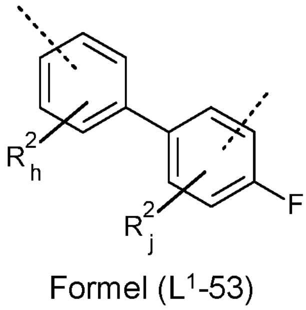
Formel ($L^1$-53)

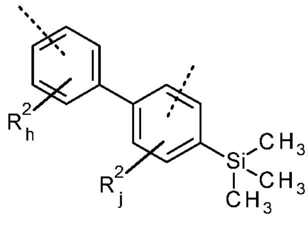
Formel ($L^1$-54)

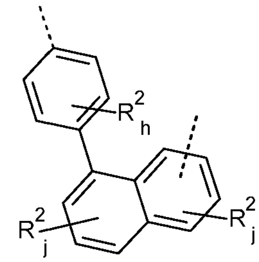
Formel (L$^1$-55)

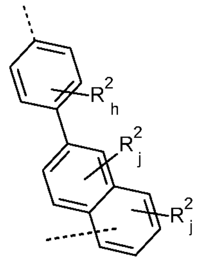
Formel (L$^1$-56)

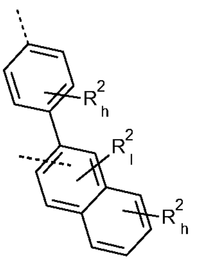
Formel (L$^1$-57)

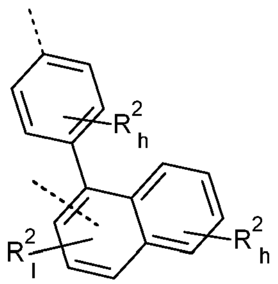
Formel (L$^1$-58)

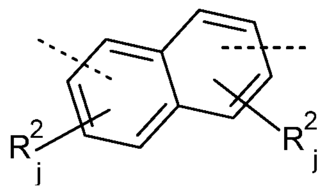
Formel (L$^1$-59)

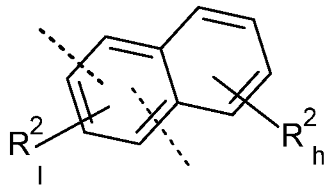
Formel (L$^1$-60)

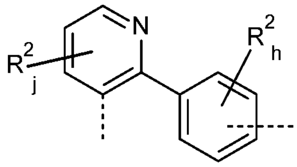
Formel (L$^1$-61)

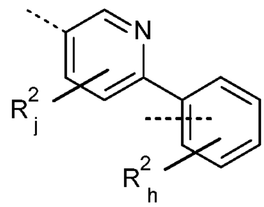
Formel (L$^1$-62)

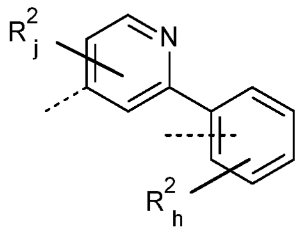
Formel (L$^1$-63)

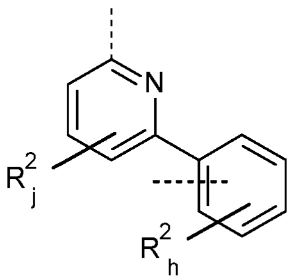
Formel (L$^1$-64)

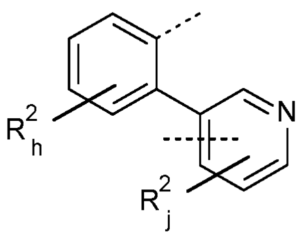
Formel (L$^1$-65)

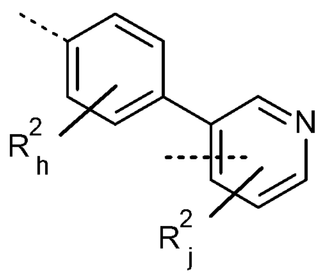
Formel (L$^1$-66)

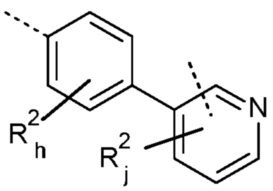
Formel (L$^1$-67)

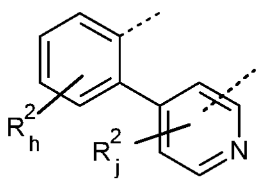
Formel (L$^1$-68)

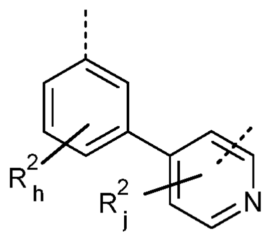
Formel (L$^1$-69)

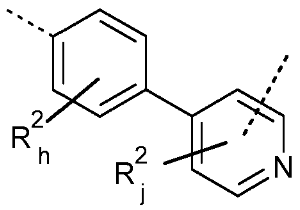

Formel ($L^1$-70)

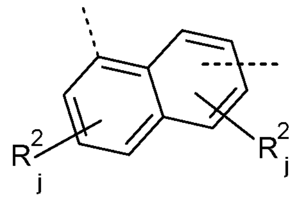

Formel ($L^1$-71)

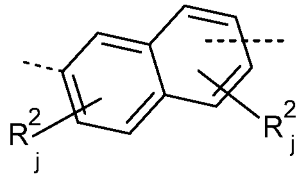

Formel ($L^1$-72)

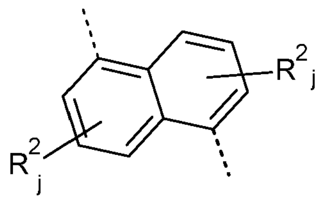

Formel ($L^1$-73)

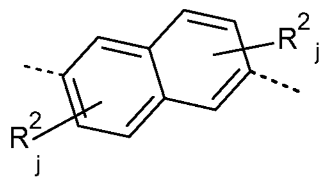

Formel ($L^1$-74)

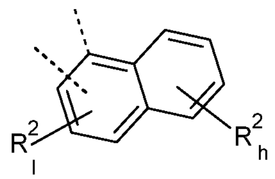

Formel ($L^1$-75)

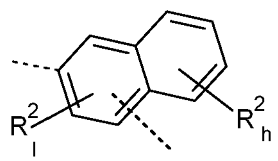

Formel ($L^1$-76)

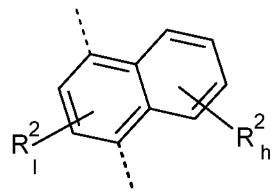

Formel ($L^1$-77)

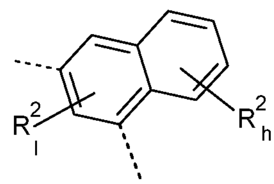

Formel ($L^1$-78)

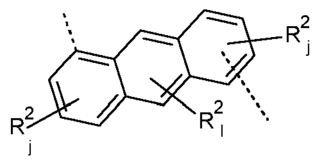

Formel ($L^1$-79)

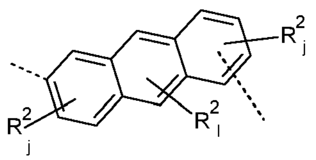

Formel ($L^1$-80)

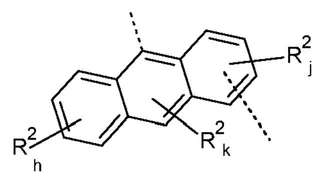

Formel ($L^1$-81)

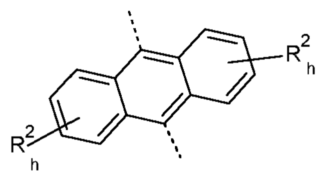

Formel ($L^1$-82)

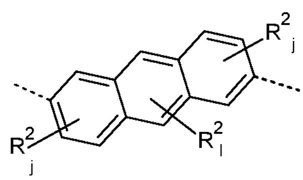

Formel ($L^1$-83)

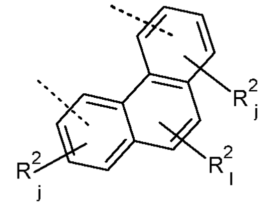

Formel ($L^1$-84)

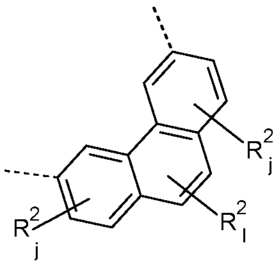

Formel (L$^1$-85)

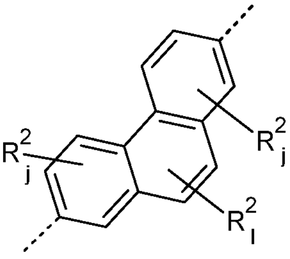

Formel (L$^1$-86)

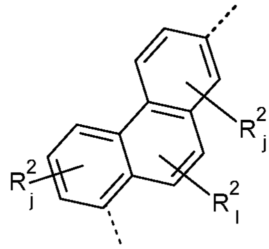

Formel (L$^1$-87)

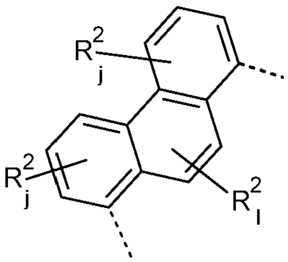

Formel (L$^1$-88)

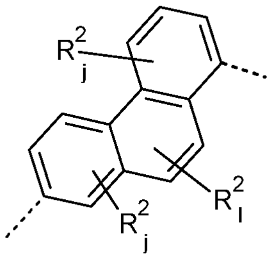

Formel (L$^1$-89)

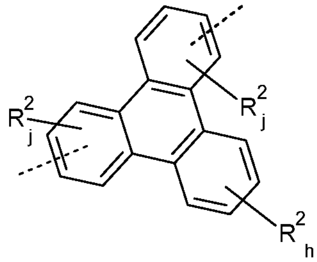

Formel (L$^1$-90)

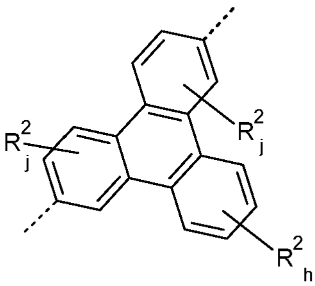

Formel (L$^1$-91)

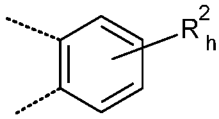

Formel (L$^1$-92)

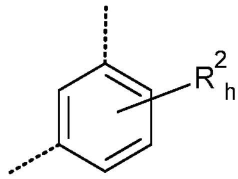

Formel (L$^1$-93)

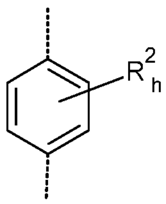

Formel (L$^1$-94)

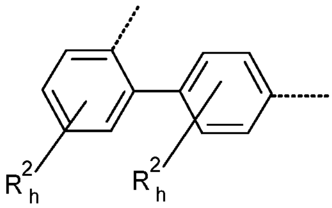

Formel (L$^1$-95)

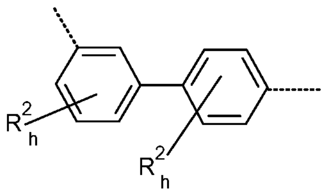

Formel (L$^1$-96)

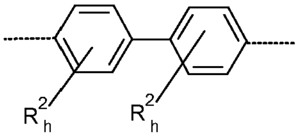

Formel (L$^1$-97)

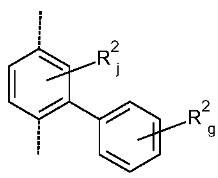

Formel (L$^1$-98)

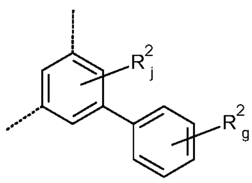

Formel (L$^1$-99)

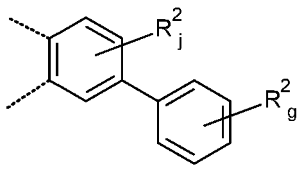

Formel ($L^1$-100)

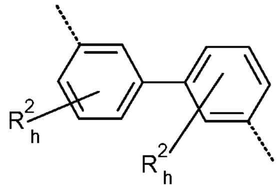

Formel ($L^1$-101)

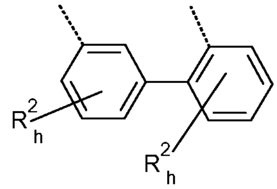

Formel ($L^1$-102)

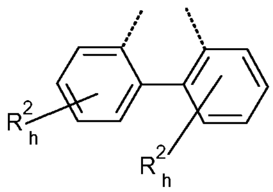

Formel ($L^1$-103)

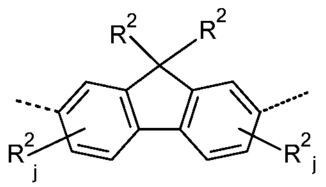

Formel ($L^1$-104)

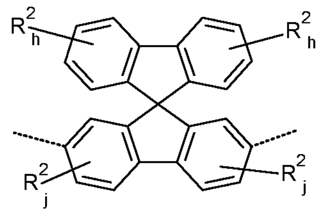

Formel ($L^1$-105)

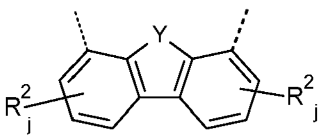

Formel ($L^1$-106)

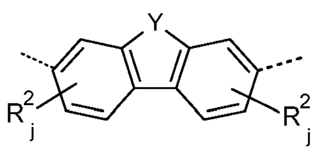

Formel ($L^1$-107)

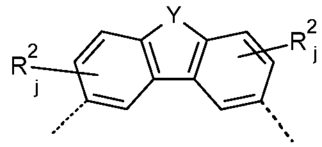

Formel ($L^1$-108)

wobei die gestrichelten Bindungen jeweils die Anbindungspositionen markieren, der Index k 0 oder 1 ist, der Index l 0, 1 oder 2 ist, der Index j bei jedem Auftreten unabhängig 0, 1, 2 oder 3 ist; der Index h bei jedem Auftreten unabhängig 0, 1, 2, 3 oder 4 ist, der Index g 0, 1, 2, 3, 4 oder 5 ist; das Symbol Y O, S oder $NR^2$, vorzugsweise O oder S ist; und das Symbol $R^2$ die zuvor, insbesondere für Formel (I) genannte Bedeutung aufweist.

Vorzugsweise kann vorgesehen sein, dass die Summe der Indices k, l, g, h und j in den Strukturen der Formel ($L^1$-1) bis ($L^1$-108) jeweils höchstens 3, vorzugsweise höchstens 2 und besonders bevorzugt höchstens 1 beträgt.

Bevorzugte erfindungsgemäße Verbindungen umfassen eine Gruppe L, die eine Bindung darstellt oder die ausgewählt ist aus einer der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103). Mit Vorteil kann die Summe der Indices k, l, g, h und j in den Strukturen der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103) jeweils höchstens 3, vorzugsweise höchstens 2 und besonders bevorzugt höchstens 1 betragen.

Bevorzugt bilden die Reste $R^2$ in den Formeln ($L^1$-1) bis ($L^1$-108) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

In einer weiteren Ausgestaltung kann vorgesehen sein, dass eine erfindungsgemäße Verbindung, umfassend mindestens eine Struktur gemäß Formel (I), (IIa), (IIb), (IIc), (IId), (IIIa), (IIIb), (IIIc), (IIId), (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4), (IIId-4), (IVa), (IVb), (IVc), (Va), (Vb), (Vc), (Va-1), (Vb-1), (Vc-1), (Va-2), (Vb-2), (Vc-2), (Va-3), (Vb-3) und/oder (Vc-3) mindestens eine lochtransportierende Gruppe umfasst, vorzugsweise eine Carbazol-Gruppe. Ferner kann als lochtransportierende Gruppe auch eine Indenocarbazol- oder Indolocarbazol-Gruppe vorgesehen sein.

Wenn die erfindungsgemäße Verbindung mit aromatischen oder heteroaromatischen Gruppen $R^1$ bzw. $R^2$ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

In einer weiteren bevorzugten Ausführungsform der Erfindung ist $R^2$, beispielsweise bei einer Struktur gemäß

Formel (I) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

In einer weiteren bevorzugten Ausführungsform der Erfindung ist $R^3$, beispielsweise bei einer Struktur gemäß Formel (I) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Beispiele für geeignete erfindungsgemäße Verbindungen sind die nachstehend gezeigten Strukturen gemäß den folgenden Formeln 1 bis 178:

| | | |
|---|---|---|
| 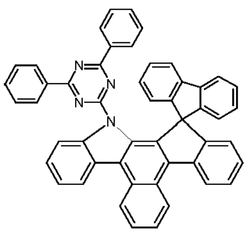 | 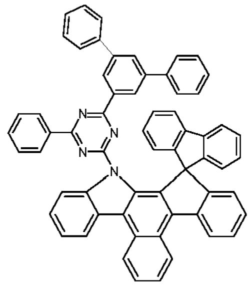 | 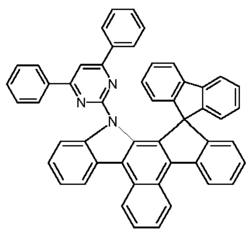 |
| Formel 1 | Formel 2 | Formel 3 |
| 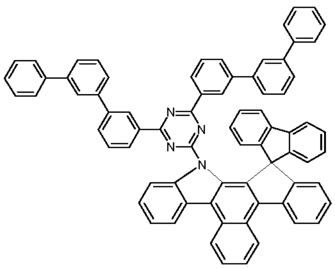 | 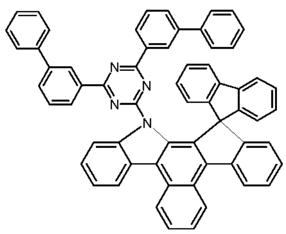 | 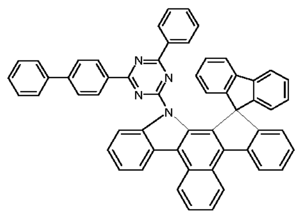 |
| Formel 4 | Formel 5 | Formel 6 |
| 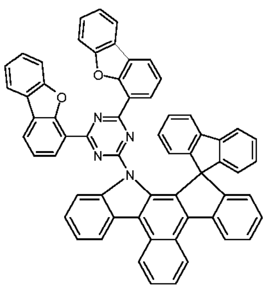 | 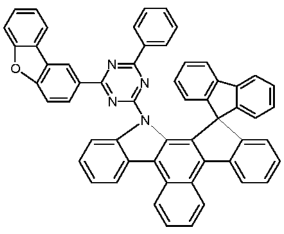 | 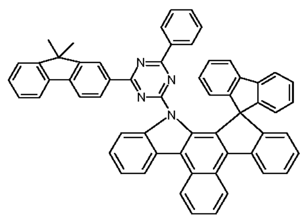 |
| Formel 7 | Formel 8 | Formel 9 |

(fortgesetzt)

| | | |
|---|---|---|
| 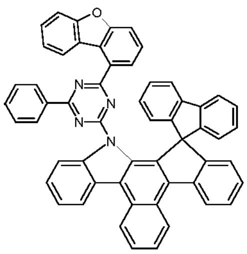 | 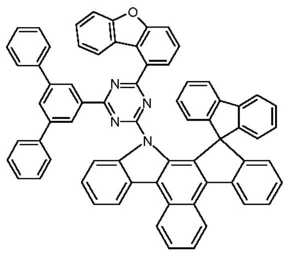 | 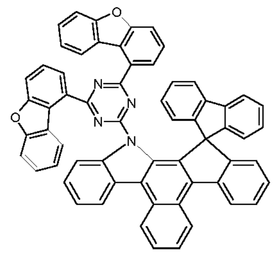 |
| Formel 10 | Formel 11 | Formel 12 |
| 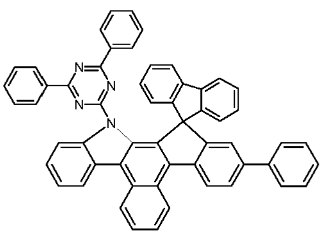 | 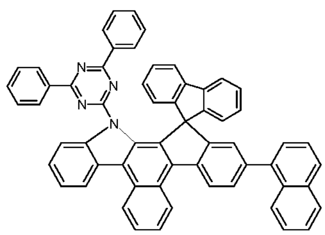 | 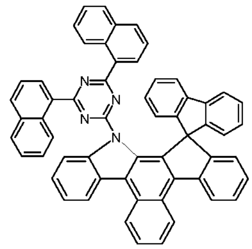 |
| Formel 13 | Formel 14 | Formel 15 |
| 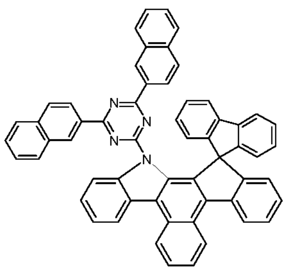 | 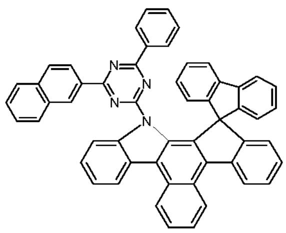 | 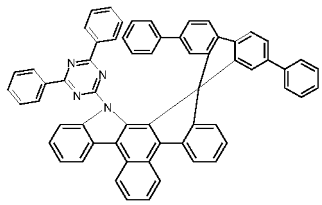 |
| Formel 16 | Formel 17 | Formel 18 |
| 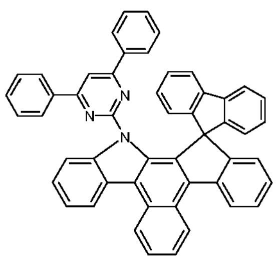 | 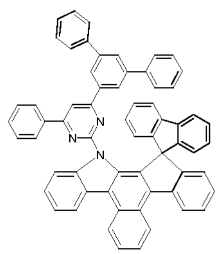 | 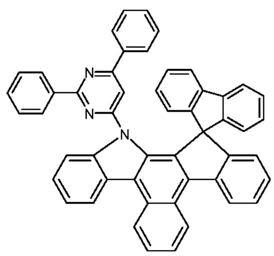 |
| Formel 19 | Formel 20 | Formel 21 |
| 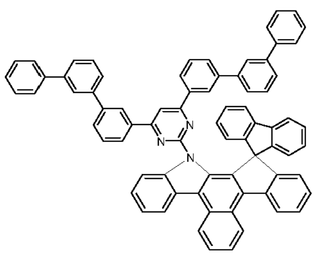 | 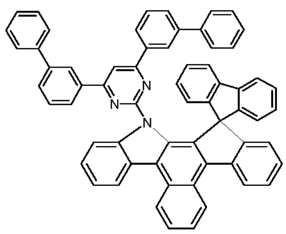 | 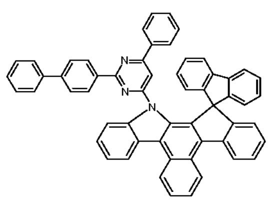 |
| Formel 22 | Formel 23 | Formel 24 |

(fortgesetzt)

| | | |
|---|---|---|
| 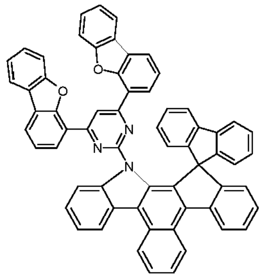 | 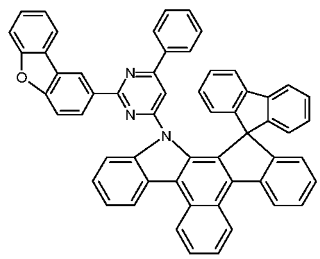 | 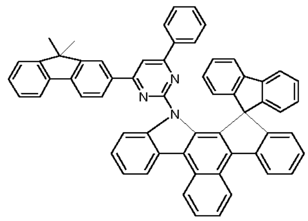 |
| Formel 25 | Formel 26 | Formel 27 |
| 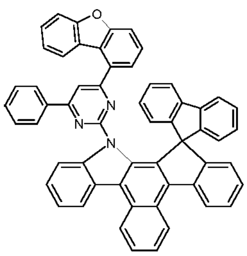 | 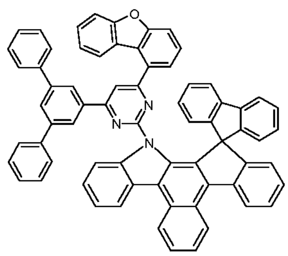 | 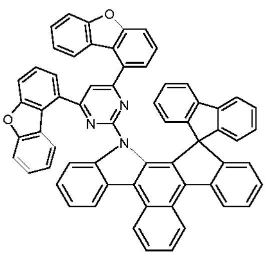 |
| Formel 28 | Formel 29 | Formel 30 |
| 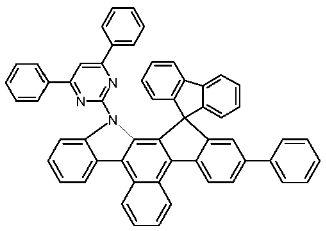 | 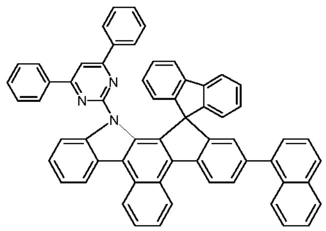 | 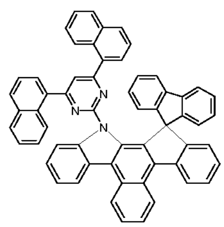 |
| Formel 31 | Formel 32 | Formel 33 |
| 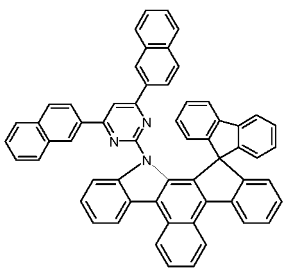 | 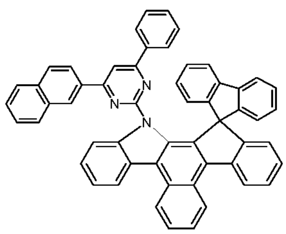 | 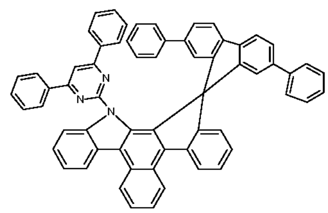 |
| Formel 34 | Formel 35 | Formel 36 |
| 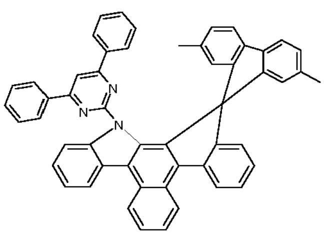 | 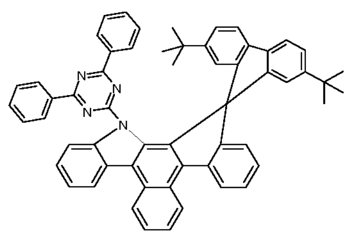 | 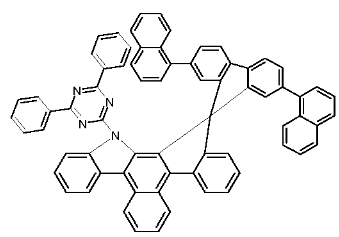 |
| Formel 37 | Formel 38 | Formel 39 |

(fortgesetzt)

| | | |
|---|---|---|
| 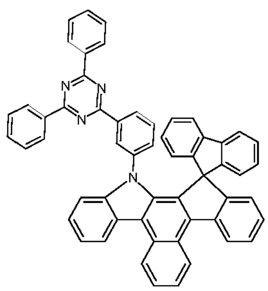 | 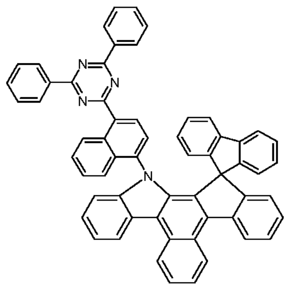 | 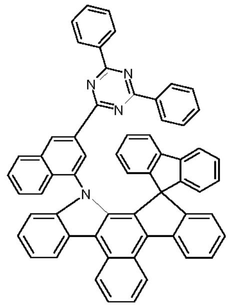 |
| Formel 40 | Formel 41 | Formel 42 |
| 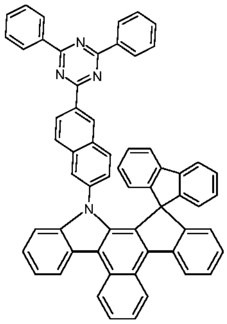 | 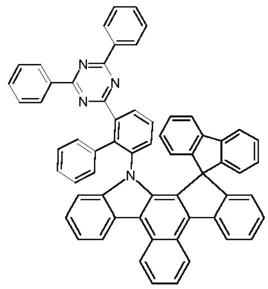 | 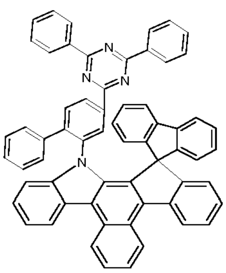 |
| Formel 43 | Formel 44 | Formel 45 |
| 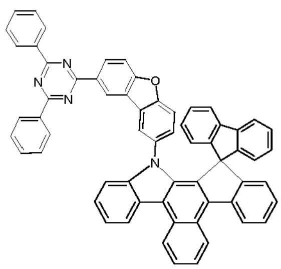 | 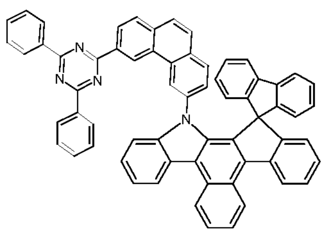 | 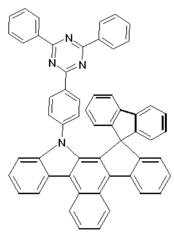 |
| Formel 46 | Formel 47 | Formel 48 |
| 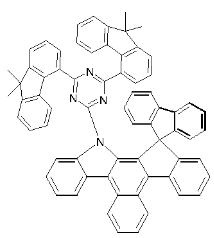 | 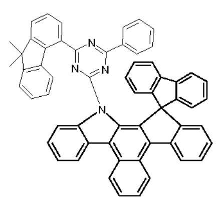 | 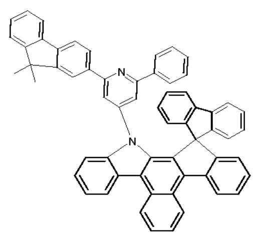 |
| Formel 49 | Formel 50 | Formel 51 |
| 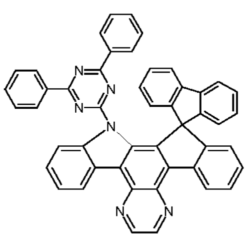 | 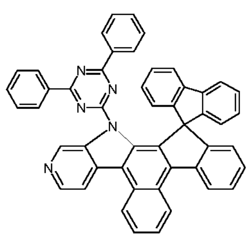 | 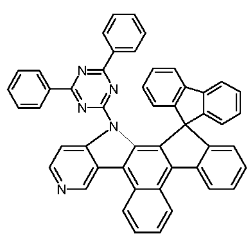 |
| Formel 52 | Formel 53 | Formel 54 |

(fortgesetzt)

| | | |
|---|---|---|
| 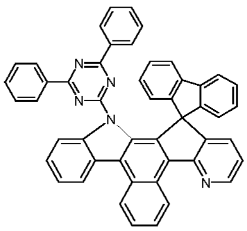 | 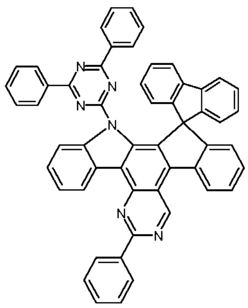 | 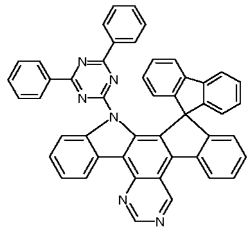 |
| Formel 55 | Formel 56 | Formel 57 |
| 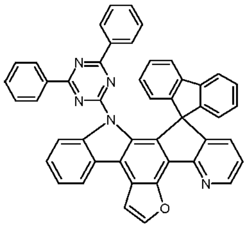 | 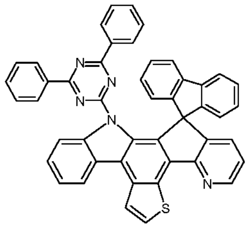 | 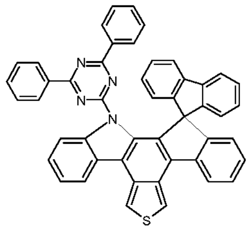 |
| Formel 58 | Formel 59 | Formel 60 |
| 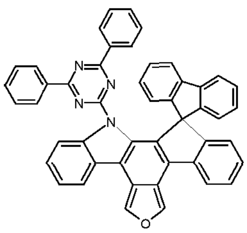 | 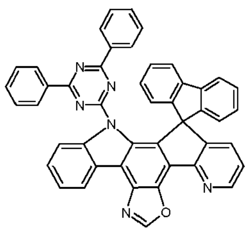 | 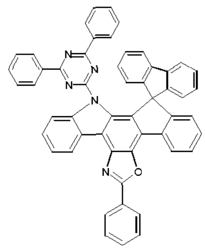 |
| Formel 61 | Formel 62 | Formel 63 |
| 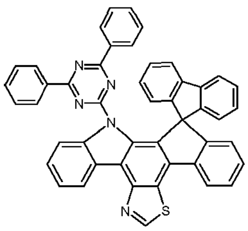 | 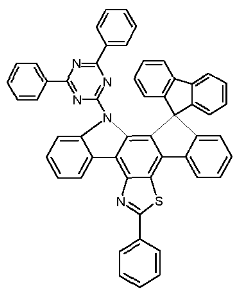 | 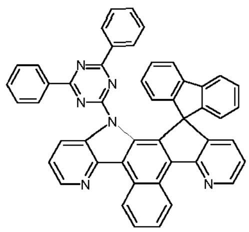 |
| Formel 64 | Formel 65 | Formel 66 |
| 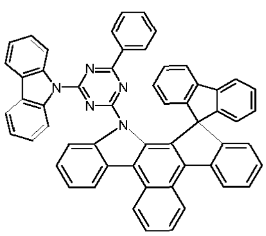 | 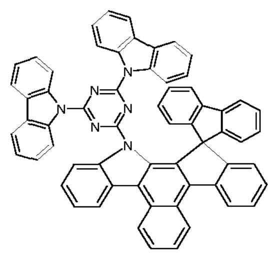 | 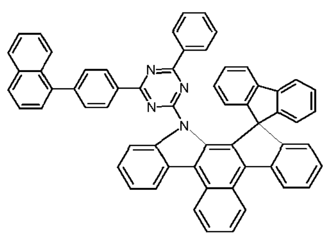 |
| Formel 67 | Formel 68 | Formel 69 |

(fortgesetzt)

| | | |
|---|---|---|
| 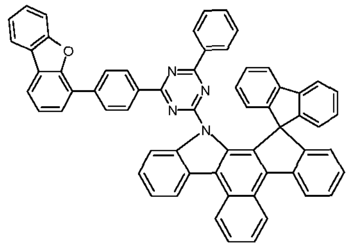 | 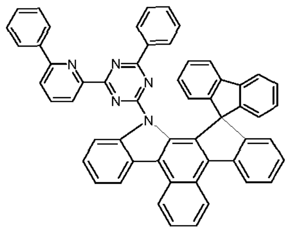 | 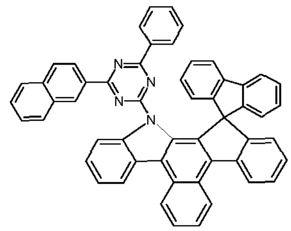 |
| Formel 70 | Formel 71 | Formel 72 |
| 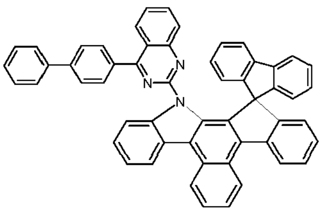 | 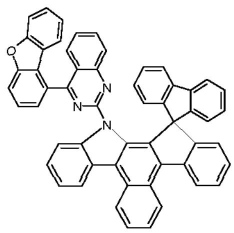 | 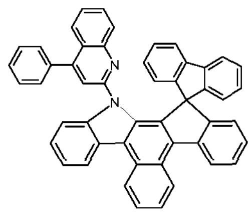 |
| Formel 73 | Formel 74 | Formel 75 |
| 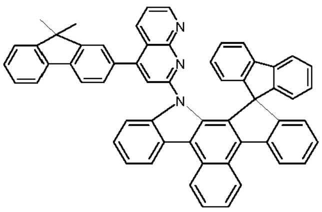 | 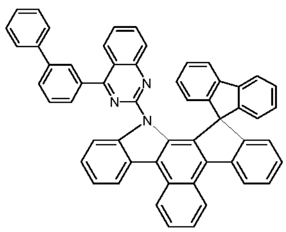 | 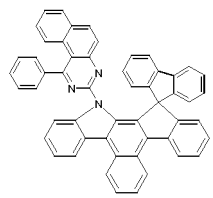 |
| Formel 76 | Formel 77 | Formel 78 |
| 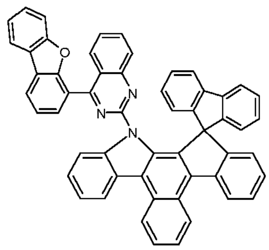 | 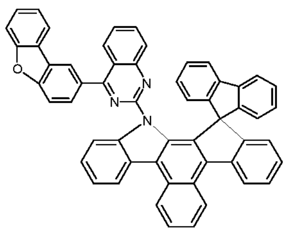 | 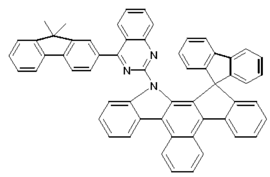 |
| Formel 79 | Formel 80 | Formel 81 |
| 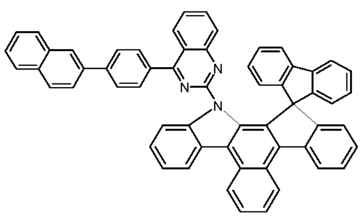 | 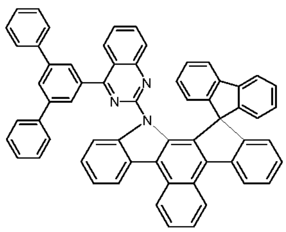 | 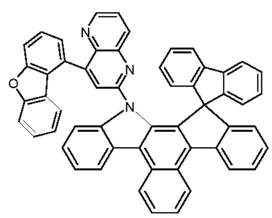 |
| Formel 82 | Formel 83 | Formel 84 |

(fortgesetzt)

| | | |
|---|---|---|
| 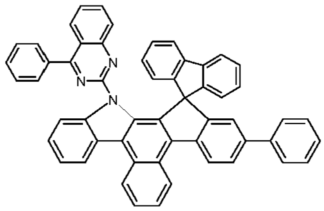 | 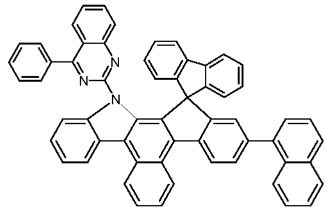 | 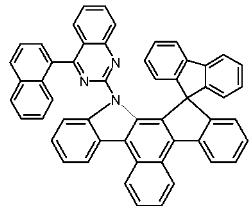 |
| Formel 85 | Formel 86 | Formel 87 |
| 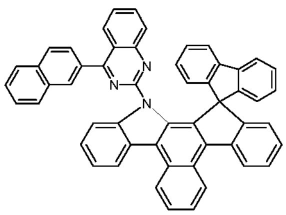 | 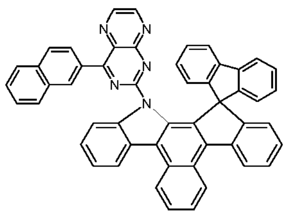 | 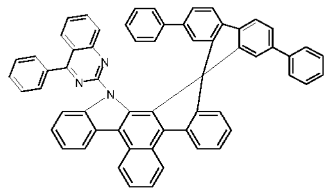 |
| Formel 88 | Formel 89 | Formel 90 |
| 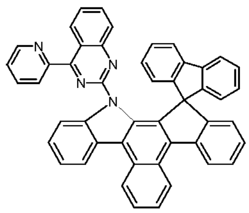 | 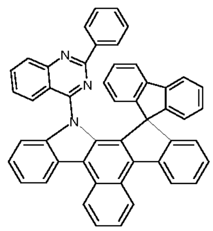 | 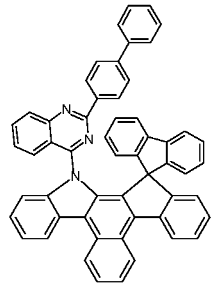 |
| Formel 91 | Formel 92 | Formel 93 |
| 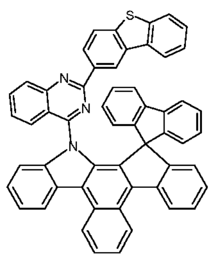 | 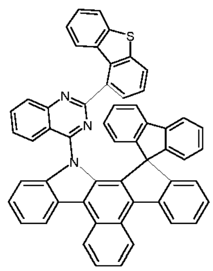 | 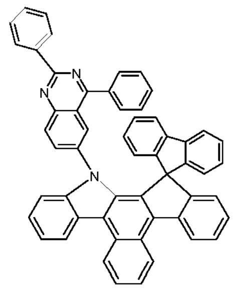 |
| Formel 94 | Formel 95 | Formel 96 |
| 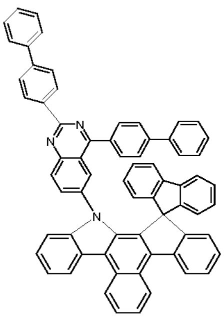 | 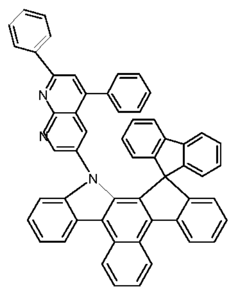 | 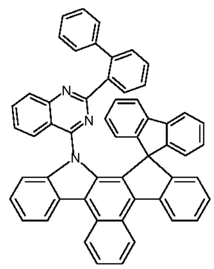 |
| Formel 97 | Formel 98 | Formel 99 |

(fortgesetzt)

| | | |
|---|---|---|
| 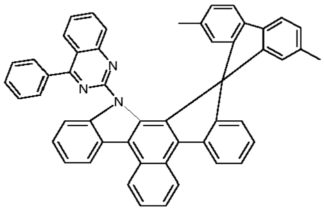 | 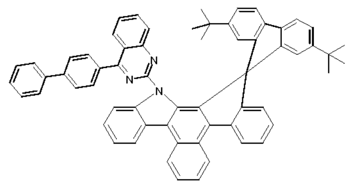 | 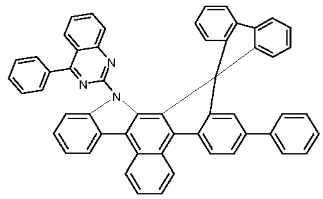 |
| Formel 100 | Formel 101 | Formel 102 |
| 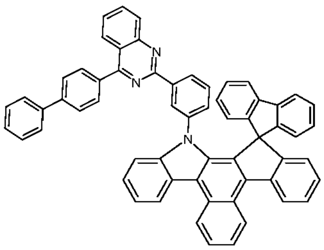 | 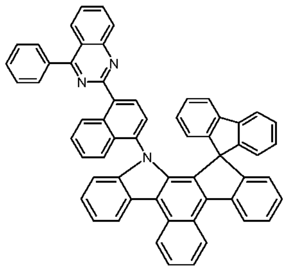 | 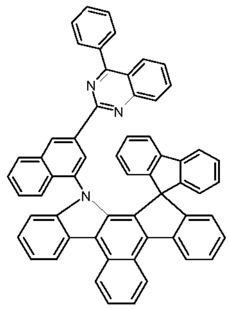 |
| Formel 103 | Formel 104 | Formel 105 |
| 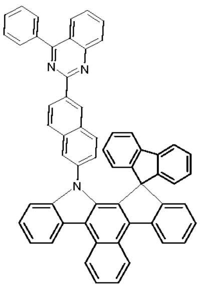 | 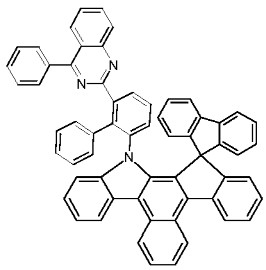 | 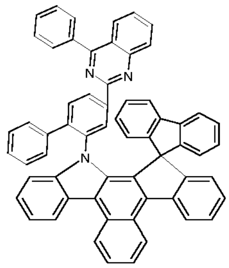 |
| Formel 106 | Formel 107 | Formel 108 |
| 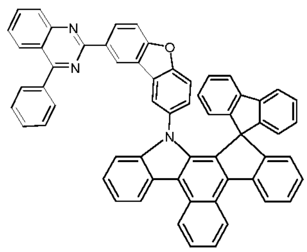 | 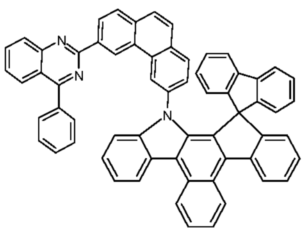 | 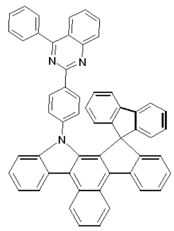 |
| Formel 109 | Formel 110 | Formel 111 |
| 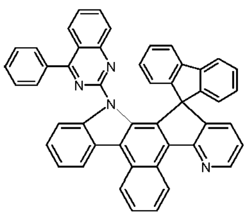 | 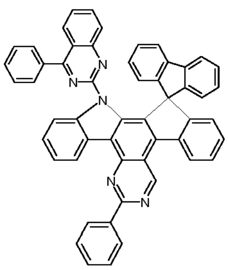 | 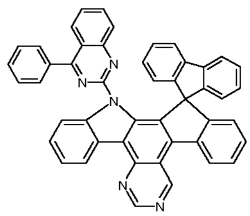 |
| Formel 112 | Formel 113 | Formel 114 |

(fortgesetzt)

| | | |
|---|---|---|
| 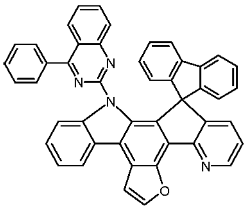 | 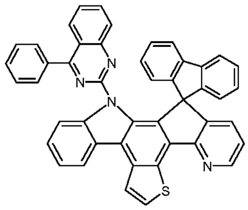 | 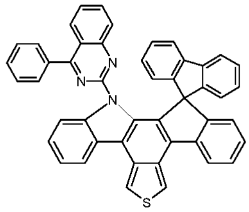 |
| Formel 115 | Formel 116 | Formel 117 |
| 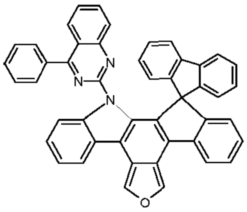 | 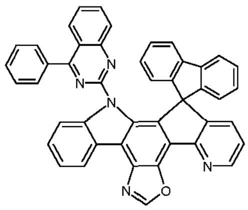 | 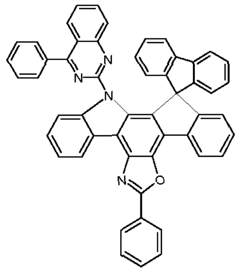 |
| Formel 118 | Formel 119 | Formel 120 |
| 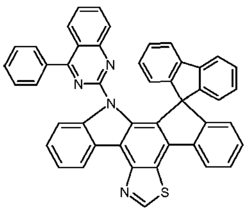 | 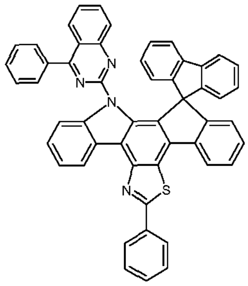 | 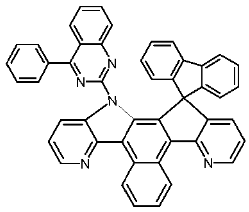 |
| Formel 121 | Formel 122 | Formel 123 |
| 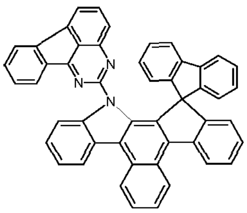 | 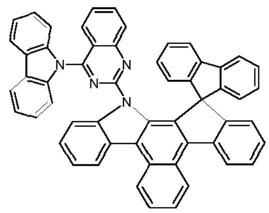 | 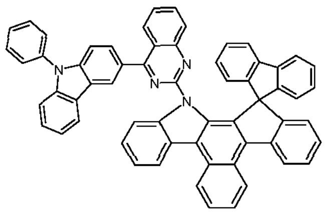 |
| Formel 124 | Formel 125 | Formel 126 |
| 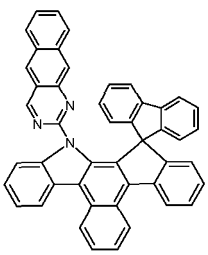 | 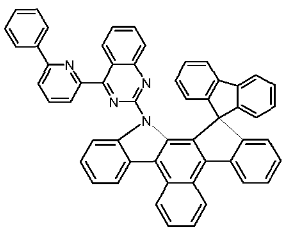 | 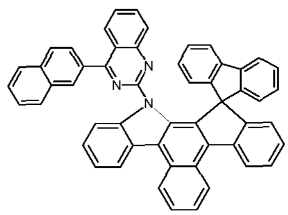 |
| Formel 127 | Formel 128 | Formel 129 |

(fortgesetzt)

| | | |
|---|---|---|
| 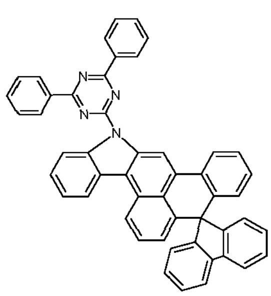 | 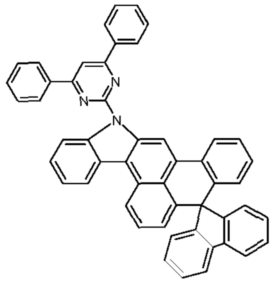 | 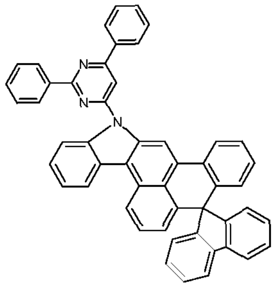 |
| Formel 130 | Formel 131 | Formel 132 |
| 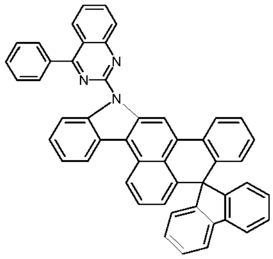 | 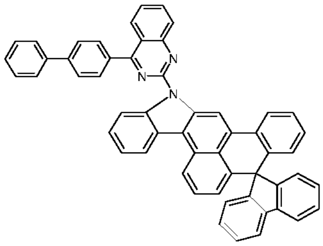 | 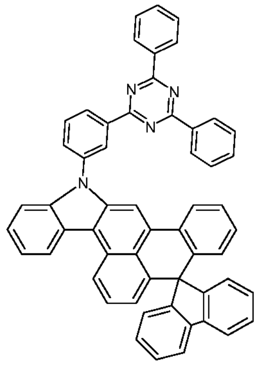 |
| Formel 133 | Formel 134 | Formel 135 |
| 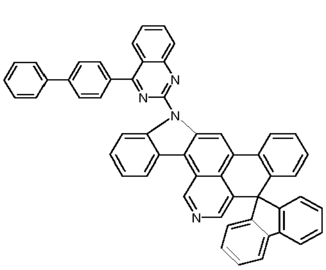 | 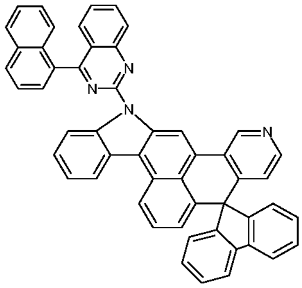 | 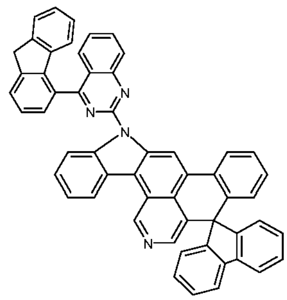 |
| Formel 136 | Formel 137 | Formel 138 |
| 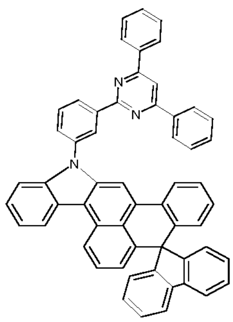 | 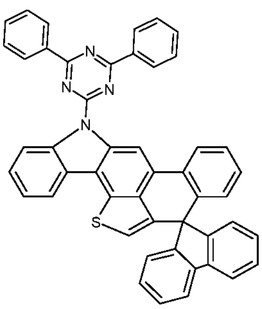 | 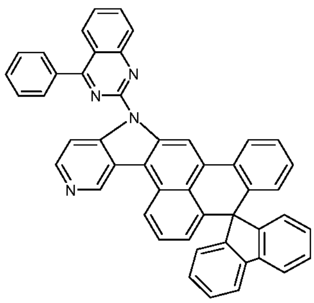 |
| Formel 139 | Formel 140 | Formel 141 |

(fortgesetzt)

| | | |
|---|---|---|
| 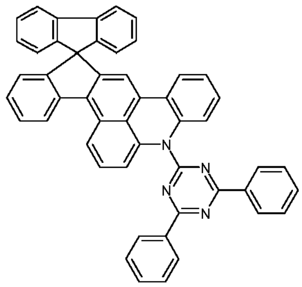 | 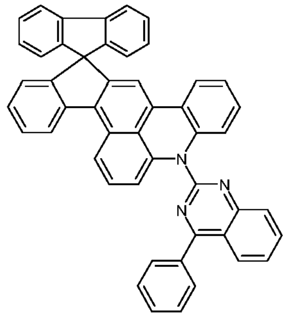 | 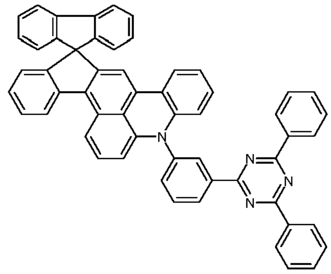 |
| Formel 142 | Formel 143 | Formel 144 |
| 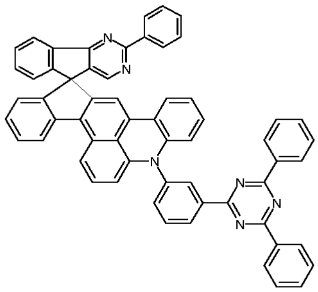 | 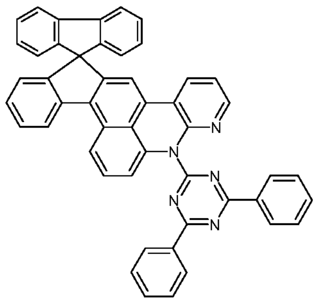 | 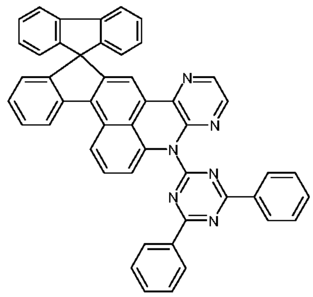 |
| Formel 145 | Formel 146 | Formel 147 |
| 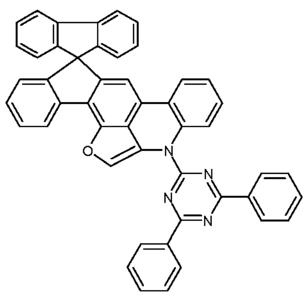 | 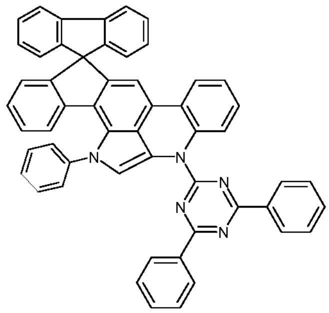 | 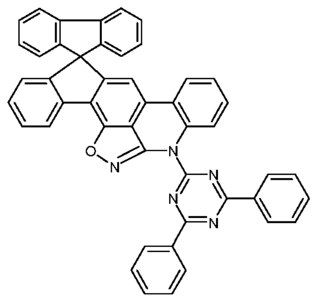 |
| Formel 148 | Formel 149 | Formel 150 |
| 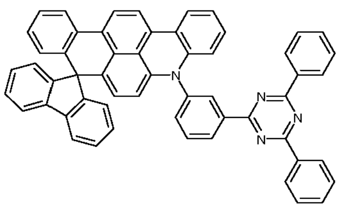 | 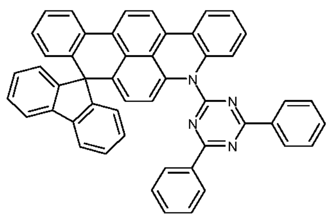 | 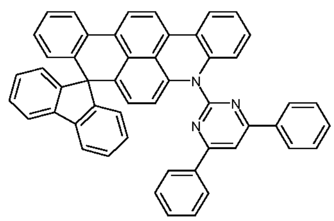 |
| Formel 151 | Formel 152 | Formel 153 |
| 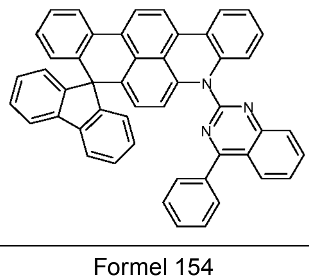 | 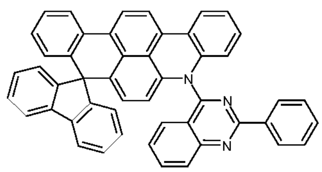 | 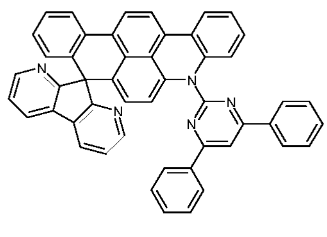 |
| Formel 154 | Formel 155 | Formel 156 |

(fortgesetzt)

| | | |
|---|---|---|
| 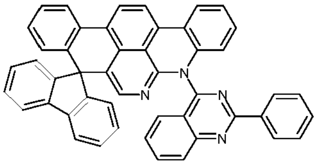 | 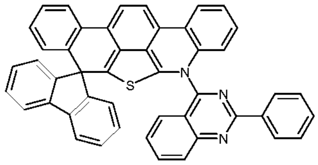 | 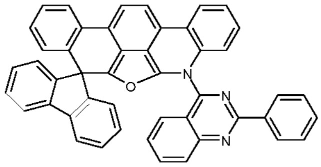 |
| Formel 157 | Formel 158 | Formel 159 |
| 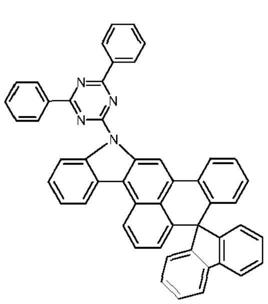 | 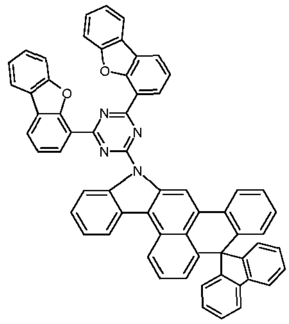 | 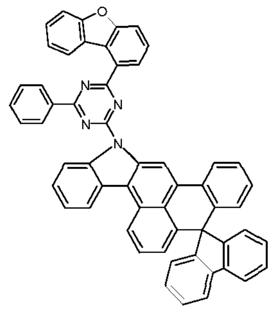 |
| Formel 160 | Formel 161 | Formel 162 |
| 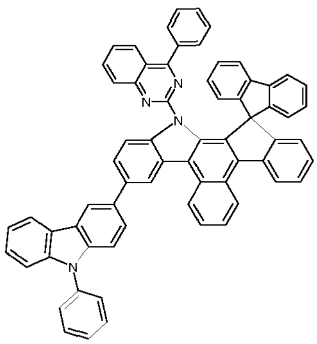 | 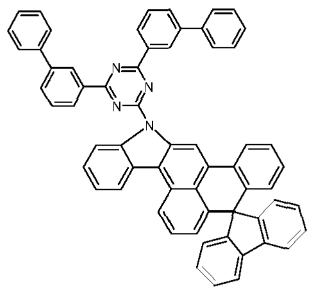 | 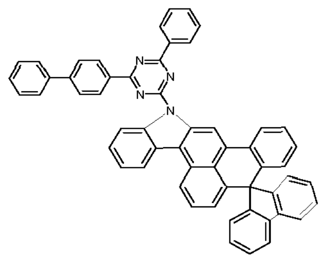 |
| Formel 163 | Formel 164 | Formel 165 |
| 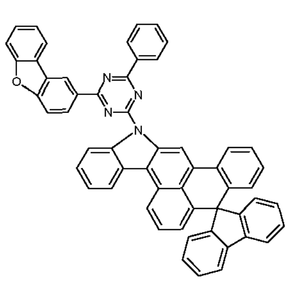 | 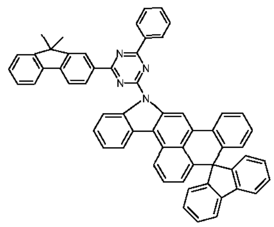 | 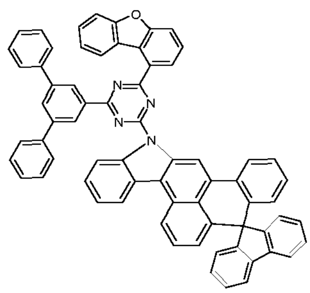 |
| Formel 166 | Formel 167 | Formel 168 |
| 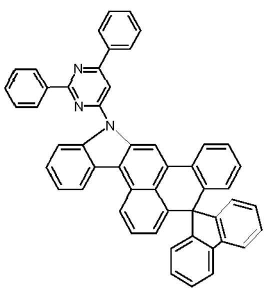 | 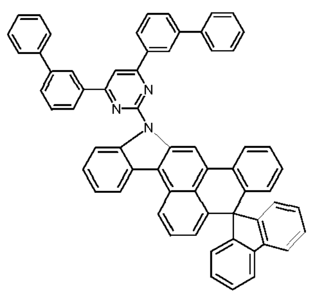 | 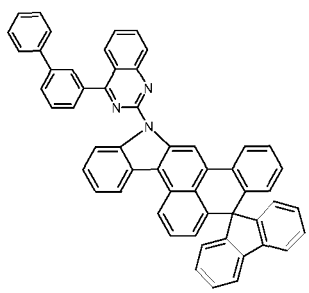 |

(fortgesetzt)

| Formel 169 | Formel 170 | Formel 171 |
|---|---|---|
| 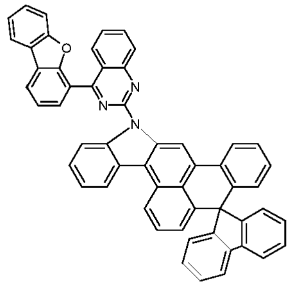 | 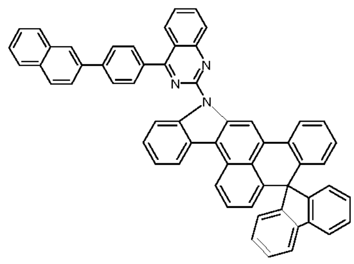 | 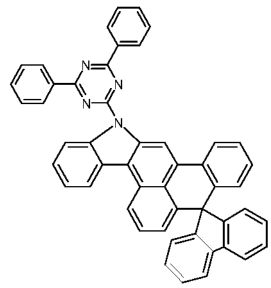 |
| Formel 172 | Formel 173 | Formel 174 |
| 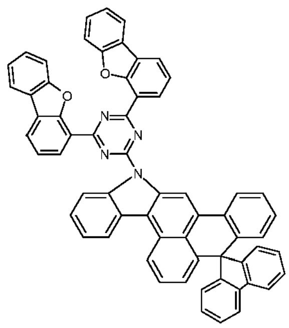 | 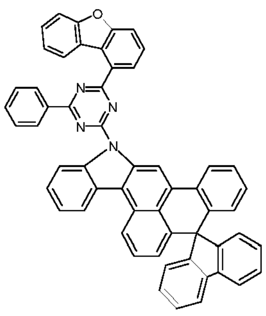 | 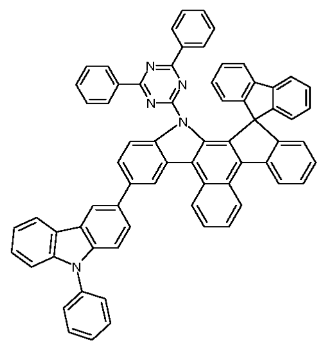 |
| Formel 175 | Formel 176 | Formel 177 |
| 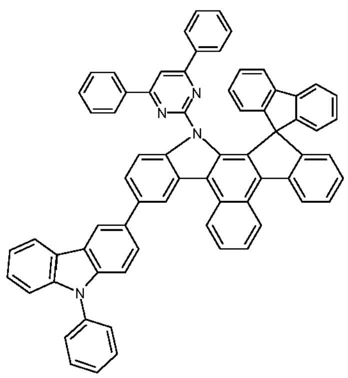 | | |
| Formel 178 | | |

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen, umfassend Strukturen gemäß Formel (I), bei dem in einer Kupplungsreaktion eine Verbindung, umfassend mindestens eine stickstoffhaltige heterocyclische Gruppe, mit einer Verbindung, umfassend mindestens eine Elektronentransportgruppe, verbunden wird. Insbesondere wird die Gruppe Q-L durch eine Kupplungsreaktion an dem Grundgerüst der Formel (I), welches keine Gruppe Q-L enthält, gebunden.

Geeignete Verbindungen mit einer Carbazol-Gruppe können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Arylverbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen. Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Im allen folgenden Syntheseschemata sind die Verbindungen zur Vereinfachung der Strukturen mit einer geringen Anzahl an Substituenten gezeigt gezeigt. Dies schließt das Vorhandensein von beliebigen weiteren Substituenten in den Verfahren nicht aus.

Eine Umsetzung ergibt sich beispielhaft gemäß folgenden Schemata, ohne dass hierdurch eine Beschränkung erfolgen soll. Die Teilschritte der einzelnen Schemata können hierbei beliebig kombiniert werden.

Schema 1

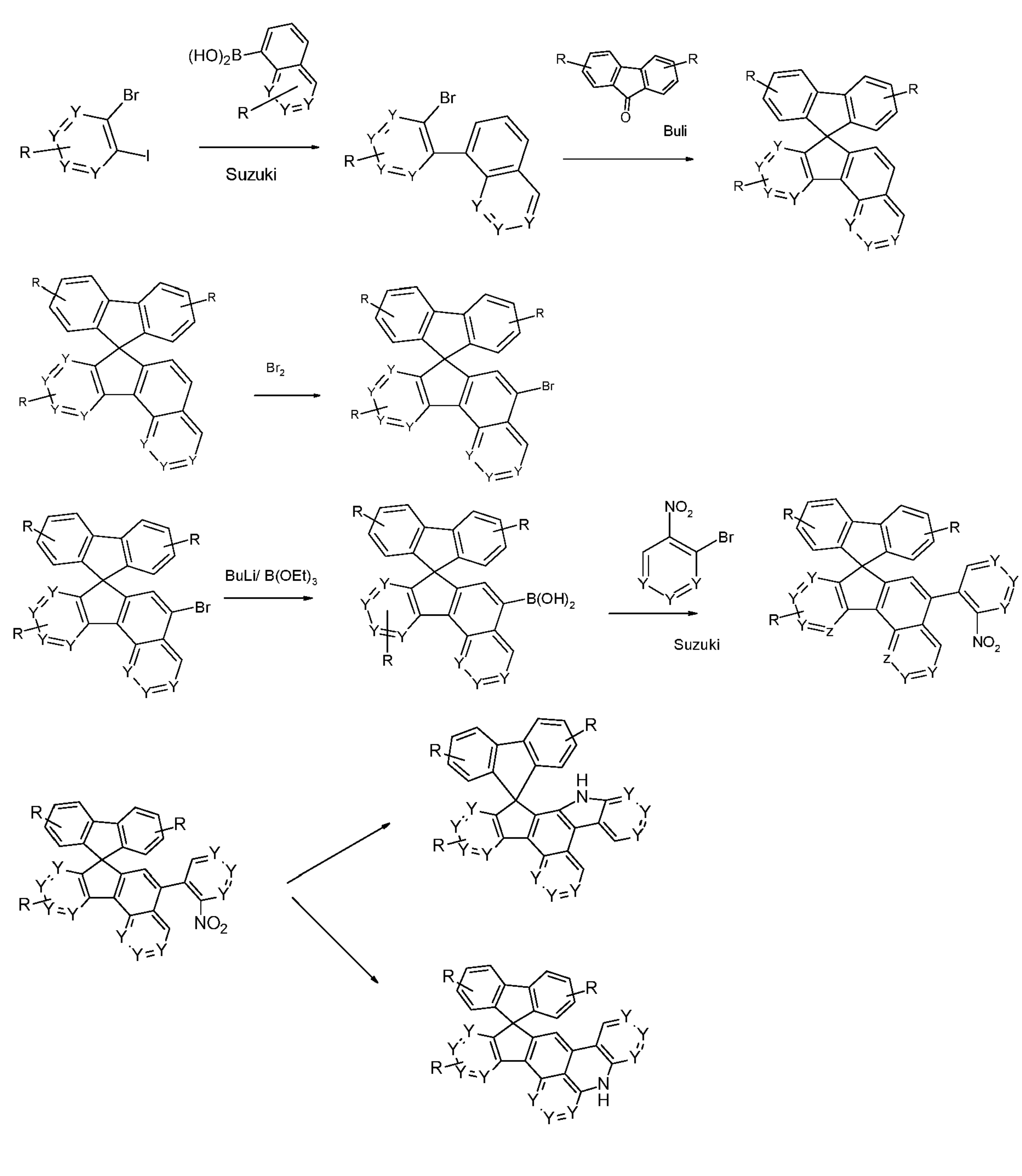

Schema 2

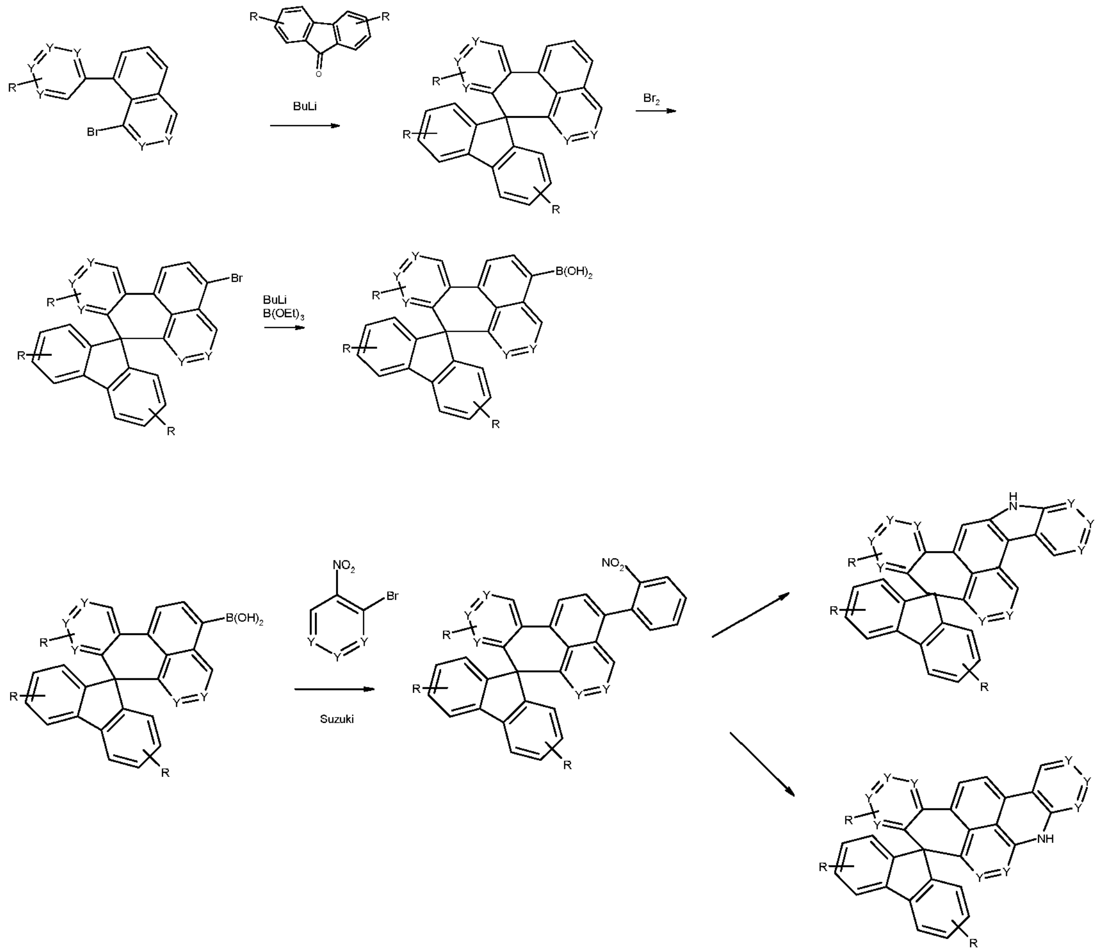

Schema 3

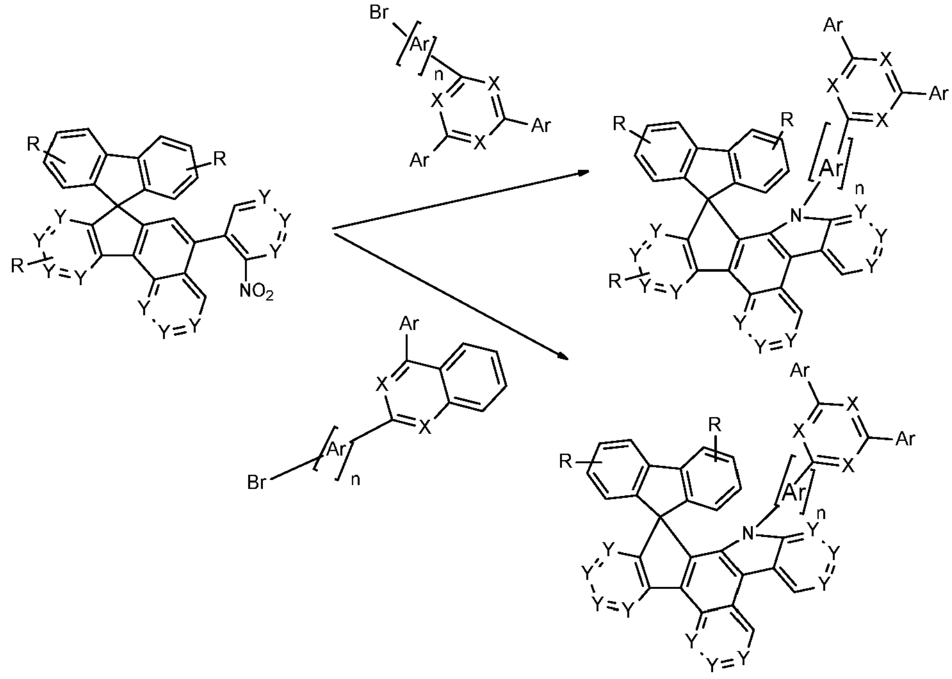

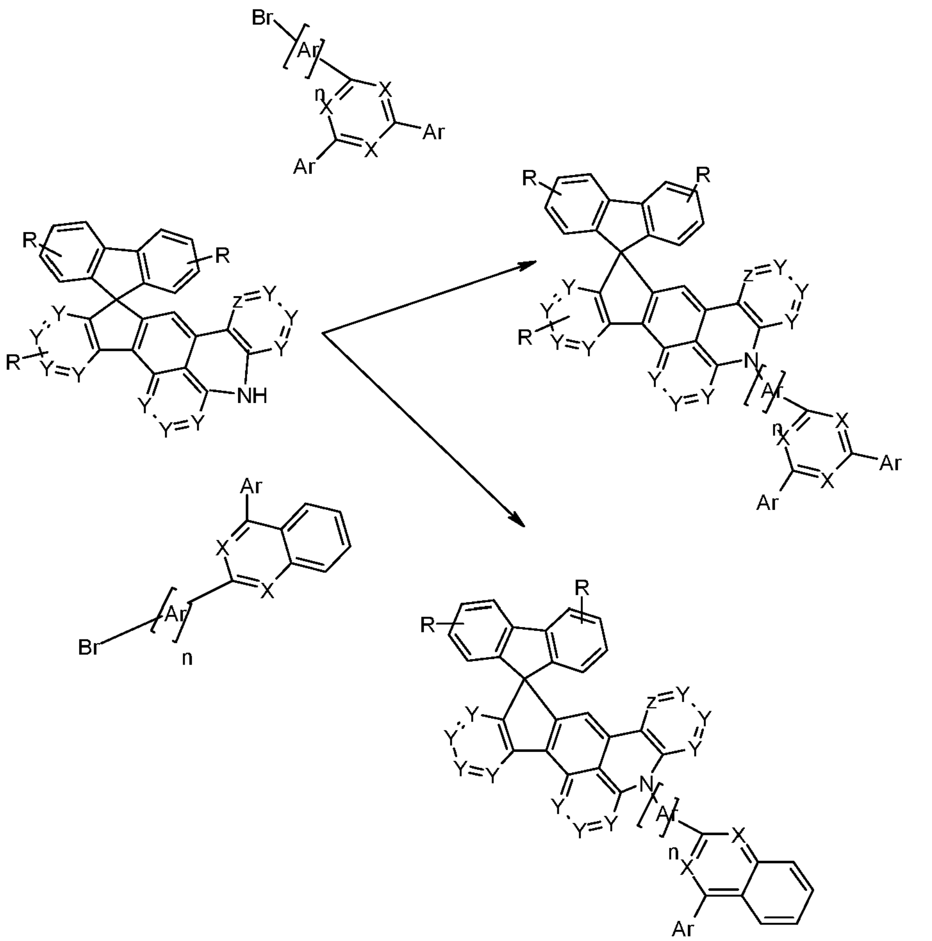

Schema 4

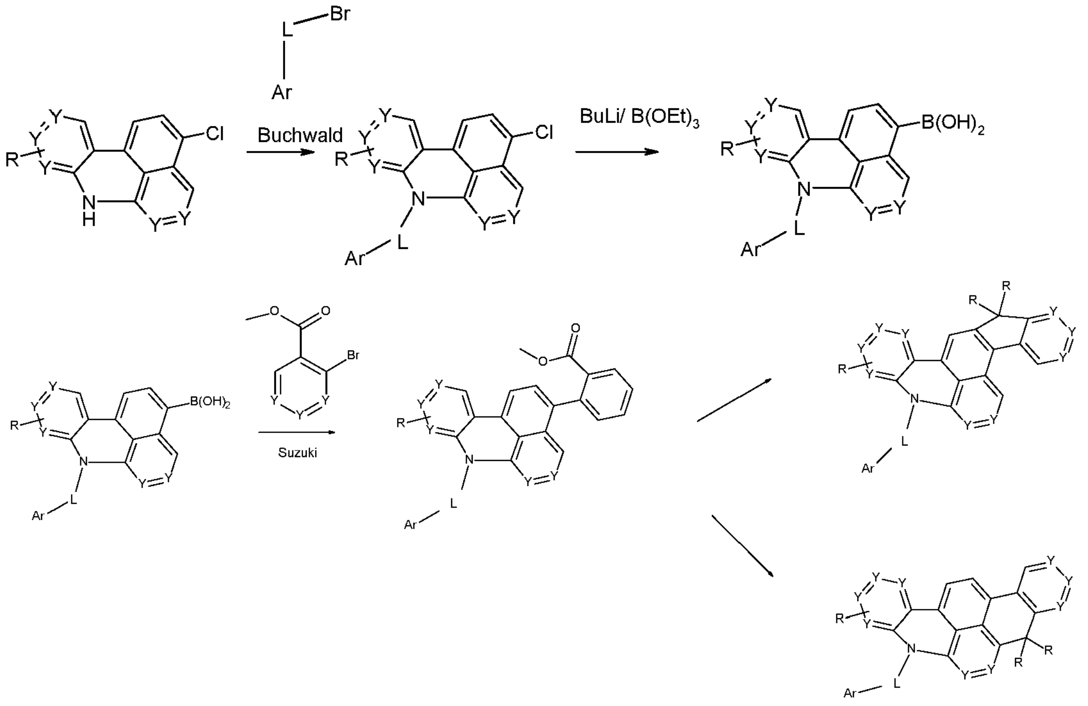


Die Bedeutung der in den Schemata 1 bis 4 verwendeten Symbole entspricht im Wesentlichen denen, die für Formel (I), (IIa), (IIb). (IIc) oder (IId) definiert wurde, wobei aus Gründen der Übersichtlichkeit auf eine Nummerierung verzichtet wurde. Der Index n ist 0 oder 1 und deutet an, dass die Gruppe Ar, die im Wesentlichen der zuvor definierten Gruppe L entspricht, optional ist, so dass gegebenenfalls eine direkte Bindung zwischen den entsprechenden Resten vorhanden ist.

Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln. Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (I) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formel (I) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (I) oder erfindungsgemäße Verbindungen, wobei ein oder mehrere Bindungen der erfindungsgemäßen Verbindungen oder der Strukturen der Formel (I) zum Polymer, Oligomer oder Dendrimer vorhanden sind. Je nach Verknüpfung der Strukturen der Formel (I) bzw. der Verbindungen bilden diese

daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen der allgemeinen Formel (I) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, HostMaterialien, Elektronentransportmaterialien, Elektroneninjektionsmaterialien, Lochleitermaterialien, Lochinjektionsmaterialien, Elektronenblockiermaterialien, Lochblockiermaterialien, Wide-Bandgap-Materialien und n-Dotanden.

Die vorliegenden Erfindung betrifft daher auch eine Zusammensetzung enthaltend wenigstens eine Verbindung umfassend Strukuren gemäß Formel (I) bzw. die zuvor und nachfolgend ausgeführten bevorzugten Ausführungsformen sowie wenigstens ein weiteres Matrixmaterial. Gemäß einem besonderen Aspekt der vorliegenden Erfindung weist das weitere Matrixmaterial lochtransportierende Eigenschaften auf.

Ein weiterer Gegenstand der vorliegenden stellt eine Zusammensetzung dar, enthaltend wenigstens eine Verbindung, umfassend mindestens eine Struktur gemäß Formel (I) bzw. die zuvor und nachfolgend ausgeführten bevorzugten Ausführungsformen sowie wenigstens ein Wide-Bandgap-Material, wobei unter Wide-Bandgap-Material ein Material im Sinne der Offenbarung von US 7,294,849 verstanden wird. Diese Systeme zeigen besondere vorteilhafte

Leistungsdaten in elektrolumineszierenden Vorrichtungen.

Vorzugsweise kann die zusätzliche Verbindung eine Bandlücke (bandgap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt. Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung umfassend Strukuren gemäß Formel (I) bzw. die zuvor und nachfolgend ausgeführten bevorzugten Ausführungsformen sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angebenen bevorzugten phosphoreszierenden Dotanden.

Vom Begriff phosphoreszierende Dotanden sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO

2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und den noch nicht offen gelegten Anmeldungen EP16179378.1 und EP16186313.9 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

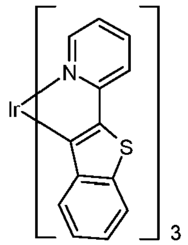
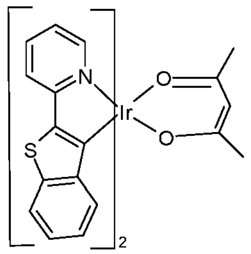
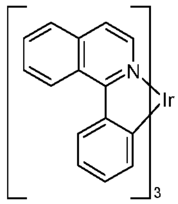

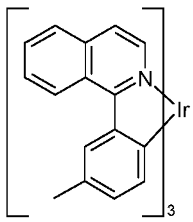
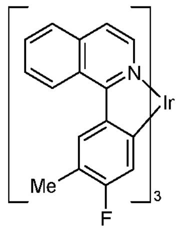
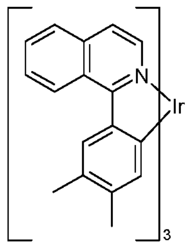

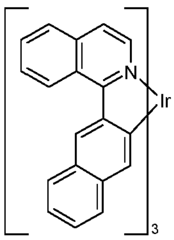
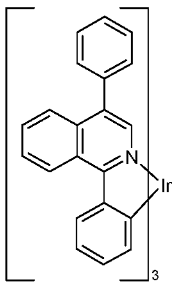
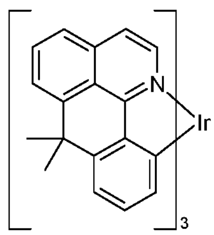

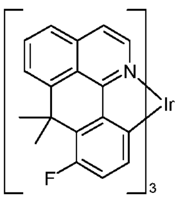
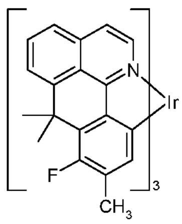
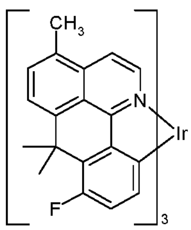

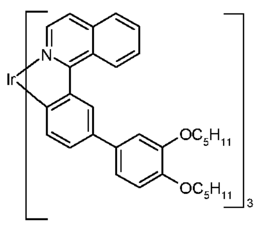
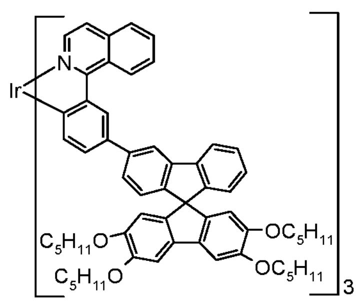
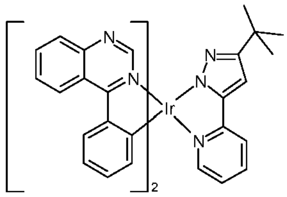

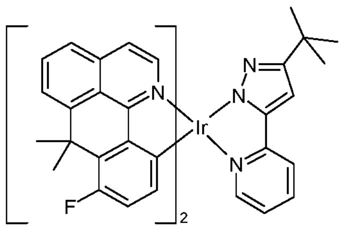
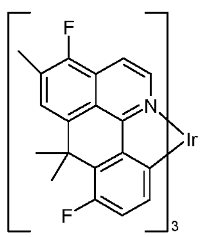
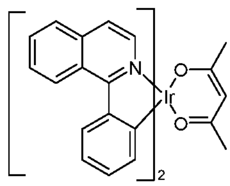

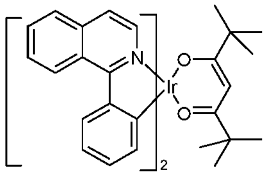
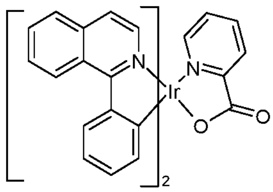
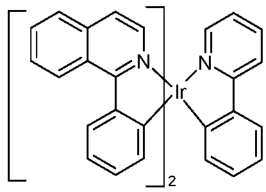

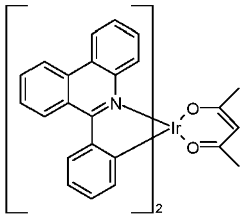
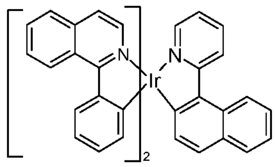
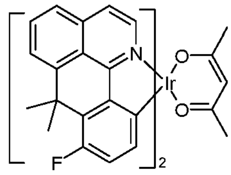

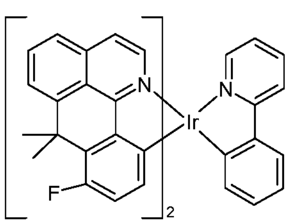
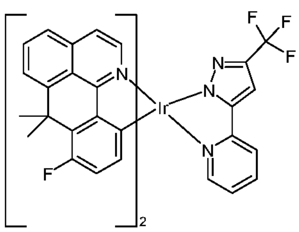
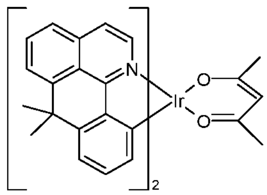

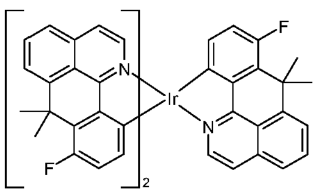
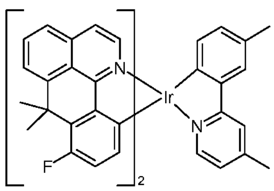
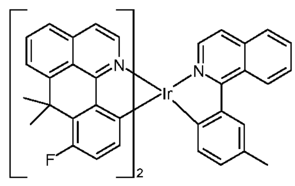

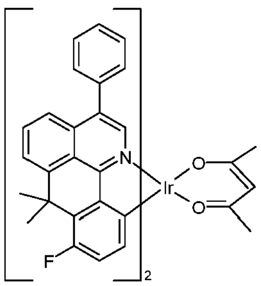
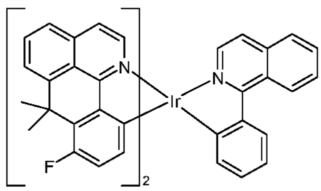
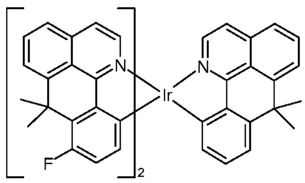

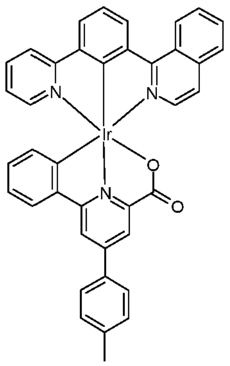

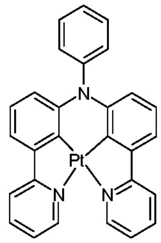

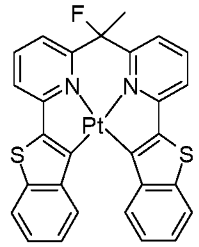

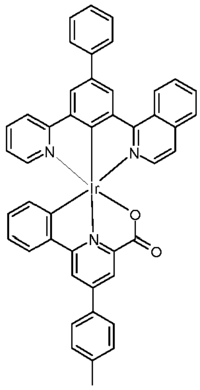

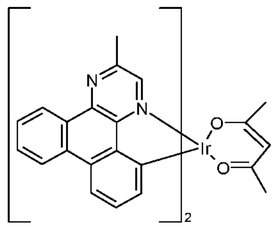

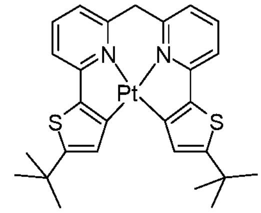

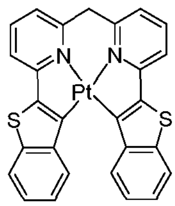

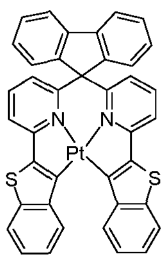

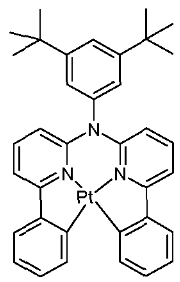

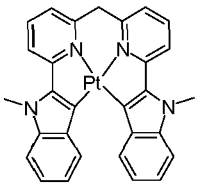

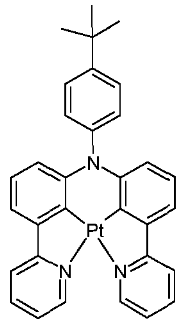

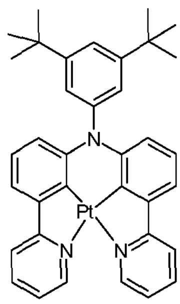

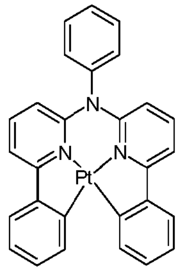

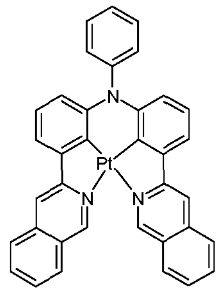

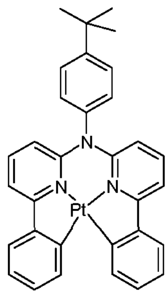

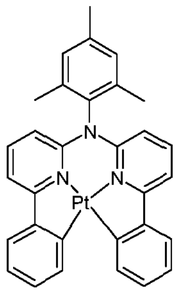

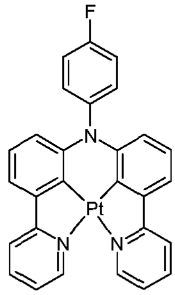

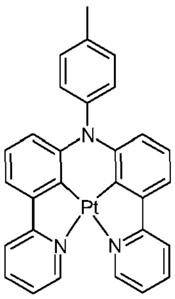

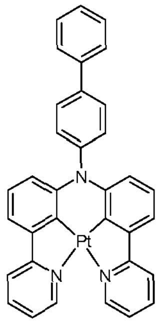

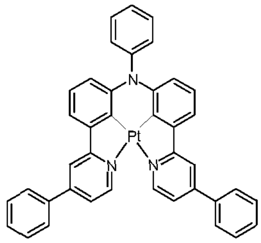

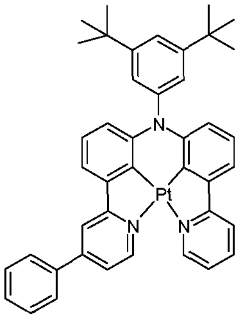

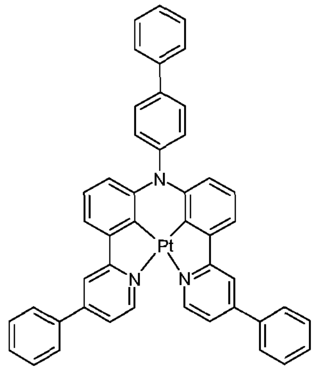

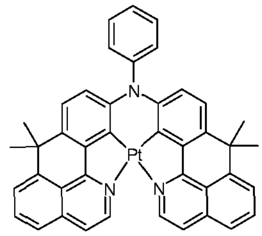

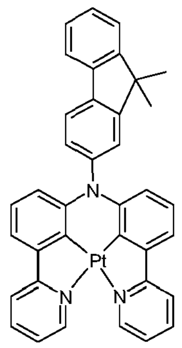
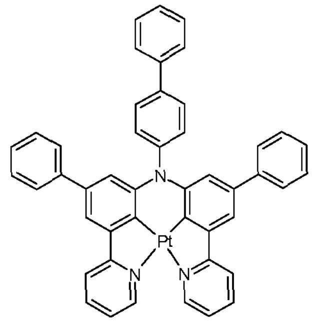
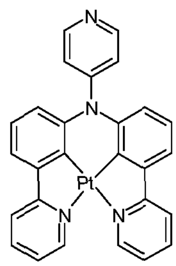

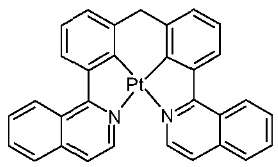
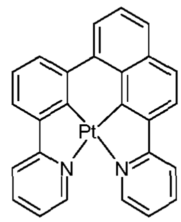
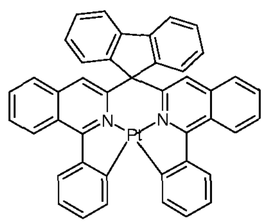

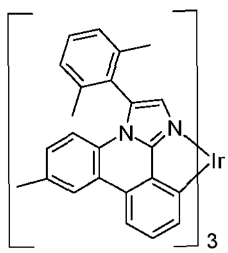
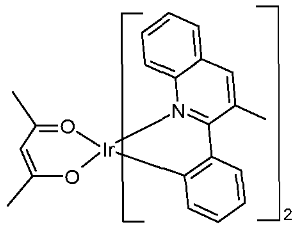
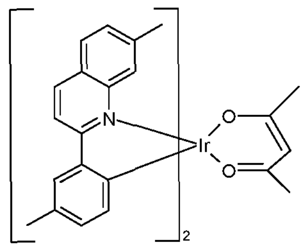

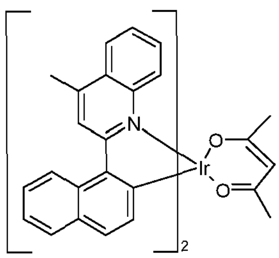
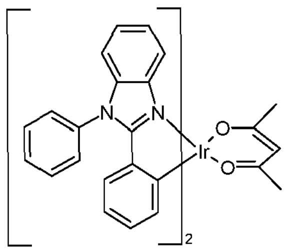
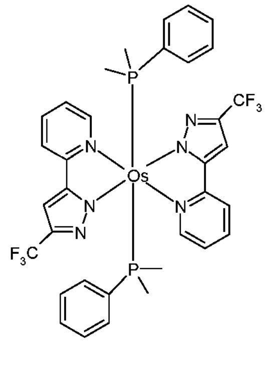

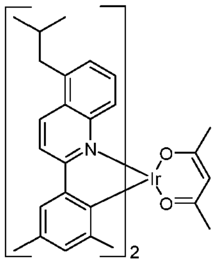
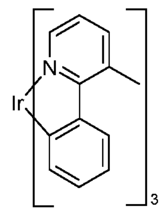
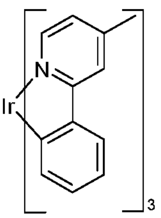

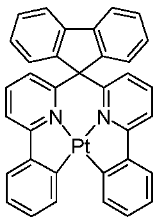

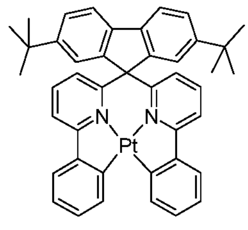

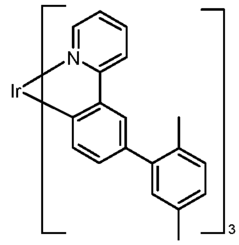

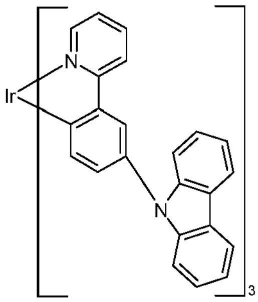

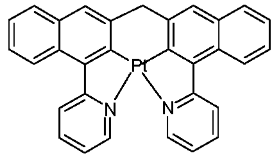

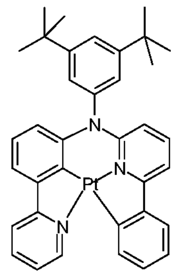

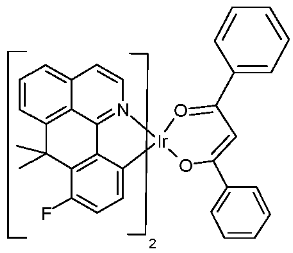

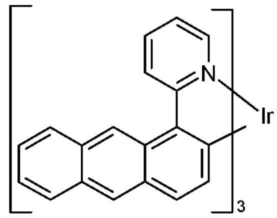

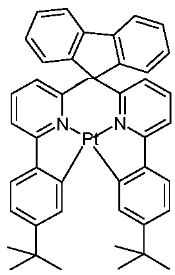

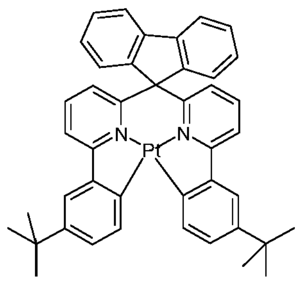

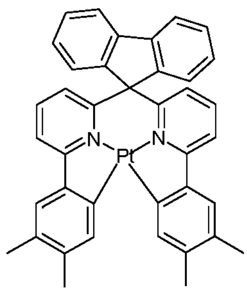

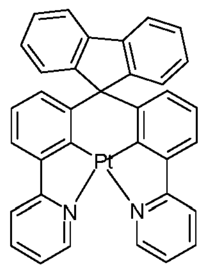

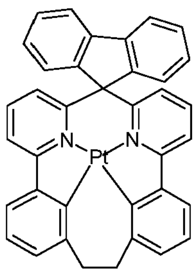

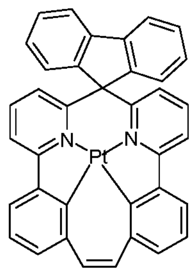

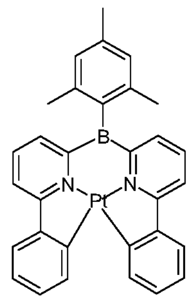

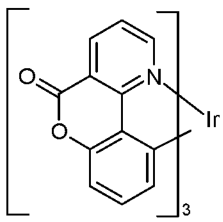
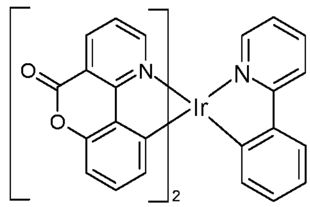
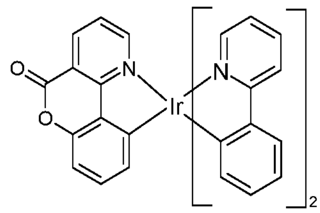

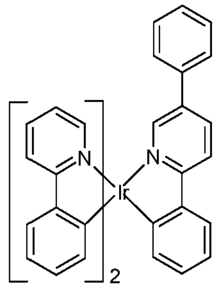
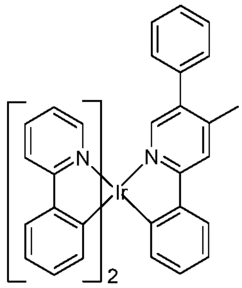
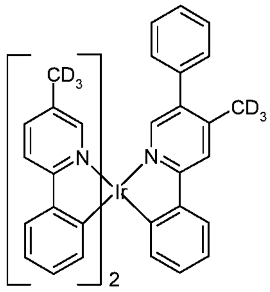

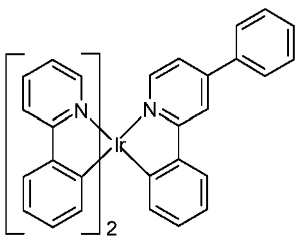
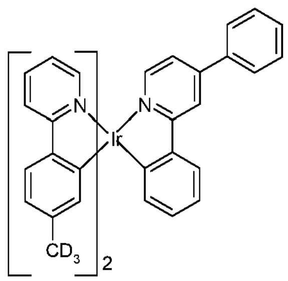
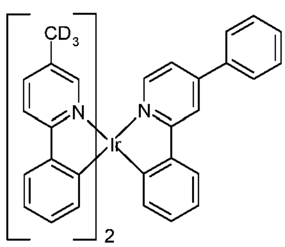

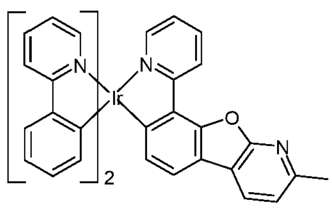
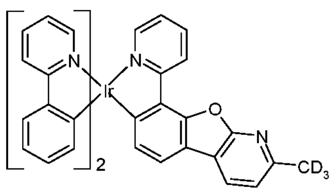
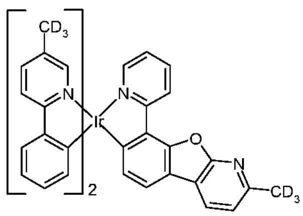

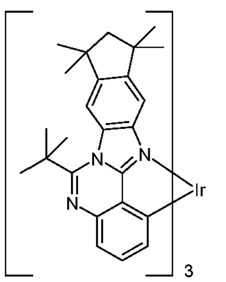
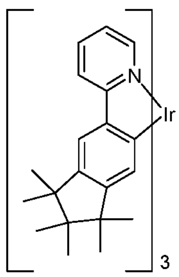
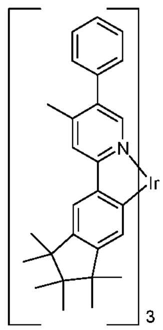

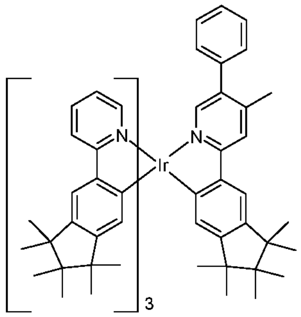
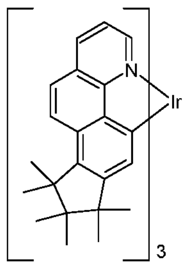
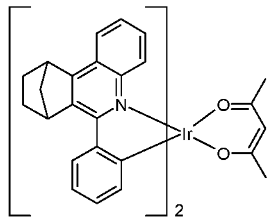

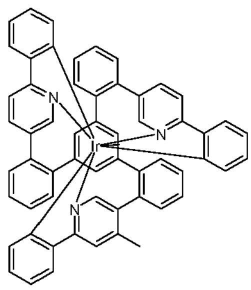
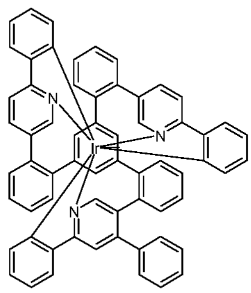
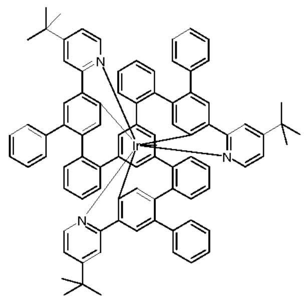

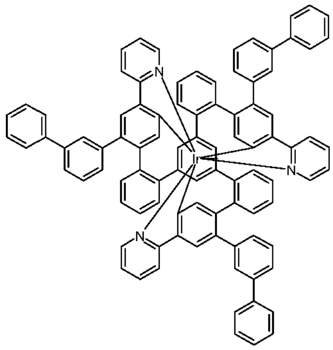
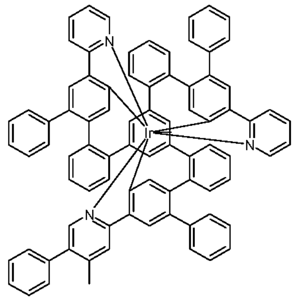
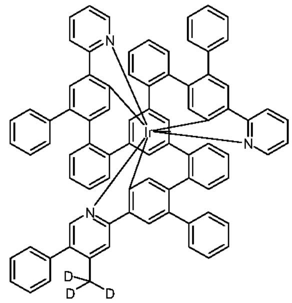

Die oben beschriebenen Verbindung, umfassend Strukturen der Formel (I) bzw. die oben aufgeführten bevorzugten Ausführungsformen, können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine Verbindung, umfassend Strukturen der Formel (I), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices", bevorzugt organischen Elektrolumineszenzvorrichtungen (OLEDs, PLEDs), insbesondere phosphoreszierenden OLEDs,enthaltend in mindestens einer Schicht mindestens eine Verbindung, umfassend Strukturen der Formel (I). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoOs oder WOs oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen bzw. Systeme, welche mehr als drei emittierende Schichten aufweisen. Weiterhin bevorzugt sind Tandem-OLEDs. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung umfassend Strukturen gemäß Formel (I) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung, die fluoreszieren oder phosphoreszieren kann oder die TADF (thermally activated delayed fluorescence) zeigt.

Geeignete Matrixmaterialien, welche in Kombination mit den Verbindungen gemäß Formel (I) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011/137951 oder WO 2013/064206, 4-SpirocarbazolDerivate, z. B. gemäß WO 2014/094963 oder WO 2015/192939, oder Dibenzofuranderivate, z. B. gemäß WO 2015/169412, WO 2016/015810, WO 2016/023608 oder den noch nicht offen gelegten Anmeldungen EP16158460.2 oder EP16159829.7. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame, Carbazolderivate und Biscarbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1),

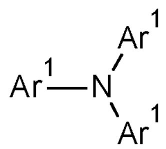

Formel (TA-1)

wobei $Ar^1$ gleich oder verschieden bei jedem Auftreten ein aromatisches oder heteroaromatisches Ringsystem mit 6 bis 40C-Atomen, das jeweils mit einem oder mehreren Resten $R^2$ substituiert sein kann, darstellt, wobei optional zwei oder mehr benachbarte Substituenten $R^2$ ein mono- oder polycyclisches, aliphatisches, heteroaliphatisches, aromatisches

oder heteroaromatisches Ringsystem, vorzugsweise ein mono- oder polycyclisches, aliphatisches Ringsystem bilden können, das mit einem oder mehreren Resten $R^3$ substituiert sein kann, wobei das Symbol $R^2$ die zuvor, insbesondere für Formel (I) genannte Bedeutung aufweist. Vorzugsweise stellt $Ar^1$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^1$ sind ausgewählt aus der Gruppe bestehend aus Phenyl, ortho-, meta- oder para-Biphenyl, Terphenyl, insbesondere verzweigtem Terphenyl, Quaterphenyl, insbesondere verzweigtem Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Bevorzugt sind die Gruppen $Ar^1$ gleich oder verschieden bei jedem Auftreten ausgewählt aus den oben genannten Gruppen $R^1$-1 bis $R^1$-80, besonders bevorzugt $R^1$-1 bis $R^1$-51.

In einer bevorzugten Ausführungsform der Verbindungen der Formel (TA-1) ist mindestens eine Gruppe $Ar^1$ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe $Ar^1$ ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe $Ar^1$ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe $Ar^1$ ausgewählt aus einer Fluoren- oder Spirobifluorengruppe, insbesondere einer 4-Fluoren- bzw. 4-Spirobifluorengruppe, und eine Gruppe $Ar^1$ ist ausgewählt aus einer Biphenylgruppe, insbesondere einer para-Biphenylgruppe, oder einer Fluorengruppe, insbesondere einer 2-Fluorengruppe, und die dritte Gruppe $Ar^1$ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2),

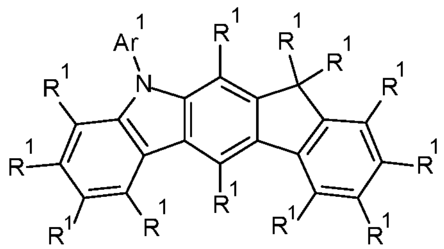

Formel (TA-2)

wobei $Ar^1$ und $R^1$ die oben insbesondere für Formeln (I) und/oder (TA-1) aufgeführten Bedeutungen aufweisen. Dabei sind bevorzugte Ausführungsformen der Gruppe $Ar^1$ die oben aufgeführten Strukturen $R^1$-1 bis $R^1$-80, besonders bevorzugt $R^1$-1 bis $R^1$-51.

Eine bevorzugte Ausführungsform der Verbindungen der Formel (TA-2) sind die Verbindungen der folgenden Formel (TA-2a),

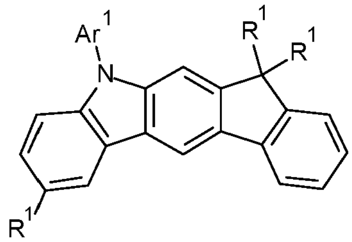

Formel (TA-2a)

wobei $Ar^1$ und $R^1$ die oben, insbesondere für Formeln (I) und/oder (TA-1) aufgeführten Bedeutungen aufweisen. Dabei stehen die beiden Gruppen $R^1$, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen $R^1$, die an das Indenokohlenstoffatom gebunden sind, für Methylgruppen. Weiterhin bevorzugt steht der Substituent $R^1$, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3),

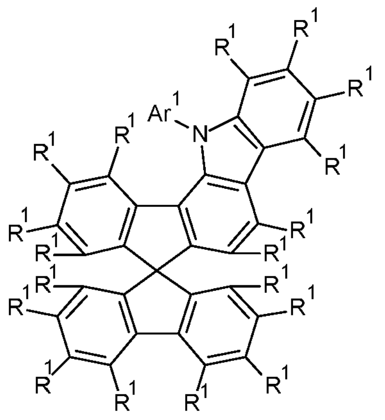

Formel (TA-3)

wobei $Ar^1$ und $R^1$ die oben, insbesondere für Formeln (I), (II) und/oder (Q-1) aufgeführten Bedeutungen aufweisen. Dabei sind bevorzugte Ausführungsformen der Gruppe $Ar^1$ die oben aufgeführten Strukturen $R^1$-1 bis $R^1$-80, besonders bevorzugt $R^1$-1 bis $R^1$-51.

Eine bevorzugte Ausführungsform der Verbindungen der Formel (TA-3) sind die Verbindungen der folgenden Formel (TA-3a),

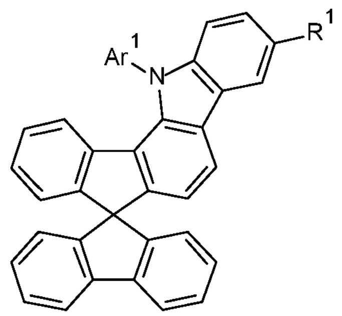

Formel (TA-3a)

wobei $Ar^1$ und $R^1$ die oben, insbesondere für Formeln (I), (II) und/oder (Q-1) aufgeführten Bedeutungen aufweisen. Dabei sind bevorzugte Ausführungsformen der Gruppe $Ar^1$ die oben aufgeführten Strukturen $R^1$-1 bis $R^1$-80, besonders bevorzugt $R^1$-1 bis $R^1$-51.

Bevorzugte Biscarbazolderivate, die als Co-Host-Materialien zusammen mit den erfindungsgemäßen Verbindungen eingesetzt werden, sind ausgewählt aus den Verbindungen der folgenden Formel (TA-4),

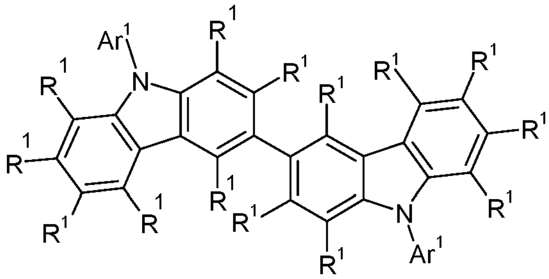

Formel (TA-4)

wobei $Ar^1$ und $R^1$ die oben, insbesondere für Formeln (I) und/oder (TA-1), aufgeführten Bedeutungen aufweisen. Dabei sind bevorzugte Ausführungsformen der Gruppe $Ar^1$ die oben aufgeführten Strukturen $R^1$-1 bis $R^1$-80, besonders bevorzugt $R^1$-1 bis $R^1$-51.

Eine bevorzugte Ausführungsform der Verbindungen der Formel (TA-4) sind die Verbindungen der folgenden Formel (TA-4a),

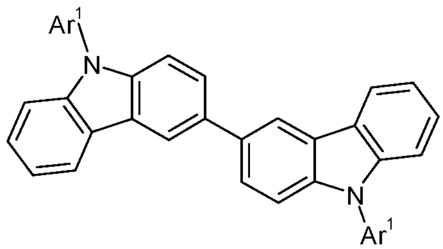

Formel (TA-4)

wobei $Ar^1$ die oben, insbesondere für Formel (TA-1), aufgeführten Bedeutungen aufweist. Dabei sind bevorzugte Ausführungsformen der Gruppe $Ar^1$ die oben aufgeführten Strukturen $R^1$-1 bis $R^1$-80, besonders bevorzugt $R^1$-1 bis $R^1$-51.

Beispiele für geeignete Biscarbazolderivate sind die in der folgenden Tabelle aufgeführten Materialien.

| | | |
|---|---|---|
| 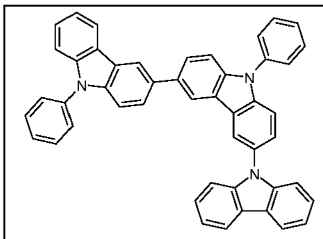 | 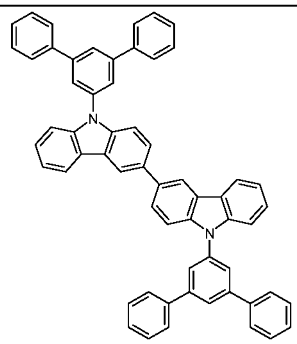 | 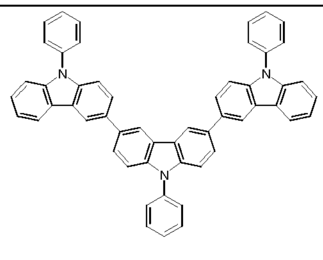 |
| 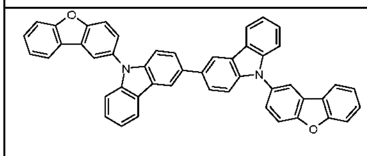 | 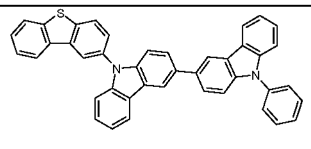 | 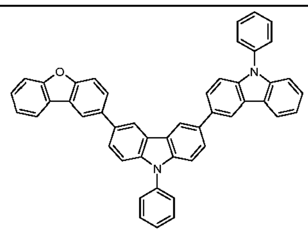 |

| | | |
|---|---|---|
| 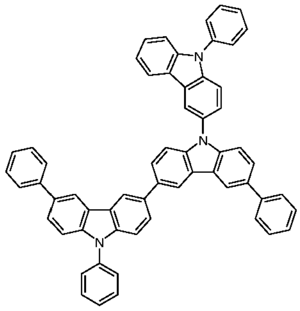 | 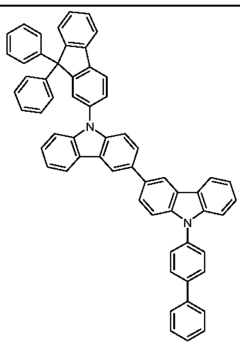 | 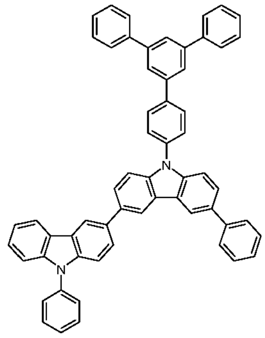 |
| 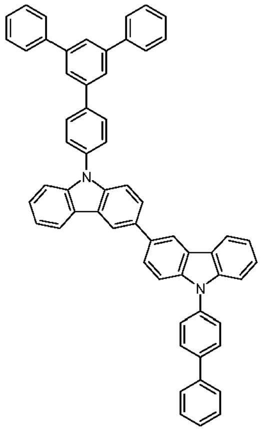 | 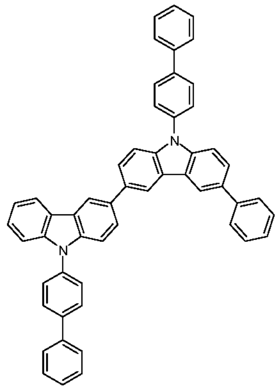 | 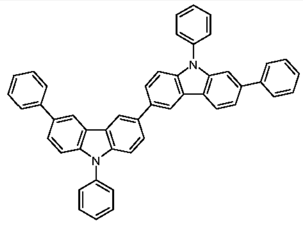 |
| 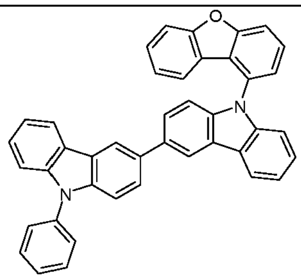 | 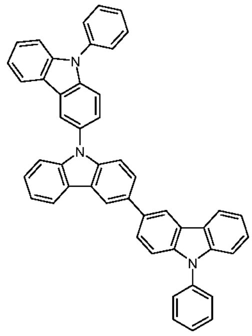 | 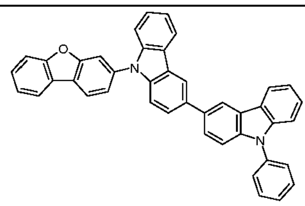 |
| 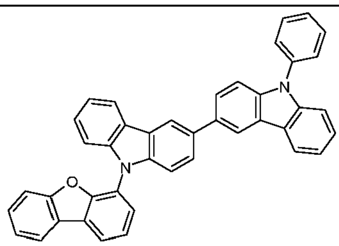 | 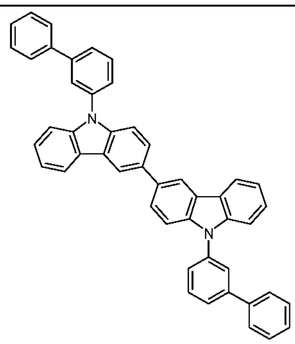 | 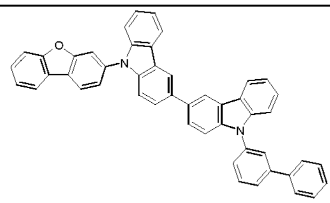 |

| | | |
|---|---|---|
| 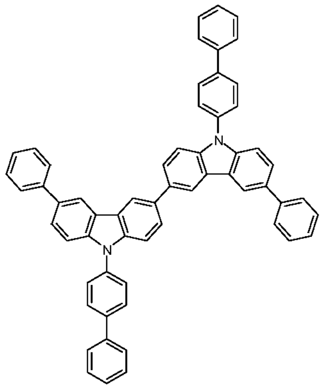 | 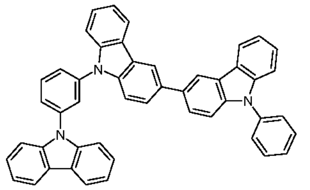 | 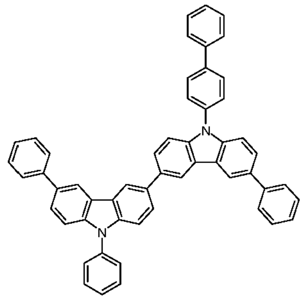 |
| 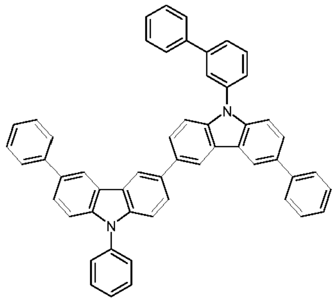 | 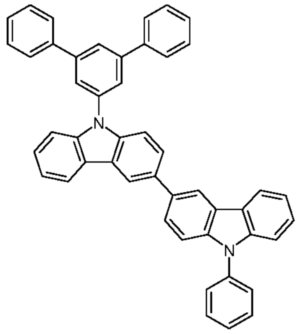 | 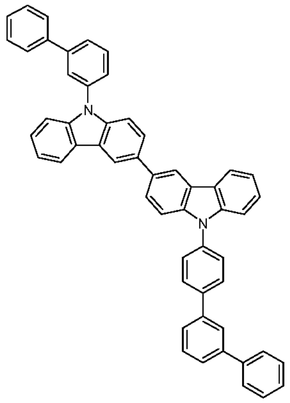 |
| 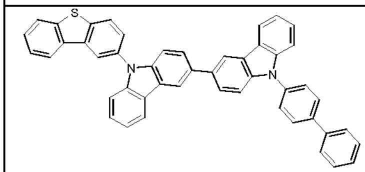 | 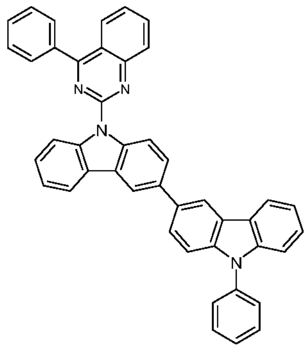 | 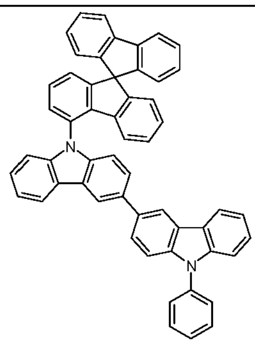 |
| 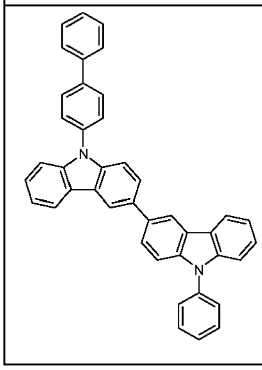 | 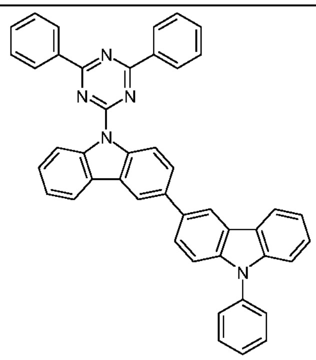 | 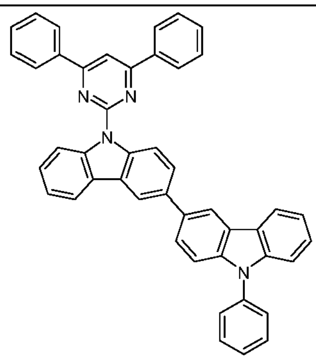 |

Bevorzugte Lactame, die als Co-Host-Materialien zusammen mit den erfindungsgemäßen Verbindungen eingesetzt werden, sind ausgewählt aus den Verbindungen der folgenden Formel (LAC-1),

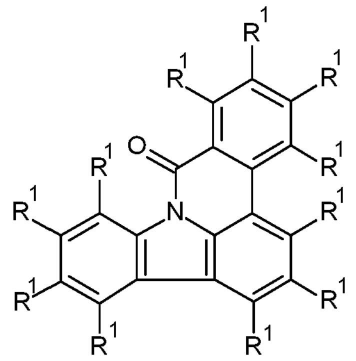

Formel (LAC-1)

wobei $R^1$ die oben, insbesondere für Formeln (I) aufgeführte Bedeutung aufweist.

Eine bevorzugte Ausführungsform der Verbindungen der Formel (LAC-1) sind die Verbindungen der folgenden Formel (LAC-1a),

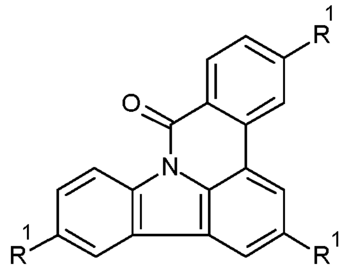

Formel (LAC-1a)

wobei $R^1$ die oben, insbesondere für Formel (I) genannte Bedeutung aufweist. Dabei steht $R^1$ bevorzugt gleich oder verschieden bei jedem Auftreten für H oder ein aromatisches oder heteroaromatisches Ringsystem mit 5 bis 40 aromatischen Ringatomen, das mit einem oder mehreren Resten $R^2$ substituiert sein kann, wobei $R^2$ die zuvor, insbesondere für Formel (I) genannte Bedeutung aufweisen kann. Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtem Terphenyl, Quaterphenyl, insbesondere verzweigtem Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei sind geeignete Strukturen $R^1$ die gleichen Strukturen, wie sie zuvor für R-1 bis R-79 abgebildet sind, besonders bevorzugt $R^1$-1 bis $R^1$-51.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der TriplettEmitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß Formel (I) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß Formel (I) bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, aber auch Dotanden,

die TADF (thermally activated delayed fluorescence) zeigen, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung umfassend Strukuren gemäß Formel (I) bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOx, Al/PtOx) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoOs oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronische Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronische Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine elektronische Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine erfindungsgemäße Verbindung umfassend Strukturen gemäß Formel (I) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend efindungsgemäße Verbindungen umfassend Strukturen gemäß Formel (I) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronischen Vorrichtungen, insbesondere organische Elektrolumineszenzvorrichtungen enthaltend Verbindungen, Oligomere, Polymere oder Dendrimere mit Strukturen gemäß Formel (I) bzw. die zuvor und nachfolgend ausgeführten bevorzugten Ausführungsformen, insbesondere als elektronenleitende Materialien, weisen eine sehr gute Lebensdauer auf.

2. Elektronischen Vorrichtungen, insbesondere organische Elektrolumineszenzvorrichtungen enthaltend Verbindungen, Oligomere, Polymere oder Dendrimere mit Strukturen gemäß Formel (I) bzw. die zuvor und nachfolgend ausgeführten bevorzugten Ausführungsformen als elektronenleitende Materialien, Elektroneninjektionsmaterialien und/oder Hostmaterialien weisen eine hervorragende Effizienz auf. Insbesondere ist die Effizienz deutlich höher gegenüber analogen Verbindungen, die keine Struktureinheit gemäß Formel (I) enthalten. Hierbei bewirken die erfindungsgemäßen Verbindungen, Oligomere, Polymere oder Dendrimere mit Strukturen gemäß Formel (I) bzw. die zuvor und nachfolgend ausgeführten bevorzugten Ausführungsformen eine geringe Betriebsspannung bei Verwendung in elektronischen Vorrichtungen. Hierbei bewirken diese Verbindungen insbesondere einen geringen Roll-off, d. h. einen geringen Abfall der Leistungseffizienz der Vorrichtung bei hohen Leuchtdichten.

3. Die erfindungsgemäßen Verbindungen, Oligomere, Polymere oder Dendrimere mit Strukturen gemäß Formel (I) bzw. die zuvor und nachfolgend ausgeführten bevorzugten Ausführungsformen zeigen eine sehr hohe Stabilität und führen zu Verbindungen mit einer sehr hohen Lebensdauer.

4. Mit Verbindungen, Oligomere, Polymere oder Dendrimere mit Strukturen gemäß Formel (I) bzw. die zuvor und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

5. Die Verwendung von Verbindungen, Oligomere, Polymere oder Dendrimere mit Strukturen gemäß Formel (I) bzw. die zuvor und nachfolgend ausgeführten bevorzugten Ausführungsformen in Schichten elektronischer Vorrichtungen, insbesondere organischer Elektrolumineszenzvorrichtungen führt zu einer hohen Mobilität der Elektronleiterstrukturen.

6. Verbindungen, Oligomere, Polymere oder Dendrimere mit Strukturen gemäß Formel (I) bzw. die zuvor und nachfolgend ausgeführten bevozugten Ausführungsformen zeichnen sich durch eine ausgezeichnete thermische Stabilität aus, wobei Verbindungen mit einer Molmasse von weniger als ca. 1200 g/mol gut sublimierbar sind.

7. Verbindungen, Oligomere, Polymere oder Dendrimere mit Strukturen gemäß Formel (I) bzw. die zuvor und nachfolgend ausgeführten bevorzugten Ausführungsformen weisen eine ausgezeichnete Glasfilmbildung auf.

8. Verbindungen, Oligomere, Polymere oder Dendrimere mit Strukturen gemäß Formel (I) bzw. die zuvor und nachfolgend ausgeführten bevorzugten Ausführungsformen bilden aus Lösungen sehr gute Filme.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als als Hostmaterial, Lochblockiermaterial, Elektroneninjektionsmaterial und/oder Elektronentransportmaterial, vorzugsweise als Hostmaterial und/oder Elektronentransportmaterial.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen. Besonders bevorzugt ist elektronische Vorrichtung ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices", bevorzugt organischen Elektrolumineszenzvorrichtungen (OLEDs, PLEDs), insbesondere phosphoreszierenden OLEDs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochblockier- oder Elektronentransportschicht einzusetzen. Diese können bevorzugt auch mit einer oder mehreren weiteren elektronentransportierenden Gruppen substituiert sein, beispielsweise Benzimidazolgruppen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (I) bzw. gemäß den bevorzugten Ausführungsformen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal,

sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße elektronische Vorrichtungen herstellen und somit die Erfindung im gesamten beanspruchten Bereich ausführen.

## Beispiele

Die nachfolgenden Synthesen werden, sofern nicht anders angegeben, unter einer Schutzgasatmosphäre in getrockneten Lösungsmitteln durchgeführt. Die Edukte können von ALDRICH bezogen werden. Die Nummern bei den literaturbekannten Edukten, die teilweise in eckigen Klammern angegeben sind, sind die entsprechenden CAS-Nummern.

## Synthesebeispiele

### a) 5-Bromo-spiro[7H-benzo[c]fluoren-7,9'-[9H]fluoren

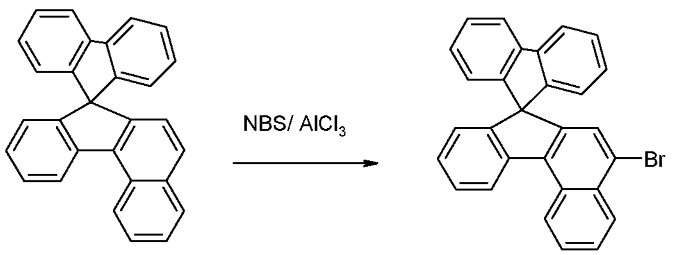


[356801-51-9 ]

[1175203-78-7 ]

19 g (51,8 mmol) Spiro[7H-benzo[c]fluoren-7,9'-[9H]fluoren werden bei Raumtemperatur unter Lichtausschluss in 380 mL Dichlormethan vorgelegt, 10,1g (57 mmol) NBS und 0,8 g (5 mmol) $AlCl_3$ innerhalb 40 min portionsweise zugegeben und anschließend unter Rückfluss gerührt. Die Reaktion wird per HPLC-Analyse von Reaktionsaliquots kontrolliert. Nach vollständigem Umsatz wird die Reaktion durch Zugabe von 20 mL EtOH gestoppt, abgesaugt, mehrfach mit EtOH gewaschen und anschließend zweimal aus Toluol umkristallisiert. Ausbeute: 22 g (51 mmol), 98% der Theorie, der laut HPLC-Analyse einen Gehalt von >99,8% aufweist.

Analog können folgende Verbindungen herestellt werden:

| | Edukt 1 | Produkt | Ausbeute |
|---|---|---|---|
| 1a | 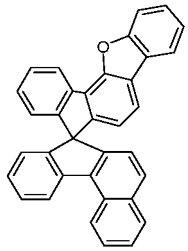 [1623076-38-9 ] | 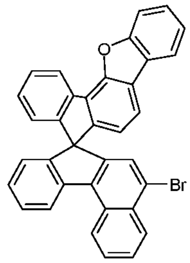 | 77% |
| 2a | 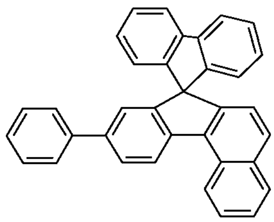 [1350966-06-1 ] | 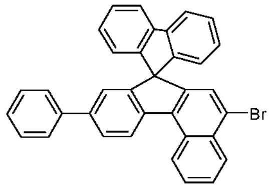 | 70% |
| 3a | 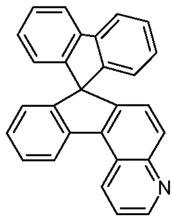 [1262435-74-4 ] | 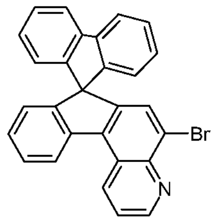 [1262435-74-4 ] | 34% |
| 4a | 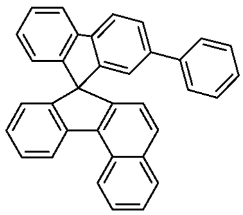 4c | 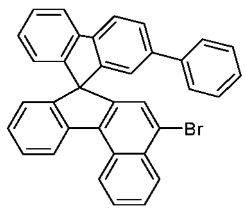 | 76% |
| 5a | 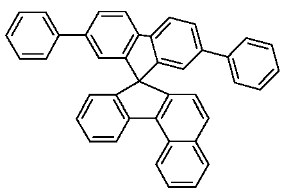 5c | 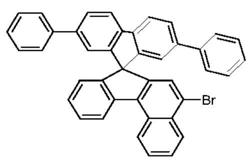 | 79% |

(fortgesetzt)

| | Edukt 1 | Produkt | Ausbeute |
|---|---|---|---|
| 6a | 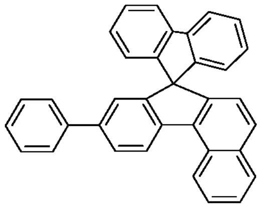 3c | 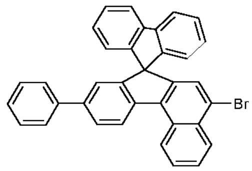 | 78% |

**b) Spiro[7H-benzo[c]fluoren-7,9'-[9H]fluoren-5-boronsäure**

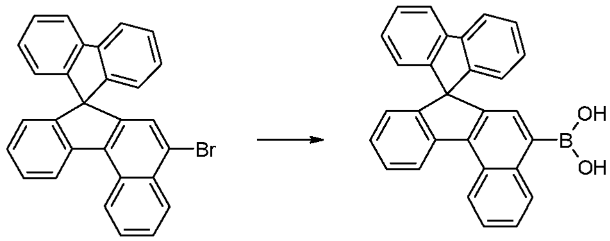

32,3 g (72,8 mmol) 5-Bromo-spiro[7H-benzo[c]fluoren-7,9'-[9H]fluoren werden in 150 mL trockenem THF gelöst und auf -78 °C gekühlt. Bei dieser Temperatur wird mit 30 mL (764 mmol / 2,5 M in Hexan) n-BuLi innerhalb von ca. 5 min. versetzt und anschließend für 2,5 h bei -78 °C nachgerührt. Bei dieser Temperatur wird mit 15,1 g (145 mmol) Borsäure-trimethylester möglichst zügig versetzt und die Reaktion langsam auf RT kommen gelassen (ca. 18 h). Die Reaktionslösung wird mit Wasser gewaschen und der ausgefallene Feststoff und die organische Phase mit Toluol azeotrop getrocknet. Das Rohprodukt wird aus Toluol/Methylenchlorid bei ca. 40 °C ausgerührt und abgesaugt. Ausbeute: 28 g (68 mmol), 95 % der Theorie, der laut HPLC-Analyse einen Gehalt von >97.3 % aufweist.

Analog können folgende Verbindungen hergestellt werden:

| | Edukt 1 | Produkt | Ausbeute |
|---|---|---|---|
| 1b | 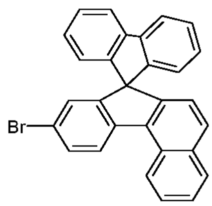 [1099593-10-8 ] | 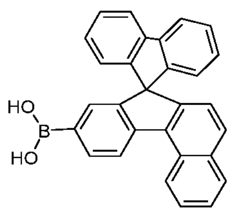 | 79% |
| 2b | 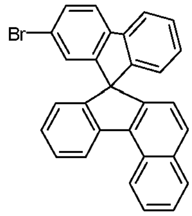 [1099593-10-8 ] | 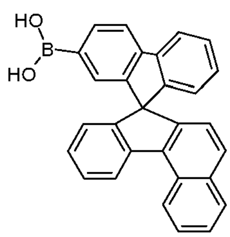 | 83% |

(fortgesetzt)

| | Edukt 1 | Produkt | Ausbeute |
|---|---|---|---|
| 3b | 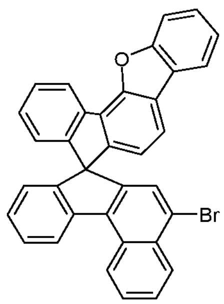 | 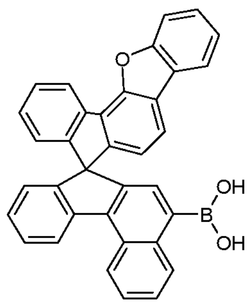 | 72% |
| 4b | 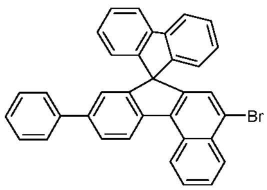 | 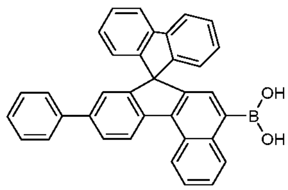 | 80% |
| 5b | 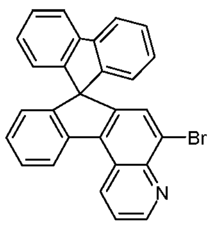 [1262435-74-4 ] | 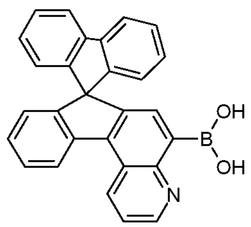 | 65% |
| 6b | 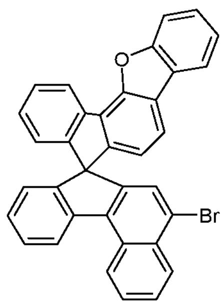 | 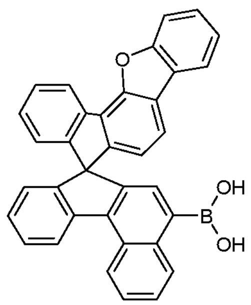 | 74% |
| 7b | 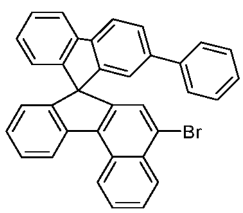 | 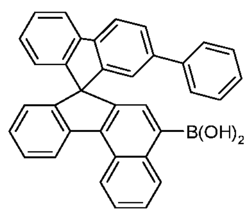 | 76% |
| 8b | 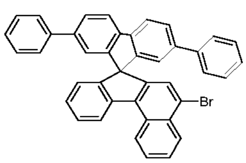 | 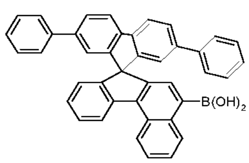 | 79% |

**c) 5-(2-Nitrophenyl)-spiro[7H-benzo[c]fluoren-7,9'-[9H]fluoren**

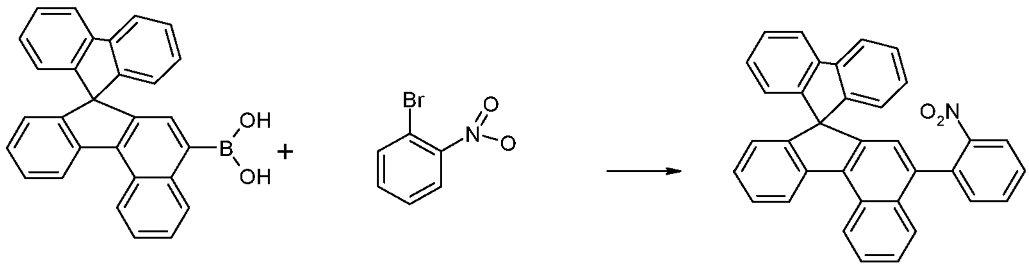

Eine gut gerührte, entgaste Suspension aus 46,7 g (114 mmol) Spiro[7H-benzo[c]fluoren-7,9'-[9H]fluoren-5-boronsäure, 13,4 g (47 mmol) 2,5-Dibromnitrobenzol und 30,3 g (143 mmol) Kaliumcarbonat in einem Gemisch aus 150 ml Wasser und 150 ml THF wird mit 0,5 g (0,4 mmol) $Pd(PPh_3)_4$ versetzt und 20 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und im Vakuum zur Trockene einrotiert. Der graue Rückstand wird aus Toluol/$CH_2Cl_2$ umkristallisiert. Die ausgefallenen Kristalle werden abgesaugt, mit etwas MeOH gewaschen und im Vakuum getrocknet. Ausbeute: 47 g, 86 % d. Th.; Reinheit: 98,3 % n. HPLC.

Analog können folgende Verbindungen hergestellt werden:

| | Edukt 1 | Edukt 2 | Produkt | Ausbeute |
|---|---|---|---|---|
| 1c | 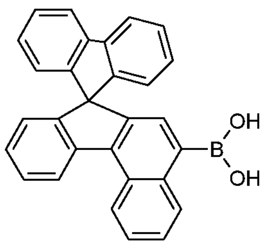 | 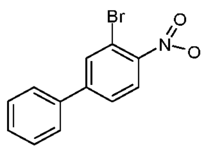 [27721-82-0 ] | 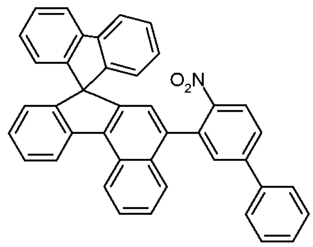 | 86% |
| 2c | 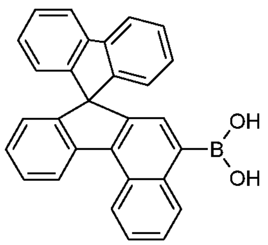 | 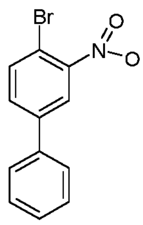 [27701-66-2 ] | 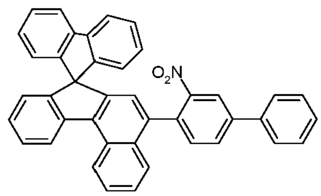 | 88% |
| 3c | 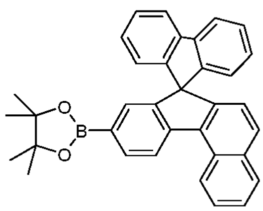 [1800333-51-0 ] | 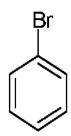 | 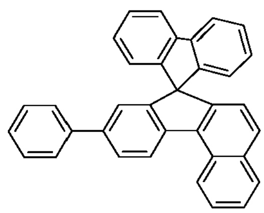 | 80% |
| 4c | 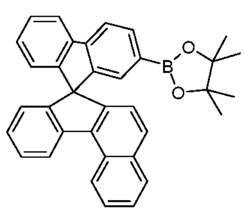 [1793062-59-5 ] | 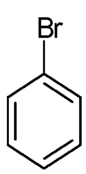 | 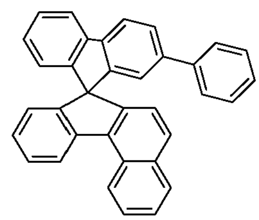 | 87% |

(fortgesetzt)

| | Edukt 1 | Edukt 2 | Produkt | Ausbeute |
|---|---|---|---|---|
| 5c | 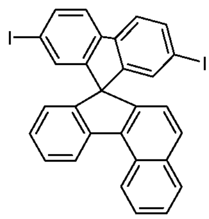 [1416083-52-7 ] | 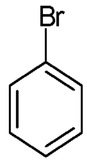 | 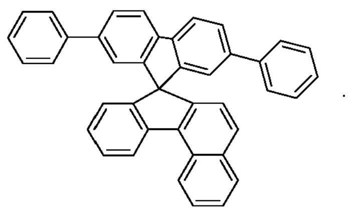 | 85% |
| 6c | 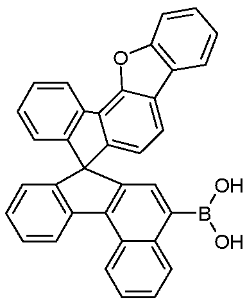 | 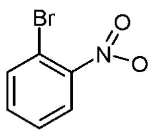 | 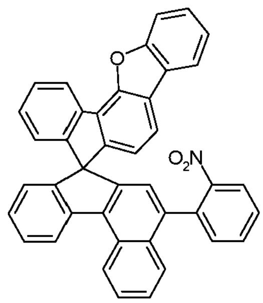 | 79% |
| 7c | 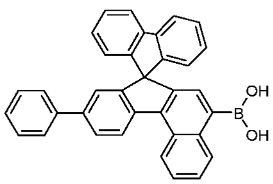 | 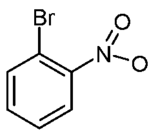 | 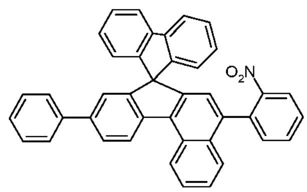 | 86% |
| 8c | 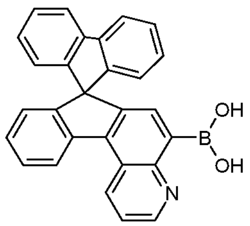 | 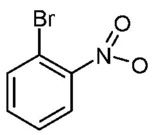 | 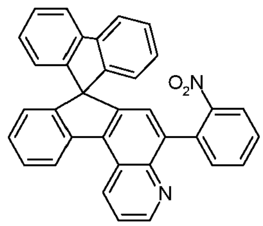 | 85% |
| 9c | 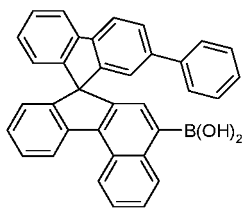 | 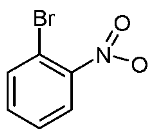 | 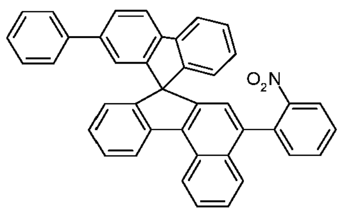 | 77% |
| 10c | 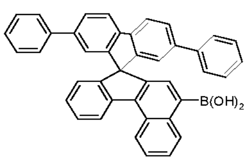 | 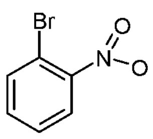 | 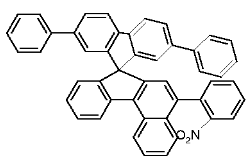 | 83% |

**d) Cyclisierung: Spiro[9H-fluoren-9,12'(11'H)-benzindeno[2,1-a] carbazol]**

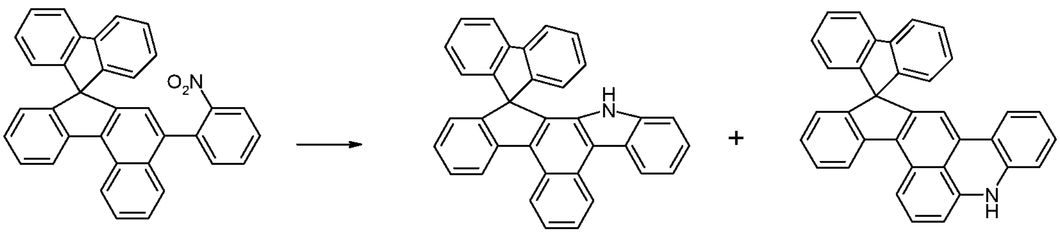

d1 d2

Eine Mischung aus 115 g (238 mmol) 5-(2-Nitrophenyl)-spiro[7H-benzo[c]-fluoren-7,9'-[9H]fluoren und 290,3 ml (1669 mmol) Triethylphosphit wird 12 h unter Rückfluss erhitzt. Anschließend wird das restliche Triethylphosphit abdestilliert (72-76 °C / 9 mm Hg). Der Rückstand wird mit Wasser/MeOH (1:1) versetzt, der Feststoff abfiltriert und aus Toluol umkristallisiert. Ausbeute: 98 g (215 mmol) der Mischung d1+d2, 92% d. Th.; Reinheit: 98,0 % n. HPLC. Nach Umkristallisation aus Essigsäureethylester/$CH_2Cl_2$ (2:1) erhält man 70% d1 und 17% d2.

Analog können folgende Verbindungen hergestellt werden:

| | Edukt 1 | Produkt 1 | Produkt 2 | Ausbeute |
|---|---|---|---|---|
| 1d | 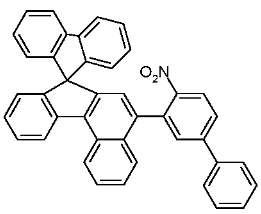 | 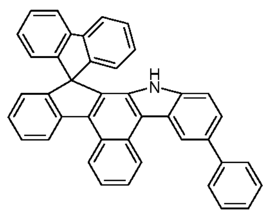 | 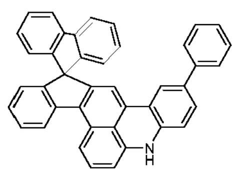 | 66%/ 18% |
| 2d | 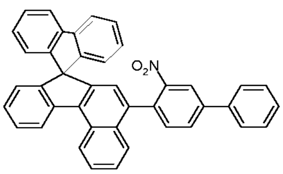 | 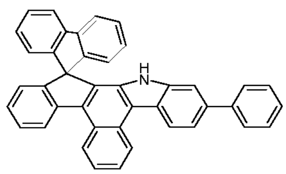 | 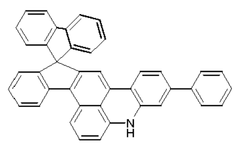 | 68%/ 14% |
| 3d | 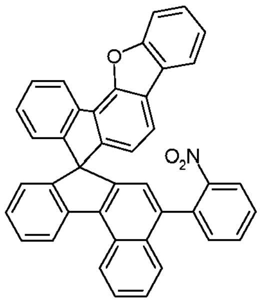 | 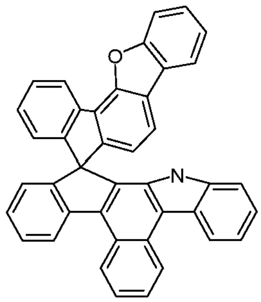 | 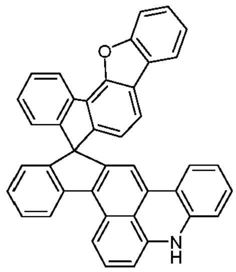 | 69%/ 15% |
| 4d | 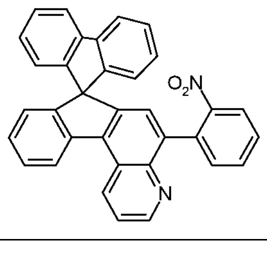 | 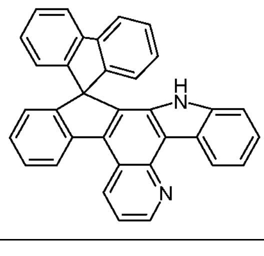 | | 78% |
| 5d | 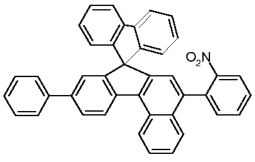 | 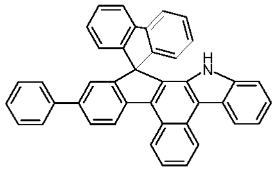 | 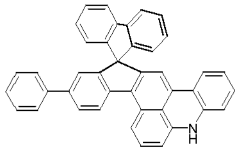 | 71%/ 14% |

(fortgesetzt)

| | Edukt 1 | Produkt 1 | Produkt 2 | Ausbeute |
|---|---|---|---|---|
| 6d | 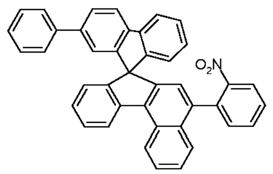 | 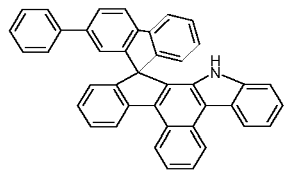 | 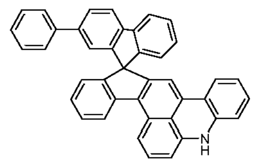 | 69%/ 13% |
| 7d | 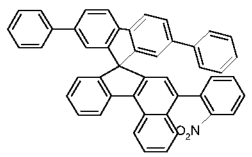 | 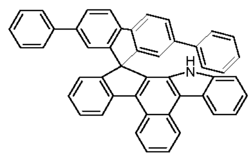 | 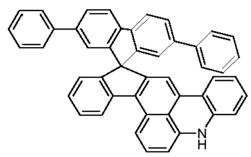 | 72%/ 16% |

**e) 13-(4,6-Diphenyl-[1,3,5]triazin-2-yl)-9**H**-fluoren-9,12'(11'**H**)-13-aza-benzo[c]indeno[2,1-a]spiro**

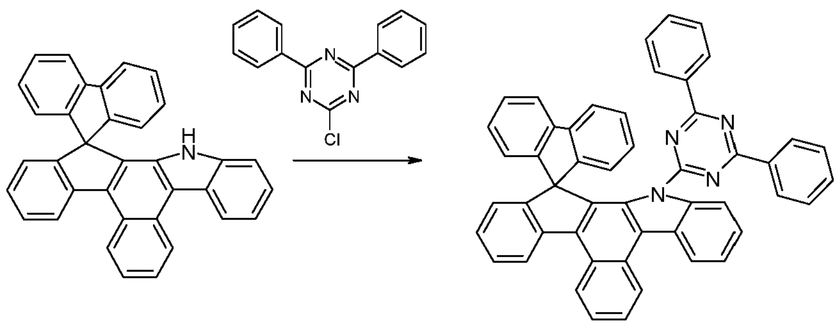

13,6 g (30 mmol) Spiro[9**H**-fluoren-9,12'(11'**H**)-benzindeno[2,1-**a**]carbazol] werden in 225 ml Dimethylformamid unter Schutzgasatmosphäre gelöst und mit 1,5 g (37,5 mmol) NaH (60%ig in Mineralöl) versetzt. Nach 1 h bei Raumtemperatur wird eine Lösung von 2-Chlor-4,6-diphenyl-[1,3,5]-triazin (8,5 g, 31,75 mmol) in 75 mL Dimethylformamid zugetropft. Das Reaktionsgemisch wird dann 12 h bei Raumtemperatur gerührt. Nach dieser Zeit wird das Reaktionsgemisch auf Eis gegossen und dreimal mit Dichlormethan extrahiert. Die vereinigten organischen Phasen werden über $Na_2SO_4$ getrocknet und eingeengt. Der Rückstand wird mit Toluol umkristallisiert und abschließend zweimal fraktioniert sublimiert (p ca. $10^{-6}$ mbar, T = 390 - 393 °C). Ausbeute: 16 g, 80 % d. Th.; Reinheit: 99.9 % n. HPLC.

Analog können folgende Verbindungen hergestellt werden:

| | Edukt 1 | Edukt 2 | Produkt | Ausbeute |
|---|---|---|---|---|
| 1e | 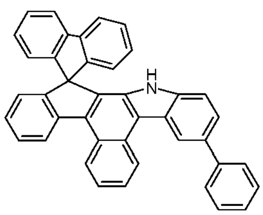 | 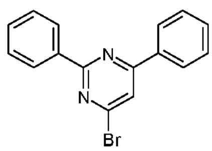 [40734-24-5] | 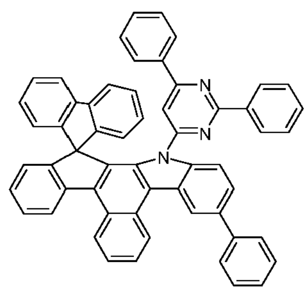 | 76% |

(fortgesetzt)

| | Edukt 1 | Edukt 2 | Produkt | Ausbeute |
|---|---|---|---|---|
| 2e | 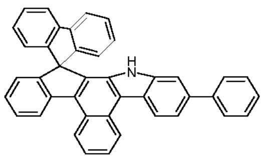 | 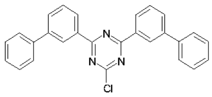 | 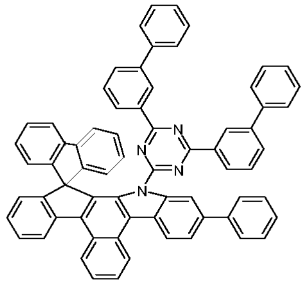 | 81% |
| 3e | 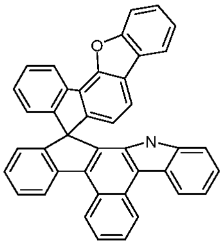 | 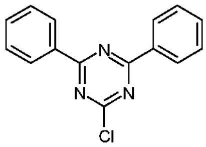 [3842-55-5] | 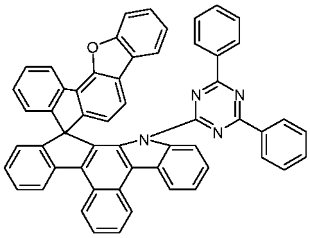 | 79% |
| 4e | 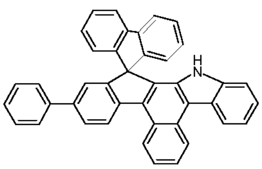 | 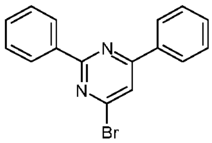 40734-4-5 | 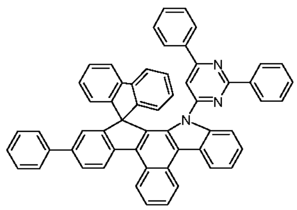 | 77% |
| 5e | 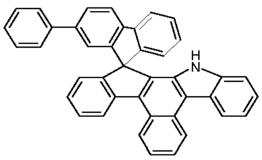 | 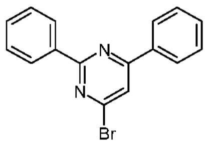 [40734-24-5] | 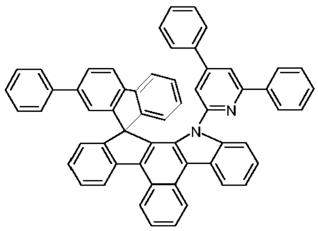 | 68% |
| 6e | 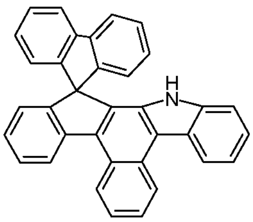 | 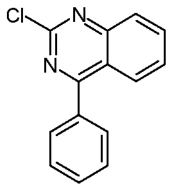 [29874-83-7] | 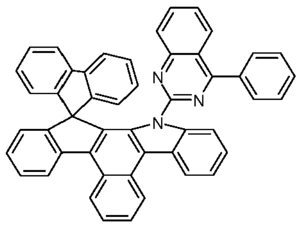 | 83% |
| 7e | 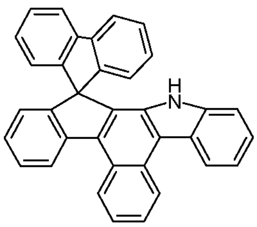 | 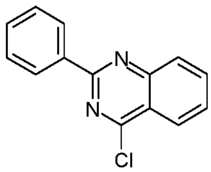 [6484-25-9] | 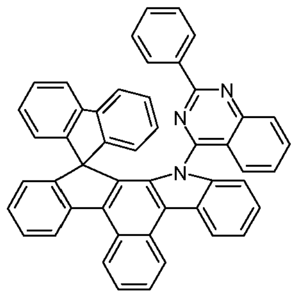 | 82% |

(fortgesetzt)

| | Edukt 1 | Edukt 2 | Produkt | Ausbeute |
|---|---|---|---|---|
| 8e | 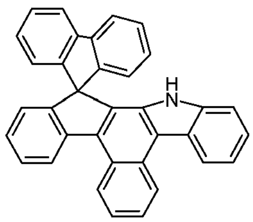 | 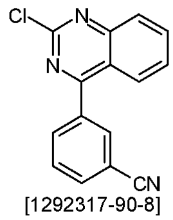 [1292317-90-8] | 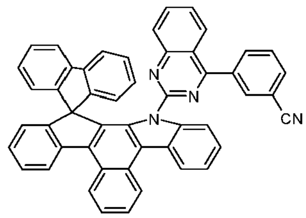 | 77% |
| 9e | 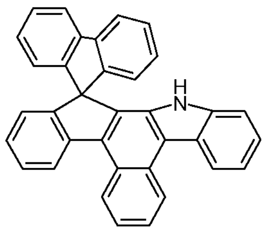 | 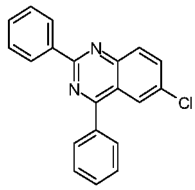 [30169-34-7] | 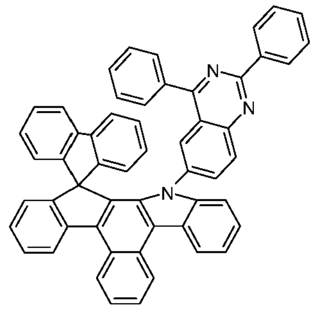 | 76% |
| 10e | 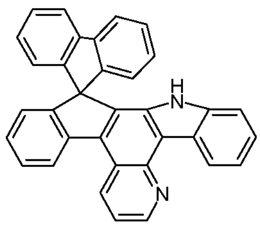 | 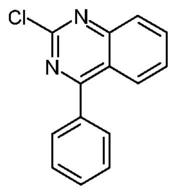 [29874-83-7] | 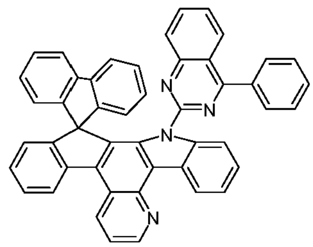 | 81% |
| 11e | 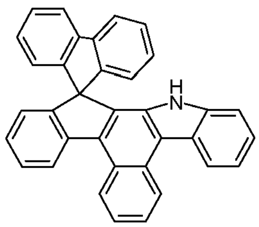 | 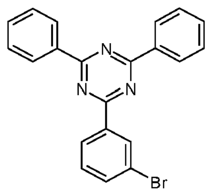 864377-31-1 | 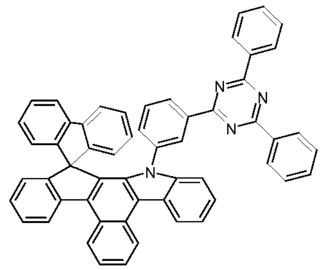 | 77% |
| 12e | 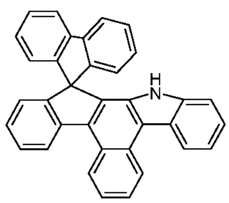 | 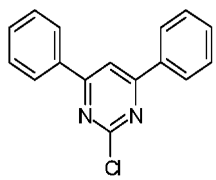 2915-16-4 | 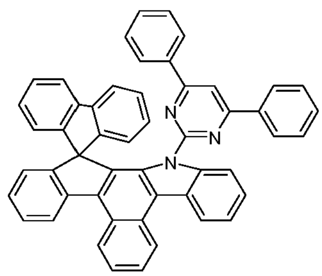 | 76% |

**f) Spiro[7H-benz[de]anthracen-7,9'-[9H]fluoren]**

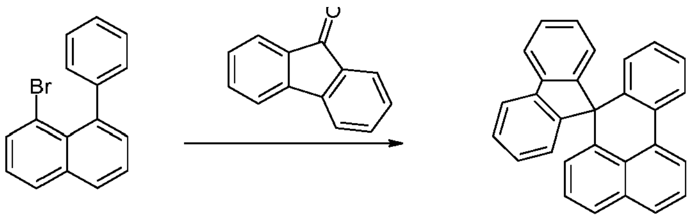

[1121545-24-1 ]

47 g (166 mmol) 1-Bromphenyl-naphthalin werden in 700 mL THF bei -78 °C vorgelegt. Bei dieser Temperatur werden 70 mL BuLi (2,5 M in Hexan) zugetropft. Nach 1 h werden 45,2 g (174 mmol) 2-Brom-fluoren-9-on in 200 mL THF zugetropft. Der Ansatz wird über Nacht bei Raumtemperatur gerührt, auf Eiswasser gegeben und mit Dichlormethan extrahiert. Die vereinigten organischen Phasen werden mit Wasser gewaschen und über Natriumsulfat getrocknet. Das Lösungsmittel wird im Vakuum entfernt und der Rückstand wird ohne weitere Aufreinigung mit 90 mL HCl und 1L AcOH bei 75 °C über Nacht erhitzt. Nach Erkalten saugt man vom ausgefallenen Feststoff ab, wäscht diesen zweimal mit je 150 ml Wasser, dreimal mit je 150 ml Ethanol und kristallisiert abschließend aus Heptan um. Ausbeute: 43 g (72 mmol), 70 %; Reinheit ca. 97 % nach $^{1}$H-NMR.

Analog werden die folgenden Verbindungen hergestellt:

| | Edukt 1 | Edukt 2 | Produkt | Ausbeute |
|---|---|---|---|---|
| 1f | 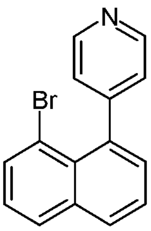 [1449393-74-1 ] | 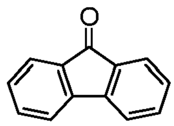 486-25-9 | 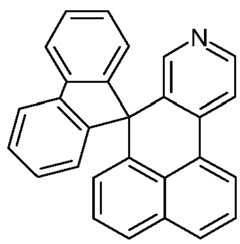 | 72% |
| 2f | 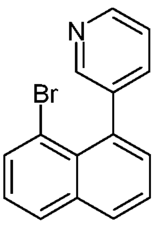 [190323-59-2 ] | 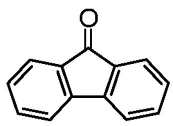 486-25-9 | 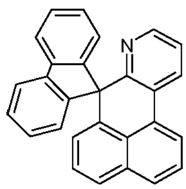 | 83% |
| 3f | 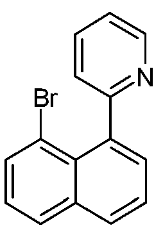 [190323-58-1 ] | 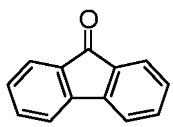 486-25-9 | 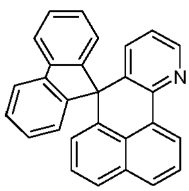 | 70% |

(fortgesetzt)

| | Edukt 1 | Edukt 2 | Produkt | Ausbeute |
|---|---|---|---|---|
| 4f | 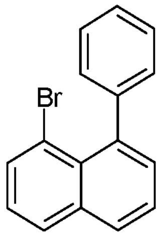 | 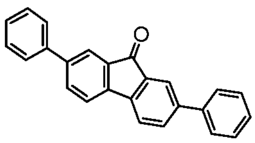 115033-91-5 | 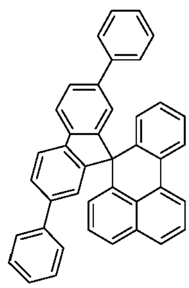 | 81% |
| 5f | 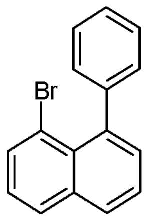 | 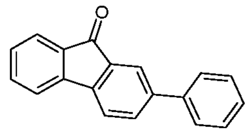 3096-49-9 | 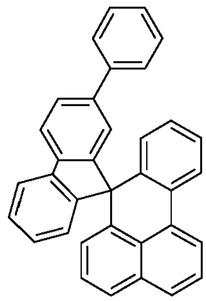 | 75% |
| 6f | 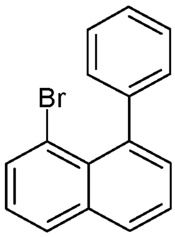 | 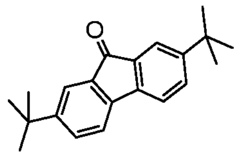 58775-13-6 | 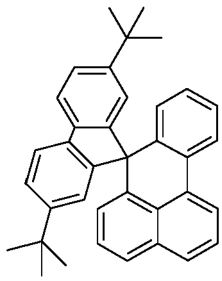 | 70% |

**q) 3-Bromo-spiro[7H-benz[de]anthracen-7,9'-[9H]fluoren]**

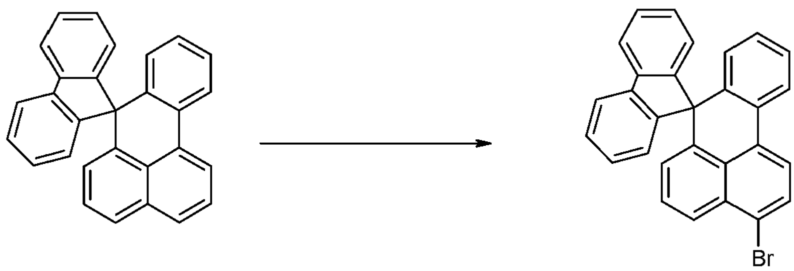

18,3 g (50 mmol) Spiro[7**H**-benz[**de**]anthracen-7,9'-[9**H**]fluoren] werden unter Lichtausschluss in 300 ml Dichlormethan vorgelegt und auf 5 °C abgekühlt. Es werden 2,7 ml (50 mmol) Brom in 25 ml Chloroform innerhalb 15 min. zugetropft und bei 5 °C weitere 7 h gerührt. Nach vollständigem Umsatz wird die Reaktion durch Zugabe von 15 ml Ethanol gestoppt, abgesaugt, mehrfach mit Ethanol gewaschen und anschließend zweimal aus NMP umkristallisiert. Ausbeute: 19 g (42 mmol), 85 %; Reinheit ca. 89 % nach $^1$H-NMR.

Analog werden die folgenden Verbindungen hergestellt:

| | Edukt 1 | Produkt | Ausbeute |
|---|---|---|---|
| 1g | 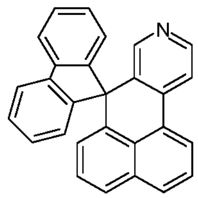 | 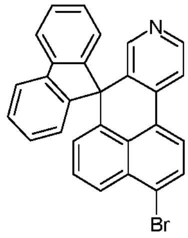 | 72% |
| 2g | 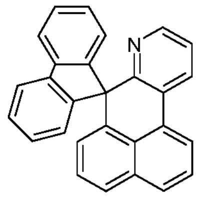 | 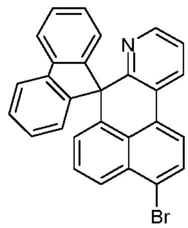 | 83% |
| 3g | 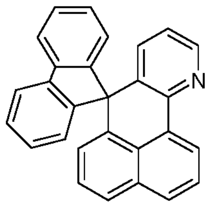 | 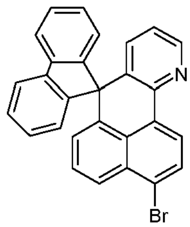 | 70% |
| 4g | 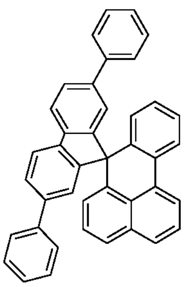 | 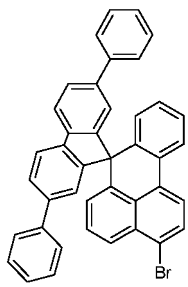 | 81% |
| 5g | 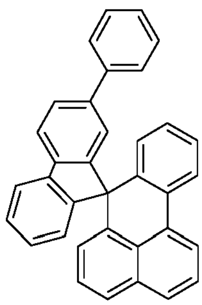 | 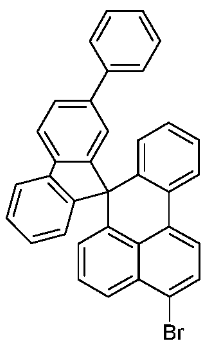 | 75% |

(fortgesetzt)

| | Edukt 1 | Produkt | Ausbeute |
|---|---|---|---|
| 6g | 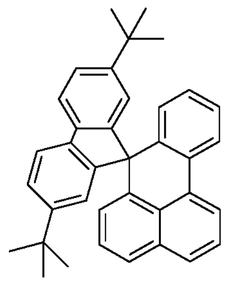 | 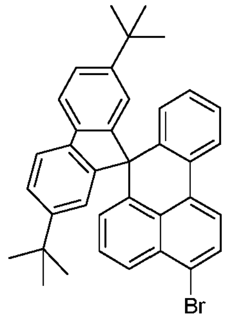 | 70% |

**h) Spiro[7H-benz[de]anthracen-7,9'-[9H]fluoren]-3-boronsäure**

Analog zu Beispiel b werden folgende Verbindungen hergestellt:

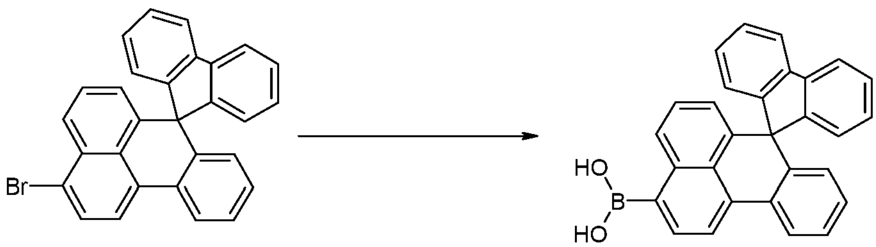

Einsatz von 72,8 mmol Arylbromid, 150 mL trockenes THF, 30 mL (764 mmol / 2,5 M in Hexan) n-BuLi und 15,1 g (145 mmol) Borsäuretrimethylester. Ausbeute: 26 g (65 mmol), 93 % der Theorie, der laut HPLC-Analyse einen Gehalt von >97.3 % aufweist.

Analog werden die folgenden Verbindungen hergestellt:

| | Edukt 1 | Produkt | Ausbeute |
|---|---|---|---|
| **1h** | 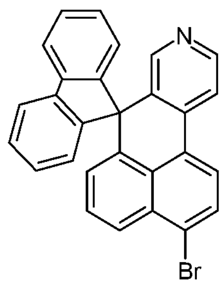 | 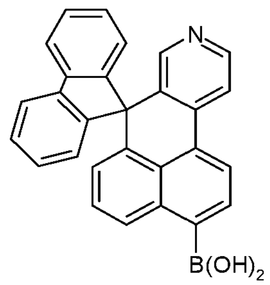 | 72% |
| **2h** | 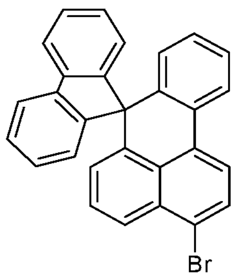 [1121545-27-4 ] | 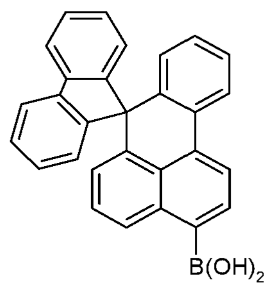 | 72% |

(fortgesetzt)

| | Edukt 1 | Produkt | Ausbeute |
|---|---|---|---|
| **3h** | 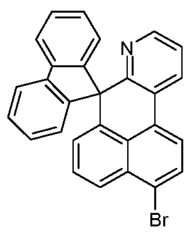 | 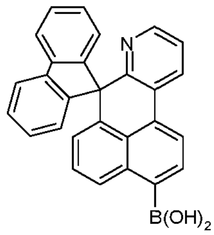 | 71% |
| **4h** | 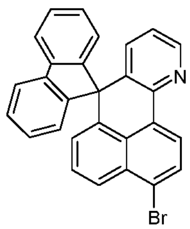 | 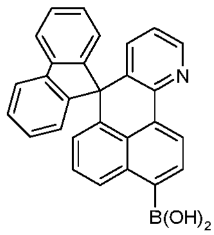 | 75% |

**i) 3-(2-Nitro-phenyl)-spiro[7H-benz[de]anthracen-7,9'-[9H]fluoren]**

Analog zu Beispiel c werden folgende Verbindungen hergestellt:

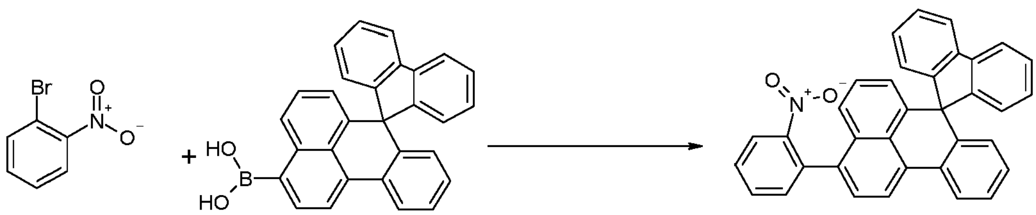

Einsatz von 114 mmol Arylboronsäure, 47 mmol 1-Brom-2-nitrobenzol und 30,3 g (143 mmol) Kaliumcarbonat in einem Gemisch aus 150 ml Wasser und 150 ml THF mit 0,5 g (0,4 mmol) $Pd(PPh_3)_4$. Ausbeute: (65 mmol), 88 % der Theorie, der laut HPLC-Analyse einen Gehalt von >96,3 % aufweist.

Analog werden die folgenden Verbindungen hergestellt

| | Edukt 1 | Edukt 2 | Produkt | Ausbeute |
|---|---|---|---|---|
| **1i** | 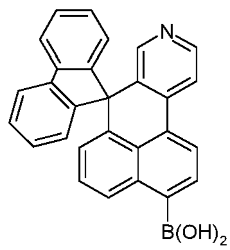 | 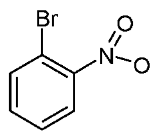 | 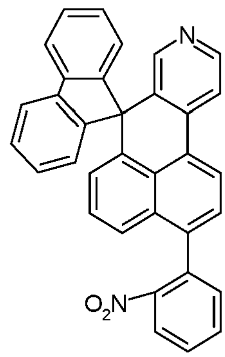 | 70% |

(fortgesetzt)

| | Edukt 1 | Edukt 2 | Produkt | Ausbeute |
|---|---|---|---|---|
| 2i | 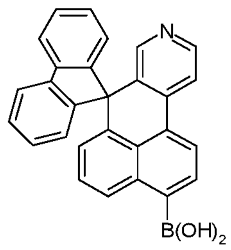 | 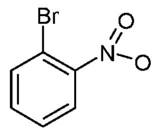 | 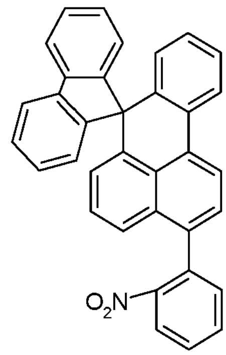 | 79% |
| 3i | 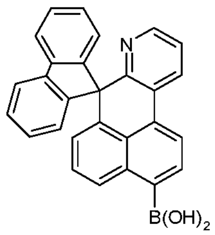 | 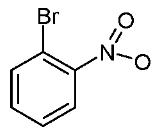 | 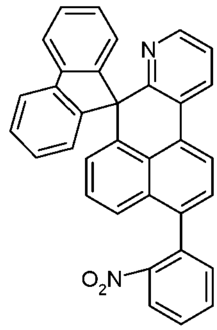 | 80% |
| 4i | 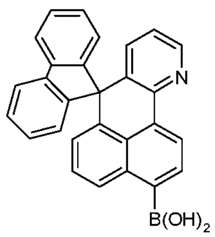 | 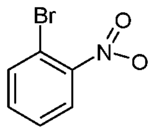 | 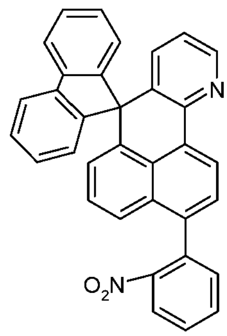 | 78% |

## j) Cyclisierung

Analog zu Beispiel d werden folgende Verbindungen hergestellt:

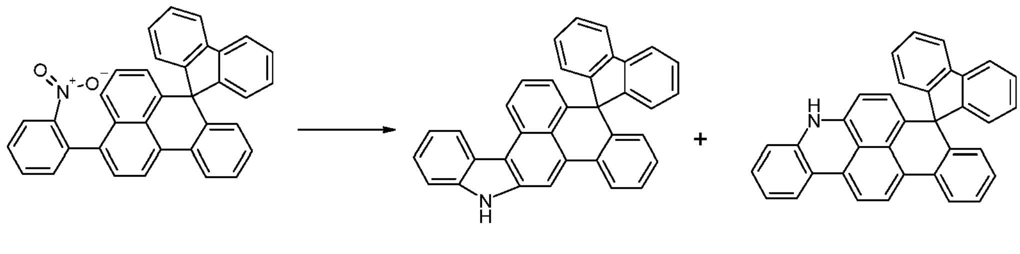

j1 j2

Eine Mischung aus 238 mmol der Nitro-Derivats und 290,3 ml (1669 mmol) Triethylphosphit wird 12 h unter Rückfluss erhitzt. Anschließend wird das restliche Triethylphosphit abdestilliert (72-76 °C / 9 mm Hg). Der Rückstand wird mit Wasser/MeOH (1:1) versetzt, der Feststoff abfiltriert und aus Toluol umkristallisiert. Ausbeute: 104 g (229 mmol) der Mischung d1+d2, 92% d. Th.; Reinheit: 98,0 % n. HPLC. Nach Umkristallisation aus Ethylacetat/Toluol (1:2) erhält

man 65% j1 und 15% j2.

Analog werden die folgenden Verbindungen hergestellt:

| | Edukt 1 | Produkt | Produkt | Ausbeute |
|---|---|---|---|---|
| **1j** | 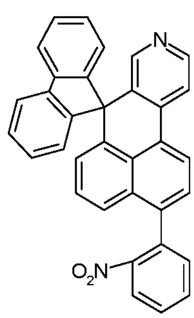 | 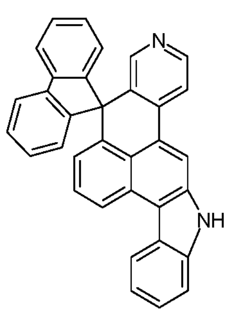 | 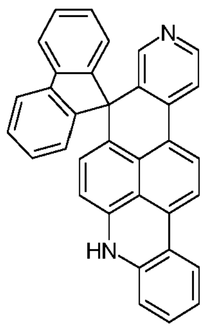 | 67%/20% |
| **2j** | 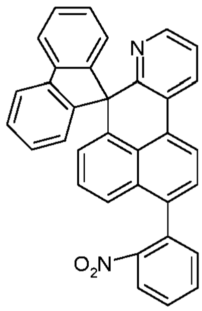 | 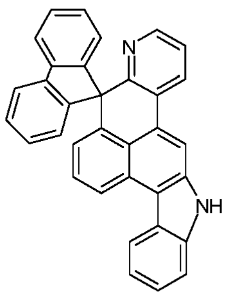 | 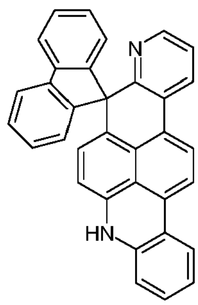 | 50%/21% |
| **3j** | 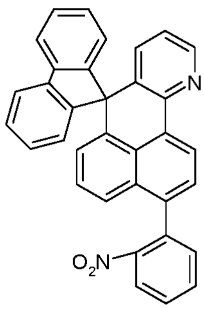 | 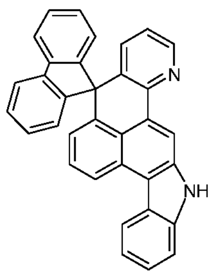 | 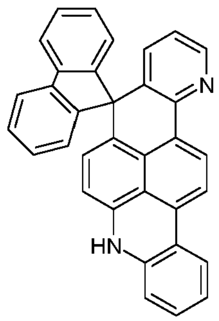 | 52%/19% |

**k) Ullmann Reaktion**

Analog zu Beispiel e werden folgende Verbindungen hergestellt:

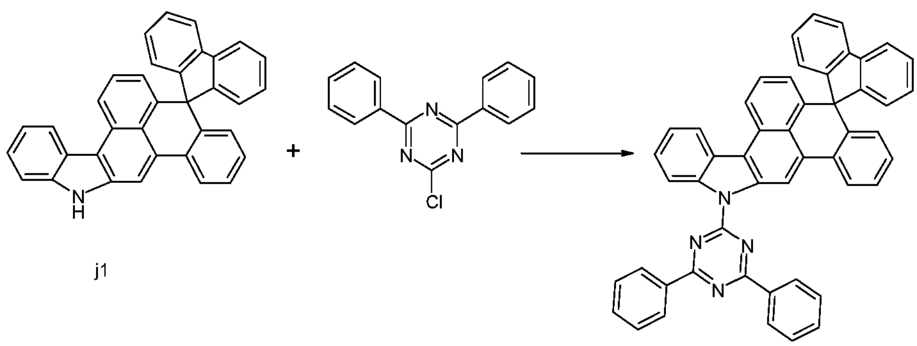

j1

30 mmol Carbazol-Derivat werden in 225 ml Dimethylformamid unter Schutzgasatmosphäre gelöst und mit 1,5 g (37,5 mmol) NaH (60%ig in Mineralöl) versetzt. Nach 1 h bei Raumtemperatur wird eine Lösung von 2-Chlor-4,6-diphenyl-[1 ,3,5]-triazin (8,5 g, 31,75 mmol) in 75 mL Dimethylformamid zugetropft. Das Reaktionsgemisch wird dann

12 h bei Raumtemperatur gerührt. Nach dieser Zeit wird das Reaktionsgemisch auf Eis gegossen und dreimal mit Dichlormethan extrahiert. Die vereinigten organischen Phasen werden über $Na_2SO_4$ getrocknet und eingeengt. Der Rückstand wird aus Toluol umkristallisiert und abschließend zweimal fraktioniert sublimiert (p ca. $10^{-6}$ mbar, T = 398 - 400 °C). Ausbeute: 78 % d. Th.; Reinheit: 99,9 % n. HPLC.

Analog werden die folgenden Verbindungen hergestellt:

| | Edukt 1 | Edukt 2 | Produkt | Ausbeute |
|---|---|---|---|---|
| **1k** | 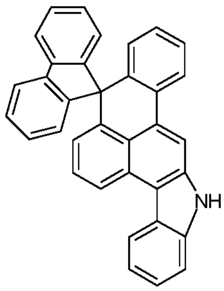 | 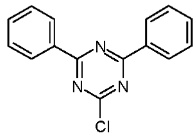 . | 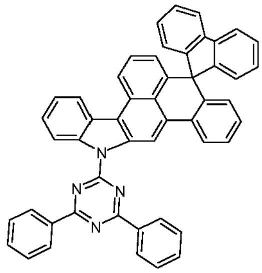 | 70% |
| **2k** | 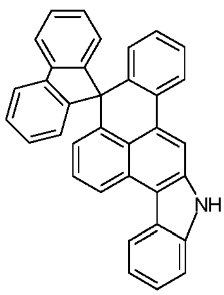 | 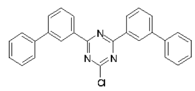 . | 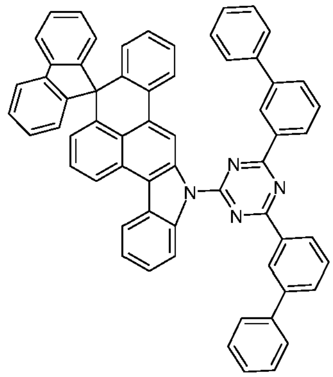 | 75% |
| **3k** | 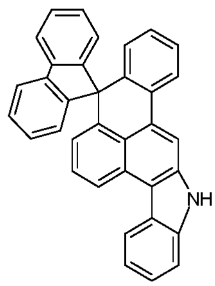 | 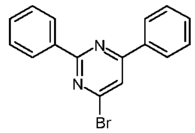 [40734-24-5] | 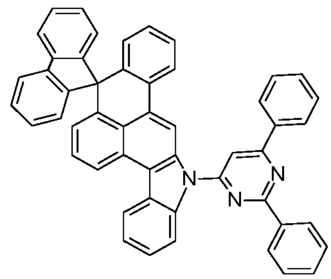 | 76% |
| **4k** | 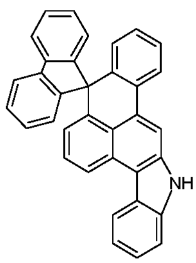 | 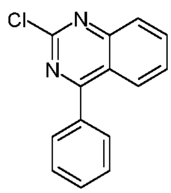 [29874-83-7] | 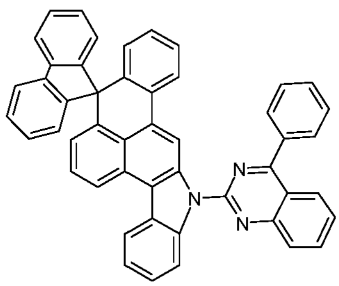 | 75% |

(fortgesetzt)

| | Edukt 1 | Edukt 2 | Produkt | Ausbeute |
|---|---|---|---|---|
| **5k** | 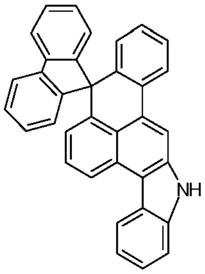 | 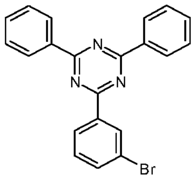 864377-31-1 | 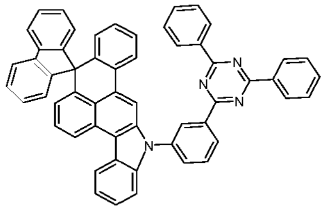 | 78% |
| **6k** | 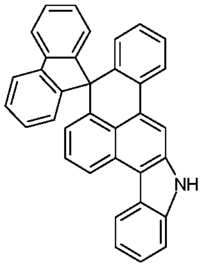 | 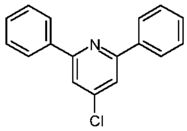 133785-60-1 | 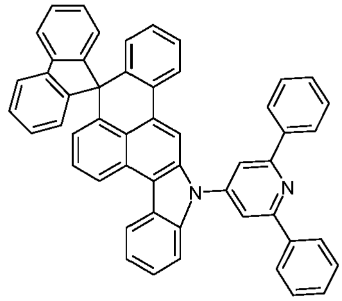 | 74% |
| **7k** | 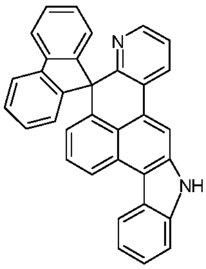 | 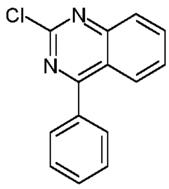 [29874-83-7] | 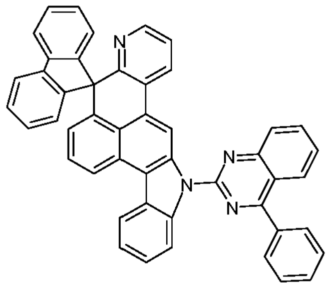 | 70% |
| **8k** | 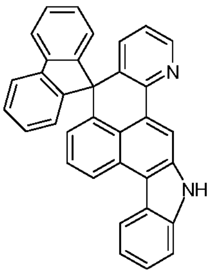 | 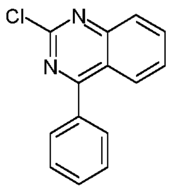 [29874-83-7] | 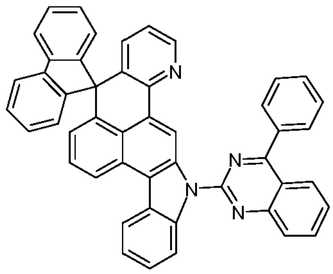 | 74% |
| **9k** | 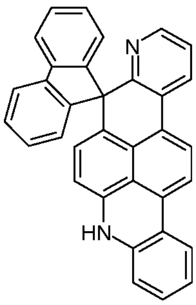 | 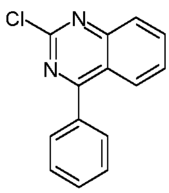 [29874-83-7] | 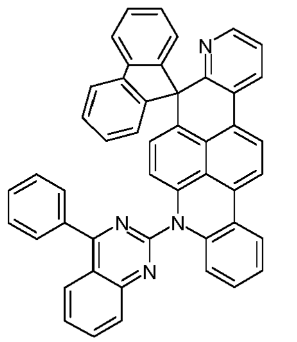 | 63% |

(fortgesetzt)

| | Edukt 1 | Edukt 2 | Produkt | Ausbeute |
|---|---|---|---|---|
| **10k** | 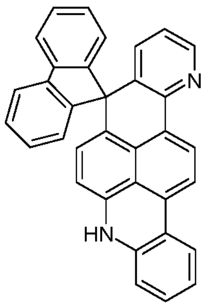 | 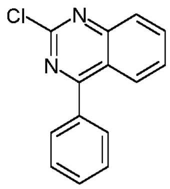 [29874-83-7] . | 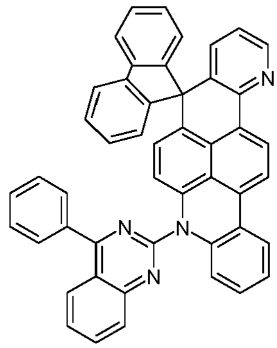 | 60% |
| **11k** | 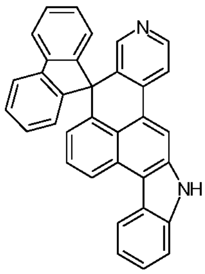 | 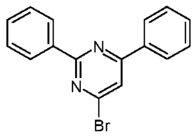 [40734-24-5] . | 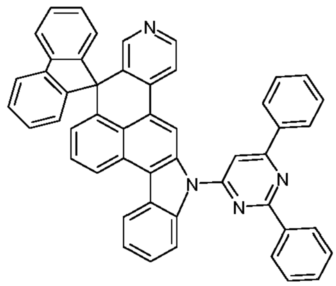 | 70% |

**l) Buchwald Reaktion**

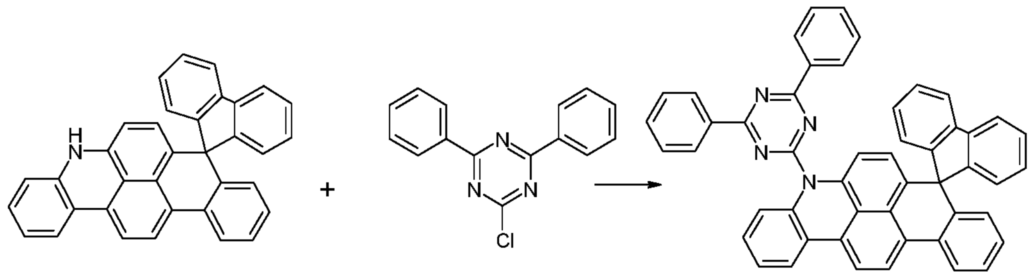

j2

Unter Schutzgas werden 36,4 g (80 mmol) der Amin-Verbindung j2, 22 g (87 mmol) 2-Chloro-4,6-diphenyl-[1,3,5]triazin, 15,9 ml (15,9 mmol, 1 molare Lösung) Tri-tert-butylphosphin und 1,79 g (7,9 mmol) Palladiumacetat in 120 ml p-Xylol suspendiert. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird mit Toluol heiß extrahiert, aus Toluol umkristallisiert und abschließend im Hochvakuum sublimiert, die Reinheit beträgt 99,9%. Ausbeute: 34 g (64 mmol), 81 % der Theorie.

Analog können folende Verbindunen herestellt werden:

| | Edukt 1 | Edukt 2 | Produkt | Ausbeute |
|---|---|---|---|---|
| 1l | 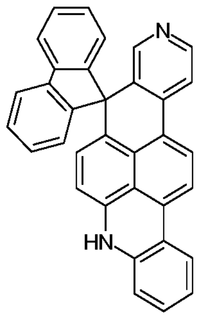 | 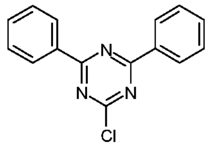 [3842-55-5] | 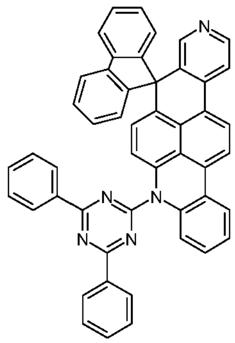 | 80% |
| 2l | 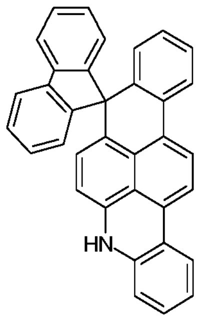 | 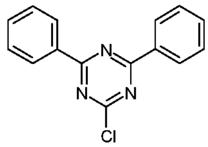 [3842-55-5] | 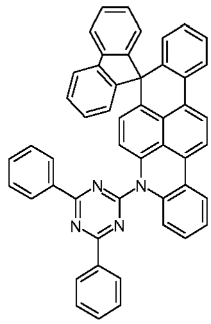 | 71% |
| 3l | 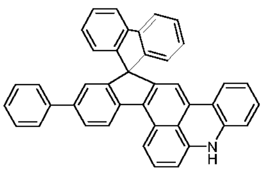 | 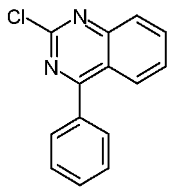 [29874-83-7] | 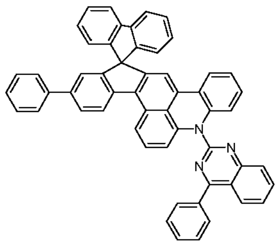 | 72% |
| 4l | 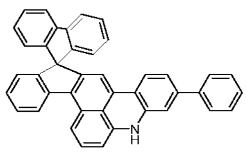 | 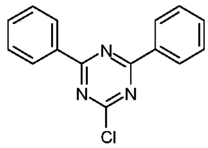 [3842-55-5] | 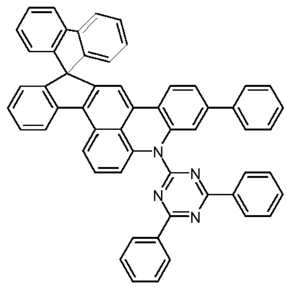 | 67% |
| 5l | 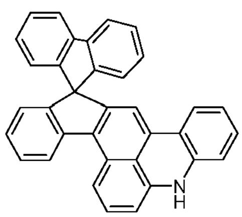 | 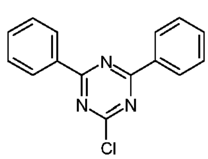 [3842-55-5] | 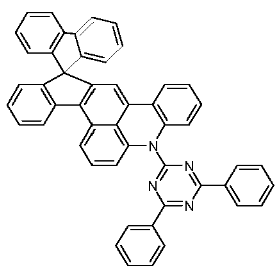 | 65% |

(fortgesetzt)

| | Edukt 1 | Edukt 2 | Produkt | Ausbeute |
|---|---|---|---|---|
| 6l | 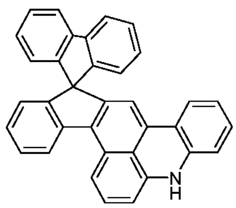 | 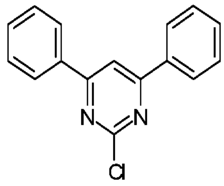 2915-16-4 | 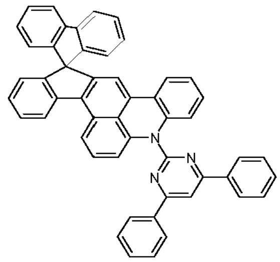 | 68% |
| 7l | 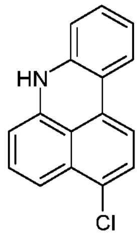 | 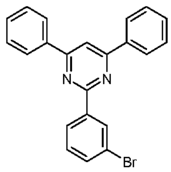 864377-22-0 | 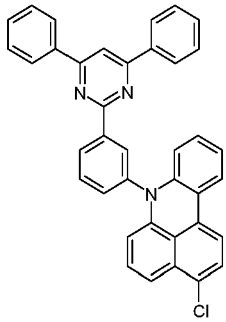 | 56% |
| 8l | 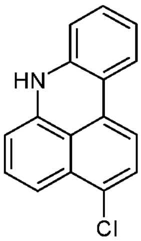 | 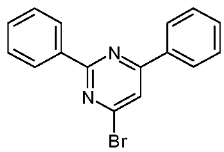 40734-4-5 | 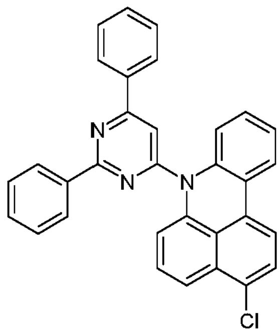 | 61% |
| 9l | 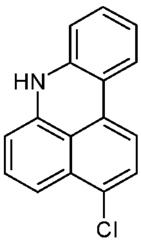 | 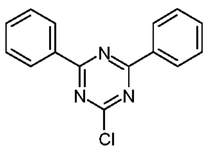 [3842-55-5] | 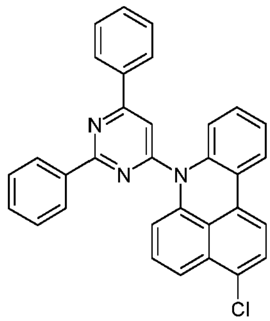 | 64% |
| 10l | 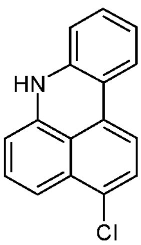 | 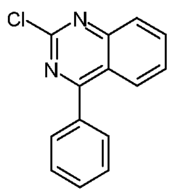 [29874-83-7] | 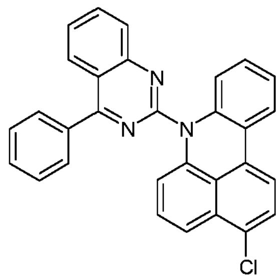 | 60% |

**m) 2-(7-{2-Phenyl-6-[(E)-((Z)-1-propenyl)-buta-1,3-dienyl]-pyrimidin-4-yl}-7H-7-aza-benzo[de]anthracen-3-yl)-benzoesäuremethylester**

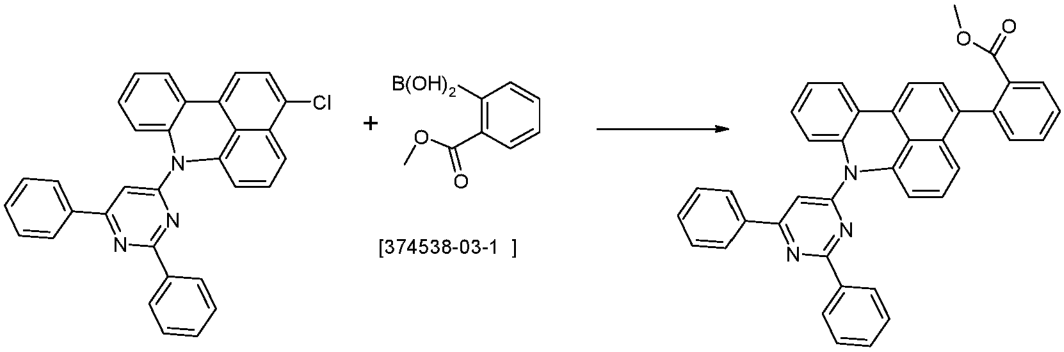


Eine gut gerührte, entgaste Suspension aus 10,6 g (71 mmol) Benzoesäuremethylester-2-boronsäure, 34 g (71 mmol) 3-Chloro-7-(2,6-diphenyl-pyrimidin-4-yl)-7H-7-aza-benzo[de]anthracen und 18,9 g (6,6 mmol) Trikaliumphosphat in einem Gemisch aus 350 ml Wasser und 350 ml THF wird mit 1,55 g (0,1 mmol) $Pd(PPh_3)_4$ versetzt und 60 h unter Rückfluss erhitzt. Nach Erkalten wird die organische Phase abgetrennt, dreimal mit 200 ml Wasser und einmal mit 200 ml gesättigter Kochsalzlösung gewaschen und anschließend über Magnesiumsulfat getrocknet. Die organische Phase wird im Vakuum zur Trockene einrotiert. Der so erhaltene graue Rückstand wird aus Dioxan umkristallisiert. Die ausgefallenen Kristalle werden abgesaugt, mit 50 ml Ethanol gewaschen und anschließend im Vakuum getrocknet. Ausbeute: 33 g, 80 % d. Th..

Analog können folgende Verbindungen hergestellt werden:

| | Edukt 1 | Edukt 2 | Produkt | Ausbeute |
|---|---|---|---|---|
| 1m | 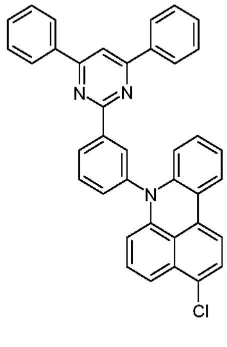 | 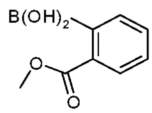 [374538-03-1 ] | 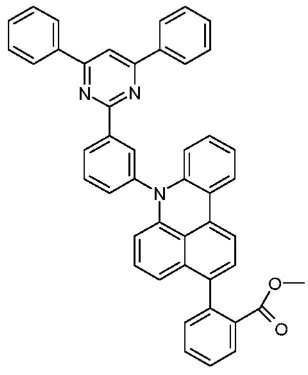 | 77% |
| 2m | 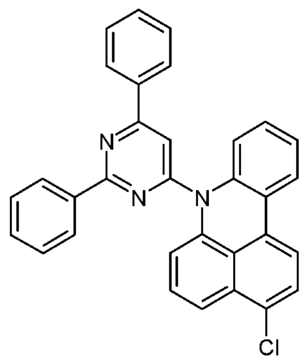 | 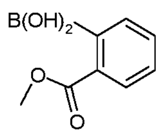 [374538-03-1 ] | 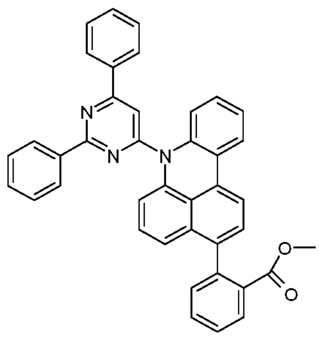 | 79% |

(fortgesetzt)

| | Edukt 1 | Edukt 2 | Produkt | Ausbeute |
|---|---|---|---|---|
| 3m | 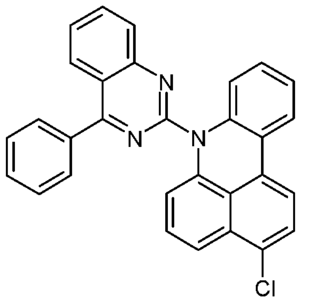 | 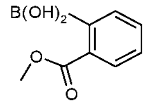 [374538-03-1 ] | 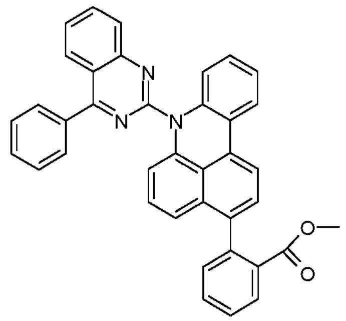 | 64% |

**n) Cyclisierung**

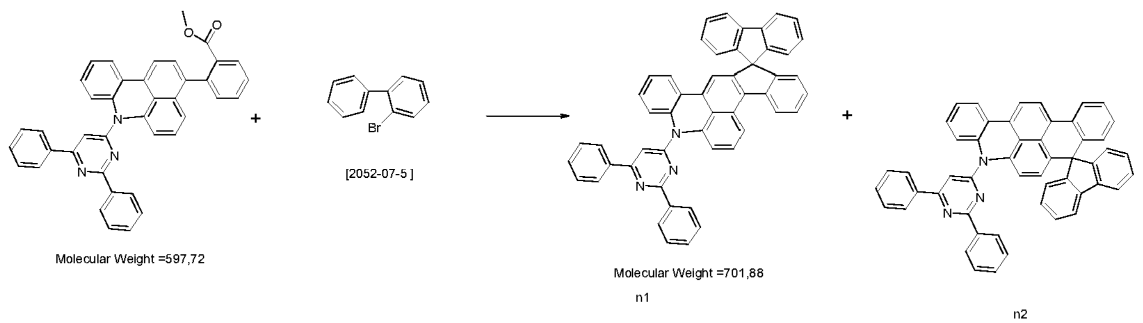


38 g (166 mmol) 2-Brombiphenyl werden in 700 mL THF bei -78 °C vorgelegt. Bei dieser Temperatur werden 70 mL BuLi (2,5 M in Hexan) zugetropft. Nach 1 h werden 99 g (166 mmol) 2-(7-{2-Phenyl-6-[(E)-((Z)-1-propenyl)-buta-1,3-dienyl]-pyrimidin-4-yl}-7H-7-azabenzo[de]anthracen-3-yl)-benzoesäuremethylester in 200 mL THF zugetropft. Der Ansatz wird über Nacht bei Raumtemperatur gerührt, auf Eiswasser gegeben und mit Dichlormethan extrahiert. Die vereinigten organischen Phasen werden mit Wasser gewaschen und über Natriumsulfat getrocknet. Das Lösungsmittel wird im Vakuum entfernt und der Rückstand wird ohne weitere Aufreinigung mit 90 mL HCl und 1L AcOH bei 75 °C über Nacht erhitzt. Nach Erkalten saugt man vom ausgefallenen Feststoff ab, wäscht diesen zweimal mit je 150 ml Wasser, dreimal mit je 150 ml Ethanol und kristallisiert abschließend aus Heptan um. Das Produkt wird mit Toluol heiß extrahiert und aus Toluol umkristallisiert und abschließend im Hochvakuum sublimiert. Ausbeute: 96 g (60 mmol) n1 und n2, 82 %. Nach der Umkristallisation aus Toluol/Ethylacetat (2:1) erhält man 59 % n1 und 19 % n2 mit einer Reinheit ca. 99,9 % nach HPLC.

Analog können folgende Verbindungen hergestellt werden:

| | Edukt 1 | Edukt 2 | Produkt 1 | Produkt 2 | Ausbeute |
|---|---|---|---|---|---|
| 1n | 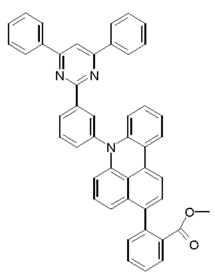 | 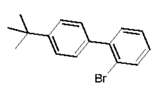 [1033692-34-0 ] | 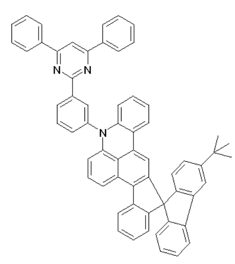 | 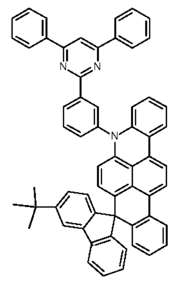 | 53%/ 16% |

(fortgesetzt)

| | Edukt 1 | Edukt 2 | Produkt 1 | Produkt 2 | Ausbeute |
|---|---|---|---|---|---|
| 2n | 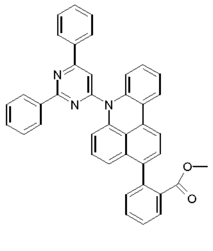 | 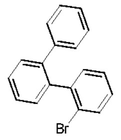 [75295-57-7 ] | 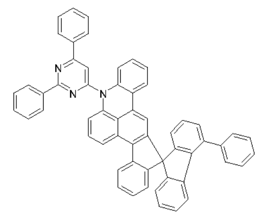 | 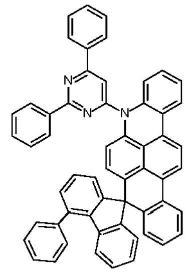 | 55%/ 19% |
| 3n | 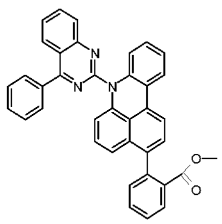 | 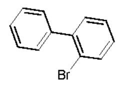 [2052-07-5 ] | 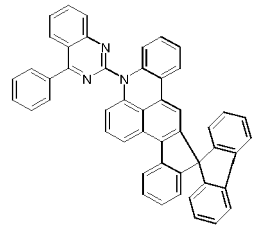 | 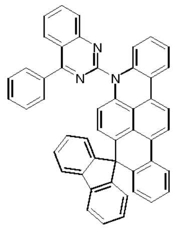 | 45%/ 14% |

**Herstellung der OLEDs**

In den folgenden Beispielen V1 bis E8 (siehe Tabellen 1 und 2) werden die Daten verschiedener OLEDs vorgestellt.

**Vorbehandlung für die Beispiele V1-E8:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC5:IC3:TEG2 (55%:35%:10%) bedeutet hierbei, dass das Material IC5 in einem Volumenanteil von 55%, IC3 in einem Anteil von 35% und TEG2in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 $cd/m^2$ bestimmt und daraus die CIE 1931 x und y Farbkoordinaten berechnet. Die Angabe U1000 in Tabelle 2 bezeichnet die Spannung, die für eine Leuchtdichte von 1000 $cd/m^2$ benötigt wird. EQE1000 schließlich bezeichnet die externe Quanteneffizienz bei einer Betriebsleuchtdichte von 1000 $cd/m^2$. Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 $cd/m^2$ und L1 = 70% in Tabelle 2 bedeutet, dass die in Spalte LD angegebene Lebensdauer der Zeit entspricht, nach der die Anfangsleuchtdichte von 4000 $cd/m^2$ auf 2800 $cd/m^2$ absinkt. Analog bedeutet L0;j0 = 20mA/$cm^2$, L1 = 80%, dass die Leuchtdichte bei Betrieb mit 20mA/$cm^2$ nach der Zeit LD auf 80% ihres Anfangswertes absinkt.

Die Daten der verschiedenen OLEDs sind in Tabelle 2 zusammengefasst. Die Beispiele V1 - V2 sind Vergleichsbeispiele gemäß dem Stand der Technik, die Beispiele E1-E8 zeigen Daten von erfindungsgemäßen OLEDs.

Im Folgenden werden einige der Beispiele näher erläutert, um die Vorteile der erfindungsgemäßen OLEDs zu verdeutlichen.

## Verwendung von erfindungsgemäßen Materialien in OLEDs

Die erfindungsgemäßen Materialien ergeben bei Einsatz in der Lochblockierschicht (HBL) in OLEDs wesentliche Verbesserungen gegenüber dem Stand der Technik, vor allem bezüglich Lebensdauer. Durch Einsatz der erfindungsgemäßen Verbindungen 13e und 6e lässt sich eine Steigerung der Lebensdauer um ca. 10-15% gegenüber dem Stand der Technik erzielen (Vergleich von Beispiel E1 mit V1; Vergleich von Beispiel E2 mit V2).

Tabelle 1: Aufbau der OLEDs

| Bsp | HIL Dicke | HTL Dicke | EBL Dicke | EML Dicke | HBL Dicke | ETL Dicke | EIL Dicke |
|---|---|---|---|---|---|---|---|
| V1 | HATCN 5nm | SpMA1 230nm | SpMA3 20nm | IC6:IC3:TEG2 (60%:30%:10 %) 30nm | SdT1 10nm | ST2:LiQ (50%:50%) 30nm | --- |
| V2 | HATCN 5nm | SpMA1 230nm | SpMA3 20nm | IC6:IC3:TEG2 (60%:30%:10 %) 30nm | SdT2 10nm | ST2:LiQ (50%:50%) 30nm | --- |
| E1 | HATCN 5nm | SpMA1 230nm | SpMA3 20nm | IC6:IC3:TEG2 (60%:30%:10 %) 30nm | 13e 10nm | ST2:LiQ (50%:50%) 30nm | --- |
| E2 | HATCN 5nm | SpMA1 230nm | SpMA3 20nm | IC6:IC3:TEG2 (60%:30%:10 %) 30nm | 6e 10nm | ST2:LiQ (50%:50%) 30nm | --- |
| E3 | HATCN 5nm | SpMA1 230nm | SpMA3 20nm | IC6:IC3:TEG2 (60%:30%:10 %) 30nm | 12e 10nm | ST2:LiQ (50%:50%) 30nm | --- |
| E4 | HATCN 5nm | SpMA1 230nm | SpMA3 20nm | IC6:IC3:TEG2 (60%:30%:10 %) 30nm | 3e 10nm | ST2:LiQ (50%:50%) 30nm | --- |
| E5 | HATCN 5nm | SpMA1 230nm | SpMA3 20nm | IC6:IC3:TEG2 (60%:30%:10 %) 30nm | --- | ST2:2l (50%:50%) 40nm | LiQ 3nm |
| E6 | HATCN 5nm | SpMA1 230nm | SpMA3 20nm | IC6:IC3:TEG2 (60%:30%:10 %) 30nm | --- | ST2:8l (50%:50%) 40nm | LiQ 3nm |
| E7 | HATCN 5nm | SpMA1 230nm | SpMA3 20nm | IC6:IC3:TEG2 (60%:30%:10 %) 30nm | --- | ST2:1n (50%:50%) 40nm | LiQ 3nm |
| E8 | HATCN 5nm | SpMA1 230nm | SpMA3 20nm | IC6:IC3:TEG2 (60%:30%:10 %) 30nm | --- | ST2:1k (50%:50%) 40nm | LiQ 3nm |

Tabelle 2: Daten der OLEDs

| Bsp. | U1000 (V) | EQE 1000 | CIE x/y bei 1000 cd/$m^2$ | $L_0$; $j_0$ | L1 % | LD (h) |
|---|---|---|---|---|---|---|
| V1 | 3.3 | 17.5% | 0.33/0.63 | 20 mA/$cm^2$ | 80 | 245 |
| V2 | 3.4 | 17.3% | 0.33/0.63 | 20 mA/$cm^2$ | 80 | 240 |
| E1 | 3.5 | 17.4% | 0.33/0.62 | 20 mA/$cm^2$ | 80 | 280 |
| E2 | 3.6 | 17.3% | 0.32/0.63 | 20 mA/$cm^2$ | 80 | 270 |

(fortgesetzt)

| Bsp. | U1000 (V) | EQE 1000 | CIE x/y bei 1000 cd/$m^2$ | $L_0$; $j_0$ | L1 % | LD (h) |
|---|---|---|---|---|---|---|
| E3 | 3.6 | 17.5% | 0.32/0.63 | 20 mA/$cm^2$ | 80 | 255 |
| E4 | 3.5 | 17.6% | 0.32/0.62 | 20 mA/$cm^2$ | 80 | 260 |
| E5 | 3.2 | 18.5% | 0.34/0.63 | 40 mA/$cm^2$ | 80 | 195 |
| E6 | 2.9 | 18.7% | 0.34/0.63 | 40 mA/$cm^2$ | 80 | 200 |
| E7 | 3.2 | 18.6% | 0.34/0.63 | 40 mA/$cm^2$ | 80 | 185 |
| E8 | 3.1 | 19.0% | 0.34/0.63 | 40 mA/$cm^2$ | 80 | 180 |

Tabelle 3: Strukturformeln der Materialien für die OLEDs

| | |
|---|---|
| 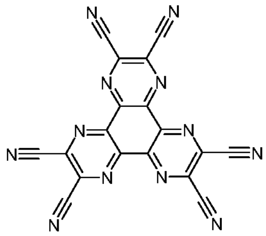 | 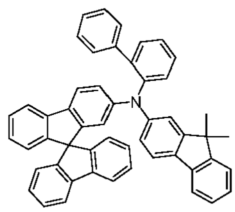 |
| HATCN | SpMA1 |
| 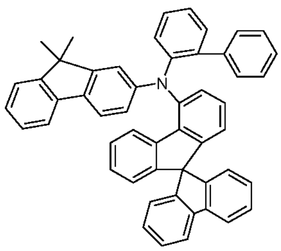 | 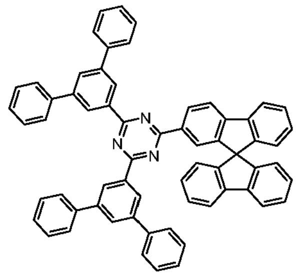 |
| SpMA3 | ST2 |
| 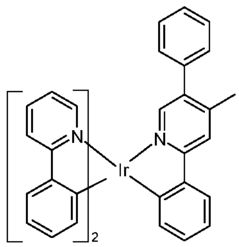 | 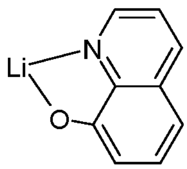 |
| TEG2 | LiQ |
| 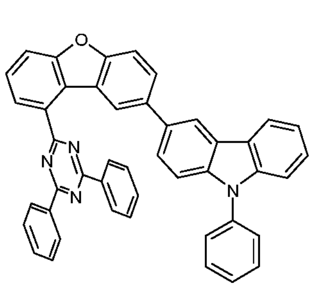 | 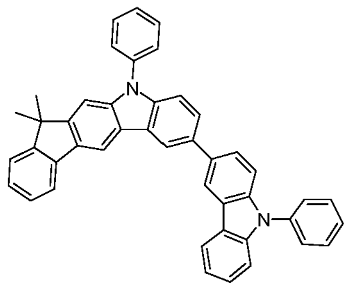 |
| IC6 | IC3 |

(fortgesetzt)

| | |
|---|---|
| 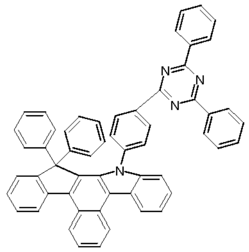 WO2015/156587A1 | 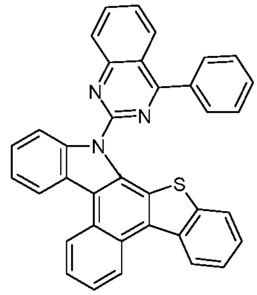 US2015/001511A1 |
| SdT1 | SdT2 |
| 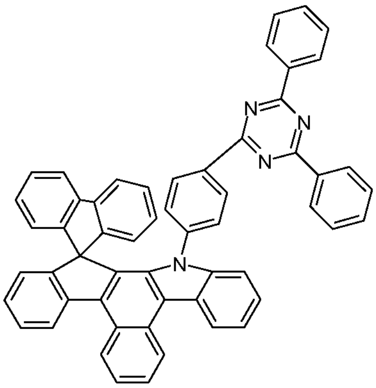 | 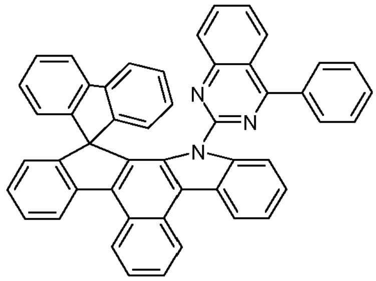 |
| 13e | 6e |
| 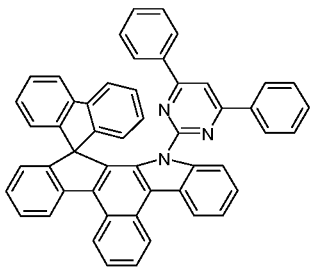 | 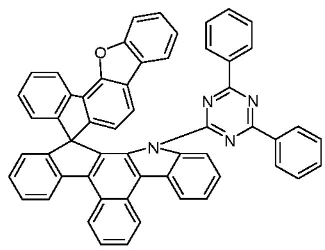 |
| 12e | 3e |
| 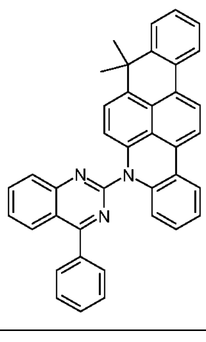 | 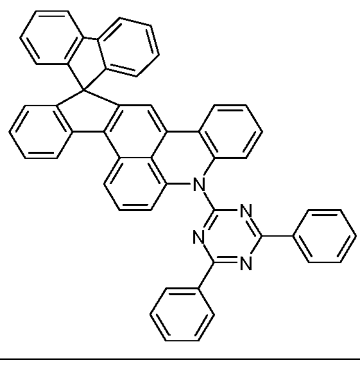 |
| 2l | 8l |

(fortgesetzt)

| | |
|---|---|
| 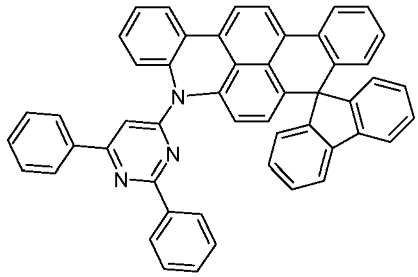 | 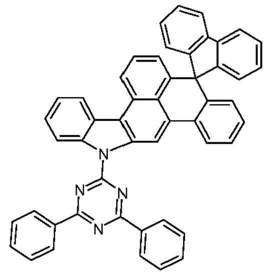 |
| 1n | 1k |

**Patentansprüche**

1. Verbindung, umfassend Strukturen der Formel (I):

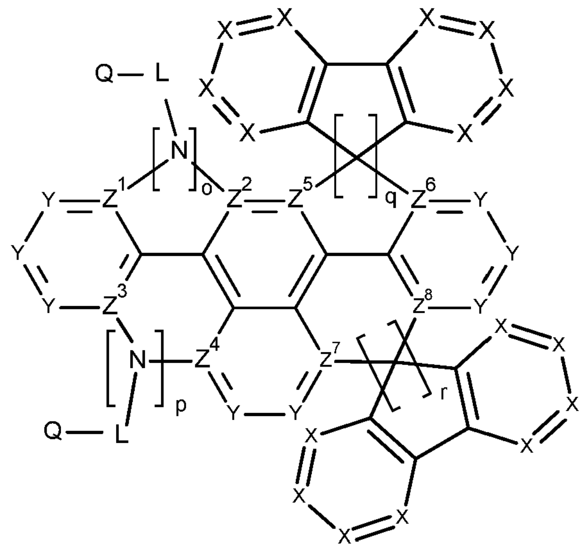

Formel (I)

wobei für die verwendeten Symbole gilt:

$Z^1$, $Z^2$, $Z^3$, $Z^4$, $Z^5$, $Z^6$, $Z^7$, $Z^8$ ist gleich oder verschieden Y oder C;
Y ist bei jedem Auftreten gleich oder verschieden N, $CR^1$, oder zwei benachbarte Gruppen Y stehen zusammen für O, S, $NR^1$ mit der Maßgabe, dass ein 5 oder ein 6 Ring gebildet wird;
X ist bei jedem Auftreten gleich oder verschieden N oder $CR^1$;
o, p, q, r ist 0 oder 1;
L ist eine Bindung oder ein aromatisches oder heteroaromatisches Ringsystem mit 5 bis 24 aromatischen Ringatomen, welches durch einen oder mehrere Reste $R^1$ substituiert sein kann;
Q ist eine Elektronentransportgruppe, ausgewählt aus einer optional substituierten Fünfring-Heteroarylgruppe, die mindestens zwei Heteroatome enthält, oder einer optional substituierten Sechsring-Heteroarylgruppe, wobei die Gruppen auch Teil eines aromatischen Ringsystems sein können und wobei an diese Gruppen auch noch weitere Aryl- oder Heteroarylgruppen ankondensiert sein können;
$R^1$ ist bei jedem Auftreten gleich oder verschieden H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $NO_2$, $P(=O)(R^2)_2$, $OSO_2R^2$, $OR^2$, $S(=O)R^2$, $S(=O)_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, wobei eine oder mehrere nicht benachbarte $CH_2$-Gruppen durch $-R^2C=CR^2-$, $-C\equiv C-$, $Si(R^2)_2$, $Ge(R^2)_2$, $Sn(R^2)_2$, C=O, C=S, C=Se, $C=NR^2$, $NR^2$,

$P(=O)(R^2)$, $-C(=O)O-$, $-C(=O)NR^2-$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Aralkyl- oder Heteroaralkylgruppe mit 5 bis 40 aromatischen Ringatomen, die durch einen oder mehrere Reste $R^2$ substituiert sein kann, oder eine Kombination dieser Systeme; dabei können zwei oder mehrere Reste $R^1$ miteinander ein Ringsystem bilden;

$R^2$ ist bei jedem Auftreten gleich oder verschieden H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C=C-, $Si(R^3)_2$, $Si(R^3)_2$, $Ge(R^3)_2$, $Sn(R^3)_2$, C=O, C=S, C=Se, $C=NR^3$, $NR^3$, $P(=O)(R^3)$, $-C(=O)O-$, $-C(=O)NR^3-$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Aralkyl- oder Heteroaralkylgruppe mit 5 bis 40 aromatischen Ringatomen, die durch einen oder mehrere Reste $R^3$ substituiert sein kann, oder eine Kombination dieser Systeme; dabei können zwei oder mehrere Substituenten $R^2$ auch miteinander ein mono- oder polycyclisches, aliphatisches, heteroaliphatisches, aromatisches oder heteroaromatisches Ringsystem bilden;

$R^3$ ist bei jedem Auftreten gleich oder verschieden H, D, F oder ein aliphatischer, aromatischer und/oder heteroaromatischer organischer Rest mit 1 bis 20 C-Atomen, in dem auch H-Atome durch F ersetzt sein können; dabei können zwei oder mehrere Substituenten $R^3$ auch miteinander ein mono- oder polycyclisches, aliphatisches, heteroaliphatisches, aromatisches oder heteroaromatisches Ringsystem bilden;

mit der Maßgabe, dass o + p = 1 ist, wobei für den Fall, dass o = 1 ist, $Z^1$, $Z^2$ C darstellen und $Z^3$, $Z^4$ Y darstellen, und für den Fall, dass p = 1 ist, $Z^1$, $Z^2$ Y darstellen und $Z^3$, $Z^4$ C darstellen; und
mit der Maßgabe, dass q + r = 1 ist, wobei für den Fall, dass q = 1 ist, $Z^5$, $Z^6$ C darstellen und $Z^7$, $Z^8$ Y darstellen, und für den Fall, dass r = 1 ist, $Z^5$, $Z^6$ Y darstellen und $Z^7$, $Z^8$ C darstellen;
weiterhin sind für o bzw. p = 0 das entsprechende Stickstoffatom und die daran gebundene Gruppen nicht vorhanden, und für q bzw. r = 0 sind das entsprechende Kohlenstoffatom und die daran gebundenen Gruppen nicht vorhanden.

**2.** Verbindung gemäß Anspruch 1, umfassend Strukturen der Formeln (IIa), (IIb), (IIc) oder (IId),

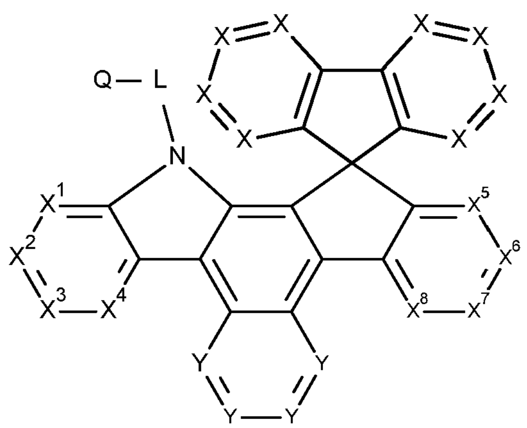

Formel (IIa)

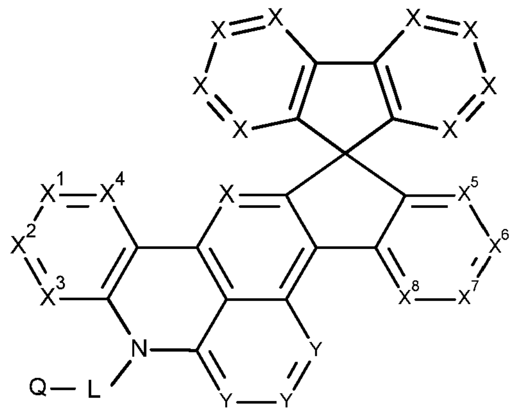

Formel (IIb)

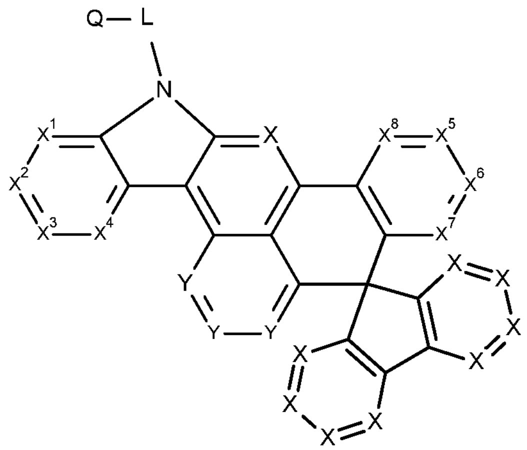

Formel (IIc)

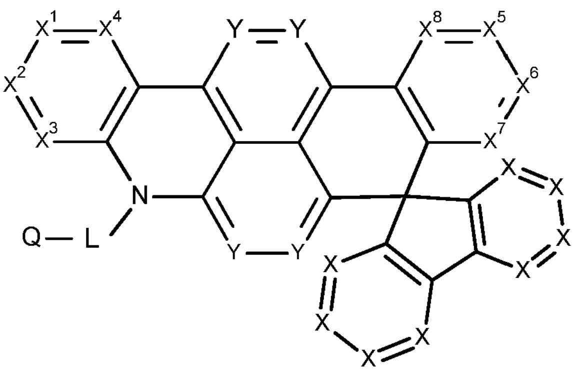

Formel (IId)

wobei die verwendeten Symbole L, Q, Y und X die in Anspruch 1 genannte Bedeutung aufweisen und $X^1$, $X^2$, $X^3$, $X^4$, $X^5$, $X^6$, $X^7$, $X^8$ gleich oder verschieden N oder $CR^1$ ist, wobei maximal zwei Gruppen X, $X^1$, $X^2$, $X^3$, $X^4$, $X^5$, $X^6$, $X^7$, $X^8$ pro Ring für N stehen.

**3.** Verbindung gemäß Anspruch 2, umfassend Strukturen der Formeln (IIIa) (IIIb), (IIIc) oder (IIId),

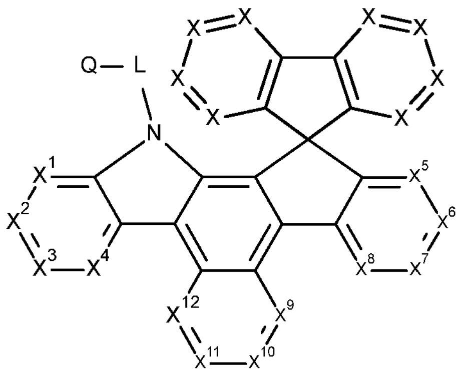

Formel (IIIa)

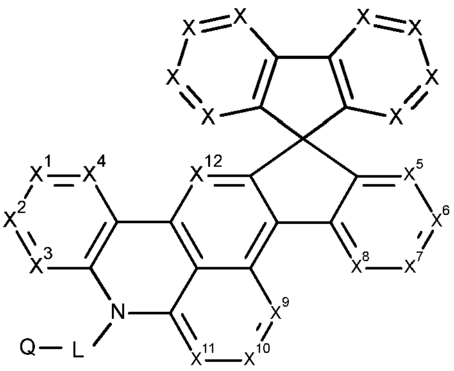

Formel (IIIb)

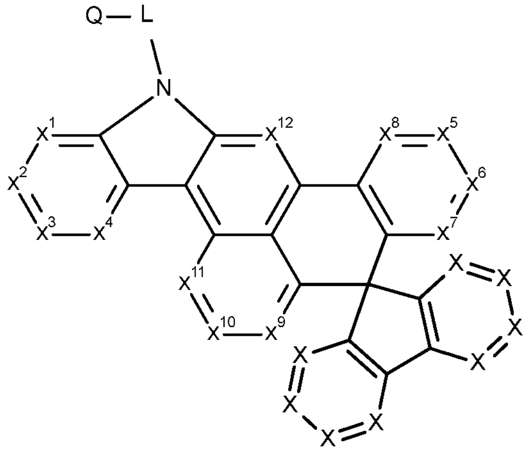

Formel (IIIc)

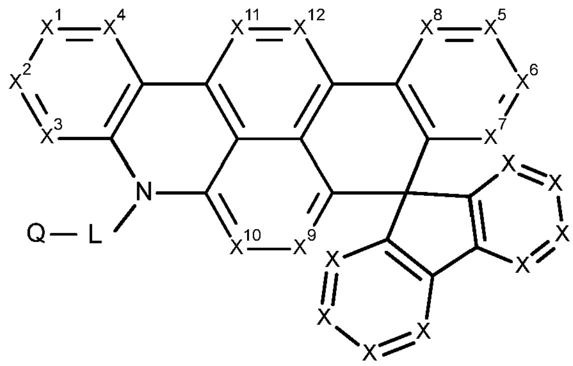

Formel (IIId)

wobei die verwendeten Symbole L, Q und X die in Anspruch 1 genannte Bedeutung aufweisen und $X^1$, $X^2$, $X^3$, $X^4$, $X^5$, $X^6$, $X^7$, $X^8$, $X^9$, $X^{10}$, $X^{11}$, $X^{12}$ gleich oder verschieden N oder $CR^1$ ist, wobei maximal zwei Gruppen X, $X^1$, $X^2$, $X^3$, $X^4$, $X^5$, $X^6$, $X^7$, $X^8$, $X^9$, $X^{10}$, $X^{11}$, $X^{12}$ pro Ring für N stehen.

**4.** Verbindung gemäß Anspruch 3, umfassend Strukturen der Formeln (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4) oder (IIId-4),

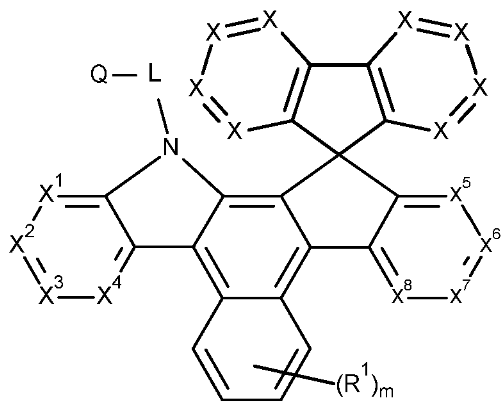

Formel (IIIa-1)

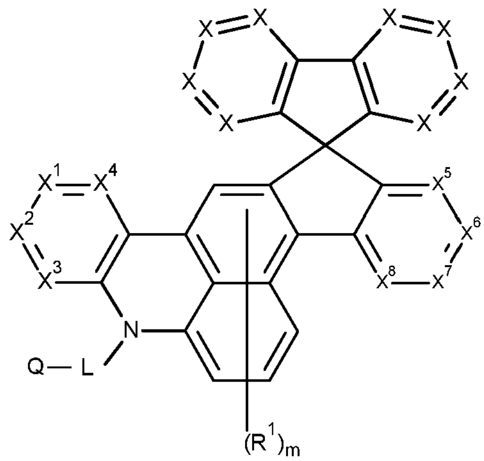

Formel (IIIb-1)

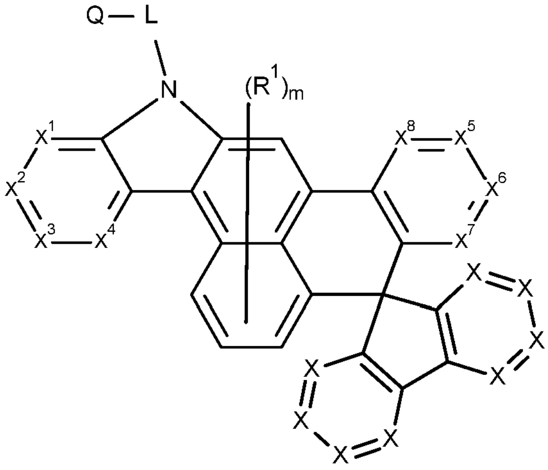

Formel (IIIc-1)

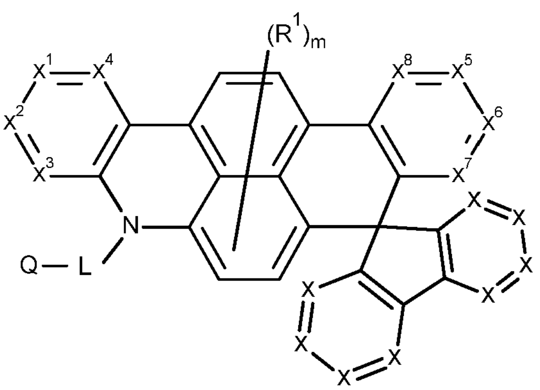

Formel (IIId-1)

wobei die verwendeten Symbole L, $R^1$, Q und X die in Anspruch 1 genannte Bedeutung aufweisen, $X^1$, $X^2$, $X^3$, $X^4$, $X^5$, $X^6$, $X^7$, $X^8$ gleich oder verschieden N oder $CR^1$ ist, und m 0, 1, 2, 3 oder 4 ist, wobei maximal zwei Gruppen X, $X^1$, $X^2$, $X^3$, $X^4$, $X^5$, $X^6$, $X^7$, $X^8$ pro Ring für N stehen;

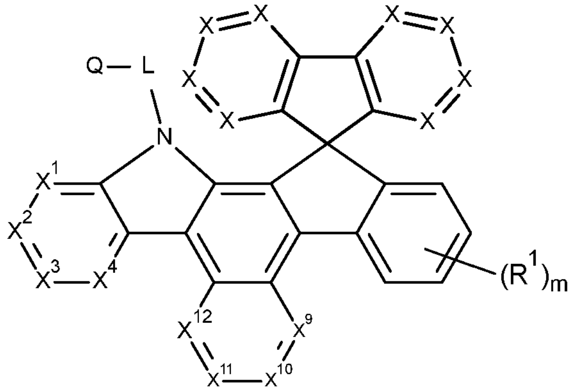

Formel (IIIa-2)

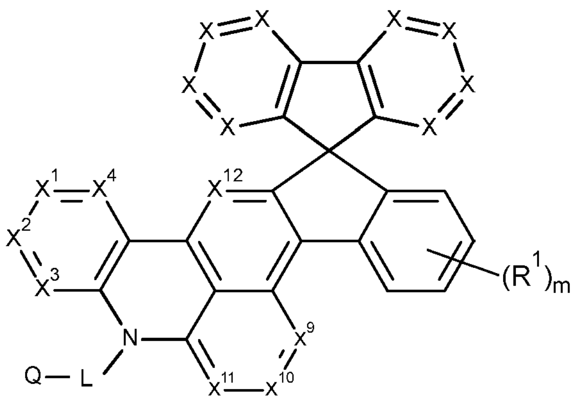

Formel (IIIb-2)

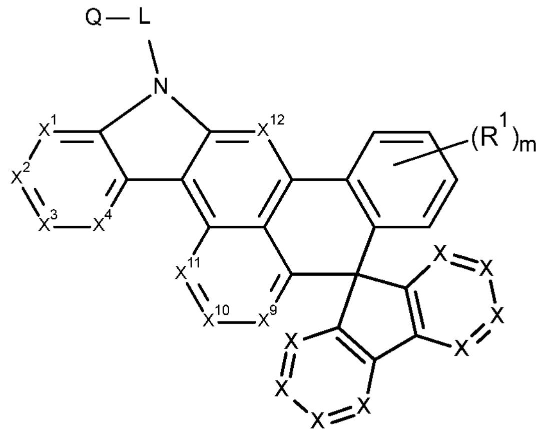

Formel (IIIc-2)

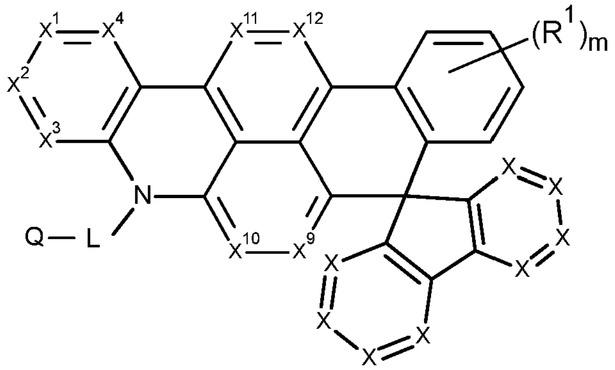

Formel (IIId-2)

wobei die verwendeten Symbole L, $R^1$, Q und X die in Anspruch 1 genannte Bedeutung aufweisen, $X^1$, $X^2$, $X^3$, $X^4$, $X^9$, $X^{10}$, $X^{11}$, $X^{12}$ gleich oder verschieden N oder $CR^1$ ist, und m 0, 1, 2, 3 oder 4 ist, wobei maximal zwei Gruppen X, $X^1$, $X^2$, $X^3$, $X^4$, $X^9$, $X^{10}$, $X^{11}$, $X^{12}$ pro Ring für N stehen;

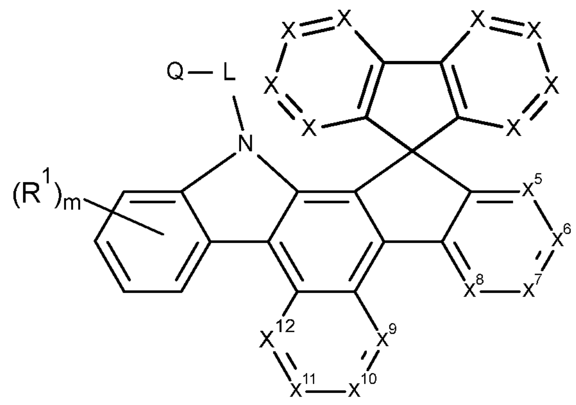

Formel (IIIa-3)

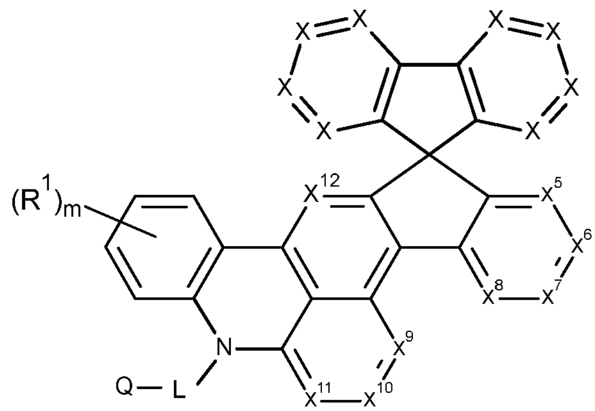

Formel (IIIb-3)

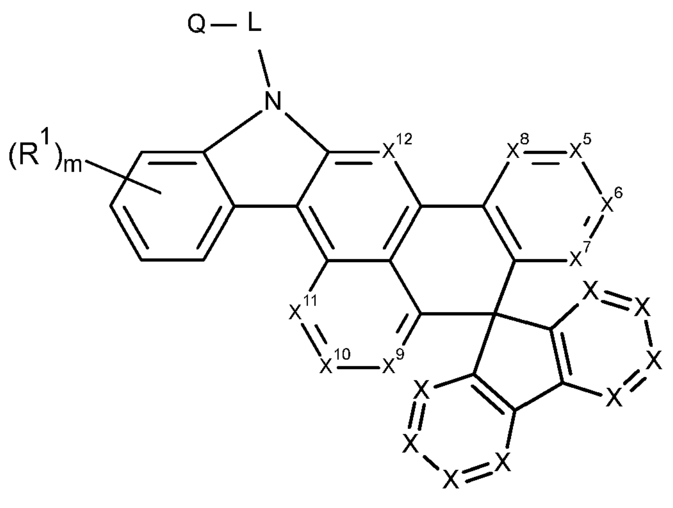

Formel (IIIc-3)

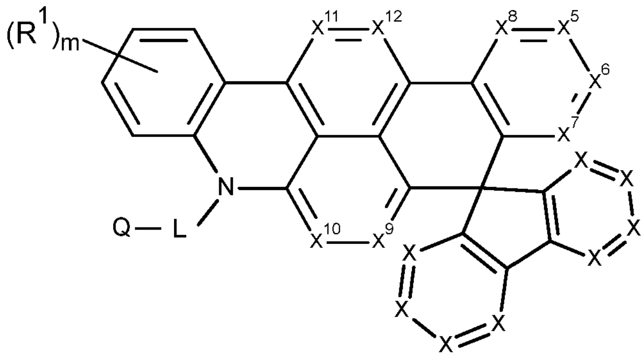

Formel (IIId-3)

wobei die verwendeten Symbole L, $R^1$, Q und X die in Anspruch 1 genannte Bedeutung aufweisen, $X^5$, $X^6$, $X^7$, $X^8$, $X^9$, $X^{10}$, $X^{11}$, $X^{12}$ gleich oder verschieden N oder $CR^1$ ist, und m 0, 1, 2, 3 oder 4 ist, wobei maximal zwei Gruppen X, $X^5$, $X^6$, $X^7$, $X^8$, $X^9$, $X^{10}$, $X^{11}$, $X^{12}$ pro Ring für N stehen;

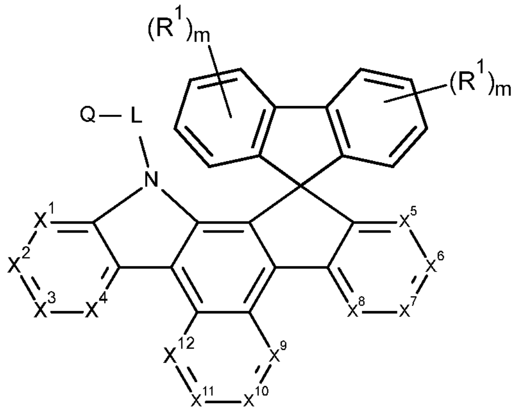

Formel (IIIa-4)

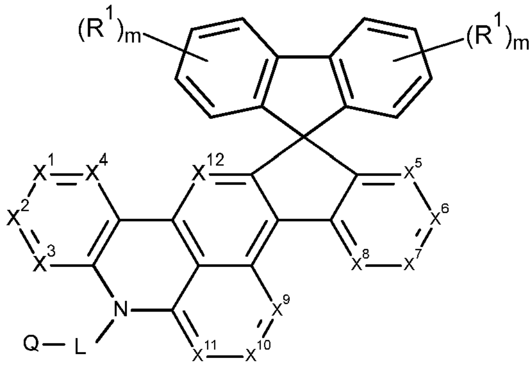

Formel (IIIb-4)

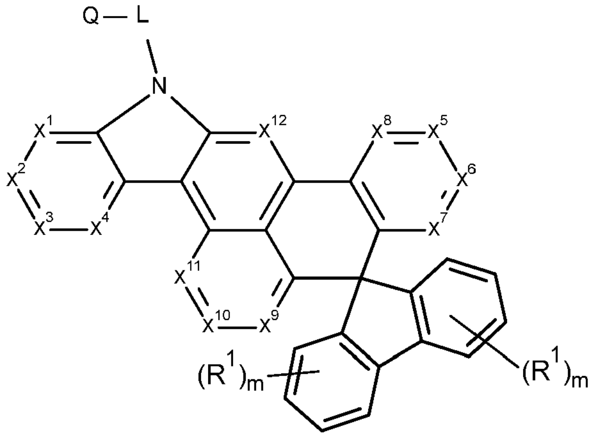

Formel (IIIc-4)

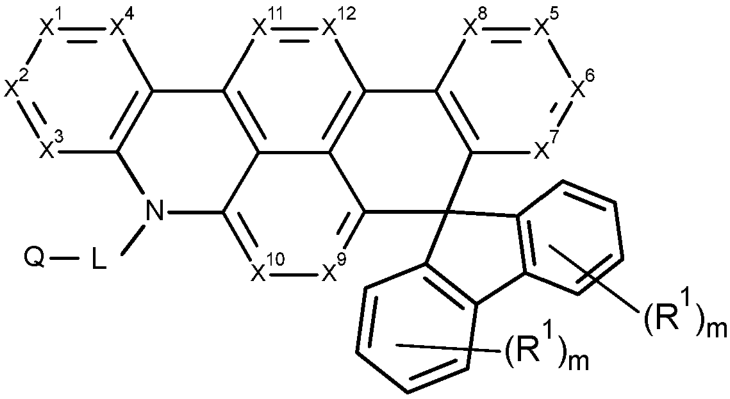

Formel (IIId-4)

wobei die verwendeten Symbole L, $R^1$ und Q die in Anspruch 1 genannte Bedeutung aufweisen, $X^1$, $X^2$, $X^3$, $X^4$, $X^5$, $X^6$, $X^7$, $X^8$, $X^9$, $X^{10}$, $X^{11}$, $X^{12}$ gleich oder verschieden N oder $CR^1$ ist, und m 0, 1, 2, 3 oder 4 ist, wobei maximal zwei Gruppen $X^1$, $X^2$, $X^3$, $X^4$, $X^5$, $X^6$, $X^7$, $X^8$, $X^9$, $X^{10}$, $X^{11}$, $X^{12}$ pro Ring für N stehen.

**5.** Verbindung gemäß Anspruch 1, umfassend Strukturen der Formeln (IVa), (IVb) oder (IVc),

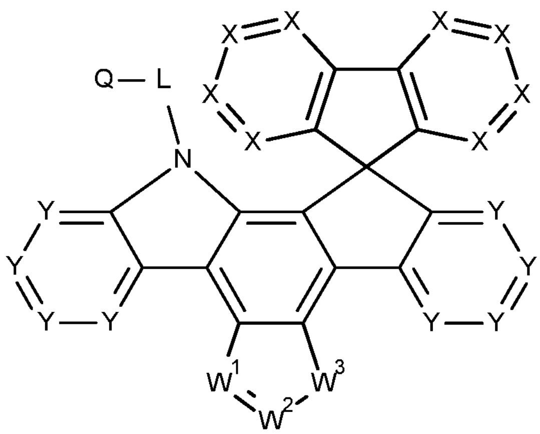

Formel (IVa)

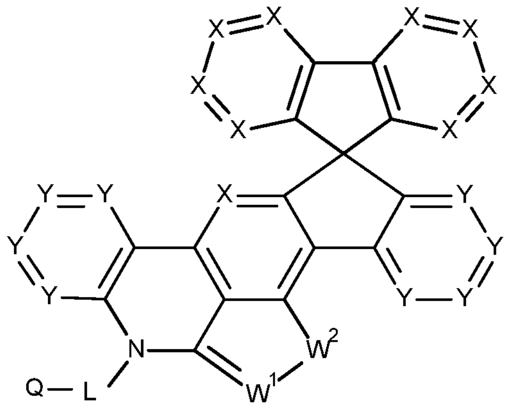

Formel (IVb)

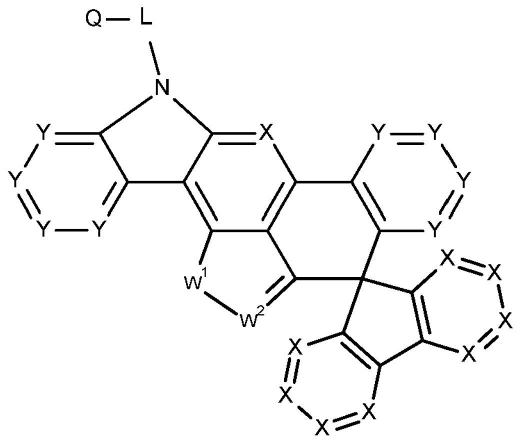

Formel (IVc)

wobei die verwendeten Symbole L, Q, X und Y die in Anspruch 1 genannte Bedeutung aufweisen und $W^1$, $W^2$ und $W^3$ bei jedem Auftreten gleich oder verschieden N, $CR^1$, O, S oder $NR^1$ ist, wobei genau eine der Gruppen $W^1$, $W^2$ und $W^3$ für O, S oder $NR^1$ steht und $W^1$ in Formel (IVb) und $W^2$ in Formel (IVc) für N oder $CR^1$ steht, wobei $R^1$ die in Anspruch 1 genannte Bedeutung aufweist.

**6.** Verbindung gemäß Anspruch 5, umfassend Strukturen der Formeln (Va), (Vb) oder (Vc),

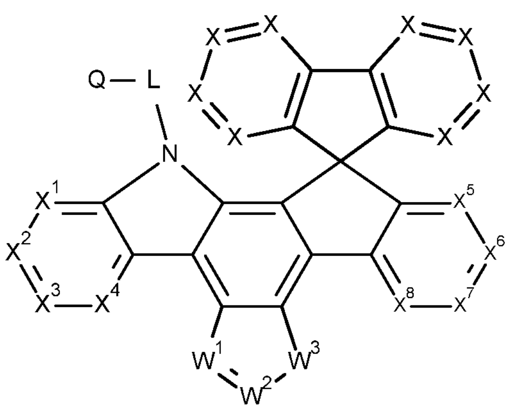

Formel (Va)

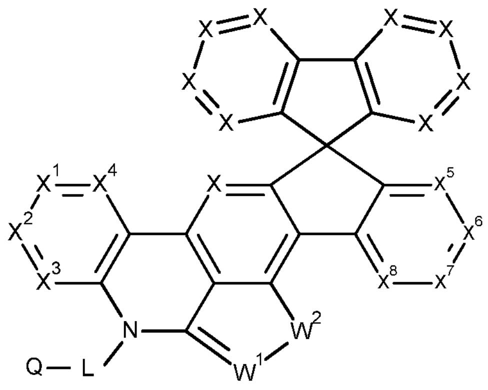

Formel (Vb)

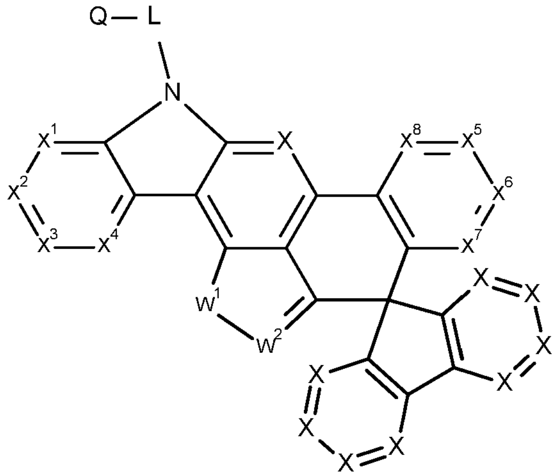

Formel (Vc)

wobei die verwendeten Symbole L, Q und X die in Anspruch 1 genannte Bedeutung, die Symbole $X^1$, $X^2$, $X^3$, $X^4$, $X^5$, $X^6$, $X^7$, $X^8$ die in Anspruch 2 genannte Bedeutung und die Symbole $W^1$, $W^2$ und $W^3$ die in Anspruch 5 genannte Bedeutung aufweisen.

**7.** Verbindung gemäß Anspruch 6, umfassend Strukturen der Formeln (Va-1), (Vb-1), (Vc-1), (Va-2), (Vb-2), (Vc-2), (Va-3), (Vb-3) oder (Vc-3)

7. Verbindung gemäß Anspruch 6, umfassend Strukturen der Formeln (Va-1), (Vb-1), (Vc-1), (Va-2), (Vb-2), (Vc-2), (Va-3), (Vb-3) oder (Vc-3),

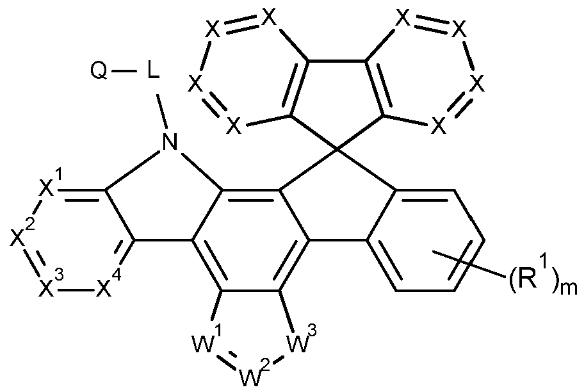

Formel (Va-1)

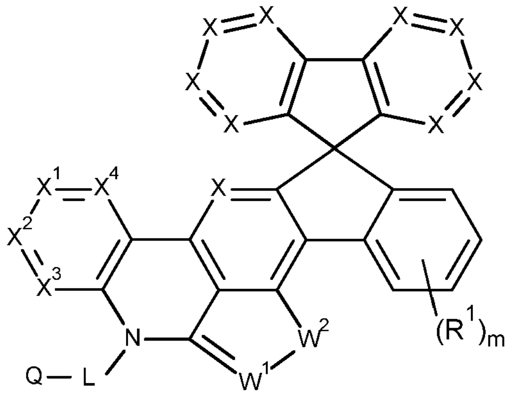

Formel (Vb-1)

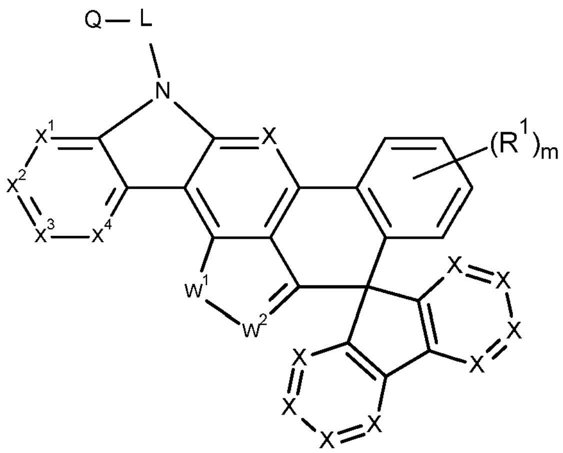

Formel (Vc-1)

wobei die verwendeten Symbole L, $R^1$, Q und X die in Anspruch 1 genannte Bedeutung, die Symbole $X^1$, $X^2$, $X^3$, $X^4$ die in Anspruch 2 genannte Bedeutung und die Symbole $W^1$, $W^2$ und $W^3$ die in Anspruch 5 genannte Bedeutung aufweisen und m 0, 1, 2, 3 oder 4 ist;

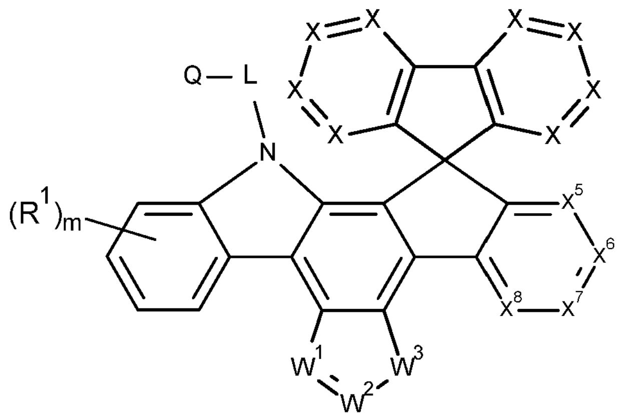

Formel (Va-2)

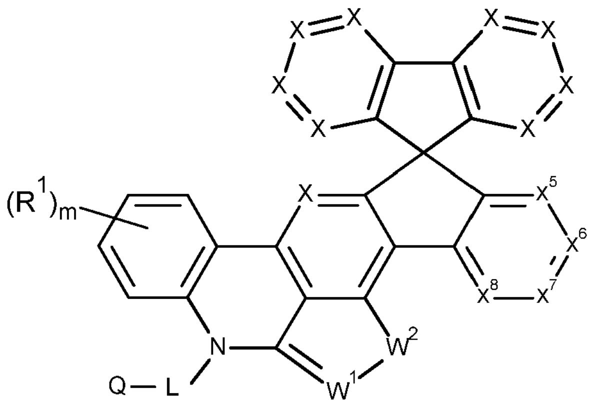

Formel (Vb-2)

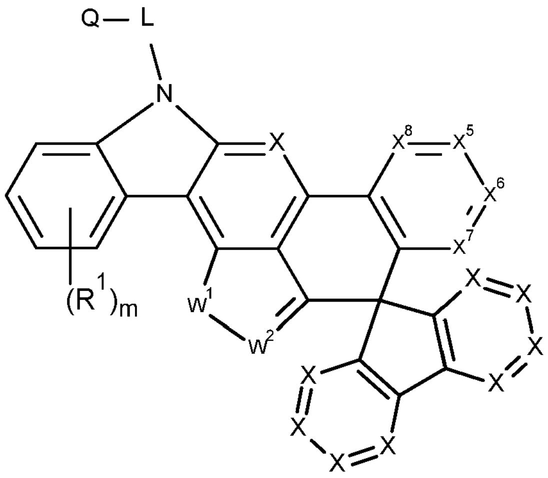

Formel (Vc-2)

wobei die verwendeten Symbole L, $R^1$, Q und X die in Anspruch 1 genannte Bedeutung, die Symbole $X^5$, $X^6$, $X^7$, $X^8$ die in Anspruch 2 genannte Bedeutung und die Symbole $W^1$, $W^2$ und $W^3$ die in Anspruch 5 genannte Bedeutung aufweisen und m 0, 1, 2, 3 oder 4 ist;

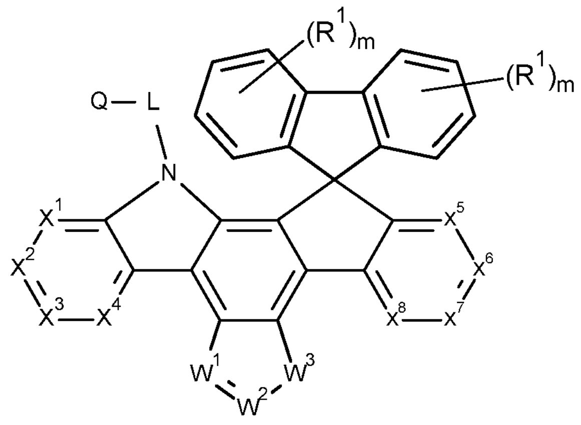

Formel (Va-3)

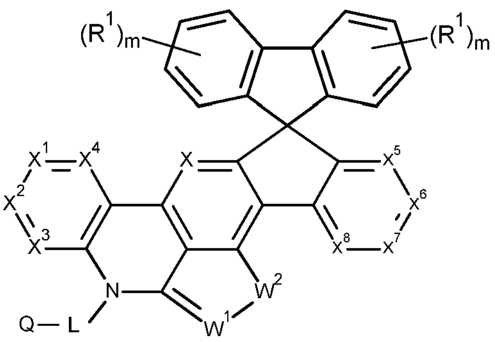

Formel (Vb-3)

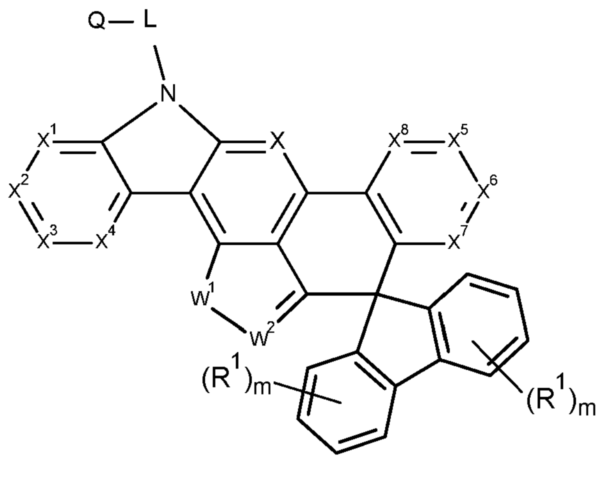

Formel (Vc-3)

wobei die verwendeten Symbole L, $R^1$, Q und X die in Anspruch 1 genannte Bedeutung, die Symbole $X^1$, $X^2$, $X^3$, $X^4$, $X^5$, $X^6$, $X^7$, $X^8$ die in Anspruch 2 genannte Bedeutung und die Symbole $W^1$, $W^2$ und $W^3$ die in Anspruch 5 genannte Bedeutung aufweisen und m 0, 1, 2, 3 oder 4 ist.

**8.** Verbindung gemäß mindestens einem der vorhergehenden Ansprüche, **dadurch gekennzeichnet, dass** die Gruppe Q ausgewählt ist aus Strukturen der Formeln (Q-1), (Q-2), (Q-3), (Q-4) oder (Q-5),

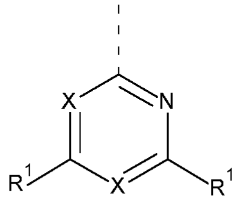

Formel (Q-1)

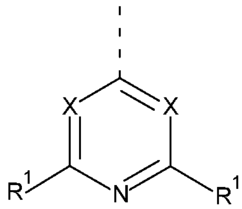

Formel (Q-2)

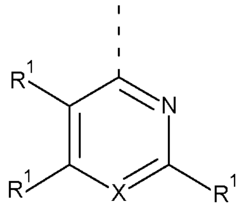

Formel (Q-3)

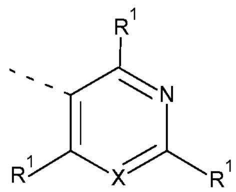

Formel (Q-4)

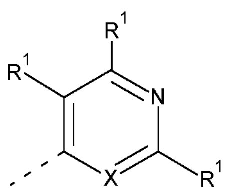

Formel (Q-5)

wobei das Symbol $R^1$ die zuvor in Anspruch 1 genannte Bedeutung aufweist, X N oder $CR^1$ ist und die gestrichelte Bindung die Anbindungsposition markiert, wobei X vorzugsweise ein Stickstoffatom darstellt.

9. Verbindung gemäß mindestens einem der vorhergehenden Ansprüche, **dadurch gekennzeichnet, dass** die Gruppe Q ausgewählt ist aus Strukturen der Formeln (Q-6), (Q-7), (Q-8), (Q-9), (Q-10), (Q-11) oder (Q-12),

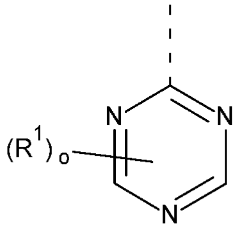

Formel (Q-6)

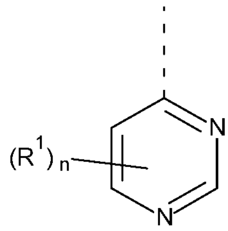

Formel (Q-7)

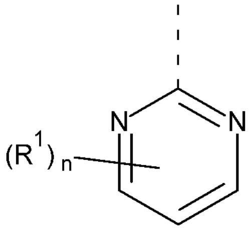

Formel (Q-8)

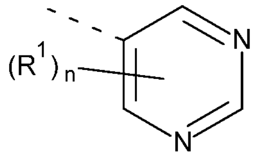

Formel (Q-9)

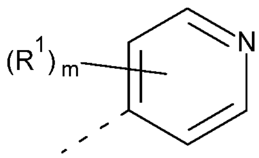

Formel (Q-10)

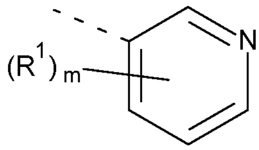

Formel (Q-11)

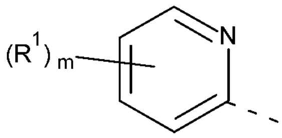

Formel (Q-12)

worin das Symbol $R^1$ die in Anspruch 1 dargelegte Bedeutung aufweist, die gestrichelte Bindung die Anbindungsposition markiert und m 0, 1, 2, 3 oder 4, n 0, 1, 2 oder 3 und o 0,1 oder 2 ist.

10. Verbindung gemäß mindestens einem der vorhergehenden Ansprüche, **dadurch gekennzeichnet, dass** die Gruppe Q ausgewählt ist aus Strukturen der Formeln (Q-16), (Q-17) oder (Q-18),

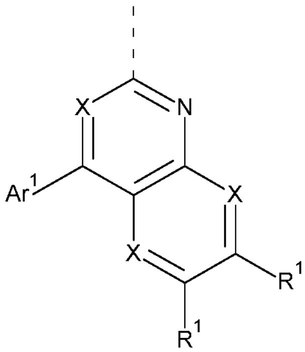

Formel (Q-16)

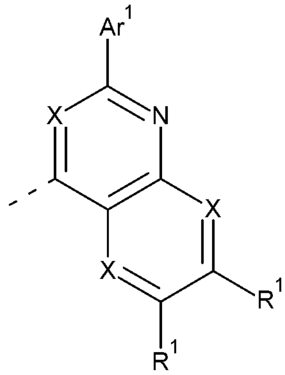

Formel (Q-17)

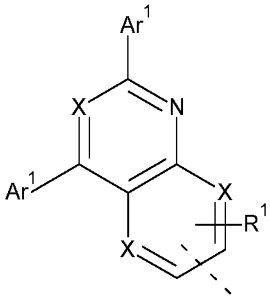

Formel (Q-18)

wobei die Symbole X und $R^1$, die zuvor in Anspruch 1 genannte Bedeutung aufweisen, die gestrichelte Bindung die Anbindungsposition markiert und $Ar^1$ ein aromatisches oder heteroaromatisches Ringsystem mit 5 bis 40 aromatischen Ringatomen, das jeweils mit einem oder mehreren Resten $R^1$ substituiert sein kann, darstellt, wobei optional zwei oder mehr benachbarte Substituenten $R^1$ ein mono- oder polycyclisches, aliphatisches, heteroaliphatisches, aromatisches oder heteroaromatisches Ringsystem bilden können, das mit einem oder mehreren Resten $R^3$ substituiert sein kann.

**11.** Oligomer, Polymer oder Dendrimer enthaltend eine oder mehrere Verbindungen nach einem der Ansprüche 1 bis 10, wobei statt eines Wasserstoffatoms oder eines Substituenten ein oder mehrere Bindungen der Verbindungen zum Polymer, Oligomer oder Dendrimer vorhanden sind.

**12.** Zusammensetzung enthaltend wenigstens eine Verbindung nach einem oder mehreren der Ansprüche 1 bis 10 oder ein Oligomer, Polymer oder Dendrimer nach Anspruch 11 und wenigstens eine weitere Verbindung ausgewählt aus der Gruppe bestehend aus fluoreszierenden Emittern, phosphoreszierenden Emittern, Hostmaterialien, Elektronentransportmaterialien, Elektroneninjektionsmaterialien, Lochleitermaterialien, Lochinjektionsmaterialien, Elektronenblockiermaterialien und Lochblockiermaterialien.

**13.** Formulierung, enthaltend mindestens eine Verbindung nach einem oder mehreren der Ansprüche 1 bis 10 oder ein Oligomer, Polymer oder Dendrimer nach Anspruch 11 oder eine Zusammensetzung nach Anspruch 12 und mindestens ein Lösemittel.

**14.** Verwendung einer Verbindung nach einem oder mehreren der Ansprüche 1 bis 10, eines Oligomers, Polymers oder Dendrimers nach Anspruch 11 oder einer Zusammensetzung nach Anspruch 12 in einer elektronischen Vorrichtung, insbesondere als Hostmaterial oder Elektronentransportmaterial.

**15.** Elektronische Vorrichtung, enthaltend mindestens eine Verbindung nach einem oder mehreren der Ansprüche 1 bis 10, ein Oligomer, Polymer oder Dendrimer nach Anspruch 11 oder eine Zusammensetzung gemäß Anspruch 12, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden.

## Claims

**1.** Compound comprising structures of the formula (I):

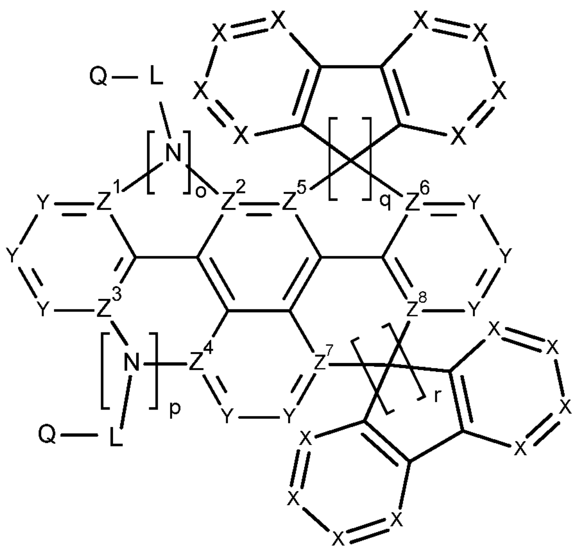

formula (I)

where the following applies to the symbols used:

$Z^1$, $Z^2$, $Z^3$, $Z^4$, $Z^5$, $Z^6$, $Z^7$, $Z^8$ are, identically or differently, Y or C;
Y is on each occurrence, identically or differently, N, $CR^1$, or two adjacent groups Y together stand for O, S, $NR^1$, with the proviso that a 5- or 6-membered ring is formed;
X is on each occurrence, identically or differently, N or $CR^1$;
o, p, q, r are 0 or 1;
L is a bond or an aromatic or heteroaromatic ring system having 5 to 24 aromatic ring atoms, which may be substituted by one or more radicals $R^1$;
Q is an electron-transport group, selected from an optionally substituted five-membered heteroaryl ring group which contains at least two heteroatoms, or an optionally substituted six-membered heteroaryl ring group, where the groups may also be part of an aromatic ring system and where further aryl or heteroaryl groups may also be condensed onto these groups;

$R^1$ is on each occurrence, identically or differently, H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $NO_2$, $P(=O)(R^2)_2$, $OSO_2R^2$, $OR^2$, $S(=O)R^2$, $S(=O)_2R^2$, a straight-chain alkyl, alkoxy or thioalkoxy group having 1 to 40 C atoms or a branched or cyclic alkyl, alkoxy or thioalkoxy group having 3 to 40 C atoms, where one or more non-adjacent $CH_2$ groups may be replaced by $-R^2C=CR^2-$, -C≡C-, $Si(R^2)_2$, $Ge(R^2)_2$, $Sn(R^2)_2$, C=O, C=S, C=Se, $C=NR^2$, $NR^2$, $P(=O)(R^2)$, -C(=O)O-, $-C(=O)NR^2-$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^2$, or an aryloxy or heteroaryloxy group having 5 to 40 aromatic ring atoms, which may be substituted by one or more radicals $R^2$, or an aralkyl or heteroaralkyl group having 5 to 40 aromatic ring atoms, which may be substituted by one or more radicals $R^2$, or a combination of these systems; two or more radicals $R^1$ may form a ring system with one another;

$R^2$ is on each occurrence, identically or differently, H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $NO_2$, $P(=O)(R^3)_2$, $OSO_2R^3$, $OR^3$, $S(=O)R^3$, $S(=O)_2R^3$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^3$, where one or more non-adjacent $CH_2$ groups may be replaced by $-R^3C=CR^3-$, -C≡C-, $Si(R^3)_2$, $Si(R^3)_2$, $Ge(R^3)_2$, $Sn(R^3)_2$, C=O, C=S, C=Se, $C=NR^3$, $NR^3$, $P(=O)(R^3)$, -C(=O)O-, $-C(=O)NR^3-$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^3$, or an aryloxy or heteroaryloxy group having 5 to 40 aromatic ring atoms, which may be substituted by one or more radicals $R^3$, or an aralkyl or heteroaralkyl group having 5 to 40 aromatic ring atoms, which may be substituted by one or more radicals $R^3$, or a combination of these systems; two or more substituents $R^2$ may also form a mono- or polycyclic, aliphatic, heteroaliphatic, aromatic or heteroaromatic ring system with one another;

$R^3$ is on each occurrence, identically or differently, H, D, F or an aliphatic, aromatic and/or heteroaromatic organic radical having 1 to 20 C atoms, in which, in addition, H atoms may be replaced by F; two or more substituents $R^3$ may also form a mono- or polycyclic, aliphatic, heteroaliphatic, aromatic or heteroaromatic ring system with one another;

with the proviso that o + p = 1, where, in the case where o = 1, $Z^1$, $Z^2$ represent C and $Z^3$, $Z^4$ represent Y, and, in the case where p = 1, $Z^1$, $Z^2$ represent Y and $Z^3$, $Z^4$ represent C; and
with the proviso that q + r = 1 is, where, in the case where q = 1, $Z^5$, $Z^6$ represent C and $Z^7$, $Z^8$ represent Y, and, in the case where r = 1, $Z^5$, $Z^6$ represent Y and $Z^7$, $Z^8$ represent C;
furthermore, for o or p = 0, the corresponding nitrogen atom and the groups bonded thereto are not present, and for q or r = 0, the corresponding carbon atom and the groups bonded thereto are not present.

2. Compound according to Claim 1, comprising structures of the formulae (IIa), (IIb), (IIc) or (IId),

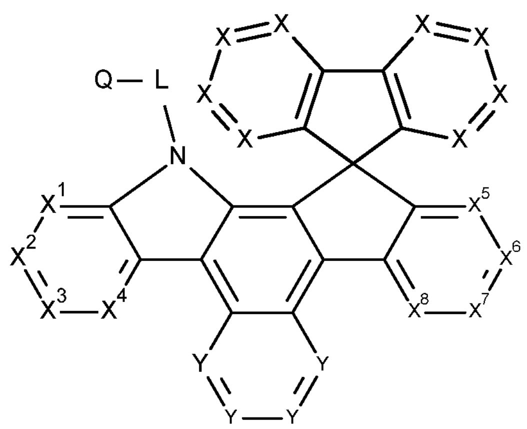

formula (IIa)

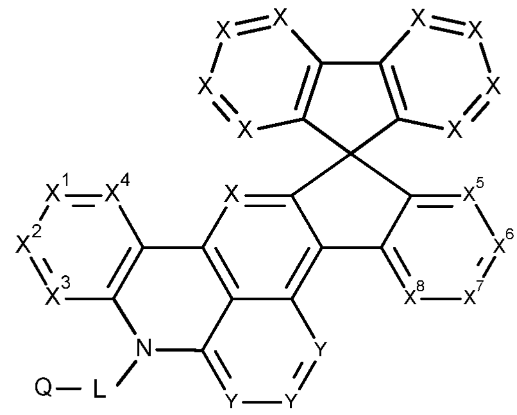

formula (IIb)

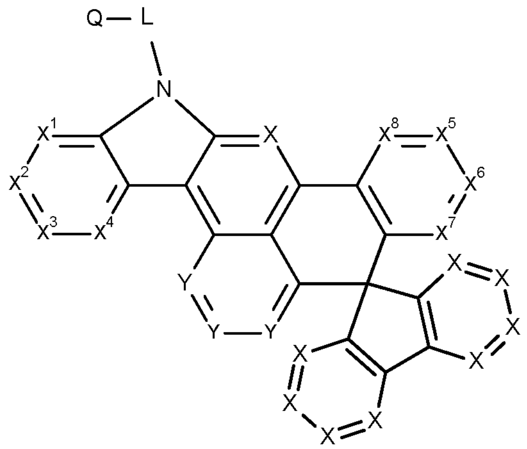

formula (IIc)

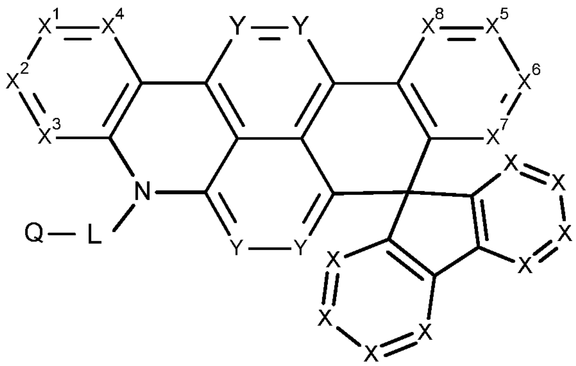

formula (IId)

where the symbols L, Q, Y and X used have the meaning given in Claim 1 and $X^1$, $X^2$, $X^3$, $X^4$, $X^5$, $X^6$, $X^7$, $X^8$ are, identically or differently, N or $CR^1$, where a maximum of two groups X, $X^1$, $X^2$, $X^3$, $X^4$, $X^5$, $X^6$, $X^7$, $X^8$ per ring stand for N.

**3.** Compound according to Claim 2, comprising structures of the formulae (IIIa), (IIIb), (IIIc) or (IIId),

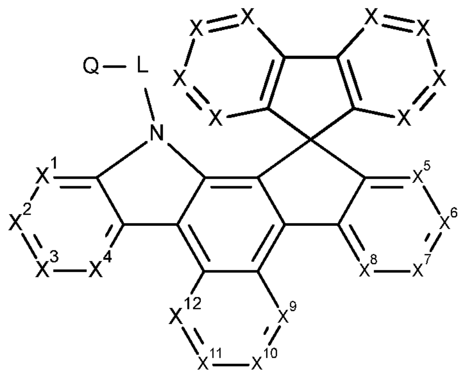

formula (IIIa)

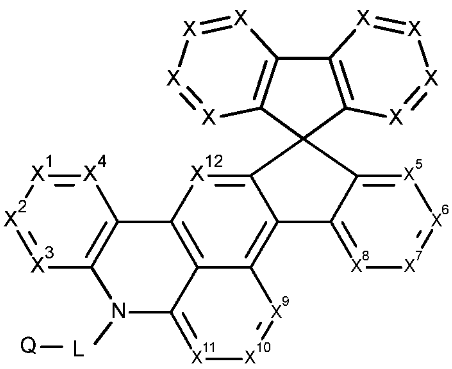

formula (IIIb)

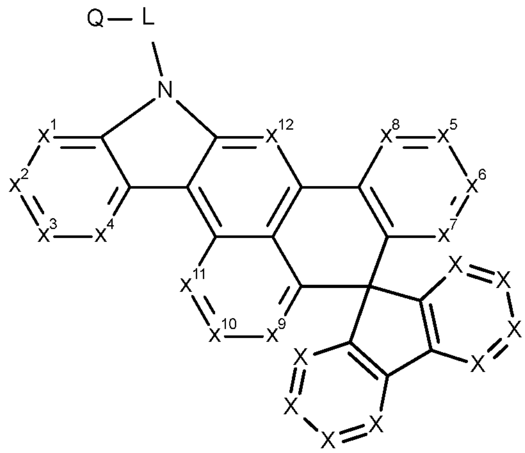

formula (IIIc)

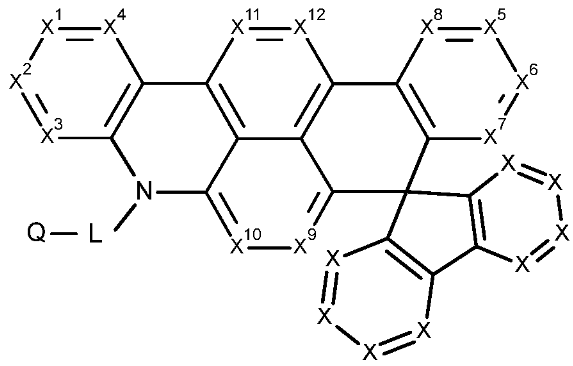

formula (IIId)

where the symbols L, Q and X used have the meaning given in Claim 1 and $X^1$, $X^2$, $X^3$, $X^4$, $X^5$, $X^6$, $X^7$, $X^8$, $X^9$, $X^{10}$, $X^{11}$, $X^{12}$ are, identically or differently, N or $CR^1$, where a maximum of two groups X, $X^1$, $X^2$, $X^3$, $X^4$, $X^5$, $X^6$, $X^7$, $X^8$, $X^9$, $X^{10}$, $X^{11}$, $X^{12}$ per ring stand for N.

**4.** Compound according to Claim 3, comprising structures of the formulae (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4) or (IIId-4),

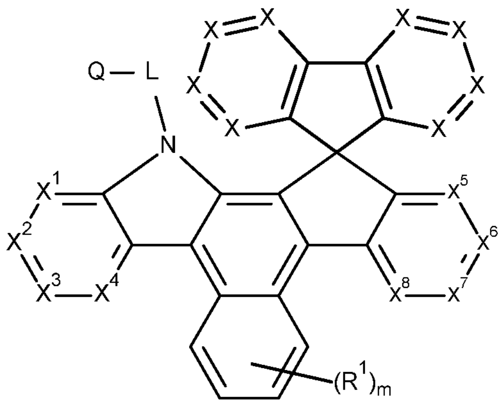

formula (IIIa-1)

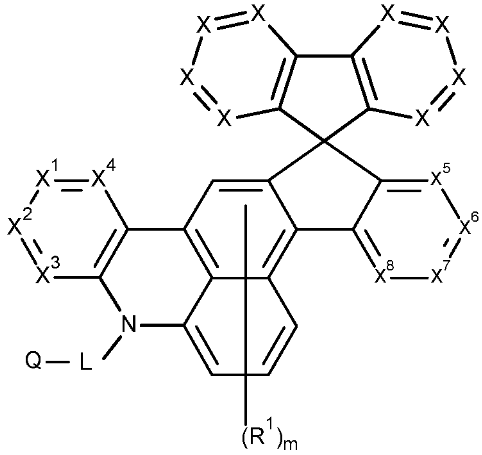

formula (IIIb-1)

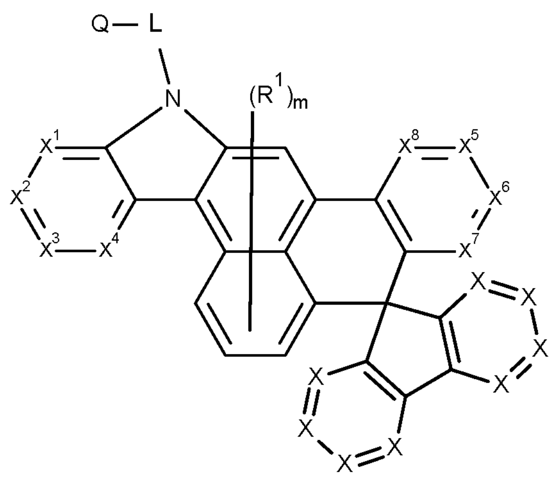

formula (IIIc-1)

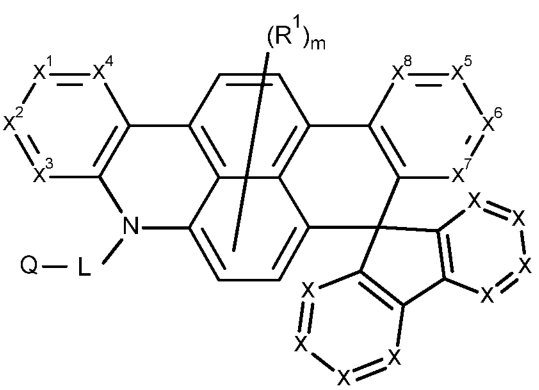

formula (IIId-1)

where the symbols L, $R^1$, Q and X used have the meaning given in Claim 1, $X^1$, $X^2$, $X^3$, $X^4$, $X^5$, $X^6$, $X^7$, $X^8$ are, identically or differently, N or $CR^1$, and m is 0, 1, 2, 3 or 4, where a maximum of two groups X, $X^1$, $X^2$, $X^3$, $X^4$, $X^5$, $X^6$, $X^7$, $X^8$ per ring stand for N;

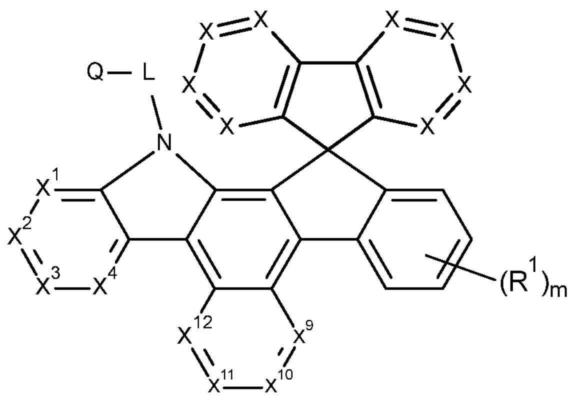

formula (IIIa-2)

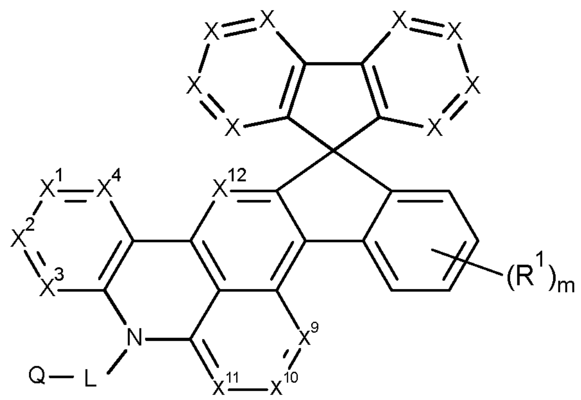

formula (IIIb-2)

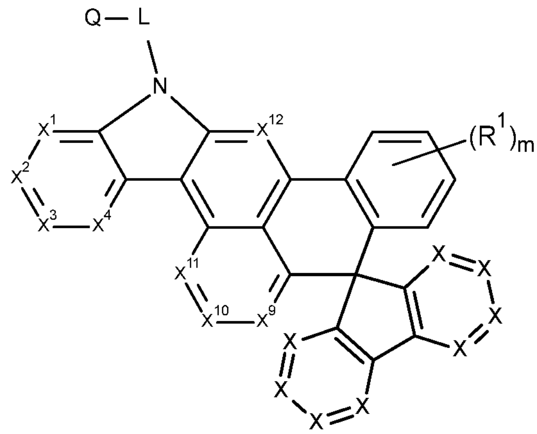

formula (IIIc-2)

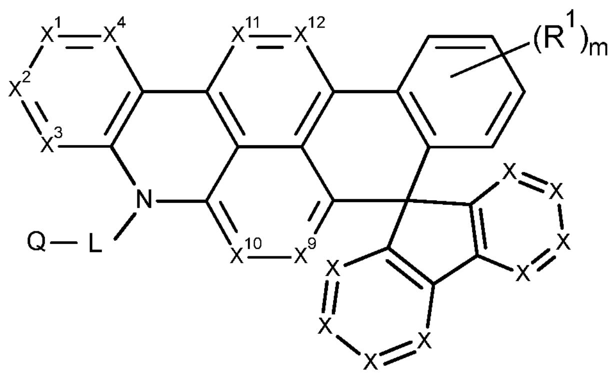

formula (IIId-2)

where the symbols L, $R^1$, Q and X used have the meaning given in Claim 1, $X^1$, $X^2$, $X^3$, $X^4$, $X^9$, $X^{10}$, $X^{11}$, $X^{12}$ are, identically or differently, N or $CR^1$, and m is 0, 1, 2, 3 or 4, where a maximum of two groups X, $X^1$, $X^2$, $X^3$, $X^4$, $X^9$, $X^{10}$, $X^{11}$, $X^{12}$ per ring stand for N;

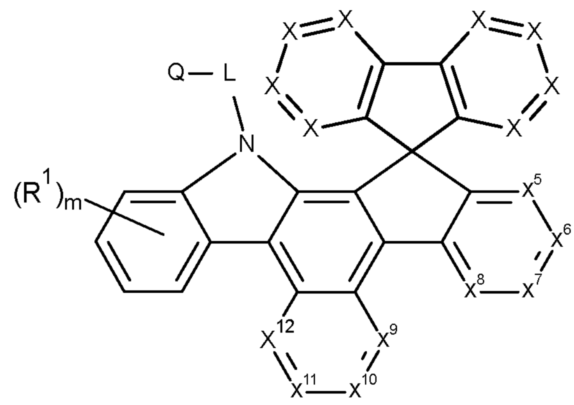

formula (IIIa-3)

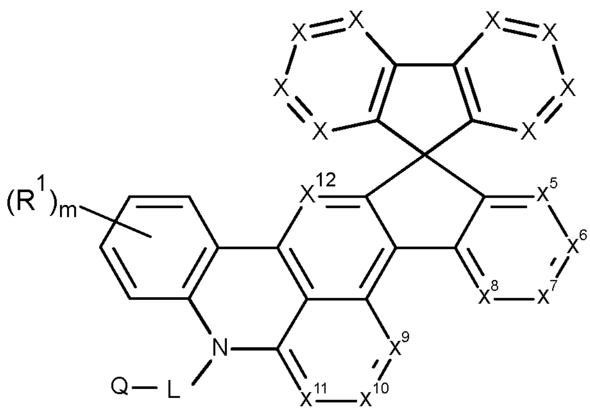

formula (IIIb-3)

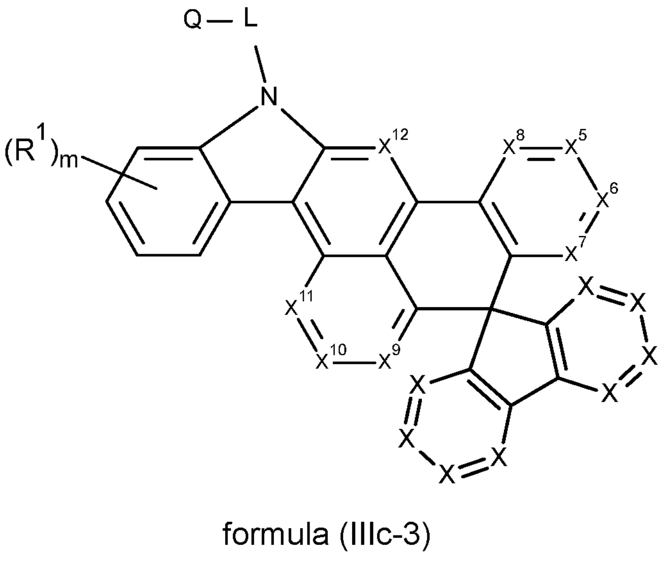

formula (IIIc-3)

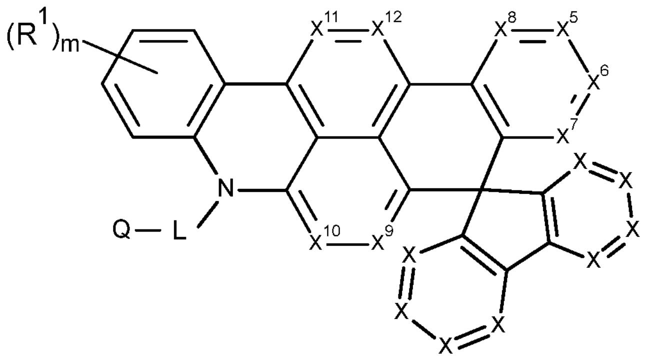

formula (IIId-3)

where the symbols L, $R^1$, Q and X used have the meaning given in Claim 1, $X^5$, $X^6$, $X^7$, $X^8$, $X^9$, $X^{10}$, $X^{11}$, $X^{12}$ are, identically or differently, N or $CR^1$, and m is 0, 1, 2, 3 or 4, where a maximum of two groups X, $X^5$, $X^6$, $X^7$, $X^8$, $X^9$, $X^{10}$, $X^{11}$, $X^{12}$ per ring stand for N;

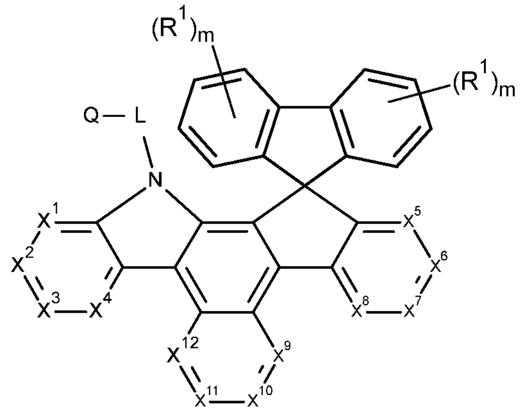

formula (IIIa-4)

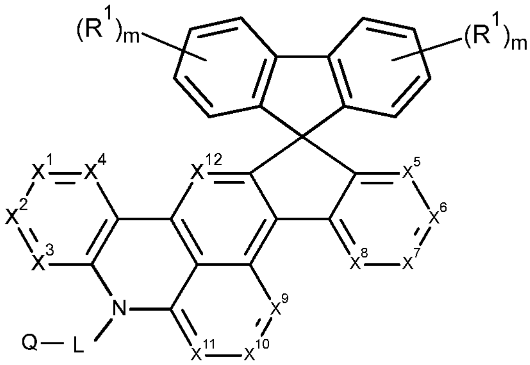

formula (IIIb-4)

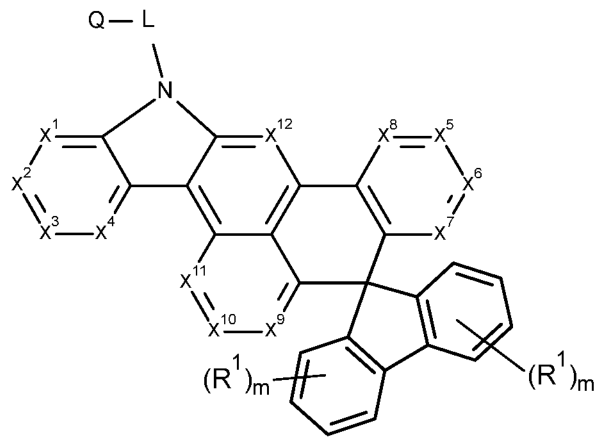

formula (IIIc-4)

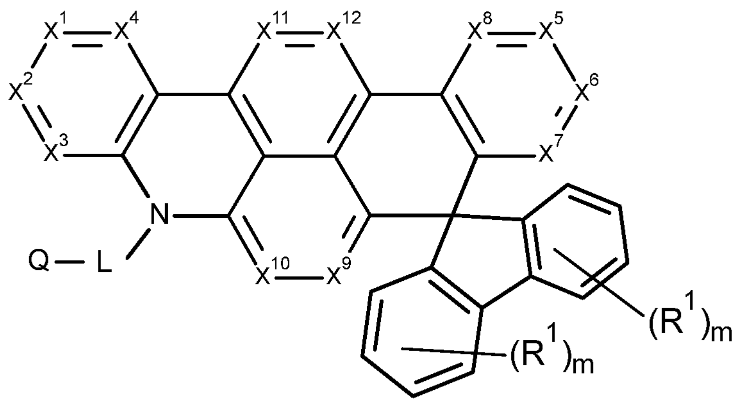

formula (IIId-4)

where the symbols L, $R^1$ and Q used have the meaning given in Claim 1, $X^1$, $X^2$, $X^3$, $X^4$, $X^5$, $X^6$, $X^7$, $X^8$, $X^9$, $X^{10}$, $X^{11}$, $X^{12}$ are, identically or differently, N or $CR^1$, and m is 0, 1, 2, 3 or 4, where a maximum of two groups $X^1$, $X^2$, $X^3$, $X^4$, $X^5$, $X^6$, $X^7$, $X^8$, $X^9$, $X^{10}$, $X^{11}$, $X^{12}$ per ring stand for N.

**5.** Compound according to Claim 1, comprising structures of the formulae (IVa), (IVb) or (IVc),

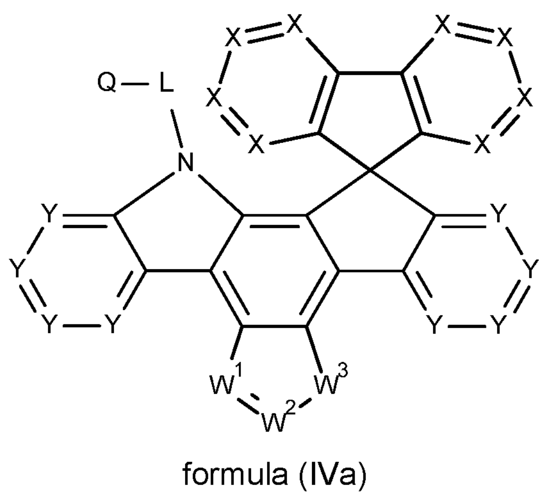

formula (IVa)

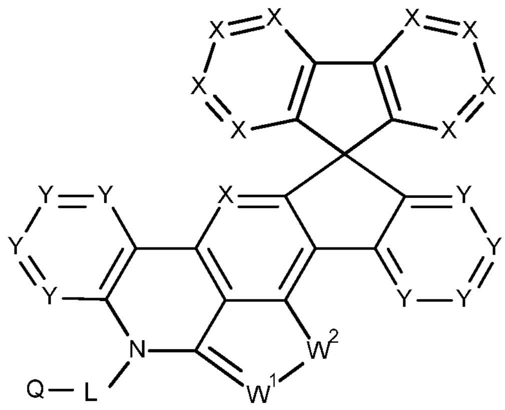

formula (IVb)

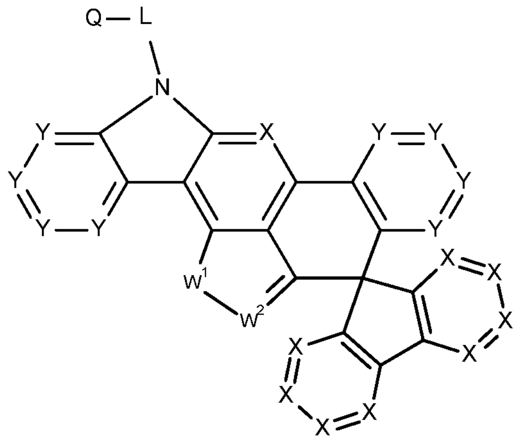

formula (IVc)

where the symbols L, Q, X and Y used have the meaning given in Claim 1 and $W^1$, $W^2$ and $W^3$ are on each occurrence, identically or differently, N, $CR^1$, O, S or $NR^1$, where precisely one of the groups $W^1$, $W^2$ and $W^3$ stands for O, S or $NR^1$ and $W^1$ in formula (IVb) and $W^2$ in formula (IVc) stand for N or $CR^1$, where $R^1$ has the meaning given in Claim 1.

6. Compound according to Claim 5, comprising structures of the formulae (Va), (Vb) or (Vc),

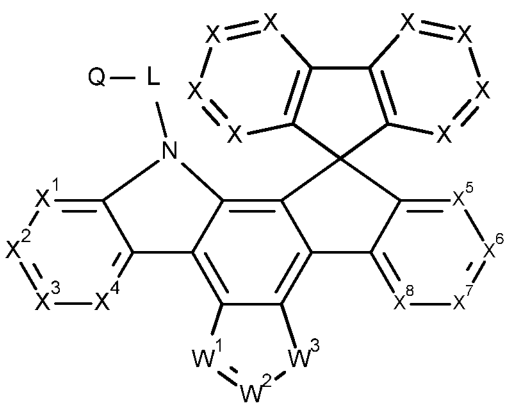

formula (Va)

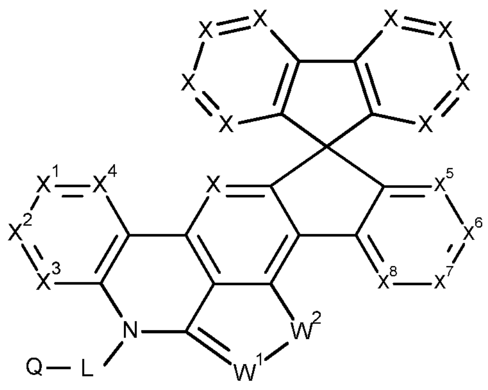

formula (Vb)

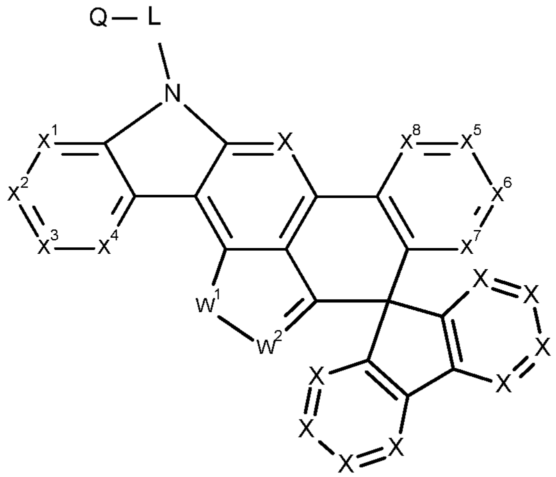

formula (Vc)

where the symbols L, Q and X used have the meaning given in Claim 1, the symbols $X^1$, $X^2$, $X^3$, $X^4$, $X^5$, $X^6$, $X^7$, $X^8$ have the meaning given in Claim 2 and the symbols $W^1$, $W^2$ and $W^3$ have the meaning given in Claim 5.

7. Compound according to Claim 6, comprising structures of the formulae (Va-1), (Vb-1), (Vc-1), (Va-2), (Vb-2), (Vc-2), (Va-3), (Vb-3) or (Vc-3),

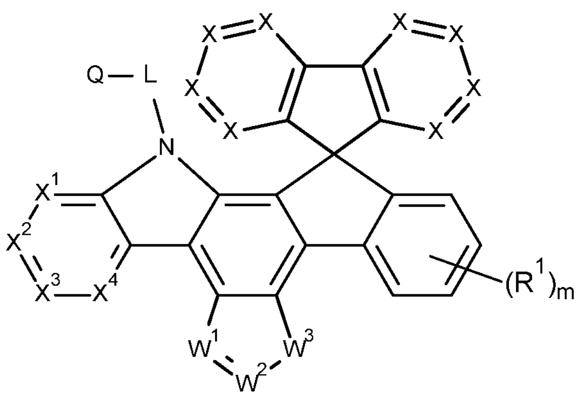

formula (Va-1)

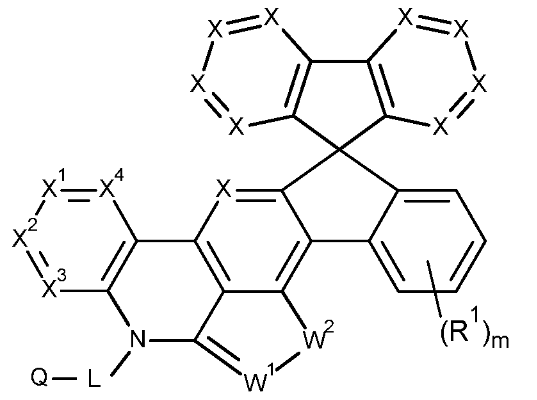

formula (Vb-1)

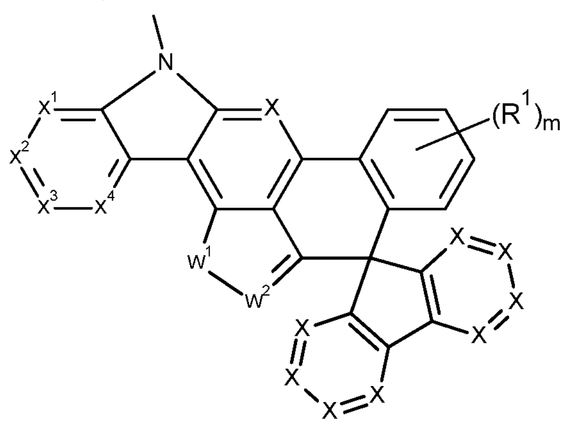

formula (Vc-1)

where the symbols L, $R^1$, Q and X used have the meaning given in Claim 1, the symbols $X^1$, $X^2$, $X^3$, $X^4$ have the meaning given in Claim 2 and the symbols $W^1$, $W^2$ and $W^3$ have the meaning given in Claim 5 and m is 0, 1, 2, 3 or 4;

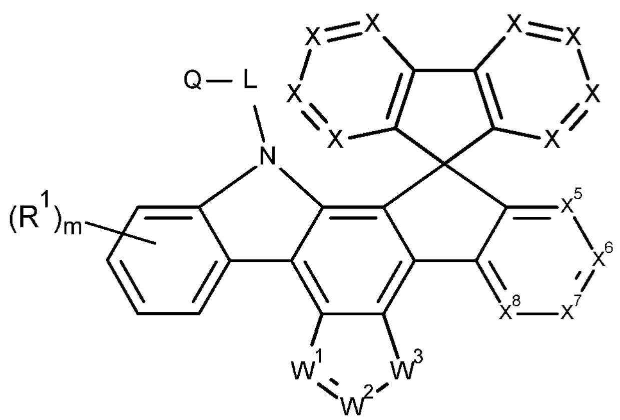

formula (Va-2)

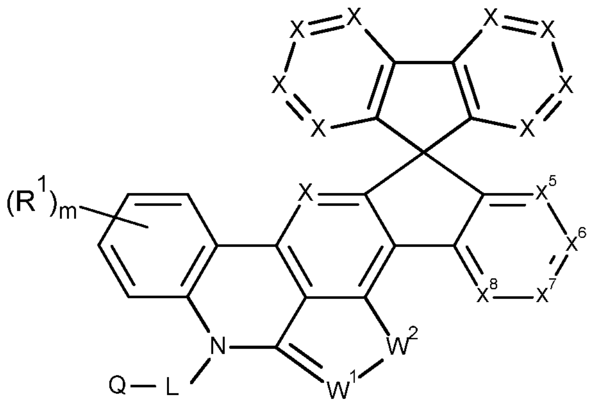

formula (Vb-2)

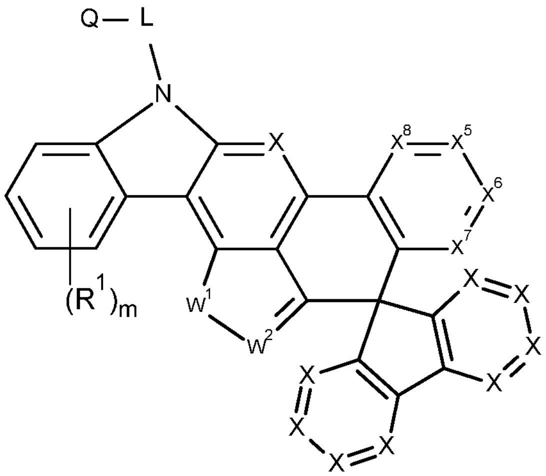

formula (Vc-2)

where the symbols L, $R^1$, Q and X used have the meaning given in Claim 1, the symbols $X^5$, $X^6$, $X^7$, $X^8$ have the meaning given in Claim 2 and the symbols $W^1$, $W^2$ and $W^3$ have the meaning given in Claim 5 and m is 0, 1, 2, 3 or 4;

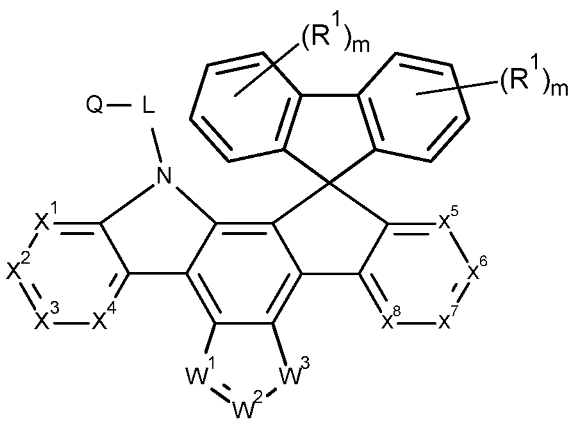

formula (Va-3)

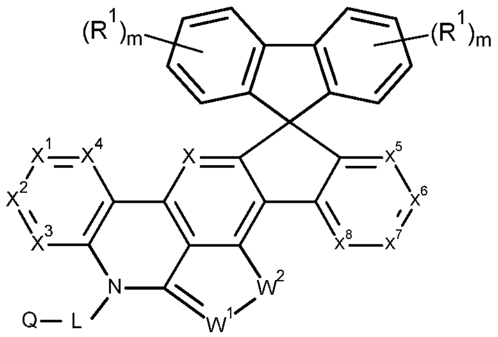

formula (Vb-3)

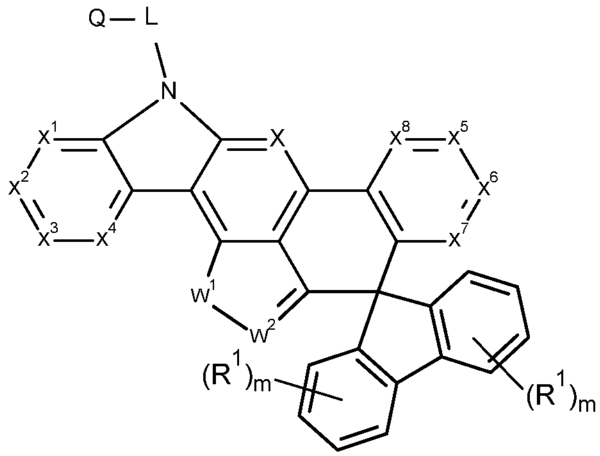

formula (Vc-3)

where the symbols L, $R^1$, Q and X used have the meaning given in Claim 1, the symbols $X^1$, $X^2$, $X^3$, $X^4$, $X^5$, $X^6$, $X^7$, $X^8$ have the meaning given in Claim 2 and the symbols $W^1$, $W^2$ and $W^3$ have the meaning given in Claim 5 and m is 0, 1, 2, 3 or 4.

**8.** Compound according to at least one of the preceding claims, **characterised in that** the group Q is selected from structures of the formulae (Q-1), (Q-2), (Q-3), (Q-4) or (Q-5),

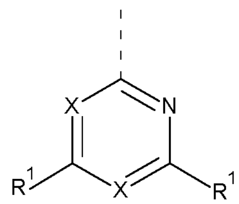

formula (Q-1)

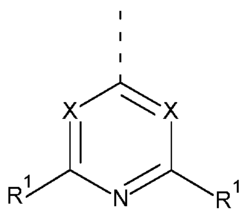

formula (Q-2)

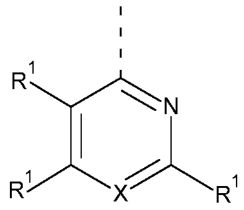

formula (Q-3)

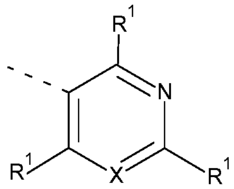

formula (Q-4)

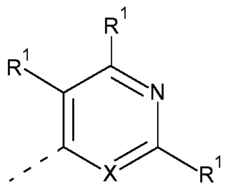

formula (Q-5)

where the symbol $R^1$ has the meaning given above in Claim 1, X is N or $CR^1$ and the dashed bond marks the bonding position, where X preferably represents a nitrogen atom.

9. Compound according to at least one of the preceding claims, **characterised in that** the group Q is selected from structures of the formulae (Q-6), (Q-7), (Q-8), (Q-9), (Q-10), (Q-11) or (Q-12),

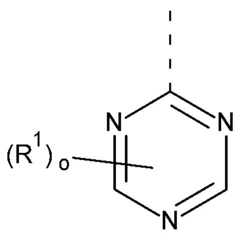

formula (Q-6)

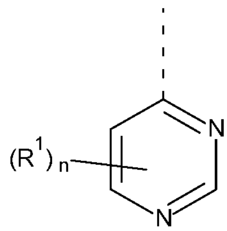

formula (Q-7)

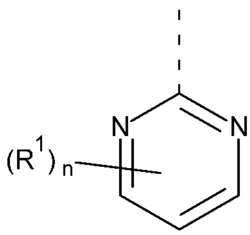

formula (Q-8)

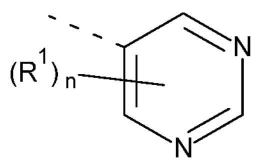

formula (Q-9)

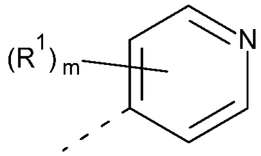

formula (Q-10)

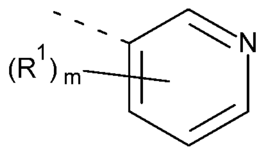

formula (Q-11)

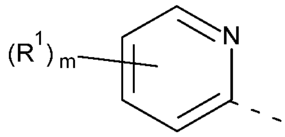

formula (Q-12)

in which the symbol $R^1$ has the meaning described in Claim 1, the dashed bond marks the bonding position and m is 0, 1, 2, 3 or 4, n is 0, 1, 2 or 3 and o is 0, 1 or 2.

**10.** Compound according to at least one of the preceding claims, **characterised in that** the group Q is selected from structures of the formulae (Q-16), (Q-17) or (Q-18),

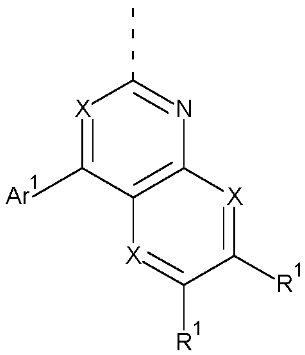

formula (Q-16)

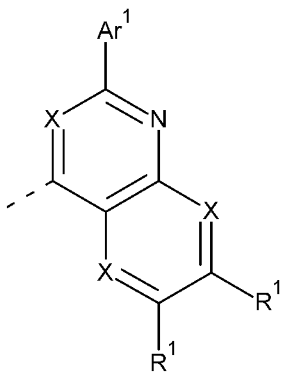

formula (Q-17)

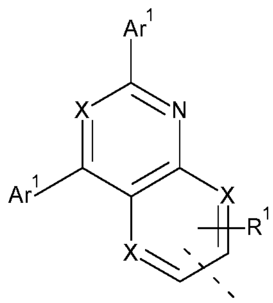

formula (Q-18)

where the symbols X and $R^1$ have the meaning given above in Claim 1, the dashed bond marks the bonding position and $Ar^1$ represents an aromatic or heteroaromatic ring system having 5 to 40 aromatic ring atoms, which may in each case be substituted by one or more radicals $R^1$, where two or more adjacent substituents $R^1$ may optionally form a mono- or polycyclic, aliphatic, heteroaliphatic, aromatic or heteroaromatic ring system, which may be substituted by one or more radicals R3.

**11.** Oligomer, polymer or dendrimer comprising one or more compounds according to one of Claims 1 to 10, where, instead of a hydrogen atom or a substituent, one or more bonds are present from the compounds to the polymer, oligomer or dendrimer.

**12.** Composition comprising at least one compound according to one or more of Claims 1 to 10 or an oligomer, polymer or dendrimer according to Claim 11 and at least one further compound selected from the group consisting of fluorescent emitters, phosphorescent emitters, host materials, electron-transport materials, electron-injection materials, hole-conductor materials, hole-injection materials, electron-blocking materials and hole-blocking materials.

**13.** Formulation comprising at least one compound according to one or more of Claims 1 to 10 or an oligomer, polymer or dendrimer according to Claim 11 or a composition according to Claim 12 and at least one solvent.

**14.** Use of a compound according to one or more of Claims 1 to 10, an oligomer, polymer or dendrimer according to Claim 11 or a composition according to Claim 12 in an electronic device, in particular as host material or electron-transport material.

**15.** Electronic device containing at least one compound according to one or more of Claims 1 to 10, an oligomer, polymer or dendrimer according to Claim 11 or a composition according to Claim 12, where the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes.

## Revendications

**1.** Composé comprenant les structures de formule (I) :

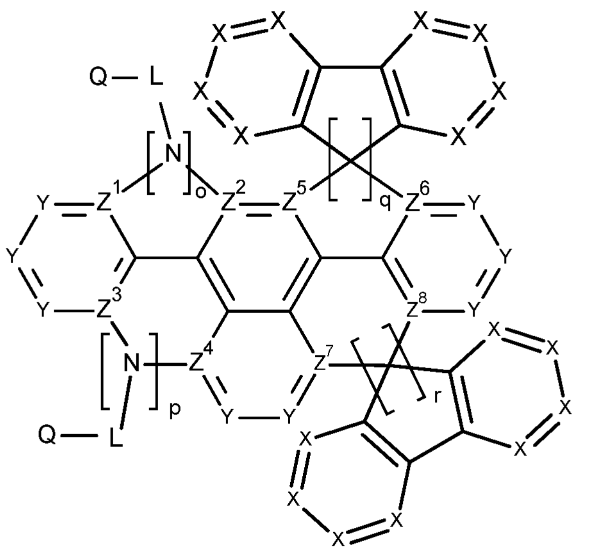

formule (I)

dans laquelle ce qui suit s'applique aux symboles utilisés :

$Z^1$, $Z^2$, $Z^3$, $Z^4$, $Z^5$, $Z^6$, $Z^7$, $Z^8$ sont, de manière identique ou différente, Y ou C ;
Y est à chaque occurrence, de manière identique ou différente, N, $CR^1$, ou deux groupements adjacents Y représentent ensemble O, S, $NR^1$, à condition qu'un cycle de 5 ou 6 chaînons soit formé ;
X est à chaque occurrence, de manière identique ou différente, N ou $CR^1$ ;
o, p, q, r valent 0 ou 1 ;
L est une liaison ou un noyau aromatique ou hétéroaromatique ayant de 5 à 24 atomes de cycle aromatique, qui peut être substitué par un ou plusieurs radicaux $R^1$ ;
Q est un groupement de transport d'électrons, choisi parmi un groupement de cycle hétéroaryle de cinq chaînons éventuellement substitué qui contient au moins deux hétéroatomes, ou un groupement de cycle hétéroaryle de six chaînons éventuellement substitué, où les groupements peuvent également faire partie d'un noyau aromatique et où d'autres groupements aryle ou hétéroaryle peuvent également être condensés sur ces groupements ;
$R^1$ est à chaque occurrence, de manière identique ou différente, H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $NO_2$, $P(=O)(R^2)_2$, $OSO_2R^2$, $OR^2$, $S(=O)R^2$, $S(=O)_2R^2$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, où un ou plusieurs groupements $CH_2$ non adjacents peuvent être remplacés par $-R^2C=CR^2-$, -C≡C-, $Si(R^2)_2$, $Ge(R^2)_2$, $Sn(R^2)_2$, C=O, C=S, C=Se, $C=NR^2$, $NR^2$, $P(=O)(R^2)$, -C(=O)O-, $-C(=O)NR^2-$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, qui peut dans chaque cas être substitué par un ou plusieurs radicaux $R^2$, ou un groupement aryloxy ou hétéroaryloxy ayant de 5 à 40 atomes de cycle aromatique, qui peut être substitué par un ou plusieurs radicaux $R^2$, ou un groupement aralkyle ou hétéroaralkyle ayant de 5 à 40 atomes de cycle aromatique, qui peut être substitué par un ou plusieurs radicaux $R^2$, ou une combinaison de ces systèmes ; deux, ou plus, radicaux $R^1$ peuvent former un noyau les uns avec les autres ;
$R^2$ est à chaque occurrence, de manière identique ou différente, H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $NO_2$, $P(=O)(R^3)_2$, $OSO_2R^3$, $OR^3$, $S(=O)R^3$, $S(=O)_2R^3$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, qui peut dans chaque cas être substitué par un ou plusieurs radicaux $R^3$, où un ou plusieurs groupements $CH_2$ non adjacents peuvent être remplacés par $-R^3C=CR^3-$, -C≡C-, $Si(R^3)_2$, $Si(R^3)_2$, $Ge(R^3)_2$, $Sn(R^3)_2$, C=O, C=S, C=Se, $C=NR^3$, $NR^3$, $P(=O)(R^3)$, -C(=O)O-, $-C(=O)NR^3-$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, qui peut dans chaque cas être substitué par un ou plusieurs radicaux $R^3$, ou un groupement aryloxy ou hétéroaryloxy ayant de 5 à 40 atomes de cycle aromatique, qui peut être substitué par un ou plusieurs radicaux $R^3$, ou un groupement aralkyle ou hétéroaralkyle ayant de 5 à 40 atomes de

cycle aromatique, qui peut être substitué par un ou plusieurs radicaux $R^3$, ou une combinaison de ces systèmes ; deux, ou plus, substituants $R^2$ peuvent également former un noyau aliphatique, hétéroaliphatique, aromatique ou hétéroaromatique, mono- ou polycyclique, les uns avec les autres ;
$R^3$ est à chaque occurrence, de manière identique ou différente, H, D, F ou un radical organique aliphatique, aromatique et/ou hétéroaromatique ayant de 1 à 20 atomes de C, dans lequel, de plus, des atomes de H peuvent être remplacés par F ; deux, ou plus, substituants $R^3$ peuvent également former un noyau aliphatique, hétéroaliphatique, aromatique ou hétéroaromatique, mono- ou polycyclique, les uns avec les autres ;

à condition que o + p = 1, où, dans le cas où o = 1, $Z^1$, $Z^2$ représentent C et $Z^3$, $Z^4$ représentent Y, et, dans le cas où p = 1, $Z^1$, $Z^2$ représentent Y et $Z^3$, $Z^4$ représentent C ; et
à condition que q + r = 1, où, dans le cas où q = 1, $Z^5$, $Z^6$ représentent C et $Z^7$, $Z^8$ représentent Y, et, dans le cas où r = 1, $Z^5$, $Z^6$ représentent Y et $Z^7$, $Z^8$ représentent C ;
en outre, pour o ou p = 0, l'atome d'azote correspondant et les groupements y étant liés ne sont pas présents, et pour q ou r = 0, l'atome de carbone correspondant et les groupements y étant liés ne sont pas présents.

**2.** Composé selon la revendication 1, comprenant les structures de formules (IIa), (IIb), (IIc) ou (IId),

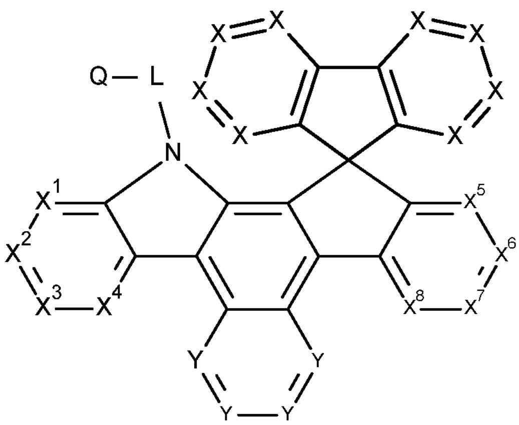

formule (IIa)

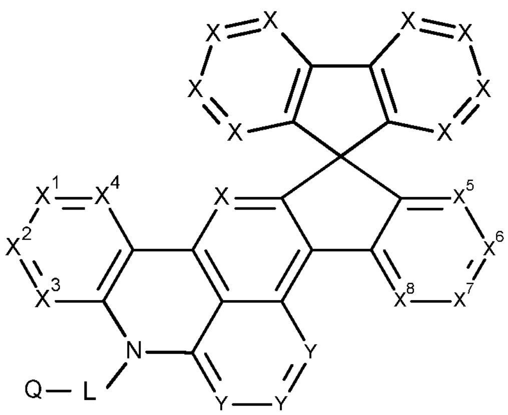

formule (IIb)

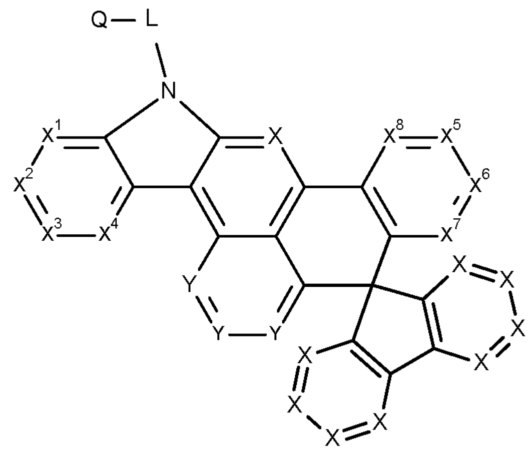

formule (IIc)

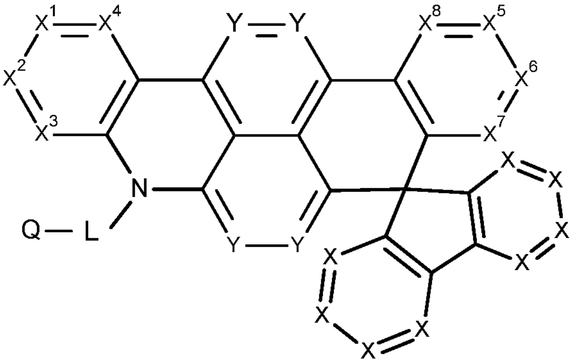

formule (IId)

dans lesquelles les symboles L, Q, Y et X utilisés revêtent la signification donnée selon la revendication 1 et $X^1$, $X^2$, $X^3$, $X^4$, $X^5$, $X^6$, $X^7$, $X^8$ sont, de manière identique ou différente, N ou $CR^1$, où un maximum de deux groupements X, $X^1$, $X^2$, $X^3$, $X^4$, $X^5$, $X^6$, $X^7$, $X^8$ par cycle représentent N.

3. Composé selon la revendication 2, comprenant les structures de formules (IIIa), (IIIb), (IIIc) ou (IIId),

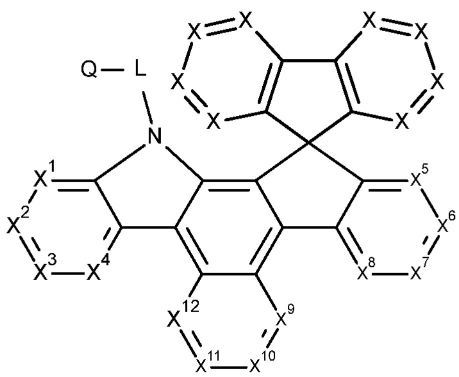

formule (IIIa)

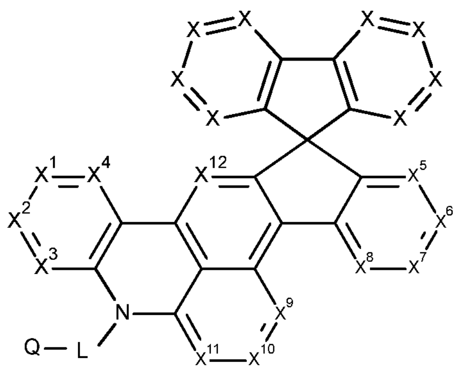

formule (IIIb)

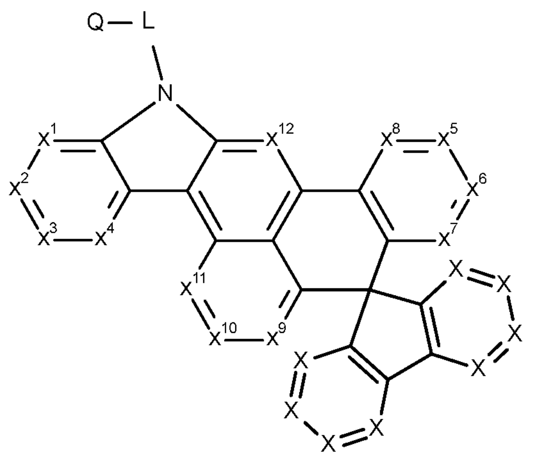

formule (IIIc)

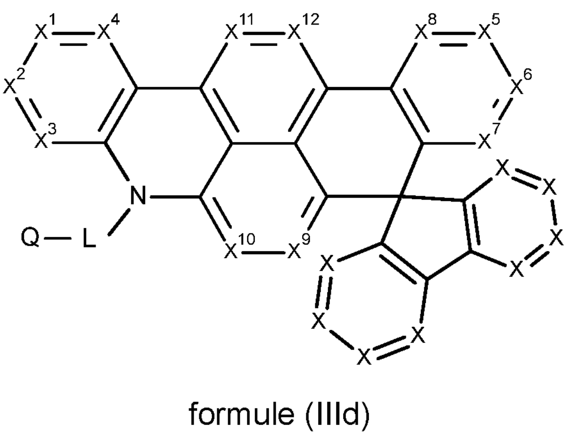

formule (IIId)

dans lesquelles les symboles L, Q et X utilisés revêtent la signification donnée selon la revendication 1 et $X^1$, $X^2$, $X^3$, $X^4$, $X^5$, $X^6$, $X^7$, $X^8$, $X^9$, $X^{10}$, $X^{11}$, $X^{12}$ sont, de manière identique ou différente, N ou $CR^1$, où un maximum de deux groupements X, $X^1$, $X^2$, $X^3$, $X^4$, $X^5$, $X^6$, $X^7$, $X^8$, $X^9$, $X^{10}$, $X^{11}$, $X^{12}$ par cycle représentent N.

**4.** Composé selon la revendication 3, comprenant les structures de formules (IIIa-1), (IIIb-1), (IIIc-1), (IIId-1), (IIIa-2), (IIIb-2), (IIIc-2), (IIId-2), (IIIa-3), (IIIb-3), (IIIc-3), (IIId-3), (IIIa-4), (IIIb-4), (IIIc-4) ou (IIId-4),

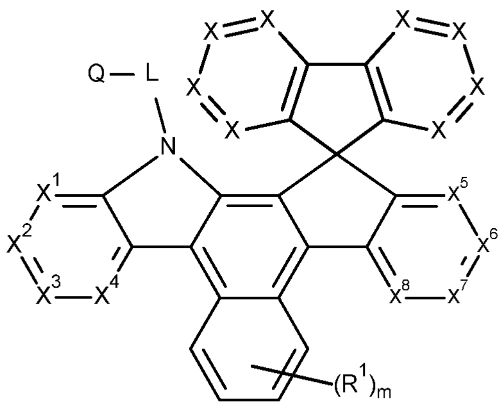

formule (IIIa-1)

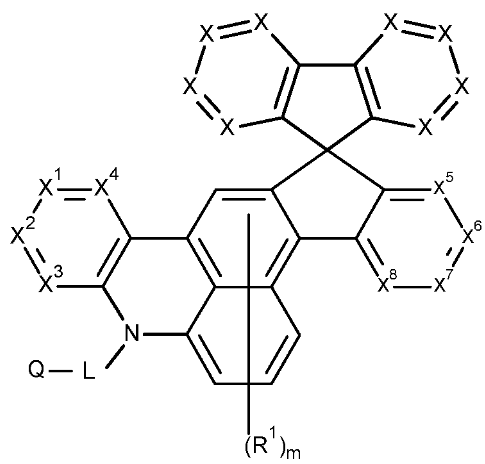

formule (IIIb-1)

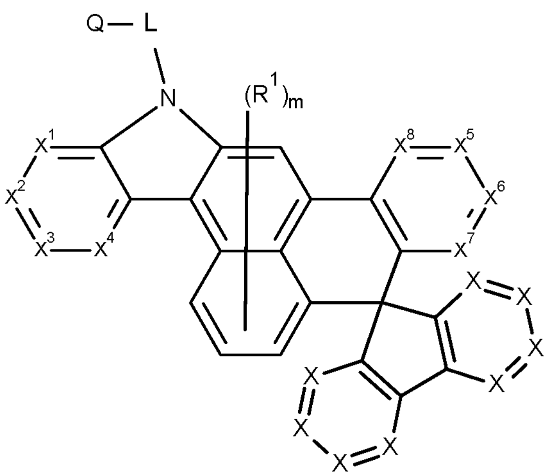

formule (IIIc-1)

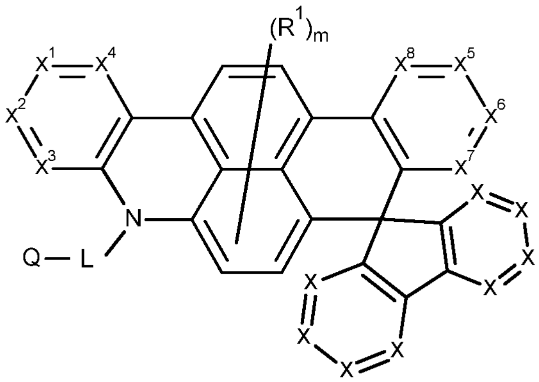

formule (IIId-1)

dans lesquelles les symboles L, $R^1$, Q et X utilisés revêtent la signification donnée selon la revendication 1, $X^1$, $X^2$, $X^3$, $X^4$, $X^5$, $X^6$, $X^7$, $X^8$ sont, de manière identique ou différente, N ou $CR^1$, et m vaut 0, 1, 2, 3 ou 4, où un maximum de deux groupements X, $X^1$, $X^2$, $X^3$, $X^4$, $X^5$, $X^6$, $X^7$, $X^8$ par cycle représentent N ;

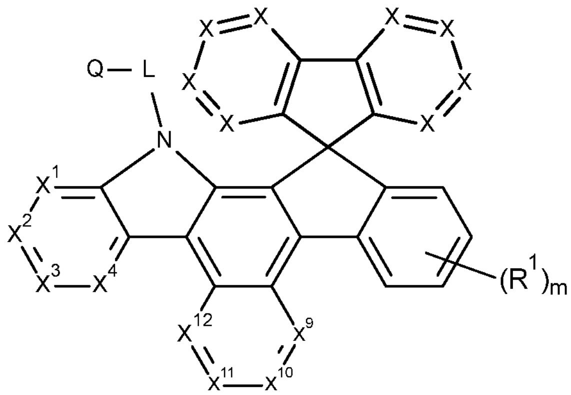

formule (IIIa-2)

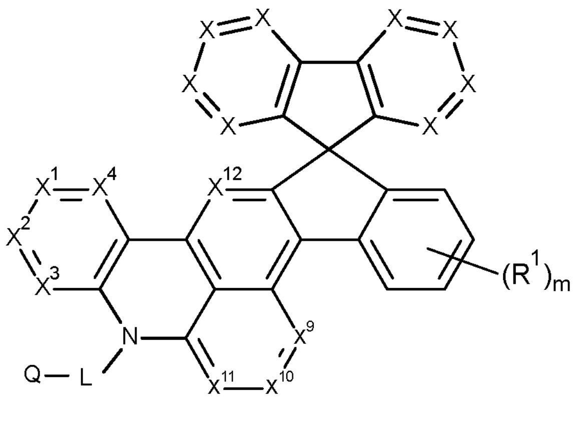

formule (IIIb-2)

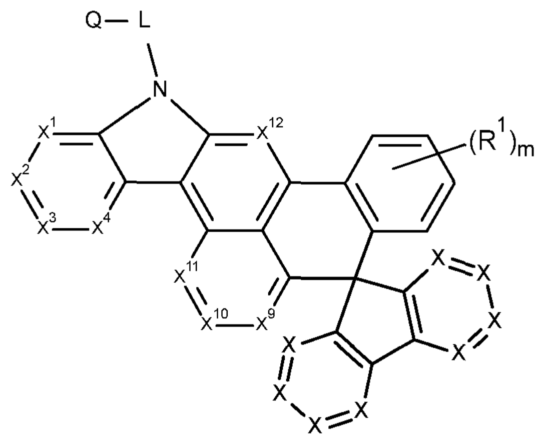

formule (IIIc-2)

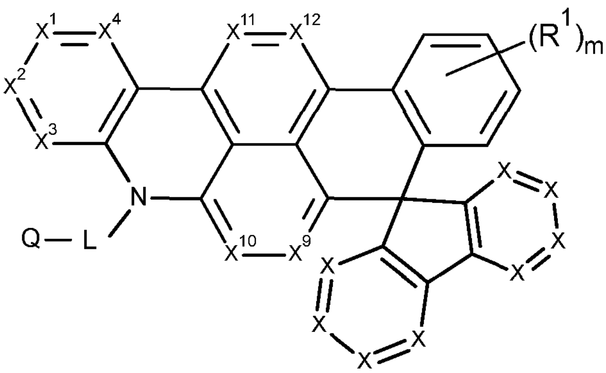

formule (IIId-2)

dans lesquelles les symboles L, $R^1$, Q et X utilisés revêtent la signification donnée selon la revendication 1, $X^1$, $X^2$, $X^3$, $X^4$, $X^9$, $X^{10}$, $X^{11}$, $X^{12}$ sont, de manière identique ou différente, N ou $CR^1$, et m vaut 0, 1, 2, 3 ou 4, où un maximum de deux groupements X, $X^1$, $X^2$, $X^3$, $X^4$, $X^9$, $X^{10}$, $X^{11}$, $X^{12}$ par cycle représentent N ;

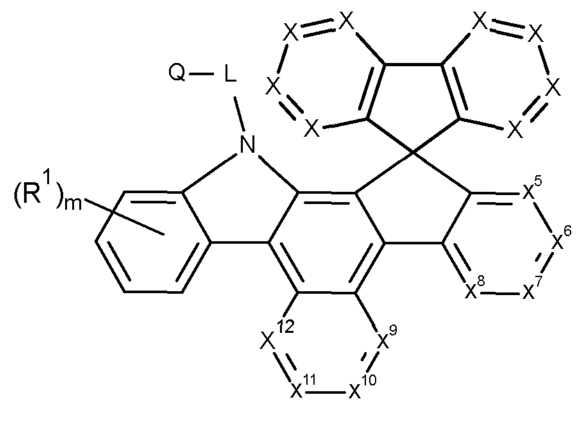

formule (IIIa-3)

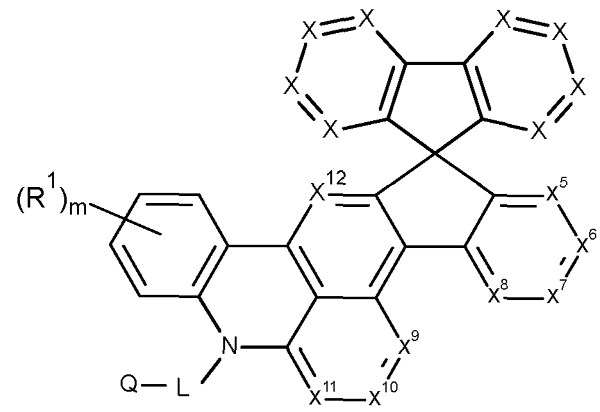

formule (IIIb-3)

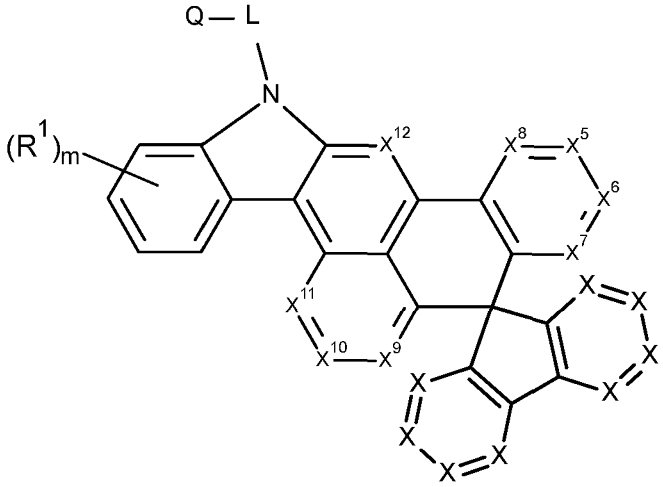

formule (IIIc-3)

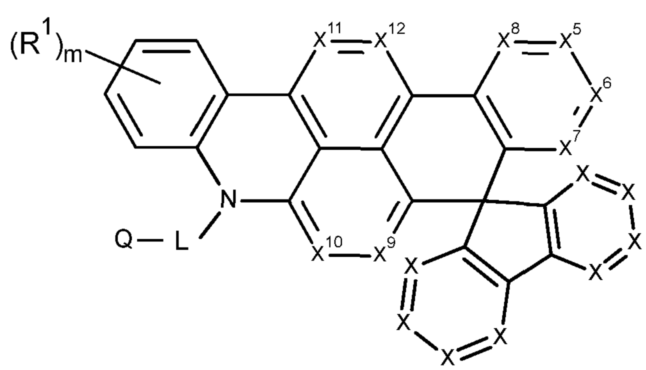

formule (IIId-3)

dans lesquelles les symboles L, $R^1$, Q et X utilisés revêtent la signification donnée selon la revendication 1, $X^5$, $X^6$, $X^7$, $X^8$, $X^9$, $X^{10}$, $X^{11}$, $X^{12}$ sont, de manière identique ou différente, N ou $CR^1$, et m vaut 0, 1, 2, 3 ou 4, où un maximum de deux groupements X, $X^5$, $X^6$, $X^7$, $X^8$, $X^9$, $X^{10}$, $X^{11}$, $X^{12}$ par cycle représentent N ;

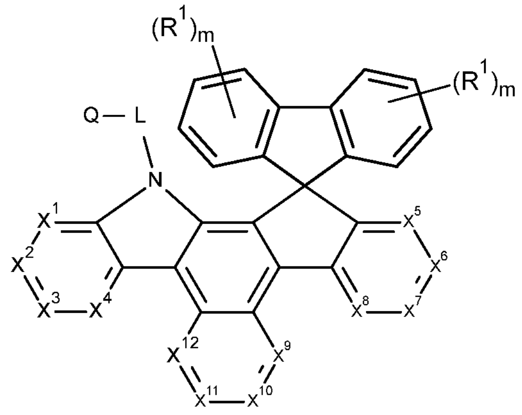

formule (IIIa-4)

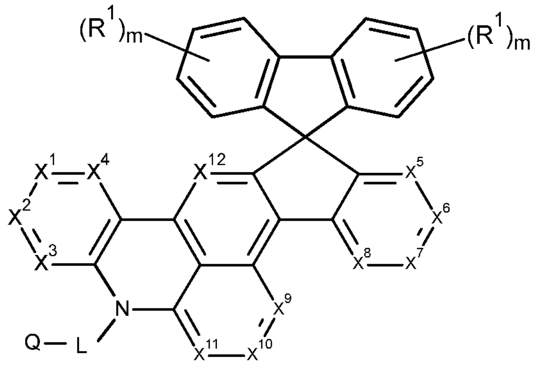

formule (IIIb-4)

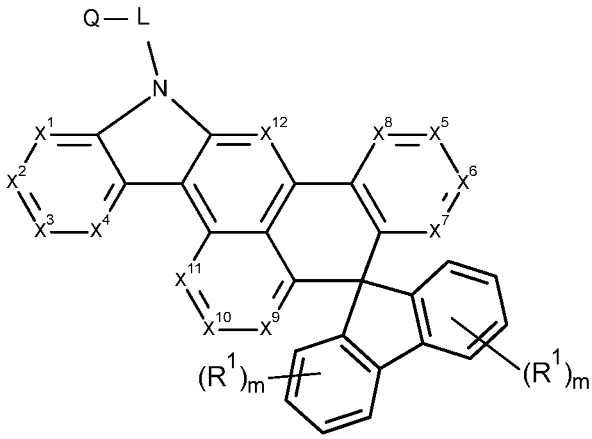

formule (IIIc-4)

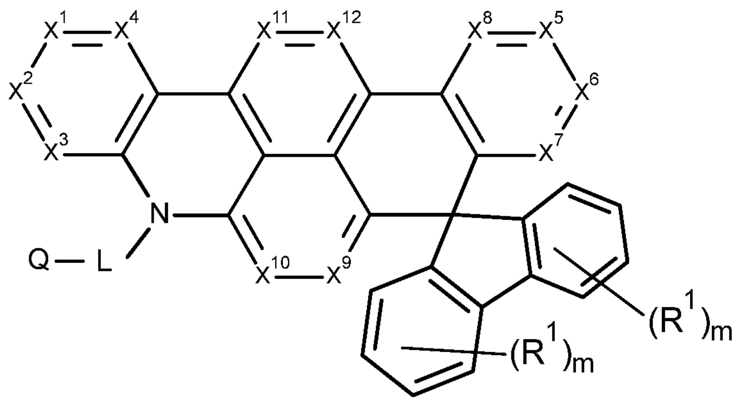

formule (IIId-4)

dans lesquelles les symboles L, $R^1$ et Q utilisés revêtent la signification donnée selon la revendication 1, $X^1$, $X^2$, $X^3$, $X^4$, $X^5$, $X^6$, $X^7$, $X^8$, $X^9$, $X^{10}$, $X^{11}$, $X^{12}$ sont, de manière identique ou différente, N ou $CR^1$, et m vaut 0, 1, 2, 3 ou 4, où un maximum de deux groupements $X^1$, $X^2$, $X^3$, $X^4$, $X^5$, $X^6$, $X^7$, $X^8$, $X^9$, $X^{10}$, $X^{11}$, $X^{12}$ par cycle représentent N.

**5.** Composé selon la revendication 1, comprenant les structures de formules (IVa), (IVb) ou (IVc),

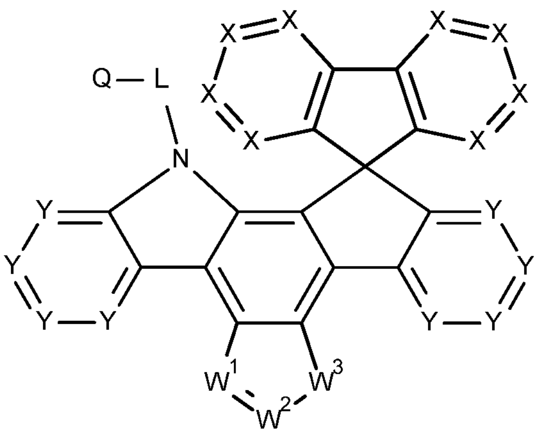

formule (IVa)

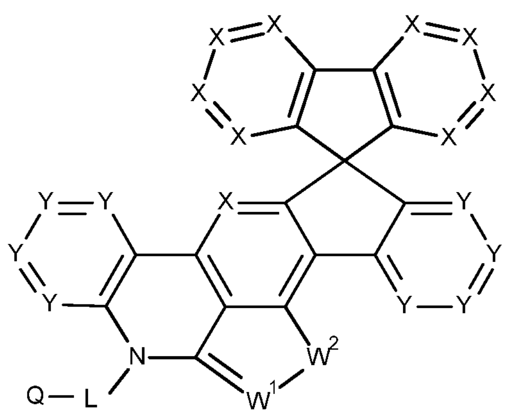

formule (IVb)

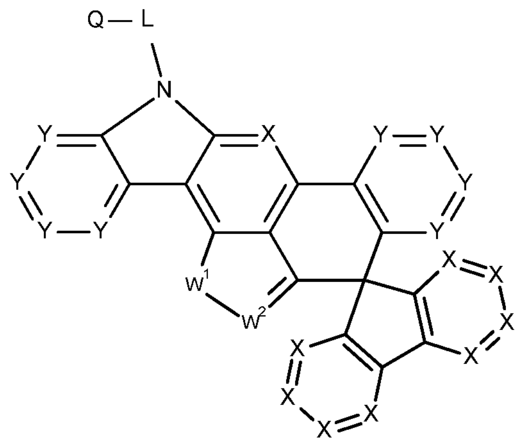

formule (IVc)

dans lesquelles les symboles L, Q, X et Y utilisés revêtent la signification donnée selon la revendication 1 et $W^1$, $W^2$ et $W^3$ sont à chaque occurrence, de manière identique ou différente, N, $CR^1$, O, S ou $NR^1$, où précisément l'un des groupements $W^1$, $W^2$ et $W^3$ représente O, S ou $NR^1$ et $W^1$ dans la formule (IVb) et $W^2$ dans la formule (IVc) représentent N ou $CR^1$, où $R^1$ revêt la signification donnée selon la revendication 1.

**6.** Composé selon la revendication 5, comprenant les structures de formules (Va), (Vb) ou (Vc),

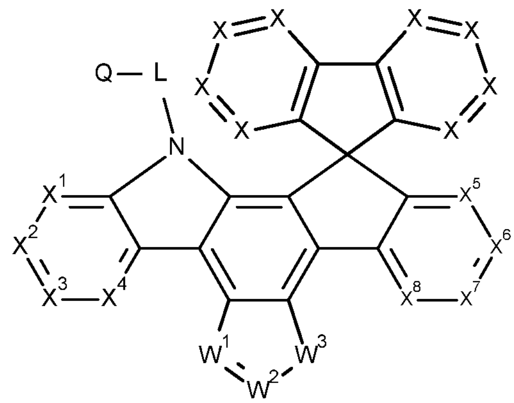

formule (Va)

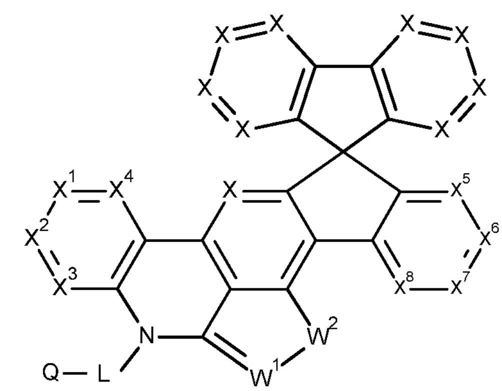

formule (Vb)

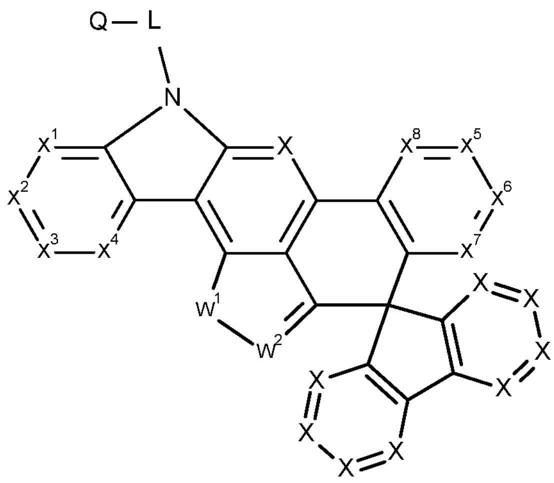

formule (Vc)

dans lesquelles les symboles L, Q et X utilisés revêtent la signification donnée selon la revendication 1, les symboles $X^1$, $X^2$, $X^3$, $X^4$, $X^5$, $X^6$, $X^7$, $X^8$ revêtent la signification donnée selon la revendication 2 et les symboles $W^1$, $W^2$ et $W^3$ revêtent la signification donnée selon la revendication 5.

**7.** Composé selon la revendication 6, comprenant les structures de formules (Va-1), (Vb-1), (Vc-1), (Va-2), (Vb-2), (Vc-2), (Va-3), (Vb-3) ou (Vc-3),

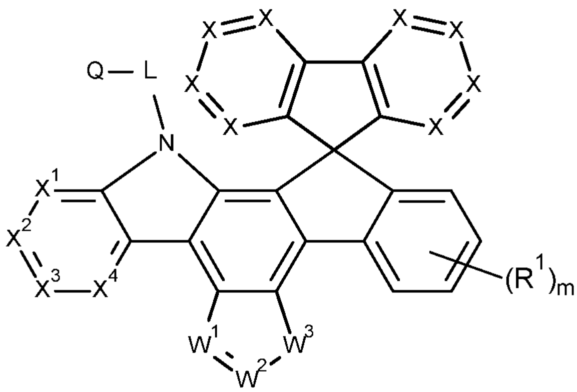

formule (Va-1)

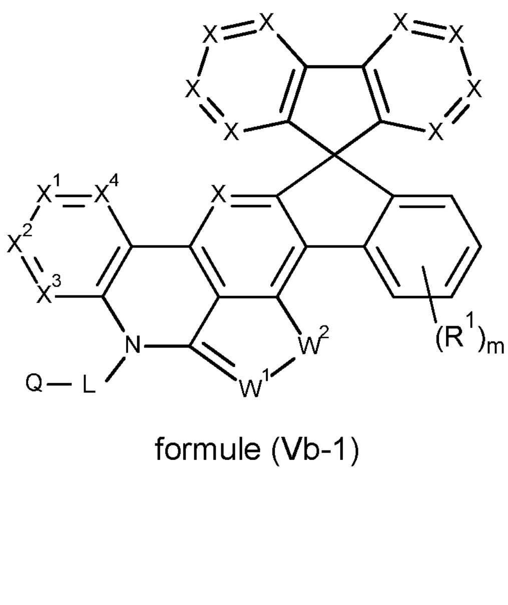

formule (Vb-1)

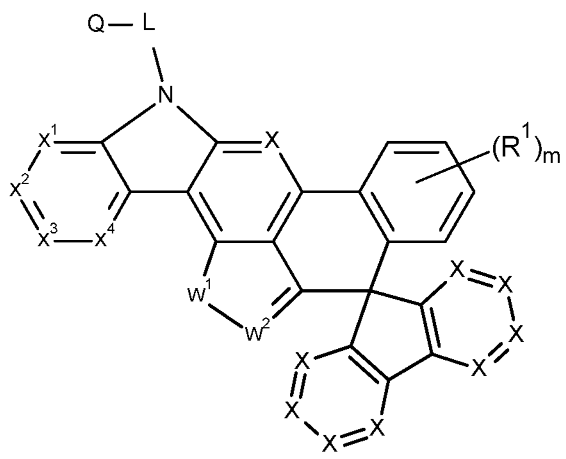

formule (Vc-1)

dans lesquelles les symboles L, $R^1$, Q et X utilisés revêtent la signification donnée selon la revendication 1, les symboles $X^1$, $X^2$, $X^3$, $X^4$ revêtent la signification donnée selon la revendication 2 et les symboles $W^1$, $W^2$ et $W^3$ revêtent la signification donnée selon la revendication 5 et m vaut 0, 1, 2, 3 ou 4 ;

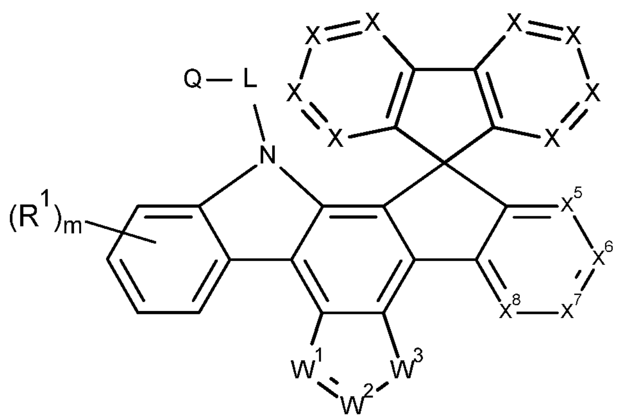

formule (Va-2)

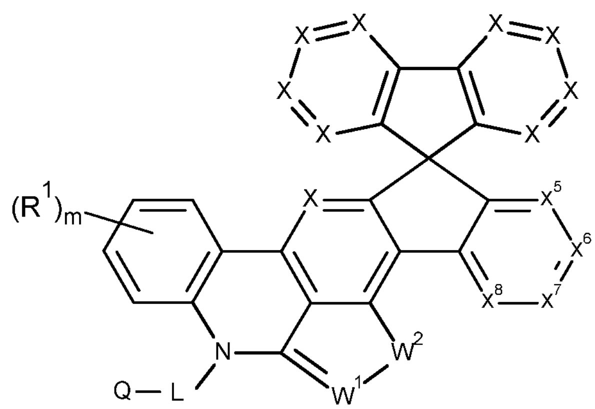

formule (Vb-2)

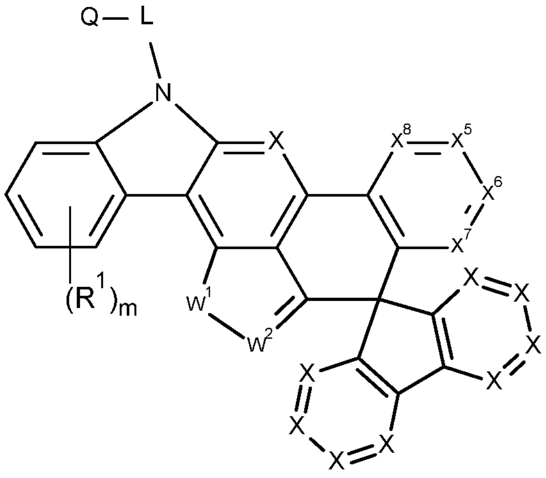

formule (Vc-2)

dans lesquelles les symboles L, $R^1$, Q et X utilisés revêtent la signification donnée selon la revendication 1, les symboles $X^5$, $X^6$, $X^7$, $X^8$ revêtent la signification donnée selon la revendication 2 et les symboles $W^1$, $W^2$ et $W^3$ revêtent la signification donnée selon la revendication 5 et m vaut 0, 1, 2, 3 ou 4 ;

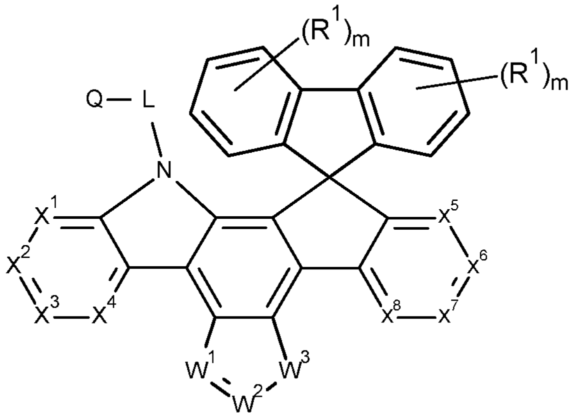

formule (Va-3)

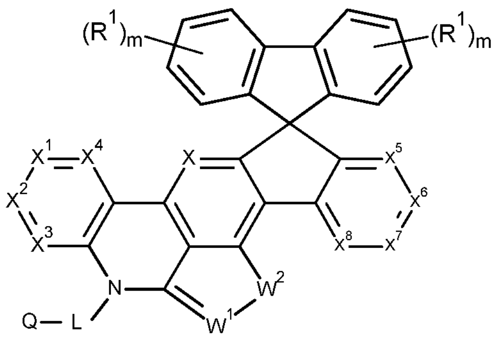

formule (Vb-3)

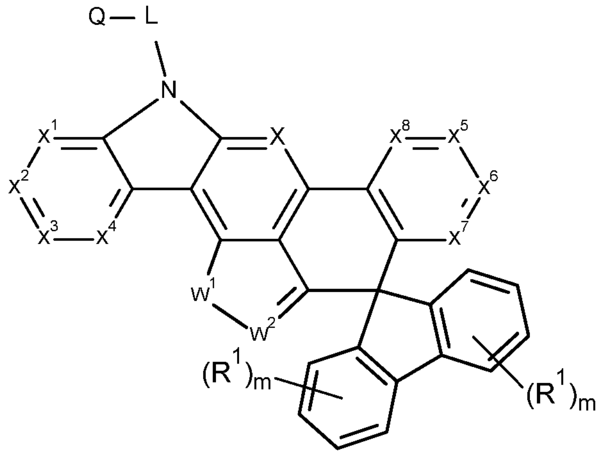

formule (Vc-3)

dans lesquelles les symboles L, $R^1$, Q et X utilisés revêtent la signification donnée selon la revendication 1, les symboles $X^1$, $X^2$, $X^3$, $X^4$, $X^5$, $X^6$, $X^7$, $X^8$ revêtent la signification donnée selon la revendication 2 et les symboles $W^1$, $W^2$ et $W^3$ revêtent la signification donnée selon la revendication 5 et m vaut 0, 1, 2, 3 ou 4.

**8.** Composé selon au moins l'une des revendications précédentes, **caractérisé en ce que** le groupement Q est choisi parmi les structures de formules (Q-1), (Q-2), (Q-3), (Q-4) ou (Q-5),

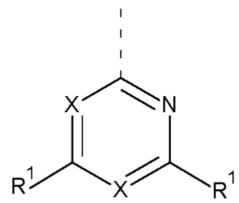

formule (Q-1)

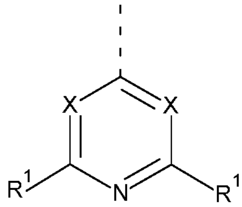

formule (Q-2)

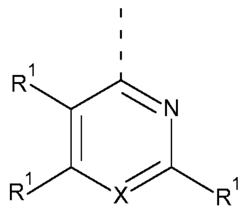

formule (Q-3)

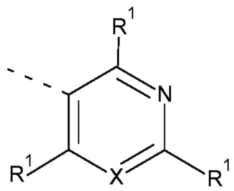

formule (Q-4)

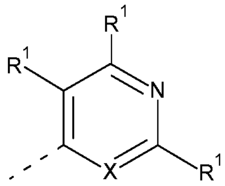

formule (Q-5)

dans lesquelles le symbole $R^1$ revêt la signification donnée ci-dessus selon la revendication 1, X est N ou $CR^1$ et la liaison en pointillés marque la position de liaison, où X représente de préférence un atome d'azote.

**9.** Composé selon au moins l'une des revendications précédentes, **caractérisé en ce que** le groupement Q est choisi parmi les structures de formules (Q-6), (Q-7), (Q-8), (Q-9), (Q-10), (Q-11) ou (Q-12),

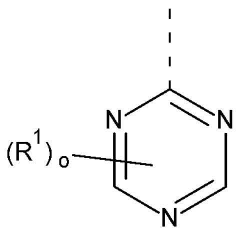

formule (Q-6)

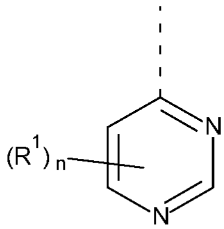

formule (Q-7)

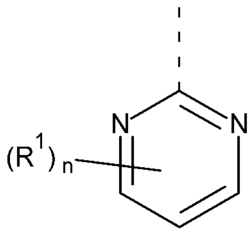

formule (Q-8)

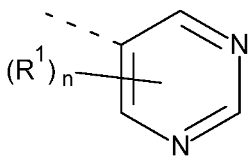

formule (Q-9)

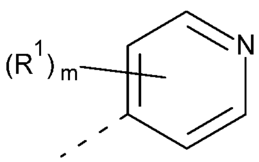

formule (Q-10)

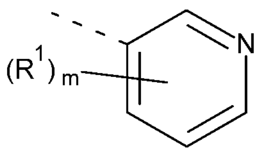

formule (Q-11)

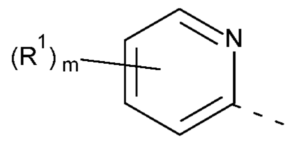

formule (Q-12)

dans lesquelles le symbole $R^1$ revêt la signification décrite selon la revendication 1, la liaison en pointillés marque la position de liaison et m vaut 0, 1, 2, 3 ou 4, n vaut 0, 1, 2 ou 3 et o vaut 0, 1 ou 2.

**10.** Composé selon au moins l'une des revendications précédentes, **caractérisé en ce que** le groupement Q est choisi parmi les structures de formules (Q-16), (Q-17) ou (Q-18),

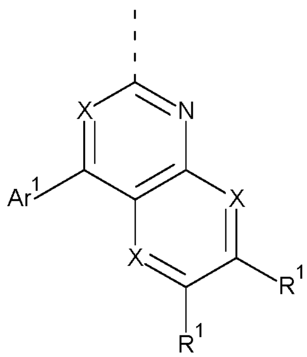

formule (Q-16)

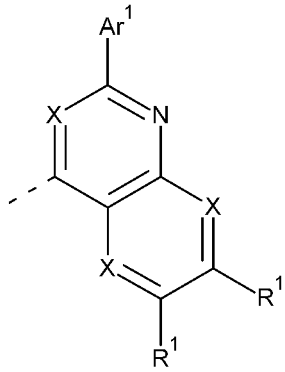

formule (Q-17)

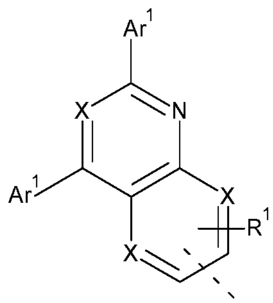

formule (Q-18)

dans lesquelles les symboles X et $R^1$ revêtent la signification donnée ci-dessus selon la revendication 1, la liaison en pointillés marque la position de liaison et $Ar^1$ représente un noyau aromatique ou hétéroaromatique ayant de 5 à 40 atomes de cycle aromatique, qui peut dans chaque cas être substitué par un ou plusieurs radicaux $R^1$, où deux, ou plus, substituants adjacents $R^1$ peuvent éventuellement former un noyau aliphatique, hétéroaliphatique, aromatique ou hétéroaromatique, mono- ou polycyclique, qui peut être substitué par un ou plusieurs radicaux $R^3$.

**11.** Oligomère, polymère ou dendrimère comprenant un ou plusieurs composés selon l'une des revendications 1 à 10, où, au lieu d'un atome d'hydrogène ou d'un substituant, une ou plusieurs liaisons sont présentes entre les composés et le polymère, l'oligomère ou le dendrimère.

**12.** Composition comprenant au moins un composé selon l'une ou plusieurs parmi les revendications 1 à 10 ou un oligomère, polymère ou dendrimère selon la revendication 11 et au moins un autre composé choisi dans le groupe constitué par les émetteurs fluorescents, les émetteurs phosphorescents, les matériaux hôtes, les matériaux de transport d'électrons, les matériaux d'injection d'électrons, les matériaux conducteurs de trous, les matériaux d'injection de trous, les matériaux de blocage d'électrons et les matériaux de blocage de trous.

**13.** Formulation comprenant au moins un composé selon l'une ou plusieurs parmi les revendications 1 à 10 ou un oligomère, polymère ou dendrimère selon la revendication 11, ou une composition selon la revendication 12, et au moins un solvant.

**14.** Utilisation d'un composé selon l'une ou plusieurs parmi les revendications 1 à 10, d'un oligomère, polymère ou dendrimère selon la revendication 11, ou d'une composition selon la revendication 12, dans un dispositif électronique, en particulier comme matériau hôte ou matériau de transport d'électrons.

**15.** Dispositif électronique contenant au moins un composé selon l'une ou plusieurs parmi les revendications 1 à 10, un oligomère, polymère ou dendrimère selon la revendication 11, ou une composition selon la revendication 12, le dispositif électronique étant préférablement choisi dans le groupe constitué par les dispositifs électroluminescents organiques, les circuits intégrés organiques, les transistors organiques à effet de champ, les transistors organiques en couche mince, les transistors organiques émetteurs de lumière, les cellules solaires organiques, les détecteurs optiques organiques, les photorécepteurs organiques, les dispositifs organiques à extinction de champ, les cellules électrochimiques émettrices de lumière ou les diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2015156587 A1
- US 2015001511 A1
- KR 20150065383 A
- KR 20140099759 A
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016015815 A
- WO 2016124304 A
- WO 2017032439 A
- EP 16179378
- EP 16186313
- WO 2004013080 A
- WO 2004093207 A
- WO 2006005627 A
- WO 2010006680 A
- WO 2014015935 A
- WO 2005039246 A
- US 20050069729 A
- JP 2004288381 A
- EP 1205527 A
- WO 2008086851 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 005111172 A
- WO 2006117052 A
- WO 2010015306 A
- EP 652273 A
- WO 2009062578 A
- WO 2010054729 A
- WO 2010054730 A
- US 20090136779 A
- WO 2010050778 A
- WO 2011042107 A
- WO 2011088877 A
- WO 2012143080 A
- WO 2012048781 A
- WO 2011116865 A
- WO 2011137951 A
- WO 2013064206 A
- WO 2014094963 A
- WO 2015192939 A
- WO 2015169412 A
- WO 2016015810 A
- WO 2016023608 A
- EP 16158460
- EP 16159829
- WO 2010108579 A
- WO 2005053051 A

- WO 2009030981 A